# EUROPEAN PATENT SPECIFICATION

(11) **EP 2 341 929 B1**
(45) Date of publication and mention of the grant of the patent: **25.01.2017**
(21) Application number: 09793315.4
(22) Date of filing: 06.10.2009
(51) Int. Cl.: A61K 39/085, A61P 31/04, A61K 39/395, C07K 16/12, A61K 39/07, A61K 39/00, C07K 14/31

(54) **COMPOSITIONS AND METHODS RELATED TO BACTERIAL EMP PROTEINS**
ZUSAMMENSETZUNGEN UND VERFAHREN IM ZUSAMMENHANG MIT BAKTERIELLEN EMP PROTEINEN
COMPOSITIONS ET PROCÉDÉS ASSOCIÉS AUX PROTÉINES BACTÉRIENNES EMP

(30) Priority: 06.10.2008 US 103190 P; 06.10.2008 US 103196 P; 20.04.2009 US 170779 P
(43) Date of publication of application: 13.07.2011
(73) Proprietor: University Of Chicago, Chicago, IL 60637 (US)
(72) Inventor: CHENG, Alice, Chicago, IL 60637 (US); THAMMAVONGSA, Vilasack, Chicago, IL 60637 (US); KERN, Justin, Chicago, IL 60637 (US); MISSIAKAS, Dominique M., Chicago, IL 60637 (US); SCHNEEWIND, Olaf, Chicago, IL 60637 (US)
(74) Representative: Roberts, Joanne Nicola
(86) International application number: PCT/US2009/059648
(87) International publication number: WO 2010/042481

(56) References cited:
- EP-A2- 1 829 892
- WO-A-02/059148
- WO-A-02/094868
- WO-A2-2007/001361
- US-A1- 2003 153 022
- ZHOU H ET AL: "An immunogenicity study of a newly fusion protein Cna-FnBP vaccinated against Staphylococcus aureus infections in a mice model" VACCINE, BUTTERWORTH SCIENTIFIC. GUILDFORD, GB, vol. 24, no. 22, 29 May 2006 (2006-05-29), pages 4830-4837, XP025151352 ISSN: 0264-410X [retrieved on 2006-05-29]
- HAUCK C R ET AL: "Sticky connections: extracellular matrix protein recognition and integrin-mediated cellular invasion by Staphylococcus aureus" CURRENT OPINION IN MICROBIOLOGY, CURRENT BIOLOGY LTD, GB, vol. 9, no. 1, 1 February 2006 (2006-02-01), pages 5-11, XP025174518 ISSN: 1369-5274 [retrieved on 2006-02-01]
- MAMO W ET AL: "Vaccination against Staphylococcus aureus mastitis: immunological response of mice vaccinated with fibronectin-binding protein (FnBP-A) to challenge with S. aureus" VACCINE, BUTTERWORTH SCIENTIFIC. GUILDFORD, GB, vol. 12, no. 11, 1 January 1994 (1994-01-01), pages 988-992, XP023712021 ISSN: 0264-410X [retrieved on 1994-01-01]
- GARCIA-LARA J ET AL: "Staphylococcus aureus: the search for novel targets" DRUG DISCOVERY TODAY, ELSEVIER, RAHWAY, NJ, US, vol. 10, no. 9, 1 May 2005 (2005-05-01), pages 643-651, XP004890888 ISSN: 1359-6446
- STRANGER-JONES YUKIKO K ET AL: "Vaccine assembly from surface proteins of Staphylococcus aureus" PROCEEDINGS OF THE NATIONAL ACADEMY OF SCIENCES OF THE UNITED STATES OF AMERICA 7 NOV 2006,, vol. 103, no. 45, 7 November 2006 (2006-11-07), pages 16942-16947, XP002517729
- NITSCHE-SCHMITZ D PATRIC ET AL: "Invasion mechanisms of Gram-positive pathogenic cocci." THROMBOSIS AND HAEMOSTASIS SEP 2007, vol. 98, no. 3, September 2007 (2007-09), pages 488-496, XP002560113 ISSN: 0340-6245
- THE FASEB JOURNAL : OFFICIAL PUBLICATION OF THE FEDERATION OF AMERICAN SOCIETIES FOR EXPERIMENTAL BIOLOGY OCT 2009, vol. 23, no. 10, October 2009 (2009-10), pages 3393-3404, XP002566140 ISSN: 1530-6860
- THE JOURNAL OF EXPERIMENTAL MEDICINE 26 OCT 2009, vol. 206, no. 11, 26 October 2009 (2009-10-26), pages 2417-2427, XP002566141 ISSN: 1540-9538
- M. Hussain ET AL: "Identification and Characterization of a Novel 38.5-Kilodalton Cell Surface Protein of Staphylococcus aureus with Extended-Spectrum Binding Activity for Extracellular Matrix and Plasma Proteins", Journal of Bacteriology, vol. 183, no. 23, 1 December 2001 (2001-12-01), pages 6778-6786, XP055065282, ISSN: 0021-9193, DOI: 10.1128/JB.183.23.6778-6786.2001

### SEQUENCE LISTING

<110> CHENG, ALICE
   MISSIAKAS, DOMINIQUE M.
   SCHNEEWIND, OSAF
<120> COMPOSITIONS AND METHODS RELATED TO BACTERIAL EAP AND/OR EMP PROTEINS
<130> ARCD:477WO
<140> UNKNOWN
   <141> 2009-10-06
<160> 53
<170> PatentIn version 3.3
<210> 1
   <211> 1023
   <212> DNA
   <213> Staphylococcus aureus
<220>
   <221> CDS
   <222> (1)..(1023)
<400> 1
   atg aaa aag aaa tta tta gtt tta act atg agc acg cta ttt gct aca         48
   Met Lys Lys Lys Leu Leu Val Leu Thr Met Ser Thr Leu Phe Ala Thr
   1               5                   10                  15
   caa att atg aat tca aat cac gct aaa gca tca gtg aca gag agt gtt         96
   Gln Ile Met Asn Ser Asn His Ala Lys Ala Ser Val Thr Glu Ser Val
               20                  25                  30
   gac aaa aaa ttt gta gtt cca gaa tca gga att aat aaa att att cca        144
   Asp Lys Lys Phe Val Val Pro Glu Ser Gly Ile Asn Lys Ile Ile Pro
           35                  40                  45
   gct tac gat gaa ttt aag aat tcg cca aaa gta aat gtt agt aat tta        192
   Ala Tyr Asp Glu Phe Lys Asn Ser Pro Lys Val Asn Val Ser Asn Leu
       50                  55                  60
   act gac aat aaa aac ttt gta gct tct gaa gat aaa ttg aat aag att        240
   Thr Asp Asn Lys Asn Phe Val Ala Ser Glu Asp Lys Leu Asn Lys Ile
   65                  70                  75                  80
   gca gat tca tcg gca gct agt aaa att gta gat aaa aac ttt gtc gta        288
   Ala Asp Ser Ser Ala Ala Ser Lys Ile Val Asp Lys Asn Phe Val Val
                   85                  90                  95
   cca gaa tca aag tta gga aac att gtg cca gag tac aaa gaa atc aat        336
   Pro Glu Ser Lys Leu Gly Asn Ile Val Pro Glu Tyr Lys Glu Ile Asn
               100                 105                 110
   aat cgc gtg aat gta gca aca aac aat cca gct tca caa caa gtt gat        384
   Asn Arg Val Asn Val Ala Thr Asn Asn Pro Ala Ser Gln Gln Val Asp
           115                 120                 125
   aag cat ttt gtt gct aaa ggc cca gaa gta aat aga ttt att acg caa        432
   Lys His Phe Val Ala Lys Gly Pro Glu Val Asn Arg Phe Ile Thr Gln
       130                 135                 140
   aac aaa gta aac cac cac ttc att act acg caa acc cac tac aag aaa       480
   Asn Lys Val Asn His His Phe Ile Thr Thr Gln Thr His Tyr Lys Lys
   145                 150                 155                 160
   gtt att act tca tac aaa tca aca cat gta cat aaa cat gta aat cat       528
   Val Ile Thr Ser Tyr Lys Ser Thr His Val His Lys His Val Asn His
                   165                 170                 175
   gca aag gat tct att aat aaa cac ttt att gtt aaa cca tca gaa tcg       576
   Ala Lys Asp Ser Ile Asn Lys His Phe Ile Val Lys Pro Ser Glu Ser
               180                 185                 190
   cct aga tat aca cat cca tct caa tct tta att atc aag cat cat ttt       624
   Pro Arg Tyr Thr His Pro Ser Gln Ser Leu Ile Ile Lys His His Phe
           195                 200                 205
   gca gtt cct gga tat cac gcg cat aaa ttt gtt aca cca ggg cat gct       672
   Ala Val Pro Gly Tyr His Ala His Lys Phe Val Thr Pro Gly His Ala
       210                 215                 220
   agc att aaa att aat cac ttt tgt gtt gtg cca caa ata aat agt ttc       720
   Ser Ile Lys Ile Asn His Phe Cys Val Val Pro Gln Ile Asn Ser Phe
   225                 230                 235                 240
   aag gta att cca cca tat ggt cac aat tca cat cgt atg cat gta cca       768
   Lys Val Ile Pro Pro Tyr Gly His Asn Ser His Arg Met His Val Pro
                   245                 250                 255
   agt ttc caa aat aac aca aca gca aca cat caa aat gct aaa gta aat       816
   Ser Phe Gln Asn Asn Thr Thr Ala Thr His Gln Asn Ala Lys Val Asn
               260                 265                 270
   aaa gca tat gac tat aaa tac ttc tat tct tat aaa gta gtt aaa ggt       864
   Lys Ala Tyr Asp Tyr Lys Tyr Phe Tyr Ser Tyr Lys Val Val Lys Gly
           275                 280                 285
   gtg aag aaa tat ttc tca ttt tca caa tca aat ggt tat aaa att ggg       912
   Val Lys Lys Tyr Phe Ser Phe Ser Gln Ser Asn Gly Tyr Lys Ile Gly
       290                 295                 300
   aaa cca tca tta aat atc aaa aat gta aat tat caa tat gct gtt cca       960
   Lys Pro Ser Leu Asn Ile Lys Asn Val Asn Tyr Gln Tyr Ala Val Pro
   305                 310                 315                 320
   agt tat agc cct aca cac tac gtt cct gaa ttt aag ggt agc tta cca      1008
   Ser Tyr Ser Pro Thr His Tyr Val Pro Glu Phe Lys Gly Ser Leu Pro
                   325                 330                 335
   gca cca cga gta taa                                                  1023
   Ala Pro Arg Val
               340
<210> 2
   <211> 340
   <212> PRT
   <213> Staphylococcus aureus
<400> 2
   Met Lys Lys Lys Leu Leu Val Leu Thr Met Ser Thr Leu Phe Ala Thr
   1               5                   10                  15
   Gln Ile Met Asn Ser Asn His Ala Lys Ala Ser Val Thr Glu Ser Val
               20                  25                  30
   Asp Lys Lys Phe Val Val Pro Glu Ser Gly Ile Asn Lys Ile Ile Pro
           35                  40                  45
   Ala Tyr Asp Glu Phe Lys Asn Ser Pro Lys Val Asn Val Ser Asn Leu
       50                  55                  60
   Thr Asp Asn Lys Asn Phe Val Ala Ser Glu Asp Lys Leu Asn Lys Ile
   65                  70                  75                  80
   Ala Asp Ser Ser Ala Ala Ser Lys Ile Val Asp Lys Asn Phe Val Val
                   85                  90                  95
   Pro Glu Ser Lys Leu Gly Asn Ile Val Pro Glu Tyr Lys Glu Ile Asn
               100                 105                 110
   Asn Arg Val Asn Val Ala Thr Asn Asn Pro Ala Ser Gln Gln Val Asp
           115                 120                 125
   Lys His Phe Val Ala Lys Gly Pro Glu Val Asn Arg Phe Ile Thr Gln
       130                 135                 140
   Asn Lys Val Asn His His Phe Ile Thr Thr Gln Thr His Tyr Lys Lys
   145                 150                 155                 160
   Val Ile Thr Ser Tyr Lys Ser Thr His Val His Lys His Val Asn His
                   165                 170                 175
   Ala Lys Asp Ser Ile Asn Lys His Phe Ile Val Lys Pro Ser Glu Ser
               180                 185                 190
   Pro Arg Tyr Thr His Pro Ser Gln Ser Leu Ile Ile Lys His His Phe
           195                 200                 205
   Ala Val Pro Gly Tyr His Ala His Lys Phe Val Thr Pro Gly His Ala
       210                 215                 220
   Ser Ile Lys Ile Asn His Phe Cys Val Val Pro Gln Ile Asn Ser Phe
   225                 230                 235                 240
   Lys Val Ile Pro Pro Tyr Gly His Asn Ser His Arg Met His Val Pro
                   245                 250                 255
   Ser Phe Gln Asn Asn Thr Thr Ala Thr His Gln Asn Ala Lys Val Asn
   260 265 270
   Lys Ala Tyr Asp Tyr Lys Tyr Phe Tyr Ser Tyr Lys Val Val Lys Gly
   275 280 285
   Val Lys Lys Tyr Phe Ser Phe Ser Gln Ser Asn Gly Tyr Lys Ile Gly
   290 295 300
   Lys Pro Ser Leu Asn Ile Lys Asn Val Asn Tyr Gln Tyr Ala Val Pro
   305 310 315 320
   Ser Tyr Ser Pro Thr His Tyr Val Pro Glu Phe Lys Gly Ser Leu Pro
   325 330 335
   Ala Pro Arg Val
   340
<210> 3
   <211> 1755
   <212> DNA
   <213> Staphylococcus aureus
<220>
   <221> CDS
   <222> (1)..(1755)
<400> 3
   atg aaa ttt aag tca ttg att aca aca act tta gca tta ggt gtt tta        48
   Met Lys Phe Lys Ser Leu Ile Thr Thr Thr Leu Ala Leu Gly Val Leu
   1               5                   10                  15
   gca tca aca ggt gca aac ttt aat aat aat gaa gcg tct gcc gca gct        96
   Ala Ser Thr Gly Ala Asn Phe Asn Asn Asn Glu Ala Ser Ala Ala Ala
               20                  25                  30
   aag cca tta gat aaa tca tca agt tcg tta cac cat gga tat tct aaa       144
   Lys Pro Leu Asp Lys Ser Ser Ser Ser Leu His His Gly Tyr Ser Lys
           35                  40                  45
   gtc cat gtt cca tat gca atc act gtg aac ggt aca agc caa aat att       192
   Val His Val Pro Tyr Ala Ile Thr Val Asn Gly Thr Ser Gln Asn Ile
       50                  55                  60
   tta tca agc tta aca ttt aat aag aat caa aat att agt tat aaa gat       240
   Leu Ser Ser Leu Thr Phe Asn Lys Asn Gln Asn Ile Ser Tyr Lys Asp
   65                  70                  75                  80
   tta gag gat aga gtt aaa tca gtt tta aaa tca gac aga ggt att agt       288
   Leu Glu Asp Arg Val Lys Ser Val Leu Lys Ser Asp Arg Gly Ile Ser
                   85                  90                  95
   gat ata gat tta aga cta tcg aag caa gcg aaa tat act gtt tac ttt       336
   Asp Ile Asp Leu Arg Leu Ser Lys Gln Ala Lys Tyr Thr Val Tyr Phe
               100                 105                 110
   aaa aat gga aca aag aaa gtt atc gat ttg aaa gca ggt att tac aca       384
   Lys Asn Gly Thr Lys Lys Val Ile Asp Leu Lys Ala Gly Ile Tyr Thr
           115                 120                 125
   gct gat tta att aat aca agt gaa att aaa gct att aat att aac gta      432
   Ala Asp Leu Ile Asn Thr Ser Glu Ile Lys Ala Ile Asn Ile Asn Val
       130                 135                 140
   gat act aaa aag caa gtt gaa gat aaa aag aaa gat aaa gca aat tac      480
   Asp Thr Lys Lys Gln Val Glu Asp Lys Lys Lys Asp Lys Ala Asn Tyr
   145                 150                 155                 160
   caa gtt cca tac aca atc act gtg aac ggt aca agc caa aat att tta      528
   Gln Val Pro Tyr Thr Ile Thr Val Asn Gly Thr Ser Gln Asn Ile Leu
                   165                 170                 175
   tca aac tta aca ttt aat aag aat caa aat att agt tac aaa gat tta      576
   Ser Asn Leu Thr Phe Asn Lys Asn Gln Asn Ile Ser Tyr Lys Asp Leu
               180                 185                 190
   gag gat aaa gtt aaa tca gtt tta gaa tca aat aga ggt att acc gat      624
   Glu Asp Lys Val Lys Ser Val Leu Glu Ser Asn Arg Gly Ile Thr Asp
           195                 200                 205
   gtt gat tta aga tta tcg aag caa gcg aaa tat aca gtt aat ttt aaa      672
   Val Asp Leu Arg Leu Ser Lys Gln Ala Lys Tyr Thr Val Asn Phe Lys
       210                 215                 220
   aat gga acg aag aaa gtt atc gat ttg aaa tca ggt att tac aca gcg      720
   Asn Gly Thr Lys Lys Val Ile Asp Leu Lys Ser Gly Ile Tyr Thr Ala
   225                 230                 235                 240
   aat tta ata aat tca agt gat att aaa agt atc aat att aac gta gat      768
   Asn Leu Ile Asn Ser Ser Asp Ile Lys Ser Ile Asn Ile Asn Val Asp
                   245                 250                 255
   aca aaa aaa cat atc gaa aat aaa gct aaa aga aac tat caa gtt cca      816
   Thr Lys Lys His Ile Glu Asn Lys Ala Lys Arg Asn Tyr Gln Val Pro
               260                 265                 270
   tat tca att aat tta aat ggt aca tct aca aac att tta tcg aat ctt      864
   Tyr Ser Ile Asn Leu Asn Gly Thr Ser Thr Asn Ile Leu Ser Asn Leu
           275                 280                 285
   tca ttt tca aat aaa cct tgg aca aat tac aaa aat tta act agt caa      912
   Ser Phe Ser Asn Lys Pro Trp Thr Asn Tyr Lys Asn Leu Thr Ser Gln
       290                 295                 300
   ata aaa tca gta ctg aag cat gat aga ggt att agt gaa caa gat tta      960
   Ile Lys Ser Val Leu Lys His Asp Arg Gly Ile Ser Glu Gln Asp Leu
   305                 310                 315                 320
   aaa tat gct aag aaa gct tat tat act gtt tat ttt aaa aat ggt ggt     1008
   Lys Tyr Ala Lys Lys Ala Tyr Tyr Thr Val Tyr Phe Lys Asn Gly Gly
                   325                 330                 335
   aaa aga atc tta cag ttg aat tca aaa aat tac aca gca aac tta gtt     1056
   Lys Arg Ile Leu Gln Leu Asn Ser Lys Asn Tyr Thr Ala Asn Leu Val
               340                 345                 350
   cat gcg aaa gat gtt aag aga att gaa att act gtt aaa aca gga act     1104
   His Ala Lys Asp Val Lys Arg Ile Glu Ile Thr Val Lys Thr Gly Thr
           355                 360                 365
   aaa gcg aaa gca gac aga tat gta cca tac aca att gca gta aat ggc      1152
   Lys Ala Lys Ala Asp Arg Tyr Val Pro Tyr Thr Ile Ala Val Asn Gly
       370                 375                 380
   aca tca aca cca att tta tca gat tta aaa ttt aca ggt gac cca cgt      1200
   Thr Ser Thr Pro Ile Leu Ser Asp Leu Lys Phe Thr Gly Asp Pro Arg
   385                 390                 395                 400
   gta ggc tac aaa gat atc tct aaa aaa gtt aaa tca gta ttg aag cat      1248
   Val Gly Tyr Lys Asp Ile Ser Lys Lys Val Lys Ser Val Leu Lys His
                   405                 410                 415
   gat aga ggt atc ggg gaa cgt gaa tta aaa tat gca aaa aaa gca act      1296
   Asp Arg Gly Ile Gly Glu Arg Glu Leu Lys Tyr Ala Lys Lys Ala Thr
               420                 425                 430
   tac aca gta cat ttt aaa aat gga acg aaa aaa gtg att aac ata aat      1344
   Tyr Thr Val His Phe Lys Asn Gly Thr Lys Lys Val Ile Asn Ile Asn
           435                 440                 445
   tca aat att agc caa ctg aat ctg ctt tat gtt caa gat att aaa aag      1392
   Ser Asn Ile Ser Gln Leu Asn Leu Leu Tyr Val Gln Asp Ile Lys Lys
       450                 455                 460
   ata gat att gat gtt aaa aca gga act aaa gcg aaa gcg gat agc tat      1440
   Ile Asp Ile Asp Val Lys Thr Gly Thr Lys Ala Lys Ala Asp Ser Tyr
   465                 470                 475                 480
   gta cca tat aca att gca gta aat ggc aca tca aca cca att tta tca      1488
   Val Pro Tyr Thr Ile Ala Val Asn Gly Thr Ser Thr Pro Ile Leu Ser
                   485                 490                 495
   aaa ctt aaa att tcg aat aaa caa tta att agt tac aaa tat tta aat      1536
   Lys Leu Lys Ile Ser Asn Lys Gln Leu Ile Ser Tyr Lys Tyr Leu Asn
               500                 505                 510
   gac aaa gtg aaa tct gta tta aaa agt gaa aga ggc atc agt gat ctt      1584
   Asp Lys Val Lys Ser Val Leu Lys Ser Glu Arg Gly Ile Ser Asp Leu
           515                 520                 525
   gac tta aaa ttt gcg aaa caa gca aaa tat aca gta tat ttc aaa aat      1632
   Asp Leu Lys Phe Ala Lys Gln Ala Lys Tyr Thr Val Tyr Phe Lys Asn
       530                 535                 540
   gga aag aaa caa gta gtg aat tta aaa tca gac atc ttt aca cct aat      1680
   Gly Lys Lys Gln Val Val Asn Leu Lys Ser Asp Ile Phe Thr Pro Asn
   545                 550                 555                 560
   tta ttt agt gcc aaa gat att aaa aag att gat att gat gta aaa caa      1728
   Leu Phe Ser Ala Lys Asp Ile Lys Lys Ile Asp Ile Asp Val Lys Gln
                   565                 570                 575
   tac act aaa tca aaa aaa aat aaa taa                                  1755
   Tyr Thr Lys Ser Lys Lys Asn Lys
               580
<210> 4
   <211> 584
   <212> PRT
   <213> Staphylococcus aureus
<400> 4
   Met Lys Phe Lys Ser Leu Ile Thr Thr Thr Leu Ala Leu Gly Val Leu
   1               5                   10                  15
   Ala Ser Thr Gly Ala Asn Phe Asn Asn Asn Glu Ala Ser Ala Ala Ala
               20                  25                  30
   Lys Pro Leu Asp Lys Ser Ser Ser Ser Leu His His Gly Tyr Ser Lys
           35                  40                  45
   Val His Val Pro Tyr Ala Ile Thr Val Asn Gly Thr Ser Gln Asn Ile
       50                  55                  60
   Leu Ser Ser Leu Thr Phe Asn Lys Asn Gln Asn Ile Ser Tyr Lys Asp
   65                  70                  75                  80
   Leu Glu Asp Arg Val Lys Ser Val Leu Lys Ser Asp Arg Gly Ile Ser
                   85                  90                  95
   Asp Ile Asp Leu Arg Leu Ser Lys Gln Ala Lys Tyr Thr Val Tyr Phe
               100                 105                 110
   Lys Asn Gly Thr Lys Lys Val Ile Asp Leu Lys Ala Gly Ile Tyr Thr
           115                 120                 125
   Ala Asp Leu Ile Asn Thr Ser Glu Ile Lys Ala Ile Asn Ile Asn Val
       130                 135                 140
   Asp Thr Lys Lys Gln Val Glu Asp Lys Lys Lys Asp Lys Ala Asn Tyr
   145                 150                 155                 160
   Gln Val Pro Tyr Thr Ile Thr Val Asn Gly Thr Ser Gln Asn Ile Leu
                   165                 170                 175
   Ser Asn Leu Thr Phe Asn Lys Asn Gln Asn Ile Ser Tyr Lys Asp Leu
               180                 185                 190
   Glu Asp Lys Val Lys Ser Val Leu Glu Ser Asn Arg Gly Ile Thr Asp
           195                 200                 205
   Val Asp Leu Arg Leu Ser Lys Gln Ala Lys Tyr Thr Val Asn Phe Lys
       210                 215                 220
   Asn Gly Thr Lys Lys Val Ile Asp Leu Lys Ser Gly Ile Tyr Thr Ala
   225                 230                 235                 240
   Asn Leu Ile Asn Ser Ser Asp Ile Lys Ser Ile Asn Ile Asn Val Asp
                   245                 250                 255
   Thr Lys Lys His Ile Glu Asn Lys Ala Lys Arg Asn Tyr Gln Val Pro
               260                 265                 270
   Tyr Ser Ile Asn Leu Asn Gly Thr Ser Thr Asn Ile Leu Ser Asn Leu
           275                 280                 285
   Ser Phe Ser Asn Lys Pro Trp Thr Asn Tyr Lys Asn Leu Thr Ser Gln
       290                 295                 300
   Ile Lys Ser Val Leu Lys His Asp Arg Gly Ile Ser Glu Gln Asp Leu
   305                 310                 315                 320
   Lys Tyr Ala Lys Lys Ala Tyr Tyr Thr Val Tyr Phe Lys Asn Gly Gly
                   325                 330                 335
   Lys Arg Ile Leu Gln Leu Asn Ser Lys Asn Tyr Thr Ala Asn Leu Val
               340                 345                 350
   His Ala Lys Asp Val Lys Arg Ile Glu Ile Thr Val Lys Thr Gly Thr
           355                 360                 365
   Lys Ala Lys Ala Asp Arg Tyr Val Pro Tyr Thr Ile Ala Val Asn Gly
       370                 375                 380
   Thr Ser Thr Pro Ile Leu Ser Asp Leu Lys Phe Thr Gly Asp Pro Arg
   385                 390                 395                 400
   Val Gly Tyr Lys Asp Ile Ser Lys Lys Val Lys Ser Val Leu Lys His
                   405                 410                 415
   Asp Arg Gly Ile Gly Glu Arg Glu Leu Lys Tyr Ala Lys Lys Ala Thr
               420                 425                 430
   Tyr Thr Val His Phe Lys Asn Gly Thr Lys Lys Val Ile Asn Ile Asn
           435                 440                 445
   Ser Asn Ile Ser Gln Leu Asn Leu Leu Tyr Val Gln Asp Ile Lys Lys
       450                 455                 460
   Ile Asp Ile Asp Val Lys Thr Gly Thr Lys Ala Lys Ala Asp Ser Tyr
   465                 470                 475                 480
   Val Pro Tyr Thr Ile Ala Val Asn Gly Thr Ser Thr Pro Ile Leu Ser
                   485                 490                 495
   Lys Leu Lys Ile Ser Asn Lys Gln Leu Ile Ser Tyr Lys Tyr Leu Asn
               500                 505                 510
   Asp Lys Val Lys Ser Val Leu Lys Ser Glu Arg Gly Ile Ser Asp Leu
               515                 520                 525
   Asp Leu Lys Phe Ala Lys Gln Ala Lys Tyr Thr Val Tyr Phe Lys Asn
           530                 535                 540
   Gly Lys Lys Gln Val Val Asn Leu Lys Ser Asp Ile Phe Thr Pro Asn
   545                 550                 555                 560
   Leu Phe Ser Ala Lys Asp Ile Lys Lys Ile Asp Ile Asp Val Lys Gln
                   565                 570                 575
   Tyr Thr Lys Ser Lys Lys Asn Lys
               580
<210> 5
   <211> 294
   <212> DNA
   <213> Staphylococcus sp.
<220>
   <221> CDS
   <222> (1)..(294)
<400> 5
   atg gca atg att aag atg agt cca gag gaa atc aga gca aaa tcg caa    48
   Met Ala Met Ile Lys Met Ser Pro Glu Glu Ile Arg Ala Lys Ser Gln
    1               5                  10                  15
   tct tac ggg caa ggt tca gac caa atc cgt caa att tta tct gat tta    96
   Ser Tyr Gly Gln Gly Ser Asp Gln Ile Arg Gln Ile Leu Ser Asp Leu
               20                  25                  30
   aca cgt gca caa ggt gaa att gca gcg aac tgg gaa ggt caa gct ttc   144
   Thr Arg Ala Gln Gly Glu Ile Ala Ala Asn Trp Glu Gly Gln Ala Phe
           35                  40                  45
   agc cgt ttc gaa gag caa ttc caa caa ctt agt cct aaa gta gaa aaa   192
   Ser Arg Phe Glu Glu Gln Phe Gln Gln Leu Ser Pro Lys Val Glu Lys
       50                  55                  60
   ttt gca caa tta tta gaa gaa att aaa caa caa ttg aat agc act gct   240
   Phe Ala Gln Leu Leu Glu Glu Ile Lys Gln Gln Leu Asn Ser Thr Ala
   65                  70                  75                  80
   gat gcc gtt caa gaa caa gac caa caa ctt tct aat aat ttc ggt ttg   288
   Asp Ala Val Gln Glu Gln Asp Gln Gln Leu Ser Asn Asn Phe Gly Leu
                   85                  90                  95
   caa taa                                                           294
   Gln
<210> 6
   <211> 97
   <212> PRT
   <213> Staphylococcus sp.
<400> 6
   Met Ala Met Ile Lys Met Ser Pro Glu Glu Ile Arg Ala Lys Ser Gln
   1               5                   10                  15
   Ser Tyr Gly Gln Gly Ser Asp Gln Ile Arg Gln Ile Leu Ser Asp Leu
               20                  25                  30
   Thr Arg Ala Gln Gly Glu Ile Ala Ala Asn Trp Glu Gly Gln Ala Phe
           35                  40                  45
   Ser Arg Phe Glu Glu Gln Phe Gln Gln Leu Ser Pro Lys Val Glu Lys
       50                  55                  60
   Phe Ala Gln Leu Leu Glu Glu Ile Lys Gln Gln Leu Asn Ser Thr Ala
   65                  70                  75                  80
   Asp Ala Val Gln Glu Gln Asp Gln Gln Leu Ser Asn Asn Phe Gly Leu
                   85                  90                  95
   Gln
<210> 7
   <211> 307
   <212> DNA
   <213> Staphylococcus sp.
<220>
   <221> CDS
   <222> (1)..(306)
<400> 7
   atg ggt gga tat aaa ggg att aaa gca gat ggt ggc aag gtg aat caa     48
   Met Gly Gly Tyr Lys Gly Ile Lys Ala Asp Gly Gly Lys Val Asn Gln
   1               5                   10                  15
   gcg aaa caa tta gcg gca aaa ata gct aaa gat att gaa gca tgt caa     96
   Ala Lys Gln Leu Ala Ala Lys Ile Ala Lys Asp Ile Glu Ala Cys Gln
               20                  25                  30
   aag caa acg caa cag ctc gct gag tat atc gaa ggt agt gat tgg gaa     144
   Lys Gln Thr Gln Gln Leu Ala Glu Tyr Ile Glu Gly Ser Asp Trp Glu
           35                  40                  45
   gga cag ttc gcc aat aag gtg aaa gat gtg tta ctt att atg gca aag     192
   Gly Gln Phe Ala Asn Lys Val Lys Asp Val Leu Leu Ile Met Ala Lys
       50                  55                  60
   ttt caa gaa gaa tta gta caa ccg atg gct gac cat caa aaa gca att     240
   Phe Gln Glu Glu Leu Val Gln Pro Met Ala Asp His Gln Lys Ala Ile
   65                  70                  75                  80
   gat aac tta agt caa aat cta gcg aaa tac gat aca tta tca att aag     288
   Asp Asn Leu Ser Gln Asn Leu Ala Lys Tyr Asp Thr Leu Ser Ile Lys
                   85                  90                  95
   caa gga ctt gat agg gtg a                                           307
   Gln Gly Leu Asp Arg Val
               100
<210> 8
   <211> 102
   <212> PRT
   <213> Staphylococcus sp.
<400> 8
   Met Gly Gly Tyr Lys Gly Ile Lys Ala Asp Gly Gly Lys Val Asn Gln
   1               5                   10                  15
   Ala Lys Gln Leu Ala Ala Lys Ile Ala Lys Asp Ile Glu Ala Cys Gln
               20                  25                  30
   Lys Gln Thr Gln Gln Leu Ala Glu Tyr Ile Glu Gly Ser Asp Trp Glu
           35                  40                  45
   Gly Gln Phe Ala Asn Lys Val Lys Asp Val Leu Leu Ile Met Ala Lys
       50                  55                  60
   Phe Gln Glu Glu Leu Val Gln Pro Met Ala Asp His Gln Lys Ala Ile
   65                  70                  75                  80
   Asp Asn Leu Ser Gln Asn Leu Ala Lys Tyr Asp Thr Leu Ser Ile Lys
                   85                  90                  95
   Gln Gly Leu Asp Arg Val
               100
<210> 9
   <211> 4158
   <212> DNA
   <213> Staphylococcus sp.
<220>
   <221> CDS
   <222> (1)..(4158)
<400> 9
   atg cta aac aga gaa aat aaa acg gca ata aca aga aaa ggc atg gta    48
   Met Leu Asn Arg Glu Asn Lys Thr Ala Ile Thr Arg Lys Gly Met Val
     1               5                  10                  15
   tcc aat cga tta aat aaa ttt tcg att aga aag tac aca gtg gga aca    96
   Ser Asn Arg Leu Asn Lys Phe Ser Ile Arg Lys Tyr Thr Val Gly Thr
               20                  25                  30
   gca tca att tta gta ggt aca aca tta att ttt ggt ctg ggg aac caa    144
   Ala Ser Ile Leu Val Gly Thr Thr Leu Ile Phe Gly Leu Gly Asn Gln
           35                  40                  45
   gaa gca aag gct gca gaa agt act aat aaa gaa ttg aac gaa gcg aca    192
   Glu Ala Lys Ala Ala Glu Ser Thr Asn Lys Glu Leu Asn Glu Ala Thr
       50                  55                  60
   act tca gca agt gat aat caa tcg agt gat aaa gtt gat atg cag caa    240
   Thr Ser Ala Ser Asp Asn Gln Ser Ser Asp Lys Val Asp Met Gln Gln
    65                  70                  75                  80
   cta aat caa gaa gac aat act aaa aat gat aat caa aaa gaa atg gta    288
   Leu Asn Gln Glu Asp Asn Thr Lys Asn Asp Asn Gln Lys Glu Met Val
                    85                  90                  95
   tca tct caa ggt aat gaa acg act tca aat ggg aat aaa tca ata gaa    336
   Ser Ser Gln Gly Asn Glu Thr Thr Ser Asn Gly Asn Lys Ser Ile Glu
               100                 105                 110
   aaa gaa agt gta caa tct acc act gga aat aaa gtt gaa gtt tca act    384
   Lys Glu Ser Val Gln Ser Thr Thr Gly Asn Lys Val Glu Val Ser Thr
           115                 120                 125
   gcc aaa tca gat gag caa gct tca cca aaa tct acg aat gaa gat tta    432
   Ala Lys Ser Asp Glu Gln Ala Ser Pro Lys Ser Thr Asn Glu Asp Leu
       130                 135                 140
   aac act aaa caa act ata agt aat caa gaa ggg tta caa cct gat ttg    480
   Asn Thr Lys Gln Thr Ile Ser Asn Gln Glu Gly Leu Gln Pro Asp Leu
   145                 150                 155                 160
   cta gag aat aaa tca gtg gta aat gtt caa cca act aat gag gaa aac    528
   Leu Glu Asn Lys Ser Val Val Asn Val Gln Pro Thr Asn Glu Glu Asn
                   165                 170                 175
   aaa aag gta gat gcg aaa act gaa tca act aca tta aat gtt aaa agt    576
   Lys Lys Val Asp Ala Lys Thr Glu Ser Thr Thr Leu Asn Val Lys Ser
               180                 185                 190
   gat gct atc aag agt aat gct gaa act ctt gtt gat aac aat agt aat    624
   Asp Ala Ile Lys Ser Asn Ala Glu Thr Leu Val Asp Asn Asn Ser Asn
           195                 200                 205
   tca aat aat gaa aat aat gca gat atc att ttg cca aaa agt aca gca    672
   Ser Asn Asn Glu Asn Asn Ala Asp Ile Ile Leu Pro Lys Ser Thr Ala
       210                 215                 220
   cct aaa agt ttg aat aca aga atg cgt atg gca gca ata caa cca aac    720
   Pro Lys Ser Leu Asn Thr Arg Met Arg Met Ala Ala Ile Gln Pro Asn
   225                 230                 235                 240
   tca aca gat tct aaa aat gtt aat gat tta atc aca tca aat aca aca    768
   Ser Thr Asp Ser Lys Asn Val Asn Asp Leu Ile Thr Ser Asn Thr Thr
                   245                 250                 255
   tta act gtc gtt gat gca gat aat agc aaa acg att gta cca gcc caa    816
   Leu Thr Val Val Asp Ala Asp Asn Ser Lys Thr Ile Val Pro Ala Gln
               260                 265                 270
   gat tat tta tca tta aaa tca caa att aca gtt gat gac aaa gtt aaa    864
   Asp Tyr Leu Ser Leu Lys Ser Gln Ile Thr Val Asp Asp Lys Val Lys
           275                 280                 285
   tca ggt gat tat ttc aca att aaa tac tca gat aca gta caa gta tat    912
   Ser Gly Asp Tyr Phe Thr Ile Lys Tyr Ser Asp Thr Val Gln Val Tyr
       290                 295                 300
   gga ttg aat ccg gaa gat att aaa aat att ggt gat att aaa gat cca    960
   Gly Leu Asn Pro Glu Asp Ile Lys Asn Ile Gly Asp Ile Lys Asp Pro
   305                 310                 315                 320
   aat aat ggt gaa aca att gcg act gca aaa cat gat act gca aat aat    1008
   Asn Asn Gly Glu Thr Ile Ala Thr Ala Lys His Asp Thr Ala Asn Asn
                   325                 330                 335
   tta att aca tat aca ttt aca gat tat gtt gat cga ttt aat tca gta    1056
   Leu Ile Thr Tyr Thr Phe Thr Asp Tyr Val Asp Arg Phe Asn Ser Val
               340                 345                 350
   aaa atg ggt att aat tac tca att tat atg gat gca gat aca att cct    1104
   Lys Met Gly Ile Asn Tyr Ser Ile Tyr Met Asp Ala Asp Thr Ile Pro
           355                 360                 365
   gtt gac aag aaa gat gtt cct ttt agt gta act att gga aat caa att    1152
   Val Asp Lys Lys Asp Val Pro Phe Ser Val Thr Ile Gly Asn Gln Ile
       370                 375                 380
   aca act aca aca gca gat atc act tat ccg gct tat aaa gaa gct gac   1200
   Thr Thr Thr Thr Ala Asp Ile Thr Tyr Pro Ala Tyr Lys Glu Ala Asp
   385                 390                 395                 400
   aat aat tca ata gga tca gct ttt aca gag aca gtt tct cat gta gga   1248
   Asn Asn Ser Ile Gly Ser Ala Phe Thr Glu Thr Val Ser His Val Gly
                   405                 410                 415
   aat gtt gaa gac cct ggt tac tat aac cag gta gta tat gtt aat cct   1296
   Asn Val Glu Asp Pro Gly Tyr Tyr Asn Gln Val Val Tyr Val Asn Pro
               420                 425                 430
   atg gat aag gat tta aaa ggt gct aag tta aaa gtt gaa gcg tac cat   1344
   Met Asp Lys Asp Leu Lys Gly Ala Lys Leu Lys Val Glu Ala Tyr His
           435                 440                 445
   ccg aaa tat cca act aat att ggt caa att aat caa aat gtt aca aat   1392
   Pro Lys Tyr Pro Thr Asn Ile Gly Gln Ile Asn Gln Asn Val Thr Asn
       450                 455                 460
   ata aaa ata tat cgt gtt cct gaa gga tat aca ttg aat aaa gga tat   1440
   Ile Lys Ile Tyr Arg Val Pro Glu Gly Tyr Thr Leu Asn Lys Gly Tyr
   465                 470                 475                 480
   gac gtt aat act aat gat ttg gta gac gta act gat gaa ttt aaa aat   1488
   Asp Val Asn Thr Asn Asp Leu Val Asp Val Thr Asp Glu Phe Lys Asn
                   485                 490                 495
   aaa atg acg tat gga tca aat caa agt gtt aat ctt gat ttt ggt gat   1536
   Lys Met Thr Tyr Gly Ser Asn Gln Ser Val Asn Leu Asp Phe Gly Asp
               500                 505                 510
   att aca tca gca tat gtt gta atg gtt aat aca aaa ttc caa tat aca   1584
   Ile Thr Ser Ala Tyr Val Val Met Val Asn Thr Lys Phe Gln Tyr Thr
           515                 520                 525
   aat agc gaa agc cca aca ctt gtt caa atg gct act tta tct tca aca   1632
   Asn Ser Glu Ser Pro Thr Leu Val Gln Met Ala Thr Leu Ser Ser Thr
       530                 535                 540
   ggt aat aaa tcc gtt tct act ggc aat gct tta gga ttt act aat aac   1680
   Gly Asn Lys Ser Val Ser Thr Gly Asn Ala Leu Gly Phe Thr Asn Asn
   545                 550                 555                 560
   caa agt ggc gga gct ggt caa gaa gta tat aaa att ggt aac tac gta   1728
   Gln Ser Gly Gly Ala Gly Gln Glu Val Tyr Lys Ile Gly Asn Tyr Val
                   565                 570                 575
   tgg gaa gat act aat aaa aac ggt gtt caa gaa tta gga gaa aaa ggc   1776
   Trp Glu Asp Thr Asn Lys Asn Gly Val Gln Glu Leu Gly Glu Lys Gly
               580                 585                 590
   gtt ggc aat gta act gta act gta ttt gat aat aat aca aat aca aaa   1824
   Val Gly Asn Val Thr Val Thr Val Phe Asp Asn Asn Thr Asn Thr Lys
           595                 600                 605
   gta gga gaa gca gtt act aaa gaa gat ggg tca tac ttg att cca aac   1872
   Val Gly Glu Ala Val Thr Lys Glu Asp Gly Ser Tyr Leu Ile Pro Asn
       610                 615                 620
   tta cct aat gga gat tac cgt gta gaa ttt tca aac tta cca aaa ggt    1920
   Leu Pro Asn Gly Asp Tyr Arg Val Glu Phe Ser Asn Leu Pro Lys Gly
   625                 630                 635                 640
   tat gaa gta acc cct tca aaa caa ggt aat aac gaa gaa tta gat tca    1968
   Tyr Glu Val Thr Pro Ser Lys Gln Gly Asn Asn Glu Glu Leu Asp Ser
                   645                 650                 655
   aac ggc tta tct tca gtt att aca gtt aat ggc aaa gat aac tta tct    2016
   Asn Gly Leu Ser Ser Val Ile Thr Val Asn Gly Lys Asp Asn Leu Ser
               660                 665                 670
   gca gac tta ggt att tac aaa cct aaa tac aac tta ggt gac tat gtc    2064
   Ala Asp Leu Gly Ile Tyr Lys Pro Lys Tyr Asn Leu Gly Asp Tyr Val
           675                 680                 685
   tgg gaa gat aca aat aaa aat ggt atc caa gac caa gat gaa aaa ggt    2112
   Trp Glu Asp Thr Asn Lys Asn Gly Ile Gln Asp Gln Asp Glu Lys Gly
       690                 695                 700
   ata tct ggc gta acg gta aca tta aaa gat gaa aac ggt aac gtg tta    2160
   Ile Ser Gly Val Thr Val Thr Leu Lys Asp Glu Asn Gly Asn Val Leu
   705                 710                 715                 720
   aaa aca gtt aca aca gac gca gat ggc aaa tat aaa ttt act gat tta    2208
   Lys Thr Val Thr Thr Asp Ala Asp Gly Lys Tyr Lys Phe Thr Asp Leu
                   725                 730                 735
   gat aat ggt aat tat aaa gtt gaa ttt act aca cca gaa ggc tat aca    2256
   Asp Asn Gly Asn Tyr Lys Val Glu Phe Thr Thr Pro Glu Gly Tyr Thr
               740                 745                 750
   ccg act aca gta aca tct ggt agc gac att gaa aaa gac tct aat ggt    2304
   Pro Thr Thr Val Thr Ser Gly Ser Asp Ile Glu Lys Asp Ser Asn Gly
           755                 760                 765
   tta aca aca aca ggt gtt att aat ggt gct gat aac atg aca tta gat    2352
   Leu Thr Thr Thr Gly Val Ile Asn Gly Ala Asp Asn Met Thr Leu Asp
       770                 775                 780
   agt gga ttc tac aaa aca cca aaa tat aat tta ggt aat tat gta tgg    2400
   Ser Gly Phe Tyr Lys Thr Pro Lys Tyr Asn Leu Gly Asn Tyr Val Trp
   785                 790                 795                 800
   gaa gat aca aat aaa gat ggt aag cag gat tca act gaa aaa ggt att    2448
   Glu Asp Thr Asn Lys Asp Gly Lys Gln Asp Ser Thr Glu Lys Gly Ile
                   805                 810                 815
   tca ggc gta aca gtt aca ttg aaa aat gaa aac ggt gaa gtt tta caa    2496
   Ser Gly Val Thr Val Thr Leu Lys Asn Glu Asn Gly Glu Val Leu Gln
               820                 825                 830
   aca act aaa aca gat aaa gat ggt aaa tat caa ttt act gga tta gaa    2544
   Thr Thr Lys Thr Asp Lys Asp Gly Lys Tyr Gln Phe Thr Gly Leu Glu
           835                 840                 845
   aat gga act tat aaa gtt gaa ttc gaa aca cca tca ggt tac aca cca    2592
   Asn Gly Thr Tyr Lys Val Glu Phe Glu Thr Pro Ser Gly Tyr Thr Pro
       850                 855                 860
   aca caa gta ggt tca gga act gat gaa ggt ata gat tca aat ggt aca    2640
   Thr Gln Val Gly Ser Gly Thr Asp Glu Gly Ile Asp Ser Asn Gly Thr
   865                 870                 875                 880
   tca aca aca ggt gtc att aaa gat aaa gat aac gat act att gac tct    2688
   Ser Thr Thr Gly Val Ile Lys Asp Lys Asp Asn Asp Thr Ile Asp Ser
                   885                 890                 895
   ggt ttc tac aaa ccg act tac aac tta ggt gac tat gta tgg gaa gat    2736
   Gly Phe Tyr Lys Pro Thr Tyr Asn Leu Gly Asp Tyr Val Trp Glu Asp
               900                 905                 910
   aca aat aaa aac ggt gtt caa gat aaa gat gaa aag ggt att tca ggt    2784
   Thr Asn Lys Asn Gly Val Gln Asp Lys Asp Glu Lys Gly Ile Ser Gly
           915                 920                 925
   gta aca gtt acg tta aaa gat gaa aac gac aaa gtt tta aaa aca gtt    2832
   Val Thr Val Thr Leu Lys Asp Glu Asn Asp Lys Val Leu Lys Thr Val
       930                 935                 940
   aca aca gat gaa aat ggt aaa tat caa ttc act gat tta aac aat gga    2880
   Thr Thr Asp Glu Asn Gly Lys Tyr Gln Phe Thr Asp Leu Asn Asn Gly
   945                 950                 955                 960
   act tat aaa gtt gaa ttc gag aca cca tca ggt tat aca cca act tca    2928
   Thr Tyr Lys Val Glu Phe Glu Thr Pro Ser Gly Tyr Thr Pro Thr Ser
                   965                 970                 975
   gta act tct gga aat gat act gaa aaa gat tct aat ggt tta aca aca    2976
   Val Thr Ser Gly Asn Asp Thr Glu Lys Asp Ser Asn Gly Leu Thr Thr
               980                 985                 990
   aca ggt gtc att aaa gat gca gat aac atg aca tta gac agt ggt ttc    3024
   Thr Gly Val Ile Lys Asp Ala Asp Asn Met Thr Leu Asp Ser Gly Phe
           995                 1000                1005
   tat aaa aca cca aaa tat agt tta ggt gat tat gtt tgg tac gac agt    3072
   Tyr Lys Thr Pro Lys Tyr Ser Leu Gly Asp Tyr Val Trp Tyr Asp Ser
      1010                1015                1020
   aat aaa gac ggc aaa caa gat tca act gaa aaa ggt atc aaa gat gtt    3120
   Asn Lys Asp Gly Lys Gln Asp Ser Thr Glu Lys Gly Ile Lys Asp Val
   1025                1030                1035                1040
   aaa gtt att tta tta aat gaa aaa ggc gaa gta att gga aca act aaa    3168
   Lys Val Ile Leu Leu Asn Glu Lys Gly Glu Val Ile Gly Thr Thr Lys
                  1045                1050                1055
   aca gat gaa aat ggt aaa tac cgc ttt gat aat tta gat agc ggt aaa    3216
   Thr Asp Glu Asn Gly Lys Tyr Arg Phe Asp Asn Leu Asp Ser Gly Lys
              1060                1065                1070
   tac aaa gtt att ttt gaa aag cct act ggc tta aca caa aca ggt aca    3264
   Tyr Lys Val Ile Phe Glu Lys Pro Thr Gly Leu Thr Gln Thr Gly Thr
          1075                1080                1085
   aat aca act gaa gat gat aaa gat gcc gat ggt ggc gaa gtt gat gta    3312
   Asn Thr Thr Glu Asp Asp Lys Asp Ala Asp Gly Gly Glu Val Asp Val
      1090                1095                1100
   aca att acg gat cat gat gat ttc aca ctt gat aat ggc tac tac gaa    3360
   Thr Ile Thr Asp His Asp Asp Phe Thr Leu Asp Asn Gly Tyr Tyr Glu
   1105                1110                1115                1120
   gaa gaa aca tca gat agc gac tca gat tcg gac agc gat tca gac tca    3408
   Glu Glu Thr Ser Asp Ser Asp Ser Asp Ser Asp Ser Asp Ser Asp Ser
               1125                1130                1135
   gac agc gat tca gac tca gat agt gat tca gat tca gat agt gat tca   3456
   Asp Ser Asp Ser Asp Ser Asp Ser Asp Ser Asp Ser Asp Ser Asp Ser
           1140                1145                1150
   gat tca gat agt gat tca gat tca gac agc gac tca gac tca gat agt   3504
   Asp Ser Asp Ser Asp Ser Asp Ser Asp Ser Asp Ser Asp Ser Asp Ser
       1155                1160                1165
   gac tca gac tca gat agc gat tca gat tca gat agc gat tca gac tca   3552
   Asp Ser Asp Ser Asp Ser Asp Ser Asp Ser Asp Ser Asp Ser Asp Ser
   1170                1175                1180
   gac agc gat tca gat tca gac agc gac tca gac tca gat agc gac tca   3600
   Asp Ser Asp Ser Asp Ser Asp Ser Asp Ser Asp Ser Asp Ser Asp Ser
   1185                1190                1195                1200
   gat tcg gac agc gat tca gac tca gat agc gac tca gac tca gac agc   3648
   Asp Ser Asp Ser Asp Ser Asp Ser Asp Ser Asp Ser Asp Ser Asp Ser
               1205                1210                1215
   gat tca gac tca gat agc gac tca gac tca gat agc gat tca gat tca   3696
   Asp Ser Asp Ser Asp Ser Asp Ser Asp Ser Asp Ser Asp Ser Asp Ser
           1220                1225                1230
   gac agc gat tca gat tca gac agt gat tca gat tca gac agc gac tca   3744
   Asp Ser Asp Ser Asp Ser Asp Ser Asp Ser Asp Ser Asp Ser Asp Ser
       1235                1240                1245
   gat tca gat agc gat tca gac tca gac tca gat agc gat tca gat tca   3792
   Asp Ser Asp Ser Asp Ser Asp Ser Asp Ser Asp Ser Asp Ser Asp Ser
   1250                1255                1260
   gac agc gac tca gat tcg gac agc gac tca gac tca gac agt gat tca   3840
   Asp Ser Asp Ser Asp Ser Asp Ser Asp Ser Asp Ser Asp Ser Asp Ser
   1265                1270                1275                1280
   gat tca gat agc gac tca gac tca gat agc gac tca gat tca gac agc   3888
   Asp Ser Asp Ser Asp Ser Asp Ser Asp Ser Asp Ser Asp Ser Asp Ser
               1285                1290                1295
   gat tca gac tca gat agt gac tca gat tcg gac agc gat tca gac tca   3936
   Asp Ser Asp Ser Asp Ser Asp Ser Asp Ser Asp Ser Asp Ser Asp Ser
           1300                1305                1310
   gat agc gac tca gat tca gac agt gat tca gac tca gat gca ggt aag   3984
   Asp Ser Asp Ser Asp Ser Asp Ser Asp Ser Asp Ser Asp Ala Gly Lys
       1315                1320                1325
   cac aca cct gtt aaa cca atg agt act act aaa gac cat cac aat aaa   4032
   His Thr Pro Val Lys Pro Met Ser Thr Thr Lys Asp His His Asn Lys
   1330                1335                1340
   gca aaa gca tta cca gaa aca ggt aat gaa aat agt ggc tca aat aac   4080
   Ala Lys Ala Leu Pro Glu Thr Gly Asn Glu Asn Ser Gly Ser Asn Asn
   1345                1350                1355                1360
   gca acg tta ttt ggc gga tta ttc gca gca tta gga tca tta ttg tta   4128
   Ala Thr Leu Phe Gly Gly Leu Phe Ala Ala Leu Gly Ser Leu Leu Leu
               1365                1370                1375
   ttc ggt cgt cgt aaa aaa caa aat aaa taa                           4158
   Phe Gly Arg Arg Lys Lys Gln Asn Lys
   1380 1385
<210> 10
   <211> 1385
   <212> PRT
   <213> Staphylococcus sp.
<400> 10
   Met Leu Asn Arg Glu Asn Lys Thr Ala Ile Thr Arg Lys Gly Met Val
   1               5                   10                  15
   Ser Asn Arg Leu Asn Lys Phe Ser Ile Arg Lys Tyr Thr Val Gly Thr
               20                  25                  30
   Ala Ser Ile Leu Val Gly Thr Thr Leu Ile Phe Gly Leu Gly Asn Gln
           35                  40                  45
   Glu Ala Lys Ala Ala Glu Ser Thr Asn Lys Glu Leu Asn Glu Ala Thr
       50                  55                  60
   Thr Ser Ala Ser Asp Asn Gln Ser Ser Asp Lys Val Asp Met Gln Gln
   65                  70                  75                  80
   Leu Asn Gln Glu Asp Asn Thr Lys Asn Asp Asn Gln Lys Glu Met Val
                   85                  90                  95
   Ser Ser Gln Gly Asn Glu Thr Thr Ser Asn Gly Asn Lys Ser Ile Glu
               100                 105                 110
   Lys Glu Ser Val Gln Ser Thr Thr Gly Asn Lys Val Glu Val Ser Thr
           115                 120                 125
   Ala Lys Ser Asp Glu Gln Ala Ser Pro Lys Ser Thr Asn Glu Asp Leu
       130                 135                 140
   Asn Thr Lys Gln Thr Ile Ser Asn Gln Glu Gly Leu Gln Pro Asp Leu
   145                 150                 155                 160
   Leu Glu Asn Lys Ser Val Val Asn Val Gln Pro Thr Asn Glu Glu Asn
                   165                 170                 175
   Lys Lys Val Asp Ala Lys Thr Glu Ser Thr Thr Leu Asn Val Lys Ser
               180                 185                 190
   Asp Ala Ile Lys Ser Asn Ala Glu Thr Leu Val Asp Asn Asn Ser Asn
           195                 200                 205
   Ser Asn Asn Glu Asn Asn Ala Asp Ile Ile Leu Pro Lys Ser Thr Ala
       210                 215                 220
   Pro Lys Ser Leu Asn Thr Arg Met Arg Met Ala Ala Ile Gln Pro Asn
   225                 230                 235                 240
   Ser Thr Asp Ser Lys Asn Val Asn Asp Leu Ile Thr Ser Asn Thr Thr
                   245                 250                 255
   Leu Thr Val Val Asp Ala Asp Asn Ser Lys Thr Ile Val Pro Ala Gln
               260                 265                 270
   Asp Tyr Leu Ser Leu Lys Ser Gln Ile Thr Val Asp Asp Lys Val Lys
           275                 280                 285
   Ser Gly Asp Tyr Phe Thr Ile Lys Tyr Ser Asp Thr Val Gln Val Tyr
       290                 295                 300
   Gly Leu Asn Pro Glu Asp Ile Lys Asn Ile Gly Asp Ile Lys Asp Pro
   305                 310                 315                 320
   Asn Asn Gly Glu Thr Ile Ala Thr Ala Lys His Asp Thr Ala Asn Asn
                   325                 330                 335
   Leu Ile Thr Tyr Thr Phe Thr Asp Tyr Val Asp Arg Phe Asn Ser Val
               340                 345                 350
   Lys Met Gly Ile Asn Tyr Ser Ile Tyr Met Asp Ala Asp Thr Ile Pro
           355                 360                 365
   Val Asp Lys Lys Asp Val Pro Phe Ser Val Thr Ile Gly Asn Gln Ile
       370                 375                 380
   Thr Thr Thr Thr Ala Asp Ile Thr Tyr Pro Ala Tyr Lys Glu Ala Asp
   385                 390                 395                 400
   Asn Asn Ser Ile Gly Ser Ala Phe Thr Glu Thr Val Ser His Val Gly
                   405                 410                 415
   Asn Val Glu Asp Pro Gly Tyr Tyr Asn Gln Val Val Tyr Val Asn Pro
                   420                 425                 430
   Met Asp Lys Asp Leu Lys Gly Ala Lys Leu Lys Val Glu Ala Tyr His
               435                 440                 445
   Pro Lys Tyr Pro Thr Asn Ile Gly Gln Ile Asn Gln Asn Val Thr Asn
       450                 455                 460
   Ile Lys Ile Tyr Arg Val Pro Glu Gly Tyr Thr Leu Asn Lys Gly Tyr
   465                 470                 475                 480
   Asp Val Asn Thr Asn Asp Leu Val Asp Val Thr Asp Glu Phe Lys Asn
                   485                 490                 495
   Lys Met Thr Tyr Gly Ser Asn Gln Ser Val Asn Leu Asp Phe Gly Asp
               500                 505                 510
   Ile Thr Ser Ala Tyr Val Val Met Val Asn Thr Lys Phe Gln Tyr Thr
           515                 520                 525
   Asn Ser Glu Ser Pro Thr Leu Val Gln Met Ala Thr Leu Ser Ser Thr
       530                 535                 540
   Gly Asn Lys Ser Val Ser Thr Gly Asn Ala Leu Gly Phe Thr Asn Asn
   545                 550                 555                 560
   Gln Ser Gly Gly Ala Gly Gln Glu Val Tyr Lys Ile Gly Asn Tyr Val
                   565                 570                 575
   Trp Glu Asp Thr Asn Lys Asn Gly Val Gln Glu Leu Gly Glu Lys Gly
               580                 585                 590
   Val Gly Asn Val Thr Val Thr Val Phe Asp Asn Asn Thr Asn Thr Lys
           595                 600                 605
   Val Gly Glu Ala Val Thr Lys Glu Asp Gly Ser Tyr Leu Ile Pro Asn
       610                 615                 620
   Leu Pro Asn Gly Asp Tyr Arg Val Glu Phe Ser Asn Leu Pro Lys Gly
   625                 630                 635                 640
   Tyr Glu Val Thr Pro Ser Lys Gln Gly Asn Asn Glu Glu Leu Asp Ser
                   645                 650                 655
   Asn Gly Leu Ser Ser Val Ile Thr Val Asn Gly Lys Asp Asn Leu Ser
               660                 665                 670
   Ala Asp Leu Gly Ile Tyr Lys Pro Lys Tyr Asn Leu Gly Asp Tyr Val
           675                 680                 685
   Trp Glu Asp Thr Asn Lys Asn Gly Ile Gln Asp Gln Asp Glu Lys Gly
       690                 695                 700
   Ile Ser Gly Val Thr Val Thr Leu Lys Asp Glu Asn Gly Asn Val Leu
   705                 710                 715                 720
   Lys Thr Val Thr Thr Asp Ala Asp Gly Lys Tyr Lys Phe Thr Asp Leu
                   725                 730                 735
   Asp Asn Gly Asn Tyr Lys Val Glu Phe Thr Thr Pro Glu Gly Tyr Thr
               740                 745                 750
   Pro Thr Thr Val Thr Ser Gly Ser Asp Ile Glu Lys Asp Ser Asn Gly
           755                 760                 765
   Leu Thr Thr Thr Gly Val Ile Asn Gly Ala Asp Asn Met Thr Leu Asp
       770                 775                 780
   Ser Gly Phe Tyr Lys Thr Pro Lys Tyr Asn Leu Gly Asn Tyr Val Trp
   785                 790                 795                 800
   Glu Asp Thr Asn Lys Asp Gly Lys Gln Asp Ser Thr Glu Lys Gly Ile
                   805                 810                 815
   Ser Gly Val Thr Val Thr Leu Lys Asn Glu Asn Gly Glu Val Leu Gln
               820                 825                 830
   Thr Thr Lys Thr Asp Lys Asp Gly Lys Tyr Gln Phe Thr Gly Leu Glu
           835                 840                 845
   Asn Gly Thr Tyr Lys Val Glu Phe Glu Thr Pro Ser Gly Tyr Thr Pro
       850                 855                 860
   Thr Gln Val Gly Ser Gly Thr Asp Glu Gly Ile Asp Ser Asn Gly Thr
   865                 870                 875                 880
   Ser Thr Thr Gly Val Ile Lys Asp Lys Asp Asn Asp Thr Ile Asp Ser
                   885                 890                 895
   Gly Phe Tyr Lys Pro Thr Tyr Asn Leu Gly Asp Tyr Val Trp Glu Asp
               900                 905                 910
   Thr Asn Lys Asn Gly Val Gln Asp Lys Asp Glu Lys Gly Ile Ser Gly
           915                 920                 925
   Val Thr Val Thr Leu Lys Asp Glu Asn Asp Lys Val Leu Lys Thr Val
       930                 935                 940
   Thr Thr Asp Glu Asn Gly Lys Tyr Gln Phe Thr Asp Leu Asn Asn Gly
   945                 950                 955                 960
   Thr Tyr Lys Val Glu Phe Glu Thr Pro Ser Gly Tyr Thr Pro Thr Ser
                   965                 970                 975
   Val Thr Ser Gly Asn Asp Thr Glu Lys Asp Ser Asn Gly Leu Thr Thr
               980                 985                 990
   Thr Gly Val Ile Lys Asp Ala Asp Asn Met Thr Leu Asp Ser Gly Phe
           995                 1000                1005
   Tyr Lys Thr Pro Lys Tyr Ser Leu Gly Asp Tyr Val Trp Tyr Asp Ser
      1010                1015                1020
   Asn Lys Asp Gly Lys Gln Asp Ser Thr Glu Lys Gly Ile Lys Asp Val
   1025                1030                1035                1040
   Lys Val Ile Leu Leu Asn Glu Lys Gly Glu Val Ile Gly Thr Thr Lys
                  1045                1050                1055
   Thr Asp Glu Asn Gly Lys Tyr Arg Phe Asp Asn Leu Asp Ser Gly Lys
              1060                1065                1070
   Tyr Lys Val Ile Phe Glu Lys Pro Thr Gly Leu Thr Gln Thr Gly Thr
          1075                1080                1085
   Asn Thr Thr Glu Asp Asp Lys Asp Ala Asp Gly Gly Glu Val Asp Val
      1090                1095                1100
   Thr Ile Thr Asp His Asp Asp Phe Thr Leu Asp Asn Gly Tyr Tyr Glu
   1105                1110                1115                1120
   Glu Glu Thr Ser Asp Ser Asp Ser Asp Ser Asp Ser Asp Ser Asp Ser
                  1125                1130                1135
   Asp Ser Asp Ser Asp Ser Asp Ser Asp Ser Asp Ser Asp Ser Asp Ser
              1140                1145                1150
   Asp Ser Asp Ser Asp Ser Asp Ser Asp Ser Asp Ser Asp Ser Asp Ser
          1155                1160                1165
   Asp Ser Asp Ser Asp Ser Asp Ser Asp Ser Asp Ser Asp Ser Asp Ser
      1170                1175                1180
   Asp Ser Asp Ser Asp Ser Asp Ser Asp Ser Asp Ser Asp Ser Asp Ser
   1185                1190                1195                1200
   Asp Ser Asp Ser Asp Ser Asp Ser Asp Ser Asp Ser Asp Ser Asp Ser
                  1205                1210                1215
   Asp Ser Asp Ser Asp Ser Asp Ser Asp Ser Asp Ser Asp Ser Asp Ser
              1220                1225                1230
   Asp Ser Asp Ser Asp Ser Asp Ser Asp Ser Asp Ser Asp Ser Asp Ser
          1235                1240                1245
   Asp Ser Asp Ser Asp Ser Asp Ser Asp Ser Asp Ser Asp Ser Asp Ser
      1250                1255                1260
   Asp Ser Asp Ser Asp Ser Asp Ser Asp Ser Asp Ser Asp Ser Asp Ser
   1265                1270                1275                1280
   Asp Ser Asp Ser Asp Ser Asp Ser Asp Ser Asp Ser Asp Ser Asp Ser
                  1285                1290                1295
   Asp Ser Asp Ser Asp Ser Asp Ser Asp Ser Asp Ser Asp Ser Asp Ser
              1300                1305                1310
   Asp Ser Asp Ser Asp Ser Asp Ser Asp Ser Asp Ser Asp Ala Gly Lys
          1315                1320                1325
   His Thr Pro Val Lys Pro Met Ser Thr Thr Lys Asp His His Asn Lys
      1330                1335                1340
   Ala Lys Ala Leu Pro Glu Thr Gly Asn Glu Asn Ser Gly Ser Asn Asn
   1345                1350                1355                1360
   Ala Thr Leu Phe Gly Gly Leu Phe Ala Ala Leu Gly Ser Leu Leu Leu
                  1365                1370                1375
   Phe Gly Arg Arg Lys Lys Gln Asn Lys
              1380                1385
<210> 11
   <211> 3426
   <212> DNA
   <213> Staphylococcus sp.
<220>
   <221> CDS
   <222> (1)..(3426)
<400> 11
   atg att aac agg gat aat aaa aag gca ata aca aaa aag ggt atg att     48
   Met Ile Asn Arg Asp Asn Lys Lys Ala Ile Thr Lys Lys Gly Met Ile
    1               5                  10                  15
   tca aat cgc tta aac aaa ttt tcg att aga aag tat act gta gga act     96
   Ser Asn Arg Leu Asn Lys Phe Ser Ile Arg Lys Tyr Thr Val Gly Thr
               20                  25                  30
   gca tcg att tta gta ggt acg aca ttg att ttt ggt cta ggg aac caa    144
   Ala Ser Ile Leu Val Gly Thr Thr Leu Ile Phe Gly Leu Gly Asn Gln
           35                  40                  45
   gaa gct aaa gct gct gaa aac act agt aca gaa aat gcg aaa caa gat    192
   Glu Ala Lys Ala Ala Glu Asn Thr Ser Thr Glu Asn Ala Lys Gln Asp
       50                  55                  60
   gat gca acg act agt gat aat aaa gaa gta gtg tcg gaa act gaa aat    240
   Asp Ala Thr Thr Ser Asp Asn Lys Glu Val Val Ser Glu Thr Glu Asn
   65                  70                  75                  80
   aat tcg aca aca gaa aat gat tca aca aat cca att aag aaa gaa aca    288
   Asn Ser Thr Thr Glu Asn Asp Ser Thr Asn Pro Ile Lys Lys Glu Thr
                   85                  90                  95
   aat act gat tca caa cca gaa gct aaa gaa gaa tca act aca tca agt    336
   Asn Thr Asp Ser Gln Pro Glu Ala Lys Glu Glu Ser Thr Thr Ser Ser
               100                 105                 110
   act caa caa cag caa aat aac gtt aca gct aca act gaa act aag cct    384
   Thr Gln Gln Gln Gln Asn Asn Val Thr Ala Thr Thr Glu Thr Lys Pro
           115                 120                 125
   caa aac att gaa aaa gaa aat gtt aaa cct tca act gat aaa act gcg    432
   Gln Asn Ile Glu Lys Glu Asn Val Lys Pro Ser Thr Asp Lys Thr Ala
       130                 135                 140
   aca gaa gat aca tct gtt att tta gaa gag aag aaa gca cca aat tat    480
   Thr Glu Asp Thr Ser Val Ile Leu Glu Glu Lys Lys Ala Pro Asn Tyr
   145                 150                 155                 160
   aca aat aac gat gta act aca aaa cca tct aca agt gaa att caa aca    528
   Thr Asn Asn Asp Val Thr Thr Lys Pro Ser Thr Ser Glu Ile Gln Thr
                   165                 170                 175
   aaa cca act aca cct caa gaa tct aca aat att gaa aat tca caa ccg    576
   Lys Pro Thr Thr Pro Gln Glu Ser Thr Asn Ile Glu Asn Ser Gln Pro
               180                 185                 190
   caa cca acg cct tca aaa gta gac aat caa gtt aca gat gca act aat    624
   Gln Pro Thr Pro Ser Lys Val Asp Asn Gln Val Thr Asp Ala Thr Asn
           195                 200                 205
   cca aaa gaa cca gta aat gtg tca aaa gaa gaa ctt aaa aat aat cct    672
   Pro Lys Glu Pro Val Asn Val Ser Lys Glu Glu Leu Lys Asn Asn Pro
       210                 215                 220
   gag aaa tta aaa gaa tta gtt aga aat gat aac aat aca gat cgt tca    720
   Glu Lys Leu Lys Glu Leu Val Arg Asn Asp Asn Asn Thr Asp Arg Ser
   225                 230                 235                 240
   act aaa cca gtt gct aca gct cca aca agt gtt gca cca aaa cga tta      768
   Thr Lys Pro Val Ala Thr Ala Pro Thr Ser Val Ala Pro Lys Arg Leu
                   245                 250                 255
   aat gcg aaa atg cgt ttt gca gtt gca caa cca gca gca gtt gct tca      816
   Asn Ala Lys Met Arg Phe Ala Val Ala Gln Pro Ala Ala Val Ala Ser
               260                 265                 270
   aat aat gta aat gac tta att aca gtt acg aaa cag acg atc aaa gtt      864
   Asn Asn Val Asn Asp Leu Ile Thr Val Thr Lys Gln Thr Ile Lys Val
           275                 280                 285
   ggc gat ggt aaa gat aat gtg gca gca gcg cat gac ggt aaa gat att      912
   Gly Asp Gly Lys Asp Asn Val Ala Ala Ala His Asp Gly Lys Asp Ile
       290                 295                 300
   gaa tat gat aca gag ttt aca att gac aat aaa gtc aaa aaa ggc gat      960
   Glu Tyr Asp Thr Glu Phe Thr Ile Asp Asn Lys Val Lys Lys Gly Asp
   305                 310                 315                 320
   aca atg acg att aat tat gat aag aat gta att cct tcg gat tta aca      1008
   Thr Met Thr Ile Asn Tyr Asp Lys Asn Val Ile Pro Ser Asp Leu Thr
                   325                 330                 335
   gat aaa aat gat cct atc gat att act gat cca tca gga gag gtc att      1056
   Asp Lys Asn Asp Pro Ile Asp Ile Thr Asp Pro Ser Gly Glu Val Ile
               340                 345                 350
   gcc aaa gga aca ttt gat aaa gcg act aag caa atc aca tat aca ttt      1104
   Ala Lys Gly Thr Phe Asp Lys Ala Thr Lys Gln Ile Thr Tyr Thr Phe
           355                 360                 365
   aca gat tat gta gat aaa tat gaa gat ata aaa gca cgt tta act tta      1152
   Thr Asp Tyr Val Asp Lys Tyr Glu Asp Ile Lys Ala Arg Leu Thr Leu
       370                 375                 380
   tac tca tat att gat aag caa gca gta cct aat gaa act agt ttg aat      1200
   Tyr Ser Tyr Ile Asp Lys Gln Ala Val Pro Asn Glu Thr Ser Leu Asn
   385                 390                 395                 400
   tta acg ttt gca aca gca ggt aaa gaa act agc caa aac gtt tct gtt      1248
   Leu Thr Phe Ala Thr Ala Gly Lys Glu Thr Ser Gln Asn Val Ser Val
                   405                 410                 415
   gat tat caa gac cca atg gtt cat ggt gat tca aac att caa tct atc      1296
   Asp Tyr Gln Asp Pro Met Val His Gly Asp Ser Asn Ile Gln Ser Ile
               420                 425                 430
   ttt aca aag tta gat gaa aac aaa caa act att gaa caa caa att tat      1344
   Phe Thr Lys Leu Asp Glu Asn Lys Gln Thr Ile Glu Gln Gln Ile Tyr
           435                 440                 445
   gtt aat cct ttg aaa aaa aca gca act aac act aaa gtt gat ata gct      1392
   Val Asn Pro Leu Lys Lys Thr Ala Thr Asn Thr Lys Val Asp Ile Ala
       450                 455                 460
   ggt agt caa gta gat gat tat gga aat att aaa cta gga aat ggt agt      1440
   Gly Ser Gln Val Asp Asp Tyr Gly Asn Ile Lys Leu Gly Asn Gly Ser
   465                 470                 475                 480
   acc att att gac caa aat aca gaa ata aaa gtt tat aaa gtt aac cct    1488
   Thr Ile Ile Asp Gln Asn Thr Glu Ile Lys Val Tyr Lys Val Asn Pro
                   485                 490                 495
   aat caa caa ttg cct caa agt aat aga atc tat gat ttt agt caa tac    1536
   Asn Gln Gln Leu Pro Gln Ser Asn Arg Ile Tyr Asp Phe Ser Gln Tyr
               500                 505                 510
   gaa gat gta aca agt caa ttt gat aat aaa aaa tca ttt agt aat aat    1584
   Glu Asp Val Thr Ser Gln Phe Asp Asn Lys Lys Ser Phe Ser Asn Asn
           515                 520                 525
   gta gca aca ttg gat ttt ggt gat att aat tca gcc tat att atc aaa    1632
   Val Ala Thr Leu Asp Phe Gly Asp Ile Asn Ser Ala Tyr Ile Ile Lys
       530                 535                 540
   gtt gtt agt aaa tat aca cct aca tca gat ggc gaa cta gat att gct    1680
   Val Val Ser Lys Tyr Thr Pro Thr Ser Asp Gly Glu Leu Asp Ile Ala
   545                 550                 555                 560
   caa ggt act agt atg aga aca act gat aaa tat ggt tat tat aat tat    1728
   Gln Gly Thr Ser Met Arg Thr Thr Asp Lys Tyr Gly Tyr Tyr Asn Tyr
                   565                 570                 575
   gca gga tat tca aac ttc atc gta act tct aat gac act ggc ggt ggc    1776
   Ala Gly Tyr Ser Asn Phe Ile Val Thr Ser Asn Asp Thr Gly Gly Gly
               580                 585                 590
   gac ggt act gtt aaa cct gaa gaa aag tta tac aaa att ggt gac tat    1824
   Asp Gly Thr Val Lys Pro Glu Glu Lys Leu Tyr Lys Ile Gly Asp Tyr
           595                 600                 605
   gta tgg gaa gac gtt gat aaa gac ggt gtc caa ggt aca gat tcg aaa    1872
   Val Trp Glu Asp Val Asp Lys Asp Gly Val Gln Gly Thr Asp Ser Lys
       610                 615                 620
   gaa aag cca atg gca aac gtt tta gtt aca tta act tac ccg gac ggt    1920
   Glu Lys Pro Met Ala Asn Val Leu Val Thr Leu Thr Tyr Pro Asp Gly
   625                 630                 635                 640
   act aca aaa tca gta aga aca gat gct aac ggt cat tat gaa ttc ggt    1968
   Thr Thr Lys Ser Val Arg Thr Asp Ala Asn Gly His Tyr Glu Phe Gly
                   645                 650                 655
   ggt ttg aaa gac gga gaa act tat aca gtt aaa ttc gaa acg cca gct    2016
   Gly Leu Lys Asp Gly Glu Thr Tyr Thr Val Lys Phe Glu Thr Pro Ala
               660                 665                 670
   gga tat ctt cca aca aaa gta aat gga aca act gat ggt gaa aaa gac    2064
   Gly Tyr Leu Pro Thr Lys Val Asn Gly Thr Thr Asp Gly Glu Lys Asp
           675                 680                 685
   tca aat ggt agt tct ata act gtt aaa att aat ggt aaa gat gat atg    2112
   Ser Asn Gly Ser Ser Ile Thr Val Lys Ile Asn Gly Lys Asp Asp Met
       690                 695                 700
   tct tta gac act ggt ttt tat aaa gaa cct aaa tat aat ctt ggt gac    2160
   Ser Leu Asp Thr Gly Phe Tyr Lys Glu Pro Lys Tyr Asn Leu Gly Asp
   705                 710                 715                 720
   tat gta tgg gaa gat aca aat aaa gat ggt atc caa gat gct aat gaa    2208
   Tyr Val Trp Glu Asp Thr Asn Lys Asp Gly Ile Gln Asp Ala Asn Glu
                   725                 730                 735
   cct ggt atc aaa gat gtt aag gtt aca tta aaa gat agt act gga aaa     2256
   Pro Gly Ile Lys Asp Val Lys Val Thr Leu Lys Asp Ser Thr Gly Lys
               740                 745                 750
   gtt att ggt aca act act act gat gcc tcg ggt aaa tat aaa ttt aca     2304
   Val Ile Gly Thr Thr Thr Thr Asp Ala Ser Gly Lys Tyr Lys Phe Thr
           755                 760                 765
   gat tta gat aat ggt aac tat aca gta gaa ttt gaa aca cca gca ggt     2352
   Asp Leu Asp Asn Gly Asn Tyr Thr Val Glu Phe Glu Thr Pro Ala Gly
       770                 775                 780
   tac acg cca acg gtt aaa aat act aca gct gaa gat aaa gat tct aat     2400
   Tyr Thr Pro Thr Val Lys Asn Thr Thr Ala Glu Asp Lys Asp Ser Asn
   785                 790                 795                 800
   ggt tta aca aca aca ggt gtc att aaa gat gca gat aat atg aca tta     2448
   Gly Leu Thr Thr Thr Gly Val Ile Lys Asp Ala Asp Asn Met Thr Leu
                   805                 810                 815
   gac agt ggt ttc tat aaa aca cca aaa tac agt tta ggt gat tat gtt     2496
   Asp Ser Gly Phe Tyr Lys Thr Pro Lys Tyr Ser Leu Gly Asp Tyr Val
               820                 825                 830
   tgg tac gac agt aat aaa gac ggt aaa caa gat tca act gaa aaa ggt     2544
   Trp Tyr Asp Ser Asn Lys Asp Gly Lys Gln Asp Ser Thr Glu Lys Gly
           835                 840                 845
   atc aaa gat gtt aaa gtt act tta tta aat gaa aaa ggc gaa gta att     2592
   Ile Lys Asp Val Lys Val Thr Leu Leu Asn Glu Lys Gly Glu Val Ile
       850                 855                 860
   gga aca act aaa aca gat gaa aat ggt aaa tat cgt ttc gat aat tta     2640
   Gly Thr Thr Lys Thr Asp Glu Asn Gly Lys Tyr Arg Phe Asp Asn Leu
   865                 870                 875                 880
   gat agc ggt aaa tac aaa gtt att ttt gaa aag cct gct ggc tta aca     2688
   Asp Ser Gly Lys Tyr Lys Val Ile Phe Glu Lys Pro Ala Gly Leu Thr
                   885                 890                 895
   caa aca gtt aca aat aca act gaa gat gat aaa gat gcc gat ggt ggc     2736
   Gln Thr Val Thr Asn Thr Thr Glu Asp Asp Lys Asp Ala Asp Gly Gly
               900                 905                 910
   gaa gtt gac gta aca att acg gat cat gat gat ttc aca ctt gat aac     2784
   Glu Val Asp Val Thr Ile Thr Asp His Asp Asp Phe Thr Leu Asp Asn
           915                 920                 925
   gga tac ttc gaa gaa gat aca tca gac agt gat tca gac tca gac agt     2832
   Gly Tyr Phe Glu Glu Asp Thr Ser Asp Ser Asp Ser Asp Ser Asp Ser
       930                 935                 940
   gat tca gac tca gac agc gac tca gat tca gac agt gat tca gac tca     2880
   Asp Ser Asp Ser Asp Ser Asp Ser Asp Ser Asp Ser Asp Ser Asp Ser
   945                 950                 955                 960
   gat agc gat tca gat tca gac agc gac tca gac tca gat agc gac tca     2928
   Asp Ser Asp Ser Asp Ser Asp Ser Asp Ser Asp Ser Asp Ser Asp Ser
                   965                 970                 975
   gac tca gac agc gac tca gac tca gat agc gac tca gat tcg gac agc     2976
   Asp Ser Asp Ser Asp Ser Asp Ser Asp Ser Asp Ser Asp Ser Asp Ser
               980                 985                 990
   gat tca gac tca gat agc gac tca gat tca gac agc gat tca gac tca    3024
   Asp Ser Asp Ser Asp Ser Asp Ser Asp Ser Asp Ser Asp Ser Asp Ser
           995                 1000                1005
   gat agc gac tca gat tca gac agt gac tca gac tca gat agc gac tca    3072
   Asp Ser Asp Ser Asp Ser Asp Ser Asp Ser Asp Ser Asp Ser Asp Ser
      1010                1015                1020
   gac tca gac agt gac tca gac tca gac agc gat tca gat tca gat agc    3120
   Asp Ser Asp Ser Asp Ser Asp Ser Asp Ser Asp Ser Asp Ser Asp Ser
   1025                1030                1035                1040
   gac tca gat tcg gac agt gat tca gac tca gat agc gac tca gat tca    3168
   Asp Ser Asp Ser Asp Ser Asp Ser Asp Ser Asp Ser Asp Ser Asp Ser
                   1045                1050                1055
   gac agc gac tca gac tca gat agc gac tca gac tca gac agt gat tca    3216
   Asp Ser Asp Ser Asp Ser Asp Ser Asp Ser Asp Ser Asp Ser Asp Ser
               1060                1065                1070
   gac tca gat agc gat tcg gac tcg gat gca gga aaa cat aca cct gtt    3264
   Asp Ser Asp Ser Asp Ser Asp Ser Asp Ala Gly Lys His Thr Pro Val
           1075                1080                1085
   aaa cca atg agt act act aaa gac cat cac aat aaa gca aaa gca tta    3312
   Lys Pro Met Ser Thr Thr Lys Asp His His Asn Lys Ala Lys Ala Leu
      1090                1095                1100
   cca gaa aca ggt agt gaa aat aac ggc tca aat aac gca acg tta ttt    3360
   Pro Glu Thr Gly Ser Glu Asn Asn Gly Ser Asn Asn Ala Thr Leu Phe
   1105                1110                1115                1120
   ggt gga tta ttt gca gca tta ggt tca tta ttg tta ttc ggt cgt cgc    3408
   Gly Gly Leu Phe Ala Ala Leu Gly Ser Leu Leu Leu Phe Gly Arg Arg
                   1125                1130                1135
   aaa aaa caa aac aaa taa                                            3426
   Lys Lys Gln Asn Lys
               1140
<210> 12
   <211> 1141
   <212> PRT
   <213> Staphylococcus sp.
<400> 12
   Met Ile Asn Arg Asp Asn Lys Lys Ala Ile Thr Lys Lys Gly Met Ile
    1               5                  10                  15
   Ser Asn Arg Leu Asn Lys Phe Ser Ile Arg Lys Tyr Thr Val Gly Thr
               20                  25                  30
   Ala Ser Ile Leu Val Gly Thr Thr Leu Ile Phe Gly Leu Gly Asn Gln
           35                  40                  45
   Glu Ala Lys Ala Ala Glu Asn Thr Ser Thr Glu Asn Ala Lys Gln Asp
       50                  55                  60
   Asp Ala Thr Thr Ser Asp Asn Lys Glu Val Val Ser Glu Thr Glu Asn
   65                  70                  75                  80
   Asn Ser Thr Thr Glu Asn Asp Ser Thr Asn Pro Ile Lys Lys Glu Thr
                   85                  90                  95
   Asn Thr Asp Ser Gln Pro Glu Ala Lys Glu Glu Ser Thr Thr Ser Ser
               100                 105                 110
   Thr Gln Gln Gln Gln Asn Asn Val Thr Ala Thr Thr Glu Thr Lys Pro
           115                 120                 125
   Gln Asn Ile Glu Lys Glu Asn Val Lys Pro Ser Thr Asp Lys Thr Ala
       130                 135                 140
   Thr Glu Asp Thr Ser Val Ile Leu Glu Glu Lys Lys Ala Pro Asn Tyr
   145                 150                 155                 160
   Thr Asn Asn Asp Val Thr Thr Lys Pro Ser Thr Ser Glu Ile Gln Thr
                   165                 170                 175
   Lys Pro Thr Thr Pro Gln Glu Ser Thr Asn Ile Glu Asn Ser Gln Pro
               180                 185                 190
   Gln Pro Thr Pro Ser Lys Val Asp Asn Gln Val Thr Asp Ala Thr Asn
           195                 200                 205
   Pro Lys Glu Pro Val Asn Val Ser Lys Glu Glu Leu Lys Asn Asn Pro
       210                 215                 220
   Glu Lys Leu Lys Glu Leu Val Arg Asn Asp Asn Asn Thr Asp Arg Ser
   225                 230                 235                 240
   Thr Lys Pro Val Ala Thr Ala Pro Thr Ser Val Ala Pro Lys Arg Leu
                   245                 250                 255
   Asn Ala Lys Met Arg Phe Ala Val Ala Gln Pro Ala Ala Val Ala Ser
               260                 265                 270
   Asn Asn Val Asn Asp Leu Ile Thr Val Thr Lys Gln Thr Ile Lys Val
           275                 280                 285
   Gly Asp Gly Lys Asp Asn Val Ala Ala Ala His Asp Gly Lys Asp Ile
       290                 295                 300
   Glu Tyr Asp Thr Glu Phe Thr Ile Asp Asn Lys Val Lys Lys Gly Asp
   305                 310                 315                 320
   Thr Met Thr Ile Asn Tyr Asp Lys Asn Val Ile Pro Ser Asp Leu Thr
                   325                 330                 335
   Asp Lys Asn Asp Pro Ile Asp Ile Thr Asp Pro Ser Gly Glu Val Ile
               340                 345                 350
   Ala Lys Gly Thr Phe Asp Lys Ala Thr Lys Gln Ile Thr Tyr Thr Phe
           355                 360                 365
   Thr Asp Tyr Val Asp Lys Tyr Glu Asp Ile Lys Ala Arg Leu Thr Leu
       370                 375                 380
   Tyr Ser Tyr Ile Asp Lys Gln Ala Val Pro Asn Glu Thr Ser Leu Asn
   385                 390                 395                 400
   Leu Thr Phe Ala Thr Ala Gly Lys Glu Thr Ser Gln Asn Val Ser Val
                   405                 410                 415
   Asp Tyr Gln Asp Pro Met Val His Gly Asp Ser Asn Ile Gln Ser Ile
               420                 425                 430
   Phe Thr Lys Leu Asp Glu Asn Lys Gln Thr Ile Glu Gln Gln Ile Tyr
           435                 440                 445
   Val Asn Pro Leu Lys Lys Thr Ala Thr Asn Thr Lys Val Asp Ile Ala
       450                 455                 460
   Gly Ser Gln Val Asp Asp Tyr Gly Asn Ile Lys Leu Gly Asn Gly Ser
   465                 470                 475                 480
   Thr Ile Ile Asp Gln Asn Thr Glu Ile Lys Val Tyr Lys Val Asn Pro
                   485                 490                 495
   Asn Gln Gln Leu Pro Gln Ser Asn Arg Ile Tyr Asp Phe Ser Gln Tyr
               500                 505                 510
   Glu Asp Val Thr Ser Gln Phe Asp Asn Lys Lys Ser Phe Ser Asn Asn
           515                 520                 525
   Val Ala Thr Leu Asp Phe Gly Asp Ile Asn Ser Ala Tyr Ile Ile Lys
       530                 535                 540
   Val Val Ser Lys Tyr Thr Pro Thr Ser Asp Gly Glu Leu Asp Ile Ala
   545                 550                 555                 560
   Gln Gly Thr Ser Met Arg Thr Thr Asp Lys Tyr Gly Tyr Tyr Asn Tyr
                   565                 570                 575
   Ala Gly Tyr Ser Asn Phe Ile Val Thr Ser Asn Asp Thr Gly Gly Gly
               580                 585                 590
   Asp Gly Thr Val Lys Pro Glu Glu Lys Leu Tyr Lys Ile Gly Asp Tyr
           595                 600                 605
   Val Trp Glu Asp Val Asp Lys Asp Gly Val Gln Gly Thr Asp Ser Lys
       610                 615                 620
   Glu Lys Pro Met Ala Asn Val Leu Val Thr Leu Thr Tyr Pro Asp Gly
   625                 630                 635                 640
   Thr Thr Lys Ser Val Arg Thr Asp Ala Asn Gly His Tyr Glu Phe Gly
                   645                 650                 655
   Gly Leu Lys Asp Gly Glu Thr Tyr Thr Val Lys Phe Glu Thr Pro Ala
               660                 665                 670
   Gly Tyr Leu Pro Thr Lys Val Asn Gly Thr Thr Asp Gly Glu Lys Asp
           675                 680                 685
   Ser Asn Gly Ser Ser Ile Thr Val Lys Ile Asn Gly Lys Asp Asp Met
       690                 695                 700
   Ser Leu Asp Thr Gly Phe Tyr Lys Glu Pro Lys Tyr Asn Leu Gly Asp
   705                 710                 715                 720
   Tyr Val Trp Glu Asp Thr Asn Lys Asp Gly Ile Gln Asp Ala Asn Glu
                   725                 730                 735
   Pro Gly Ile Lys Asp Val Lys Val Thr Leu Lys Asp Ser Thr Gly Lys
               740                 745                 750
   Val Ile Gly Thr Thr Thr Thr Asp Ala Ser Gly Lys Tyr Lys Phe Thr
           755                 760                 765
   Asp Leu Asp Asn Gly Asn Tyr Thr Val Glu Phe Glu Thr Pro Ala Gly
       770                 775                 780
   Tyr Thr Pro Thr Val Lys Asn Thr Thr Ala Glu Asp Lys Asp Ser Asn
   785                 790                 795                 800
   Gly Leu Thr Thr Thr Gly Val Ile Lys Asp Ala Asp Asn Met Thr Leu
                   805                 810                 815
   Asp Ser Gly Phe Tyr Lys Thr Pro Lys Tyr Ser Leu Gly Asp Tyr Val
               820                 825                 830
   Trp Tyr Asp Ser Asn Lys Asp Gly Lys Gln Asp Ser Thr Glu Lys Gly
           835                 840                 845
   Ile Lys Asp Val Lys Val Thr Leu Leu Asn Glu Lys Gly Glu Val Ile
       850                 855                 860
   Gly Thr Thr Lys Thr Asp Glu Asn Gly Lys Tyr Arg Phe Asp Asn Leu
   865                 870                 875                 880
   Asp Ser Gly Lys Tyr Lys Val Ile Phe Glu Lys Pro Ala Gly Leu Thr
                   885                 890                 895
   Gln Thr Val Thr Asn Thr Thr Glu Asp Asp Lys Asp Ala Asp Gly Gly
               900                 905                 910
   Glu Val Asp Val Thr Ile Thr Asp His Asp Asp Phe Thr Leu Asp Asn
           915                 920                 925
   Gly Tyr Phe Glu Glu Asp Thr Ser Asp Ser Asp Ser Asp Ser Asp Ser
       930                 935                 940
   Asp Ser Asp Ser Asp Ser Asp Ser Asp Ser Asp Ser Asp Ser Asp Ser
   945                 950                 955                 960
   Asp Ser Asp Ser Asp Ser Asp Ser Asp Ser Asp Ser Asp Ser Asp Ser
                   965                 970                 975
   Asp Ser Asp Ser Asp Ser Asp Ser Asp Ser Asp Ser Asp Ser Asp Ser
               980                 985                 990
   Asp Ser Asp Ser Asp Ser Asp Ser Asp Ser Asp Ser Asp Ser Asp Ser
           995                 1000                1005
   Asp Ser Asp Ser Asp Ser Asp Ser Asp Ser Asp Ser Asp Ser Asp Ser
      1010                 1015                1020
   Asp Ser Asp Ser Asp Ser Asp Ser Asp Ser Asp Ser Asp Ser Asp Ser
   1025                1030                1035                1040
   Asp Ser Asp Ser Asp Ser Asp Ser Asp Ser Asp Ser Asp Ser Asp Ser
                  1045                1050                1055
   Asp Ser Asp Ser Asp Ser Asp Ser Asp Ser Asp Ser Asp Ser Asp Ser
              1060                1065                1070
   Asp Ser Asp Ser Asp Ser Asp Ser Asp Ala Gly Lys His Thr Pro Val
          1075                1080                1085
   Lys Pro Met Ser Thr Thr Lys Asp His His Asn Lys Ala Lys Ala Leu
      1090                1095                1100
   Pro Glu Thr Gly Ser Glu Asn Asn Gly Ser Asn Asn Ala Thr Leu Phe
   1105                1110                1115                1120
   Gly Gly Leu Phe Ala Ala Leu Gly Ser Leu Leu Leu Phe Gly Arg Arg
                   1125                1130                1135
   Lys Lys Gln Asn Lys
               1140
<210> 13
   <211> 1052
   <212> DNA
   <213> Staphylococcus sp.
<220>
   <221> CDS
   <222> (1)..(1050)
<400> 13
   atg aca aaa cat tat tta aac agt aag tat caa tca gaa caa cgt tca    48
   Met Thr Lys His Tyr Leu Asn Ser Lys Tyr Gln Ser Glu Gln Arg Ser
    1               5                  10                  15
   tca gct atg aaa aag att aca atg ggt aca gca tct atc att tta ggt    96
   Ser Ala Met Lys Lys Ile Thr Met Gly Thr Ala Ser Ile Ile Leu Gly
               20                  25                  30
   tcc ctt gta tac ata ggc gca gac agc caa caa gtc aat gcg gca aca    144
   Ser Leu Val Tyr Ile Gly Ala Asp Ser Gln Gln Val Asn Ala Ala Thr
           35                  40                  45
   gaa gct acg aac gca act aat aat caa agc aca caa gtt tct caa gca    192
   Glu Ala Thr Asn Ala Thr Asn Asn Gln Ser Thr Gln Val Ser Gln Ala
       50                  55                  60
   aca tca caa cca att aat ttc caa gtg caa aaa gat ggc tct tca gag    240
   Thr Ser Gln Pro Ile Asn Phe Gln Val Gln Lys Asp Gly Ser Ser Glu
   65                  70                  75                  80
   aag tca cac atg gat gac tat atg caa cac cct ggt aaa gta att aaa    288
   Lys Ser His Met Asp Asp Tyr Met Gln His Pro Gly Lys Val Ile Lys
                   85                  90                  95
   caa aat aat aaa tat tat ttc caa acc gtg tta aac aat gca tca ttc    336
   Gln Asn Asn Lys Tyr Tyr Phe Gln Thr Val Leu Asn Asn Ala Ser Phe
               100                 105                 110
   tgg aaa gaa tac aaa ttt tac aat gca aac aat caa gaa tta gca aca    384
   Trp Lys Glu Tyr Lys Phe Tyr Asn Ala Asn Asn Gln Glu Leu Ala Thr
           115                 120                 125
   act gtt gtt aac gat aat aaa aaa gcg gat act aga aca atc aat gtt    432
   Thr Val Val Asn Asp Asn Lys Lys Ala Asp Thr Arg Thr Ile Asn Val
       130                 135                 140
   gca gtt gaa cct gga tat aag agc tta act act aaa gta cat att gtc    480
   Ala Val Glu Pro Gly Tyr Lys Ser Leu Thr Thr Lys Val His Ile Val
   145                 150                 155                 160
   gtg cca caa att aat tac aat cat aga tat act acg cat ttg gaa ttt    528
   Val Pro Gln Ile Asn Tyr Asn His Arg Tyr Thr Thr His Leu Glu Phe
                   165                 170                 175
   gaa aaa gca att cct aca tta gct gac gca gca aaa cca aac aat gtt    576
   Glu Lys Ala Ile Pro Thr Leu Ala Asp Ala Ala Lys Pro Asn Asn Val
               180                 185                 190
   aaa ccg gtt caa cca aaa cca gct caa cct aaa aca cct act gag caa      624
   Lys Pro Val Gln Pro Lys Pro Ala Gln Pro Lys Thr Pro Thr Glu Gln
           195                 200                 205
   act aaa cca gtt caa cct aaa gtt gaa aaa gtt aaa cct act gta act      672
   Thr Lys Pro Val Gln Pro Lys Val Glu Lys Val Lys Pro Thr Val Thr
       210                 215                 220
   aca aca agc aaa gtt gaa gac aat cac tct act aaa gtt gta agt act      720
   Thr Thr Ser Lys Val Glu Asp Asn His Ser Thr Lys Val Val Ser Thr
   225                 230                 235                 240
   gac aca aca aaa gat caa act aaa aca caa act gct cat aca gtt aaa      768
   Asp Thr Thr Lys Asp Gln Thr Lys Thr Gln Thr Ala His Thr Val Lys
                   245                 250                 255
   aca gca caa act gct caa gaa caa aat aaa gtt caa aca cct gtt aaa      816
   Thr Ala Gln Thr Ala Gln Glu Gln Asn Lys Val Gln Thr Pro Val Lys
               260                 265                 270
   gat gtt gca aca gcg aaa tct gaa agc aac aat caa gct gta agt gat      864
   Asp Val Ala Thr Ala Lys Ser Glu Ser Asn Asn Gln Ala Val Ser Asp
           275                 280                 285
   aat aaa tca caa caa act aac aaa gtt aca aaa cat aac gaa acg cct      912
   Asn Lys Ser Gln Gln Thr Asn Lys Val Thr Lys His Asn Glu Thr Pro
       290                 295                 300
   aaa caa gca tct aaa gct aaa gaa tta cca aaa act ggt tta act tca      960
   Lys Gln Ala Ser Lys Ala Lys Glu Leu Pro Lys Thr Gly Leu Thr Ser
   305                 310                 315                 320
   gtt gat aac ttt att agc aca gtt gcc ttc gca aca ctt gcc ctt tta      1008
   Val Asp Asn Phe Ile Ser Thr Val Ala Phe Ala Thr Leu Ala Leu Leu
                   325                 330                 335
   ggt tca tta tct tta tta ctt ttc aaa aga aaa gaa tct aaa ta           1052
   Gly Ser Leu Ser Leu Leu Leu Phe Lys Arg Lys Glu Ser Lys
               340                 345                 350
<210> 14
   <211> 350
   <212> PRT
   <213> Staphylococcus sp.
<400> 14
   Met Thr Lys His Tyr Leu Asn Ser Lys Tyr Gln Ser Glu Gln Arg Ser
   1               5                   10                  15
   Ser Ala Met Lys Lys Ile Thr Met Gly Thr Ala Ser Ile Ile Leu Gly
               20                  25                  30
   Ser Leu Val Tyr Ile Gly Ala Asp Ser Gln Gln Val Asn Ala Ala Thr
           35                  40                  45
   Glu Ala Thr Asn Ala Thr Asn Asn Gln Ser Thr Gln Val Ser Gln Ala
       50                  55                  60
   Thr Ser Gln Pro Ile Asn Phe Gln Val Gln Lys Asp Gly Ser Ser Glu
   65                  70                  75                  80
   Lys Ser His Met Asp Asp Tyr Met Gln His Pro Gly Lys Val Ile Lys
                   85                  90                  95
   Gln Asn Asn Lys Tyr Tyr Phe Gln Thr Val Leu Asn Asn Ala Ser Phe
               100                 105                 110
   Trp Lys Glu Tyr Lys Phe Tyr Asn Ala Asn Asn Gln Glu Leu Ala Thr
           115                 120                 125
   Thr Val Val Asn Asp Asn Lys Lys Ala Asp Thr Arg Thr Ile Asn Val
       130                 135                 140
   Ala Val Glu Pro Gly Tyr Lys Ser Leu Thr Thr Lys Val His Ile Val
   145                 150                 155                 160
   Val Pro Gln Ile Asn Tyr Asn His Arg Tyr Thr Thr His Leu Glu Phe
                   165                 170                 175
   Glu Lys Ala Ile Pro Thr Leu Ala Asp Ala Ala Lys Pro Asn Asn Val
               180                 185                 190
   Lys Pro Val Gln Pro Lys Pro Ala Gln Pro Lys Thr Pro Thr Glu Gln
           195                 200                 205
   Thr Lys Pro Val Gln Pro Lys Val Glu Lys Val Lys Pro Thr Val Thr
       210                 215                 220
   Thr Thr Ser Lys Val Glu Asp Asn His Ser Thr Lys Val Val Ser Thr
   225                 230                 235                 240
   Asp Thr Thr Lys Asp Gln Thr Lys Thr Gln Thr Ala His Thr Val Lys
                   245                 250                 255
   Thr Ala Gln Thr Ala Gln Glu Gln Asn Lys Val Gln Thr Pro Val Lys
               260                 265                 270
   Asp Val Ala Thr Ala Lys Ser Glu Ser Asn Asn Gln Ala Val Ser Asp
           275                 280                 285
   Asn Lys Ser Gln Gln Thr Asn Lys Val Thr Lys His Asn Glu Thr Pro
       290                 295                 300
   Lys Gln Ala Ser Lys Ala Lys Glu Leu Pro Lys Thr Gly Leu Thr Ser
   305                 310                 315                 320
   Val Asp Asn Phe Ile Ser Thr Val Ala Phe Ala Thr Leu Ala Leu Leu
                   325                 330                 335
   Gly Ser Leu Ser Leu Leu Leu Phe Lys Arg Lys Glu Ser Lys
               340                 345                 350
<210> 15
   <211> 1938
   <212> DNA
   <213> Staphylococcus sp.
<400> 15
   ttagttttta cgttttctag gtaatacgaa tgcaacgatg ctacttaaag ctagtaatgc 60
   cattaatggt aatgtcatat ctttatttga ttcttcacca gtttgtggta atgattttgc 120
   tttattttct tgtgtatttt tattgttttg gctttgagtg tgtccatcat ttgtgttttt 180
   aatgtttgct ttttgtaatg gagcactatc ttttgcttcg ctagaacctg ctgaagtttg 240
   aacaacatct tttgttgttt ttgatgaagc agttgttggt tttgcaacat tttgagtcgt 300
   agatactacc ttagttggag ttgtactact tgattctact tcacctttag ttggttttgt 360
   agcaggcgtt ttgtctttac ctgactcact agatgcgtca ttttcttttt caacacttgg 420
   taattgttta ttgtcatctt tttggctgtc ttgtttttgt gattcttttt caacaggtga 480
   tggtgttggt ttgctaggcg tagctggagt agcttccttc ttagctgagt tatcttgttg 540
   ttcttttttg ttagatttat cggtattggc ttttgtaaat gcttctttat caacgattct 600
   gacatggtat tgtccatcat aatcaatcgt ttttacgtga actttaacga tagcatcata 660
   tagagtttta ccttcaacat atgggaaaat aattgttcta gtattatttt tagcatcttt 720
   gcttatagtt ctaacacgtt gaccttcaac catgaaatct ttccagtaat cgtcattagt 780
   agtttccatg accatatatt ttttgccgtt aagcatacct gttttaatag ggtgtttaac 840
   aaaagtatcc atcatagatt cgttattctc aacactttca taaacaacat attttgtatc 900
   ttgtaaatca gtcatttttt catttgttgg ttgtacattt tggaattcag taatagctga 960
   tttcacttgc tcatctaaag ctttctttgt atcctctaat ttcttcttgt actcagcctt 1020
   taatttttca ggaagtttat cttgaatttt atttaattca taaacttgtc tttctagtgt 1080
   tttcgctttt ttatatggcg ctaataattt ttcagcttta taatcttctt cagttttgaa 1140
   tttatctgca ctgttataaa ttggttgtgc gaattccatt aatgtgtaat cgtatttttc 1200
   ttctttgtta ttgaagtgag ttgaacttac aattttaacg gcttttgttc catttgaaac 1260
   agagaagcga atgtaagcgt aatctttaac agtatcgtat gatactaatt taattggcaa 1320
   ctttttgtca ccttcataaa cttcaaattt tctccaaaat tgacctgatt gtaatcctaa 1380
   ttcaatttct ggttttgaat cagtgaaaat aactctagca ggtttaacag agctggcata 1440
   atgataaaat tgttgctcac cattttcttt tttcatttca aaatcaattg gacgagagtt 1500
   tggtgcgcta tgatctttat cttttattgc agggttttta atcgcttctc taagttcctg 1560
   attcaaaata ggatatgtat tgttagtggc ttttgctgct ggtttaactg cttttgtttc 1620
   cttaggggct ttaacttctt taacttcttt agcttctttt gtttcagaag taggggcctc 1680
   aacttcttta ttagatactg agacagcatt agctactggt ttagtttctg gagctttttc 1740
   agatgttgtt gttggacttg caactgcttc agtttttggt tgtgcttctg tatttgtacc 1800
   acctgtttct tcagctgctg cttgtgcttc gccatttgac attaataata aaagtgtact 1860
   aatcgctaca gatgcaacgc ctagtgatga ctttctaatt gaataaaatg atttaaattc 1920
   tttttgctgt ttgttcat                                               1938
<210> 16
   <211> 645
   <212> PRT
   <213> Staphylococcus sp.
<400> 16
   Met Asn Lys Gln Gln Lys Glu Phe Lys Ser Phe Tyr Ser Ile Arg Lys
   1               5                   10                  15
   Ser Ser Leu Gly Val Ala Ser Val Ala Ile Ser Thr Leu Leu Leu Leu
               20                  25                  30
   Met Ser Asn Gly Glu Ala Gln Ala Ala Ala Glu Glu Thr Gly Gly Thr
           35                  40                  45
   Asn Thr Glu Ala Gln Pro Lys Thr Glu Ala Val Ala Ser Pro Thr Thr
       50                  55                  60
   Thr Ser Glu Lys Ala Pro Glu Thr Lys Pro Val Ala Asn Ala Val Ser
   65                  70                  75                  80
   Val Ser Asn Lys Glu Val Glu Ala Pro Thr Ser Glu Thr Lys Glu Ala
                   85                  90                  95
   Lys Glu Val Lys Glu Val Lys Ala Pro Lys Glu Thr Lys Ala Val Lys
               100                 105                 110
   Pro Ala Ala Lys Ala Thr Asn Asn Thr Tyr Pro Ile Leu Asn Gln Glu
           115                 120                 125
   Leu Arg Glu Ala Ile Lys Asn Pro Ala Ile Lys Asp Lys Asp His Ser
       130                 135                 140
   Ala Pro Asn Ser Arg Pro Ile Asp Phe Glu Met Lys Lys Glu Asn Gly
   145                 150                 155                 160
   Glu Gln Gln Phe Tyr His Tyr Ala Ser Ser Val Lys Pro Ala Arg Val
                   165                 170                 175
   Ile Phe Thr Asp Ser Lys Pro Glu Ile Glu Leu Gly Leu Gln Ser Gly
               180                 185                 190
   Gln Phe Trp Arg Lys Phe Glu Val Tyr Glu Gly Asp Lys Lys Leu Pro
           195                 200                 205
   Ile Lys Leu Val Ser Tyr Asp Thr Val Lys Asp Tyr Ala Tyr Ile Arg
       210                 215                 220
   Phe Ser Val Ser Asn Gly Thr Lys Ala Val Lys Ile Val Ser Ser Thr
   225                 230                 235                 240
   His Phe Asn Asn Lys Glu Glu Lys Tyr Asp Tyr Thr Leu Met Glu Phe
                   245                 250                 255
   Ala Gln Pro Ile Tyr Asn Ser Ala Asp Lys Phe Lys Thr Glu Glu Asp
               260                 265                 270
   Tyr Lys Ala Glu Lys Leu Leu Ala Pro Tyr Lys Lys Ala Lys Thr Leu
           275                 280                 285
   Glu Arg Gln Val Tyr Glu Leu Asn Lys Ile Gln Asp Lys Leu Pro Glu
       290                 295                 300
   Lys Leu Lys Ala Glu Tyr Lys Lys Lys Leu Glu Asp Thr Lys Lys Ala
   305                 310                 315                 320
   Leu Asp Glu Gln Val Lys Ser Ala Ile Thr Glu Phe Gln Asn Val Gln
                   325                 330                 335
   Pro Thr Asn Glu Lys Met Thr Asp Leu Gln Asp Thr Lys Tyr Val Val
               340                 345                 350
   Tyr Glu Ser Val Glu Asn Asn Glu Ser Met Met Asp Thr Phe Val Lys
           355                 360                 365
   His Pro Ile Lys Thr Gly Met Leu Asn Gly Lys Lys Tyr Met Val Met
       370                 375                 380
   Glu Thr Thr Asn Asp Asp Tyr Trp Lys Asp Phe Met Val Glu Gly Gln
   385                 390                 395                 400
   Arg Val Arg Thr Ile Ser Lys Asp Ala Lys Asn Asn Thr Arg Thr Ile
                   405                 410                 415
   Ile Phe Pro Tyr Val Glu Gly Lys Thr Leu Tyr Asp Ala Ile Val Lys
               420                 425                 430
   Val His Val Lys Thr Ile Asp Tyr Asp Gly Gln Tyr His Val Arg Ile
           435                 440                 445
   Val Asp Lys Glu Ala Phe Thr Lys Ala Asn Thr Asp Lys Ser Asn Lys
       450                 455                 460
   Lys Glu Gln Gln Asp Asn Ser Ala Lys Lys Glu Ala Thr Pro Ala Thr
   465                 470                 475                 480
   Pro Ser Lys Pro Thr Pro Ser Pro Val Glu Lys Glu Ser Gln Lys Gln
                   485                 490                 495
   Asp Ser Gln Lys Asp Asp Asn Lys Gln Leu Pro Ser Val Glu Lys Glu
               500                 505                 510
   Asn Asp Ala Ser Ser Glu Ser Gly Lys Asp Lys Thr Pro Ala Thr Lys
           515                 520                 525
   Pro Thr Lys Gly Glu Val Glu Ser Ser Ser Thr Thr Pro Thr Lys Val
       530                 535                 540
   Val Ser Thr Thr Gln Asn Val Ala Lys Pro Thr Thr Ala Ser Ser Lys
   545                 550                 555                 560
   Thr Thr Lys Asp Val Val Gln Thr Ser Ala Gly Ser Ser Glu Ala Lys
                   565                 570                 575
   Asp Ser Ala Pro Leu Gln Lys Ala Asn Ile Lys Asn Thr Asn Asp Gly
               580                 585                 590
   His Thr Gln Ser Gln Asn Asn Lys Asn Thr Gln Glu Asn Lys Ala Lys
           595                 600                 605
   Ser Leu Pro Gln Thr Gly Glu Glu Ser Asn Lys Asp Met Thr Leu Pro
       610                 615                 620
   Leu Met Ala Leu Leu Ala Leu Ser Ser Ile Val Ala Phe Val Leu Pro
   625                 630                 635                 640
   Arg Lys Arg Lys Asn
                   645
<210> 17
   <211> 1353
   <212> DNA
   <213> Staphylococcus sp.
<220>
   <221> CDS
   <222> (1)..(1353)
<400> 17
   ttg aaa aag aaa aac att tat tca att cgt aaa cta ggt gta ggt att    48
   Met Lys Lys Lys Asn Ile Tyr Ser Ile Arg Lys Leu Gly Val Gly Ile
     1               5                  10                  15
   gca tct gta act tta ggt aca tta ctt ata tct ggt ggc gta aca cct    96
   Ala Ser Val Thr Leu Gly Thr Leu Leu Ile Ser Gly Gly Val Thr Pro
                20                  25                  30
   gct gca aat gct gcg caa cac gat gaa gct caa caa aat gct ttt tat   144
   Ala Ala Asn Ala Ala Gln His Asp Glu Ala Gln Gln Asn Ala Phe Tyr
            35                  40                  45
   caa gtg tta aat atg cct aac tta aac gct gat caa cgt aat ggt ttt   192
   Gln Val Leu Asn Met Pro Asn Leu Asn Ala Asp Gln Arg Asn Gly Phe
        50                  55                  60
   atc caa agc ctt aaa gat gat cca agc caa agt gct aac gtt tta ggt   240
   Ile Gln Ser Leu Lys Asp Asp Pro Ser Gln Ser Ala Asn Val Leu Gly
    65                  70                  75                  80
   gaa gct caa aaa ctt aat gac tct caa gct cca aaa gct gat gcg caa   288
   Glu Ala Gln Lys Leu Asn Asp Ser Gln Ala Pro Lys Ala Asp Ala Gln
                   85                  90                  95
   caa aat aac ttc aac aaa gat caa caa agc gcc ttc tat gaa atc ttg   336
   Gln Asn Asn Phe Asn Lys Asp Gln Gln Ser Ala Phe Tyr Glu Ile Leu
               100                 105                 110
   aac atg cct aac tta aac gaa gcg caa cgt aac ggc ttc att caa agt   384
   Asn Met Pro Asn Leu Asn Glu Ala Gln Arg Asn Gly Phe Ile Gln Ser
           115                 120                 125
   ctt aaa gac gac cca agc caa agc act aat gtt tta ggt gaa gct aaa   432
   Leu Lys Asp Asp Pro Ser Gln Ser Thr Asn Val Leu Gly Glu Ala Lys
       130                 135                 140
   aaa tta aac gaa tct caa gca ccg aaa gct gat aac aat ttc aac aaa   480
   Lys Leu Asn Glu Ser Gln Ala Pro Lys Ala Asp Asn Asn Phe Asn Lys
   145                 150                 155                 160
   gaa caa caa aat gct ttc tat gaa atc ttg aat atg cct aac tta aac   528
   Glu Gln Gln Asn Ala Phe Tyr Glu Ile Leu Asn Met Pro Asn Leu Asn
                   165                 170                 175
   gaa gaa caa cgc aat ggt ttc atc caa agc tta aaa gat gac cca agc   576
   Glu Glu Gln Arg Asn Gly Phe Ile Gln Ser Leu Lys Asp Asp Pro Ser
               180                 185                 190
   caa agt gct aac cta ttg tca gaa gct aaa aag tta aat gaa tct caa   624
   Gln Ser Ala Asn Leu Leu Ser Glu Ala Lys Lys Leu Asn Glu Ser Gln
           195                 200                 205
   gca ccg aaa gcg gat aac aaa ttc aac aaa gaa caa caa aat gct ttc   672
   Ala Pro Lys Ala Asp Asn Lys Phe Asn Lys Glu Gln Gln Asn Ala Phe
       210                 215                 220
   tat gaa atc tta cat tta cct aac tta aac gaa gaa caa cgt aac ggc   720
   Tyr Glu Ile Leu His Leu Pro Asn Leu Asn Glu Glu Gln Arg Asn Gly
   225                 230                 235                 240
   ttc atc caa agc ctt aaa gac gat cct tca gtg agc aaa gaa att tta   768
   Phe Ile Gln Ser Leu Lys Asp Asp Pro Ser Val Ser Lys Glu Ile Leu
                   245                 250                 255
   gca gaa gct aaa aag cta aac gat gct caa gca cca aaa gag gaa gac   816
   Ala Glu Ala Lys Lys Leu Asn Asp Ala Gln Ala Pro Lys Glu Glu Asp
               260                 265                 270
   aac aaa aaa cct ggt aaa gaa gac ggc aac aaa cct ggc aaa gaa gac   864
   Asn Lys Lys Pro Gly Lys Glu Asp Gly Asn Lys Pro Gly Lys Glu Asp
           275                 280                 285
   ggc aac aag cct ggt aaa gaa gac aac aaa aaa cct ggt aaa gaa gac   912
   Gly Asn Lys Pro Gly Lys Glu Asp Asn Lys Lys Pro Gly Lys Glu Asp
       290                 295                 300
   ggc aac aag cct ggt aaa gaa gac aac aac aaa cct ggc aaa gaa gac   960
   Gly Asn Lys Pro Gly Lys Glu Asp Asn Asn Lys Pro Gly Lys Glu Asp
   305                 310                 315                 320
   ggc aac aag cct ggt aaa gaa gac aac aac aag cct ggt aaa gaa gac   1008
   Gly Asn Lys Pro Gly Lys Glu Asp Asn Asn Lys Pro Gly Lys Glu Asp
                   325                 330                 335
   ggc aac aag cct ggt aaa gaa gac ggc aac aaa cct ggt aaa gaa gac     1056
   Gly Asn Lys Pro Gly Lys Glu Asp Gly Asn Lys Pro Gly Lys Glu Asp
               340                 345                 350
   ggc aac gga gta cat gtc gtt aaa cct ggt gat aca gta aat gac att     1104
   Gly Asn Gly Val His Val Val Lys Pro Gly Asp Thr Val Asn Asp Ile
           355                 360                 365
   gca aaa gca aac ggc act act gct gac aaa att gct gca gat aac aaa     1152
   Ala Lys Ala Asn Gly Thr Thr Ala Asp Lys Ile Ala Ala Asp Asn Lys
       370                 375                 380
   tta gct gat aaa aac atg atc aaa cct ggt caa gaa ctt gtt gtt gat     1200
   Leu Ala Asp Lys Asn Met Ile Lys Pro Gly Gln Glu Leu Val Val Asp
   385                 390                 395                 400
   aag aag caa cca gca aac cat gca gat gct aac aaa gct caa gca tta     1248
   Lys Lys Gln Pro Ala Asn His Ala Asp Ala Asn Lys Ala Gln Ala Leu
                   405                 410                 415
   cca gaa act ggt gaa gaa aat cca ttc atc ggt aca act gta ttt ggt     1296
   Pro Glu Thr Gly Glu Glu Asn Pro Phe Ile Gly Thr Thr Val Phe Gly
               420                 425                 430
   gga tta tca tta gcc tta ggt gca gcg tta tta gct gga cgt cgt cgc     1344
   Gly Leu Ser Leu Ala Leu Gly Ala Ala Leu Leu Ala Gly Arg Arg Arg
           435                 440                 445
   gaa cta taa                                                         1353
   Glu Leu
       450
<210> 18
   <211> 450
   <212> PRT
   <213> Staphylococcus sp.
<400> 18
   Met Lys Lys Lys Asn Ile Tyr Ser Ile Arg Lys Leu Gly Val Gly Ile
   1               5                   10                  15
   Ala Ser Val Thr Leu Gly Thr Leu Leu Ile Ser Gly Gly Val Thr Pro
               20                  25                  30
   Ala Ala Asn Ala Ala Gln His Asp Glu Ala Gln Gln Asn Ala Phe Tyr
           35                  40                  45
   Gln Val Leu Asn Met Pro Asn Leu Asn Ala Asp Gln Arg Asn Gly Phe
       50                  55                  60
   Ile Gln Ser Leu Lys Asp Asp Pro Ser Gln Ser Ala Asn Val Leu Gly
   65                  70                  75                  80
   Glu Ala Gln Lys Leu Asn Asp Ser Gln Ala Pro Lys Ala Asp Ala Gln
                   85                  90                  95
   Gln Asn Asn Phe Asn Lys Asp Gln Gln Ser Ala Phe Tyr Glu Ile Leu
               100                 105                 110
   Asn Met Pro Asn Leu Asn Glu Ala Gln Arg Asn Gly Phe Ile Gln Ser
           115                 120                 125
   Leu Lys Asp Asp Pro Ser Gln Ser Thr Asn Val Leu Gly Glu Ala Lys
       130                 135                 140
   Lys Leu Asn Glu Ser Gln Ala Pro Lys Ala Asp Asn Asn Phe Asn Lys
   145                 150                 155                 160
   Glu Gln Gln Asn Ala Phe Tyr Glu Ile Leu Asn Met Pro Asn Leu Asn
                   165                 170                 175
   Glu Glu Gln Arg Asn Gly Phe Ile Gln Ser Leu Lys Asp Asp Pro Ser
               180                 185                 190
   Gln Ser Ala Asn Leu Leu Ser Glu Ala Lys Lys Leu Asn Glu Ser Gln
           195                 200                 205
   Ala Pro Lys Ala Asp Asn Lys Phe Asn Lys Glu Gln Gln Asn Ala Phe
       210                 215                 220
   Tyr Glu Ile Leu His Leu Pro Asn Leu Asn Glu Glu Gln Arg Asn Gly
   225                 230                 235                 240
   Phe Ile Gln Ser Leu Lys Asp Asp Pro Ser Val Ser Lys Glu Ile Leu
                   245                 250                 255
   Ala Glu Ala Lys Lys Leu Asn Asp Ala Gln Ala Pro Lys Glu Glu Asp
               260                 265                 270
   Asn Lys Lys Pro Gly Lys Glu Asp Gly Asn Lys Pro Gly Lys Glu Asp
           275                 280                 285
   Gly Asn Lys Pro Gly Lys Glu Asp Asn Lys Lys Pro Gly Lys Glu Asp
       290                 295                 300
   Gly Asn Lys Pro Gly Lys Glu Asp Asn Asn Lys Pro Gly Lys Glu Asp
   305                 310                 315                 320
   Gly Asn Lys Pro Gly Lys Glu Asp Asn Asn Lys Pro Gly Lys Glu Asp
                   325                 330                 335
   Gly Asn Lys Pro Gly Lys Glu Asp Gly Asn Lys Pro Gly Lys Glu Asp
               340                 345                 350
   Gly Asn Gly Val His Val Val Lys Pro Gly Asp Thr Val Asn Asp Ile
           355                 360                 365
   Ala Lys Ala Asn Gly Thr Thr Ala Asp Lys Ile Ala Ala Asp Asn Lys
       370                 375                 380
   Leu Ala Asp Lys Asn Met Ile Lys Pro Gly Gln Glu Leu Val Val Asp
   385                 390                 395                 400
   Lys Lys Gln Pro Ala Asn His Ala Asp Ala Asn Lys Ala Gln Ala Leu
                   405                 410                 415
   Pro Glu Thr Gly Glu Glu Asn Pro Phe Ile Gly Thr Thr Val Phe Gly
               420                 425                 430
   Gly Leu Ser Leu Ala Leu Gly Ala Ala Leu Leu Ala Gly Arg Arg Arg
           435                 440                 445
   Glu Leu
       450
<210> 19
   <211> 2634
   <212> DNA
   <213> Staphylococcus sp.
<220>
   <221> CDS
   <222> (1)..(2634)
<400> 19
   ttg aaa aaa aga att gat tat ttg tcg aat aag cag aat aag tat tcg   48
   Met Lys Lys Arg Ile Asp Tyr Leu Ser Asn Lys Gln Asn Lys Tyr Ser
     1               5                  10                  15
   att aga cgt ttt aca gta ggt acc aca tca gta ata gta ggg gca act   96
   Ile Arg Arg Phe Thr Val Gly Thr Thr Ser Val Ile Val Gly Ala Thr
                20                  25                  30
   ata cta ttt ggg ata ggc aat cat caa gca caa gct tca gaa caa tcg   144
   Ile Leu Phe Gly Ile Gly Asn His Gln Ala Gln Ala Ser Glu Gln Ser
            35                  40                  45
   aac gat aca acg caa tct tcg aaa aat aat gca agt gca gat tcc gaa   192
   Asn Asp Thr Thr Gln Ser Ser Lys Asn Asn Ala Ser Ala Asp Ser Glu
        50                  55                  60
   aaa aac aat atg ata gaa aca cct caa tta aat aca acg gct aat gat    240
   Lys Asn Asn Met Ile Glu Thr Pro Gln Leu Asn Thr Thr Ala Asn Asp
    65                  70                  75                  80
   aca tct gat att agt gca aac aca aac agt gcg aat gta gat agc aca    288
   Thr Ser Asp Ile Ser Ala Asn Thr Asn Ser Ala Asn Val Asp Ser Thr
                    85                  90                  95
   aca aaa cca atg tct aca caa acg agc aat acc act aca aca gag cca    336
   Thr Lys Pro Met Ser Thr Gln Thr Ser Asn Thr Thr Thr Thr Glu Pro
               100                 105                 110
   gct tca aca aat gaa aca cct caa ccg acg gca att aaa aat caa gca    384
   Ala Ser Thr Asn Glu Thr Pro Gln Pro Thr Ala Ile Lys Asn Gln Ala
           115                 120                 125
   act gct gca aaa atg caa gat caa act gtt cct caa gaa gca aat tct    432
   Thr Ala Ala Lys Met Gln Asp Gln Thr Val Pro Gln Glu Ala Asn Ser
       130                 135                 140
   caa gta gat aat aaa aca acg aat gat gct aat agc ata gca aca aac    480
   Gln Val Asp Asn Lys Thr Thr Asn Asp Ala Asn Ser Ile Ala Thr Asn
   145                 150                 155                 160
   agt gag ctt aaa aat tct caa aca tta gat tta cca caa tca tca cca    528
   Ser Glu Leu Lys Asn Ser Gln Thr Leu Asp Leu Pro Gln Ser Ser Pro
                   165                 170                 175
   caa acg att tcc aat gcg caa gga act agt aaa cca agt gtt aga acg    576
   Gln Thr Ile Ser Asn Ala Gln Gly Thr Ser Lys Pro Ser Val Arg Thr
               180                 185                 190
   aga gct gta cgt agt tta gct gtt gct gaa ccg gta gta aat gct gct    624
   Arg Ala Val Arg Ser Leu Ala Val Ala Glu Pro Val Val Asn Ala Ala
           195                 200                 205
   gat gct aaa ggt aca aat gta aat gat aaa gtt acg gca agt aat ttc    672
   Asp Ala Lys Gly Thr Asn Val Asn Asp Lys Val Thr Ala Ser Asn Phe
       210                 215                 220
   aag tta gaa aag act aca ttt gac cct aat caa agt ggt aac aca ttt    720
   Lys Leu Glu Lys Thr Thr Phe Asp Pro Asn Gln Ser Gly Asn Thr Phe
   225                 230                 235                 240
   atg gcg gca aat ttt aca gtg aca gat aaa gtg aaa tca ggg gat tat    768
   Met Ala Ala Asn Phe Thr Val Thr Asp Lys Val Lys Ser Gly Asp Tyr
                   245                 250                 255
   ttt aca gcg aag tta cca gat agt tta act ggt aat gga gac gtg gat    816
   Phe Thr Ala Lys Leu Pro Asp Ser Leu Thr Gly Asn Gly Asp Val Asp
               260                 265                 270
   tat tct aat tca aat aat acg atg cca att gca gac att aaa agt acg    864
   Tyr Ser Asn Ser Asn Asn Thr Met Pro Ile Ala Asp Ile Lys Ser Thr
           275                 280                 285
   aat ggc gat gtt gta gct aaa gca aca tat gat atc ttg act aag acg    912
   Asn Gly Asp Val Val Ala Lys Ala Thr Tyr Asp Ile Leu Thr Lys Thr
       290                 295                 300
   tat aca ttt gtc ttt aca gat tat gta aat aat aaa gaa aat att aac    960
   Tyr Thr Phe Val Phe Thr Asp Tyr Val Asn Asn Lys Glu Asn Ile Asn
   305                 310                 315                 320
   gga caa ttt tca tta cct tta ttt aca gac cga gca aag gca cct aaa    1008
   Gly Gln Phe Ser Leu Pro Leu Phe Thr Asp Arg Ala Lys Ala Pro Lys
                   325                 330                 335
   tca gga aca tat gat gcg aat att aat att gcg gat gaa atg ttt aat    1056
   Ser Gly Thr Tyr Asp Ala Asn Ile Asn Ile Ala Asp Glu Met Phe Asn
               340                 345                 350
   aat aaa att act tat aac tat agt tcg cca att gca gga att gat aaa    1104
   Asn Lys Ile Thr Tyr Asn Tyr Ser Ser Pro Ile Ala Gly Ile Asp Lys
           355                 360                 365
   cca aat ggc gcg aac att tct tct caa att att ggt gta gat aca gct    1152
   Pro Asn Gly Ala Asn Ile Ser Ser Gln Ile Ile Gly Val Asp Thr Ala
       370                 375                 380
   tca ggt caa aac aca tac aag caa aca gta ttt gtt aac cct aag caa    1200
   Ser Gly Gln Asn Thr Tyr Lys Gln Thr Val Phe Val Asn Pro Lys Gln
   385                 390                 395                 400
   cga gtt tta ggt aat acg tgg gtg tat att aaa ggc tac caa gat aaa    1248
   Arg Val Leu Gly Asn Thr Trp Val Tyr Ile Lys Gly Tyr Gln Asp Lys
                   405                 410                 415
   atc gaa gaa agt agc ggt aaa gta agt gct aca gat aca aaa ctg aga    1296
   Ile Glu Glu Ser Ser Gly Lys Val Ser Ala Thr Asp Thr Lys Leu Arg
               420                 425                 430
   att ttt gaa gtg aat gat aca tct aaa tta tca gat agc tac tat gca    1344
   Ile Phe Glu Val Asn Asp Thr Ser Lys Leu Ser Asp Ser Tyr Tyr Ala
           435                 440                 445
   gat cca aat gac tct aac ctt aaa gaa gta aca gac caa ttt aaa aat    1392
   Asp Pro Asn Asp Ser Asn Leu Lys Glu Val Thr Asp Gln Phe Lys Asn
       450                 455                 460
   aga atc tat tat gag cat cca aat gta gct agt att aaa ttt ggt gat    1440
   Arg Ile Tyr Tyr Glu His Pro Asn Val Ala Ser Ile Lys Phe Gly Asp
   465                 470                 475                 480
   att act aaa aca tat gta gta tta gta gaa ggg cat tac gac aat aca    1488
   Ile Thr Lys Thr Tyr Val Val Leu Val Glu Gly His Tyr Asp Asn Thr
                   485                 490                 495
   ggt aag aac tta aaa act cag gtt att caa gaa aat gtt gat cct gta    1536
   Gly Lys Asn Leu Lys Thr Gln Val Ile Gln Glu Asn Val Asp Pro Val
               500                 505                 510
   aca aat aga gac tac agt att ttc ggt tgg aat aat gag aat gtt gta    1584
   Thr Asn Arg Asp Tyr Ser Ile Phe Gly Trp Asn Asn Glu Asn Val Val
           515                 520                 525
   cgt tat ggt ggt gga agt gct gat ggt gat tca gca gta aat ccg aaa    1632
   Arg Tyr Gly Gly Gly Ser Ala Asp Gly Asp Ser Ala Val Asn Pro Lys
       530                 535                 540
   gac cca act cca ggg ccg ccg gtt gac cca gaa cca agt cca gac cca    1680
   Asp Pro Thr Pro Gly Pro Pro Val Asp Pro Glu Pro Ser Pro Asp Pro
   545                 550                 555                 560
   gaa cca gaa cca acg cca gat cca gaa cca agt cca gac cca gaa ccg    1728
   Glu Pro Glu Pro Thr Pro Asp Pro Glu Pro Ser Pro Asp Pro Glu Pro
                   565                 570                 575
   gaa cca agc cca gac ccg gat ccg gat tcg gat tca gac agt gac tca    1776
   Glu Pro Ser Pro Asp Pro Asp Pro Asp Ser Asp Ser Asp Ser Asp Ser
               580                 585                 590
   ggc tca gac agc gac tca ggt tca gat agc gac tca gaa tca gat agc    1824
   Gly Ser Asp Ser Asp Ser Gly Ser Asp Ser Asp Ser Glu Ser Asp Ser
           595                 600                 605
   gat tcg gat tca gac agt gat tca gat tca gac agc gac tca gaa tca    1872
   Asp Ser Asp Ser Asp Ser Asp Ser Asp Ser Asp Ser Asp Ser Glu Ser
       610                 615                 620
   gat agc gat tca gaa tca gat agc gac tca gat tca gat agc gat tca    1920
   Asp Ser Asp Ser Glu Ser Asp Ser Asp Ser Asp Ser Asp Ser Asp Ser
   625                 630                 635                 640
   gat tca gat agc gat tca gaa tca gat agc gat tcg gat tca gac agt    1968
   Asp Ser Asp Ser Asp Ser Glu Ser Asp Ser Asp Ser Asp Ser Asp Ser
                   645                 650                 655
   gat tca gat tca gac agc gac tca gaa tca gat agc gac tca gaa tca    2016
   Asp Ser Asp Ser Asp Ser Asp Ser Glu Ser Asp Ser Asp Ser Glu Ser
               660                 665                 670
   gat agt gag tca gat tca gac agt gac tcg gac tca gac agt gat tca    2064
   Asp Ser Glu Ser Asp Ser Asp Ser Asp Ser Asp Ser Asp Ser Asp Ser
           675                 680                 685
   gac tca gat agc gat tca gac tca gat agc gat tca gac tca gac agc    2112
   Asp Ser Asp Ser Asp Ser Asp Ser Asp Ser Asp Ser Asp Ser Asp Ser
       690                 695                 700
   gat tca gat tca gac agc gac tca gaa tca gac agc gac tca gac tca    2160
   Asp Ser Asp Ser Asp Ser Asp Ser Glu Ser Asp Ser Asp Ser Asp Ser
   705                 710                 715                 720
   gat agc gac tca gac tca gac agc gac tca gat tca gat agc gat tca    2208
   Asp Ser Asp Ser Asp Ser Asp Ser Asp Ser Asp Ser Asp Ser Asp Ser
                   725                 730                 735
   gac tca gac agc gac tca gac tca gac agc gac tca gac tca gat agc    2256
   Asp Ser Asp Ser Asp Ser Asp Ser Asp Ser Asp Ser Asp Ser Asp Ser
               740                 745                 750
   gat tca gac tca gac agc gac tca gat tca gat agc gat tcg gac tca    2304
   Asp Ser Asp Ser Asp Ser Asp Ser Asp Ser Asp Ser Asp Ser Asp Ser
           755                 760                 765
   gac agc gat tca gat tca gac agc gac tca gac tcg gat agc gat tca    2352
   Asp Ser Asp Ser Asp Ser Asp Ser Asp Ser Asp Ser Asp Ser Asp Ser
       770                 775                 780
   gat tca gac agc gac tca gac tcg gat agc gac tcg gat tca gat agt    2400
   Asp Ser Asp Ser Asp Ser Asp Ser Asp Ser Asp Ser Asp Ser Asp Ser
   785                 790                 795                 800
   gac tcc gat tca aga gtt aca cca cca aat aat gaa cag aaa gca cca    2448
   Asp Ser Asp Ser Arg Val Thr Pro Pro Asn Asn Glu Gln Lys Ala Pro
                   805                 810                 815
   tca aat cct aaa ggt gaa gta aac cat tct aat aag gta tca aaa caa   2496
   Ser Asn Pro Lys Gly Glu Val Asn His Ser Asn Lys Val Ser Lys Gln
               820                 825                 830
   cac aaa act gat gct tta cca gaa aca gga gat aag agc gaa aac aca   2544
   His Lys Thr Asp Ala Leu Pro Glu Thr Gly Asp Lys Ser Glu Asn Thr
           835                 840                 845
   aat gca act tta ttt ggt gca atg atg gca tta tta gga tca tta cta   2592
   Asn Ala Thr Leu Phe Gly Ala Met Met Ala Leu Leu Gly Ser Leu Leu
       850                 855                 860
   ttg ttt aga aaa cgc aag caa gat cat aaa gaa aaa gcg taa           2634
   Leu Phe Arg Lys Arg Lys Gln Asp His Lys Glu Lys Ala
   865                 870                 875
<210> 20
   <211> 877
   <212> PRT
   <213> Staphylococcus sp.
<400> 20
   Met Lys Lys Arg Ile Asp Tyr Leu Ser Asn Lys Gln Asn Lys Tyr Ser
   1 5 10 15
   Ile Arg Arg Phe Thr Val Gly Thr Thr Ser Val Ile Val Gly Ala Thr
   20 25 30
   Ile Leu Phe Gly Ile Gly Asn His Gln Ala Gln Ala Ser Glu Gln Ser
   35 40 45
   Asn Asp Thr Thr Gln Ser Ser Lys Asn Asn Ala Ser Ala Asp Ser Glu
   50 55 60
   Lys Asn Asn Met Ile Glu Thr Pro Gln Leu Asn Thr Thr Ala Asn Asp
   65 70 75 80
   Thr Ser Asp Ile Ser Ala Asn Thr Asn Ser Ala Asn Val Asp Ser Thr
   85 90 95
   Thr Lys Pro Met Ser Thr Gln Thr Ser Asn Thr Thr Thr Thr Glu Pro
   100 105 110
   Ala Ser Thr Asn Glu Thr Pro Gln Pro Thr Ala Ile Lys Asn Gln Ala
   115 120 125
   Thr Ala Ala Lys Met Gln Asp Gln Thr Val Pro Gln Glu Ala Asn Ser
   130 135 140
   Gln Val Asp Asn Lys Thr Thr Asn Asp Ala Asn Ser Ile Ala Thr Asn
   145 150 155 160
   Ser Glu Leu Lys Asn Ser Gln Thr Leu Asp Leu Pro Gln Ser Ser Pro
   165 170 175
   Gln Thr Ile Ser Asn Ala Gln Gly Thr Ser Lys Pro Ser Val Arg Thr
   180 185 190
   Arg Ala Val Arg Ser Leu Ala Val Ala Glu Pro Val Val Asn Ala Ala
   195 200 205
   Asp Ala Lys Gly Thr Asn Val Asn Asp Lys Val Thr Ala Ser Asn Phe
   210 215 220
   Lys Leu Glu Lys Thr Thr Phe Asp Pro Asn Gln Ser Gly Asn Thr Phe
   225 230 235 240
   Met Ala Ala Asn Phe Thr Val Thr Asp Lys Val Lys Ser Gly Asp Tyr
   245 250 255
   Phe Thr Ala Lys Leu Pro Asp Ser Leu Thr Gly Asn Gly Asp Val Asp
   260 265 270
   Tyr Ser Asn Ser Asn Asn Thr Met Pro Ile Ala Asp Ile Lys Ser Thr
   275 280 285
   Asn Gly Asp Val Val Ala Lys Ala Thr Tyr Asp Ile Leu Thr Lys Thr
   290 295 300
   Tyr Thr Phe Val Phe Thr Asp Tyr Val Asn Asn Lys Glu Asn Ile Asn
   305                 310                 315                 320
   Gly Gln Phe Ser Leu Pro Leu Phe Thr Asp Arg Ala Lys Ala Pro Lys
                   325                 330                 335
   Ser Gly Thr Tyr Asp Ala Asn Ile Asn Ile Ala Asp Glu Met Phe Asn
               340                 345                 350
   Asn Lys Ile Thr Tyr Asn Tyr Ser Ser Pro Ile Ala Gly Ile Asp Lys
           355                 360                 365
   Pro Asn Gly Ala Asn Ile Ser Ser Gln Ile Ile Gly Val Asp Thr Ala
       370                 375                 380
   Ser Gly Gln Asn Thr Tyr Lys Gln Thr Val Phe Val Asn Pro Lys Gln
   385                 390                 395                 400
   Arg Val Leu Gly Asn Thr Trp Val Tyr Ile Lys Gly Tyr Gln Asp Lys
                   405                 410                 415
   Ile Glu Glu Ser Ser Gly Lys Val Ser Ala Thr Asp Thr Lys Leu Arg
               420                 425                 430
   Ile Phe Glu Val Asn Asp Thr Ser Lys Leu Ser Asp Ser Tyr Tyr Ala
           435                 440                 445
   Asp Pro Asn Asp Ser Asn Leu Lys Glu Val Thr Asp Gln Phe Lys Asn
       450                 455                 460
   Arg Ile Tyr Tyr Glu His Pro Asn Val Ala Ser Ile Lys Phe Gly Asp
   465                 470                 475                 480
   Ile Thr Lys Thr Tyr Val Val Leu Val Glu Gly His Tyr Asp Asn Thr
                   485                 490                 495
   Gly Lys Asn Leu Lys Thr Gln Val Ile Gln Glu Asn Val Asp Pro Val
               500                 505                 510
   Thr Asn Arg Asp Tyr Ser Ile Phe Gly Trp Asn Asn Glu Asn Val Val
           515                 520                 525
   Arg Tyr Gly Gly Gly Ser Ala Asp Gly Asp Ser Ala Val Asn Pro Lys
       530                 535                 540
   Asp Pro Thr Pro Gly Pro Pro Val Asp Pro Glu Pro Ser Pro Asp Pro
   545                 550                 555                 560
   Glu Pro Glu Pro Thr Pro Asp Pro Glu Pro Ser Pro Asp Pro Glu Pro
                   565                 570                 575
   Glu Pro Ser Pro Asp Pro Asp Pro Asp Ser Asp Ser Asp Ser Asp Ser
               580                 585                 590
   Gly Ser Asp Ser Asp Ser Gly Ser Asp Ser Asp Ser Glu Ser Asp Ser
           595                 600                 605
   Asp Ser Asp Ser Asp Ser Asp Ser Asp Ser Asp Ser Asp Ser Glu Ser
       610                 615                 620
   Asp Ser Asp Ser Glu Ser Asp Ser Asp Ser Asp Ser Asp Ser Asp Ser
   625                 630                 635                 640
   Asp Ser Asp Ser Asp Ser Glu Ser Asp Ser Asp Ser Asp Ser Asp Ser
                   645                 650                 655
   Asp Ser Asp Ser Asp Ser Asp Ser Glu Ser Asp Ser Asp Ser Glu Ser
               660                 665                 670
   Asp Ser Glu Ser Asp Ser Asp Ser Asp Ser Asp Ser Asp Ser Asp Ser
           675                 680                 685
   Asp Ser Asp Ser Asp Ser Asp Ser Asp Ser Asp Ser Asp Ser Asp Ser
       690                 695                 700
   Asp Ser Asp Ser Asp Ser Asp Ser Glu Ser Asp Ser Asp Ser Asp Ser
   705                 710                 715                 720
   Asp Ser Asp Ser Asp Ser Asp Ser Asp Ser Asp Ser Asp Ser Asp Ser
                   725                 730                 735
   Asp Ser Asp Ser Asp Ser Asp Ser Asp Ser Asp Ser Asp Ser Asp Ser
               740                 745                 750
   Asp Ser Asp Ser Asp Ser Asp Ser Asp Ser Asp Ser Asp Ser Asp Ser
           755                 760                 765
   Asp Ser Asp Ser Asp Ser Asp Ser Asp Ser Asp Ser Asp Ser Asp Ser
       770                 775                 780
   Asp Ser Asp Ser Asp Ser Asp Ser Asp Ser Asp Ser Asp Ser Asp Ser
   785                 790                 795                 800
   Asp Ser Asp Ser Arg Val Thr Pro Pro Asn Asn Glu Gln Lys Ala Pro
                   805                 810                 815
   Ser Asn Pro Lys Gly Glu Val Asn His Ser Asn Lys Val Ser Lys Gln
               820                 825                 830
   His Lys Thr Asp Ala Leu Pro Glu Thr Gly Asp Lys Ser Glu Asn Thr
           835                 840                 845
   Asn Ala Thr Leu Phe Gly Ala Met Met Ala Leu Leu Gly Ser Leu Leu
       850                 855                 860
   Leu Phe Arg Lys Arg Lys Gln Asp His Lys Glu Lys Ala
   865                 870                 875
<210> 21
   <211> 684
   <212> DNA
   <213> Staphylococcus sp.
<220>
   <221> CDS
   <222> (1)..(684)
<400> 21
   ttg aaa aat att tta aaa gtt ttt aat aca acg att tta gcg tta att      48
   Met Lys Asn Ile Leu Lys Val Phe Asn Thr Thr Ile Leu Ala Leu Ile
     1               5                  10                  15
   atc atc atc gcg aca ttc agt aat tct gca aat gcc gca gat agc ggt      96
   Ile Ile Ile Ala Thr Phe Ser Asn Ser Ala Asn Ala Ala Asp Ser Gly
                20                  25                  30
   act ttg aat tat gag gtt tac aaa tac aat acc aat gac acg tca att     144
   Thr Leu Asn Tyr Glu Val Tyr Lys Tyr Asn Thr Asn Asp Thr Ser Ile
            35                  40                  45
   gct aat gac tat ttt aat aaa ccg gca aag tac att aag aaa aat ggt     192
   Ala Asn Asp Tyr Phe Asn Lys Pro Ala Lys Tyr Ile Lys Lys Asn Gly
        50                  55                  60
   aaa ttg tat gtt caa ata act gtc aac cac agt cat tgg att act gga     240
   Lys Leu Tyr Val Gln Ile Thr Val Asn His Ser His Trp Ile Thr Gly
    65                  70                  75                  80
   atg agt atc gaa gga cat aaa gaa aat att att agt aaa aac act gcc     288
   Met Ser Ile Glu Gly His Lys Glu Asn Ile Ile Ser Lys Asn Thr Ala
                    85                  90                  95
   aaa gat gaa cgc act tct gaa ttt gaa gta agt aag ttg aac ggt aaa     336
   Lys Asp Glu Arg Thr Ser Glu Phe Glu Val Ser Lys Leu Asn Gly Lys
               100                 105                 110
   ata gat gga aaa att gac gtt tat atc gat gaa aaa gta aat gga aag     384
   Ile Asp Gly Lys Ile Asp Val Tyr Ile Asp Glu Lys Val Asn Gly Lys
           115                 120                 125
   cca ttc aaa tat gac cat cat tac aac att aca tat aaa ttt aat gga     432
   Pro Phe Lys Tyr Asp His His Tyr Asn Ile Thr Tyr Lys Phe Asn Gly
       130                 135                 140
   cca act gat gta gca ggt gct aat gca cca ggt aaa gat gat aaa aat     480
   Pro Thr Asp Val Ala Gly Ala Asn Ala Pro Gly Lys Asp Asp Lys Asn
   145                 150                 155                 160
   tct gct tca ggt agt gac aaa gga tct gat gga acg act act ggt caa     528
   Ser Ala Ser Gly Ser Asp Lys Gly Ser Asp Gly Thr Thr Thr Gly Gln
                   165                 170                 175
   agt gaa tct aat agt tcg aat aaa gac aaa gta gaa aat cca caa aca     576
   Ser Glu Ser Asn Ser Ser Asn Lys Asp Lys Val Glu Asn Pro Gln Thr
               180                 185                 190
   aat gct ggt aca cct gca tat ata tat gca ata cca gtt gca tcc tta     624
   Asn Ala Gly Thr Pro Ala Tyr Ile Tyr Ala Ile Pro Val Ala Ser Leu
           195                 200                 205
   gca tta tta atc gca atc aca ttg ttt gtt aga aaa aaa tct aaa ggc     672
   Ala Leu Leu Ile Ala Ile Thr Leu Phe Val Arg Lys Lys Ser Lys Gly
       210                 215                 220
   aat gtg gaa taa                                                     684
   Asn Val Glu
   225
<210> 22
   <211> 227
   <212> PRT
   <213> Staphylococcus sp.
<400> 22
   Met Lys Asn Ile Leu Lys Val Phe Asn Thr Thr Ile Leu Ala Leu Ile
    1               5                  10                  15
   Ile Ile Ile Ala Thr Phe Ser Asn Ser Ala Asn Ala Ala Asp Ser Gly
               20                  25                  30
   Thr Leu Asn Tyr Glu Val Tyr Lys Tyr Asn Thr Asn Asp Thr Ser Ile
           35                  40                  45
   Ala Asn Asp Tyr Phe Asn Lys Pro Ala Lys Tyr Ile Lys Lys Asn Gly
       50                  55                  60
   Lys Leu Tyr Val Gln Ile Thr Val Asn His Ser His Trp Ile Thr Gly
   65                  70                  75                  80
   Met Ser Ile Glu Gly His Lys Glu Asn Ile Ile Ser Lys Asn Thr Ala
                   85                  90                  95
   Lys Asp Glu Arg Thr Ser Glu Phe Glu Val Ser Lys Leu Asn Gly Lys
               100                 105                 110
   Ile Asp Gly Lys Ile Asp Val Tyr Ile Asp Glu Lys Val Asn Gly Lys
           115                 120                 125
   Pro Phe Lys Tyr Asp His His Tyr Asn Ile Thr Tyr Lys Phe Asn Gly
       130                 135                 140
   Pro Thr Asp Val Ala Gly Ala Asn Ala Pro Gly Lys Asp Asp Lys Asn
   145                 150                 155                 160
   Ser Ala Ser Gly Ser Asp Lys Gly Ser Asp Gly Thr Thr Thr Gly Gln
                   165                 170                 175
   Ser Glu Ser Asn Ser Ser Asn Lys Asp Lys Val Glu Asn Pro Gln Thr
               180                 185                 190
   Asn Ala Gly Thr Pro Ala Tyr Ile Tyr Ala Ile Pro Val Ala Ser Leu
           195                 200                 205
   Ala Leu Leu Ile Ala Ile Thr Leu Phe Val Arg Lys Lys Ser Lys Gly
       210                 215                 220
   Asn Val Glu
   225
<210> 23
   <211> 1908
   <212> DNA
   <213> Staphylococcus sp.
<220>
   <221> CDS
   <222> (1)..(1908)
<400> 23
   atg gct aaa tat cga ggg aaa ccg ttt caa tta tat gta aag tta tcg    48
   Met Ala Lys Tyr Arg Gly Lys Pro Phe Gln Leu Tyr Val Lys Leu Ser
    1               5                  10                  15
   tgt tcg aca atg atg gcg tca agt atc att tta acg aat atc ttg ccg    96
   Cys Ser Thr Met Met Ala Ser Ser Ile Ile Leu Thr Asn Ile Leu Pro
               20                  25                  30
   tac gat gcc caa gct gca tct gaa aag gat act gaa att tca aaa gag   144
   Tyr Asp Ala Gln Ala Ala Ser Glu Lys Asp Thr Glu Ile Ser Lys Glu
           35                  40                  45
   ata tta tct aag caa gat tta tta gac aaa gtt gac aaa gca att cgt   192
   Ile Leu Ser Lys Gln Asp Leu Leu Asp Lys Val Asp Lys Ala Ile Arg
       50                  55                  60
   caa att gag caa tta aaa cag tta tcg gct tca tct aaa gca cat tat   240
   Gln Ile Glu Gln Leu Lys Gln Leu Ser Ala Ser Ser Lys Ala His Tyr
   65                  70                  75                  80
   aaa gca caa cta aat gaa gcg aaa aca gca tcg caa ata gat gaa atc   288
   Lys Ala Gln Leu Asn Glu Ala Lys Thr Ala Ser Gln Ile Asp Glu Ile
                   85                  90                  95
   ata aaa cga gct aat gag ttg gat agc aaa gaa aat aaa agt tct cac   336
   Ile Lys Arg Ala Asn Glu Leu Asp Ser Lys Glu Asn Lys Ser Ser His
               100                 105                 110
   act gaa atg aac ggt caa agt gat ata gac agt aaa tta gat caa ttg   384
   Thr Glu Met Asn Gly Gln Ser Asp Ile Asp Ser Lys Leu Asp Gln Leu
           115                 120                 125
   ctt aaa gat tta aat gag gtt tct tca aat gtt gat agg ggt caa caa   432
   Leu Lys Asp Leu Asn Glu Val Ser Ser Asn Val Asp Arg Gly Gln Gln
       130                 135                 140
   agt ggc gag gac gat ctt aat gca atg aaa aat gat atg tca caa acg   480
   Ser Gly Glu Asp Asp Leu Asn Ala Met Lys Asn Asp Met Ser Gln Thr
   145                 150                 155                 160
   gct aca aca aaa tat gga gaa aaa gat gat aaa aat gat gaa gca atg   528
   Ala Thr Thr Lys Tyr Gly Glu Lys Asp Asp Lys Asn Asp Glu Ala Met
                   165                 170                 175
   gta aat aag gcg tta gaa gac cta gac cat ttg aat cag caa ata cac   576
   Val Asn Lys Ala Leu Glu Asp Leu Asp His Leu Asn Gln Gln Ile His
               180                 185                 190
   aaa tcg aaa gat gca ttg aaa gat gca tcg aaa gat ccg gca gtg tct   624
   Lys Ser Lys Asp Ala Leu Lys Asp Ala Ser Lys Asp Pro Ala Val Ser
           195                 200                 205
   aca aca gat agt aat cat gaa gta gct aaa acg cca aat aat gat ggt   672
   Thr Thr Asp Ser Asn His Glu Val Ala Lys Thr Pro Asn Asn Asp Gly
       210                 215                 220
   tct gga cat gtt gtg tta aat aaa ttt ctt tca aat gaa gag aat caa   720
   Ser Gly His Val Val Leu Asn Lys Phe Leu Ser Asn Glu Glu Asn Gln
   225                 230                 235                 240
   agc cat agt aat caa ctc act gat aaa tta caa gga agc gat aaa att      768
   Ser His Ser Asn Gln Leu Thr Asp Lys Leu Gln Gly Ser Asp Lys Ile
                   245                 250                 255
   aat cat gct atg att gaa aaa ttg gct aaa agt aat gcc tca acg caa      816
   Asn His Ala Met Ile Glu Lys Leu Ala Lys Ser Asn Ala Ser Thr Gln
               260                 265                 270
   cat tac aca tat cat aaa ctg aat acg tta caa tct tta gat caa cgt      864
   His Tyr Thr Tyr His Lys Leu Asn Thr Leu Gln Ser Leu Asp Gln Arg
           275                 280                 285
   att gca aat acg caa ctt cct aaa aat caa aaa tca gac tta atg agc      912
   Ile Ala Asn Thr Gln Leu Pro Lys Asn Gln Lys Ser Asp Leu Met Ser
       290                 295                 300
   gaa gta aat aag acg aaa gag cgt ata aaa agt caa cga aat att att      960
   Glu Val Asn Lys Thr Lys Glu Arg Ile Lys Ser Gln Arg Asn Ile Ile
   305                 310                 315                 320
   ttg gaa gaa ctt gca cgt act gat gat aaa aag tat gct aca caa agc      1008
   Leu Glu Glu Leu Ala Arg Thr Asp Asp Lys Lys Tyr Ala Thr Gln Ser
                   325                 330                 335
   att tta gaa agt ata ttt aat aaa gac gag gca gat aaa att cta aaa      1056
   Ile Leu Glu Ser Ile Phe Asn Lys Asp Glu Ala Asp Lys Ile Leu Lys
               340                 345                 350
   gat ata cgt gtt gat ggt aaa aca gat caa caa att gca gat caa att      1104
   Asp Ile Arg Val Asp Gly Lys Thr Asp Gln Gln Ile Ala Asp Gln Ile
           355                 360                 365
   act cgt cat att gat caa cta tct ctg aca acg agt gat gat tta tta      1152
   Thr Arg His Ile Asp Gln Leu Ser Leu Thr Thr Ser Asp Asp Leu Leu
       370                 375                 380
   acg tca ttg att gat caa tca caa gat aag tcg cta ttg att tct caa      1200
   Thr Ser Leu Ile Asp Gln Ser Gln Asp Lys Ser Leu Leu Ile Ser Gln
   385                 390                 395                 400
   atc tta caa acg aaa tta gga aaa gct gaa gca gat aaa ttg gct aaa      1248
   Ile Leu Gln Thr Lys Leu Gly Lys Ala Glu Ala Asp Lys Leu Ala Lys
                   405                 410                 415
   gat tgg acg aat aaa gga tta tca aat cgc caa atc gtt gac caa ttg      1296
   Asp Trp Thr Asn Lys Gly Leu Ser Asn Arg Gln Ile Val Asp Gln Leu
               420                 425                 430
   aag aaa cat ttt gca tca act ggc gac acg tct tca gat gat ata tta      1344
   Lys Lys His Phe Ala Ser Thr Gly Asp Thr Ser Ser Asp Asp Ile Leu
           435                 440                 445
   aaa gca att ttg aat aat gcc aaa gat aaa aag caa gca att gaa acg      1392
   Lys Ala Ile Leu Asn Asn Ala Lys Asp Lys Lys Gln Ala Ile Glu Thr
       450                 455                 460
   att tta gca aca cgt ata gaa aga caa aag gca aaa tta ctg gca gat      1440
   Ile Leu Ala Thr Arg Ile Glu Arg Gln Lys Ala Lys Leu Leu Ala Asp
   465                 470                 475                 480
   tta att act aaa ata gaa aca gat caa aat aaa att ttt aat tta gtt      1488
   Leu Ile Thr Lys Ile Glu Thr Asp Gln Asn Lys Ile Phe Asn Leu Val
                   485                 490                 495
   aaa tcg gca ttg aat ggt aaa gcg gat gat tta ttg aat tta caa aag    1536
   Lys Ser Ala Leu Asn Gly Lys Ala Asp Asp Leu Leu Asn Leu Gln Lys
               500                 505                 510
   aga ctc aat caa acg aaa aaa gat ata gac tat att tta tca cca ata    1584
   Arg Leu Asn Gln Thr Lys Lys Asp Ile Asp Tyr Ile Leu Ser Pro Ile
           515                 520                 525
   gta aat cgt cca agt tta cta gat cga ttg aat aaa aat ggg aaa aca    1632
   Val Asn Arg Pro Ser Leu Leu Asp Arg Leu Asn Lys Asn Gly Lys Thr
       530                 535                 540
   acg gat tta aat aag tta gca aat tta atg aat caa gga tca aat tta    1680
   Thr Asp Leu Asn Lys Leu Ala Asn Leu Met Asn Gln Gly Ser Asn Leu
   545                 550                 555                 560
   tta gac agt att cca gat ata ccc aca cca aag cca gaa aag acg tta    1728
   Leu Asp Ser Ile Pro Asp Ile Pro Thr Pro Lys Pro Glu Lys Thr Leu
                   565                 570                 575
   aca ctt ggt aaa ggt aat gga ttg tta agt gga tta tta aat gct gat    1776
   Thr Leu Gly Lys Gly Asn Gly Leu Leu Ser Gly Leu Leu Asn Ala Asp
               580                 585                 590
   ggt aat gta tct ttg cct aaa gcg ggg gaa acg ata aaa gaa cat tgg    1824
   Gly Asn Val Ser Leu Pro Lys Ala Gly Glu Thr Ile Lys Glu His Trp
           595                 600                 605
   ttg ccg ata tct gta att gtt ggt gca atg ggt gta cta atg att tgg    1872
   Leu Pro Ile Ser Val Ile Val Gly Ala Met Gly Val Leu Met Ile Trp
       610                 615                 620
   tta tca cga cgc aat aag ttg aaa aat aaa gca taa                    1908
   Leu Ser Arg Arg Asn Lys Leu Lys Asn Lys Ala
   625                 630                 635
<210> 24
   <211> 635
   <212> PRT
   <213> Staphylococcus sp.
<400> 24
   Met Ala Lys Tyr Arg Gly Lys Pro Phe Gln Leu Tyr Val Lys Leu Ser
   1               5                   10                  15
   Cys Ser Thr Met Met Ala Ser Ser Ile Ile Leu Thr Asn Ile Leu Pro
               20                  25                  30
   Tyr Asp Ala Gln Ala Ala Ser Glu Lys Asp Thr Glu Ile Ser Lys Glu
           35                  40                  45
   Ile Leu Ser Lys Gln Asp Leu Leu Asp Lys Val Asp Lys Ala Ile Arg
       50                  55                  60
   Gln Ile Glu Gln Leu Lys Gln Leu Ser Ala Ser Ser Lys Ala His Tyr
   65                  70                  75                  80
   Lys Ala Gln Leu Asn Glu Ala Lys Thr Ala Ser Gln Ile Asp Glu Ile
                   85                  90                  95
   Ile Lys Arg Ala Asn Glu Leu Asp Ser Lys Glu Asn Lys Ser Ser His
               100                 105                 110
   Thr Glu Met Asn Gly Gln Ser Asp Ile Asp Ser Lys Leu Asp Gln Leu
           115                 120                 125
   Leu Lys Asp Leu Asn Glu Val Ser Ser Asn Val Asp Arg Gly Gln Gln
       130                 135                 140
   Ser Gly Glu Asp Asp Leu Asn Ala Met Lys Asn Asp Met Ser Gln Thr
   145                 150                 155                 160
   Ala Thr Thr Lys Tyr Gly Glu Lys Asp Asp Lys Asn Asp Glu Ala Met
                   165                 170                 175
   Val Asn Lys Ala Leu Glu Asp Leu Asp His Leu Asn Gln Gln Ile His
               180                 185                 190
   Lys Ser Lys Asp Ala Leu Lys Asp Ala Ser Lys Asp Pro Ala Val Ser
           195                 200                 205
   Thr Thr Asp Ser Asn His Glu Val Ala Lys Thr Pro Asn Asn Asp Gly
       210                 215                 220
   Ser Gly His Val Val Leu Asn Lys Phe Leu Ser Asn Glu Glu Asn Gln
   225                 230                 235                 240
   Ser His Ser Asn Gln Leu Thr Asp Lys Leu Gln Gly Ser Asp Lys Ile
                   245                 250                 255
   Asn His Ala Met Ile Glu Lys Leu Ala Lys Ser Asn Ala Ser Thr Gln
               260                 265                 270
   His Tyr Thr Tyr His Lys Leu Asn Thr Leu Gln Ser Leu Asp Gln Arg
           275                 280                 285
   Ile Ala Asn Thr Gln Leu Pro Lys Asn Gln Lys Ser Asp Leu Met Ser
       290                 295                 300
   Glu Val Asn Lys Thr Lys Glu Arg Ile Lys Ser Gln Arg Asn Ile Ile
   305                 310                 315                 320
   Leu Glu Glu Leu Ala Arg Thr Asp Asp Lys Lys Tyr Ala Thr Gln Ser
                   325                 330                 335
   Ile Leu Glu Ser Ile Phe Asn Lys Asp Glu Ala Asp Lys Ile Leu Lys
               340                 345                 350
   Asp Ile Arg Val Asp Gly Lys Thr Asp Gln Gln Ile Ala Asp Gln Ile
           355                 360                 365
   Thr Arg His Ile Asp Gln Leu Ser Leu Thr Thr Ser Asp Asp Leu Leu
       370                 375                 380
   Thr Ser Leu Ile Asp Gln Ser Gln Asp Lys Ser Leu Leu Ile Ser Gln
   385                 390                 395                 400
   Ile Leu Gln Thr Lys Leu Gly Lys Ala Glu Ala Asp Lys Leu Ala Lys
                   405                 410                 415
   Asp Trp Thr Asn Lys Gly Leu Ser Asn Arg Gln Ile Val Asp Gln Leu
               420                 425                 430
   Lys Lys His Phe Ala Ser Thr Gly Asp Thr Ser Ser Asp Asp Ile Leu
           435                 440                 445
   Lys Ala Ile Leu Asn Asn Ala Lys Asp Lys Lys Gln Ala Ile Glu Thr
       450                 455                 460
   Ile Leu Ala Thr Arg Ile Glu Arg Gln Lys Ala Lys Leu Leu Ala Asp
   465                 470                 475                 480
   Leu Ile Thr Lys Ile Glu Thr Asp Gln Asn Lys Ile Phe Asn Leu Val
                   485                 490                 495
   Lys Ser Ala Leu Asn Gly Lys Ala Asp Asp Leu Leu Asn Leu Gln Lys
               500                 505                 510
   Arg Leu Asn Gln Thr Lys Lys Asp Ile Asp Tyr Ile Leu Ser Pro Ile
           515                 520                 525
   Val Asn Arg Pro Ser Leu Leu Asp Arg Leu Asn Lys Asn Gly Lys Thr
       530                 535                 540
   Thr Asp Leu Asn Lys Leu Ala Asn Leu Met Asn Gln Gly Ser Asn Leu
   545                 550                 555                 560
   Leu Asp Ser Ile Pro Asp Ile Pro Thr Pro Lys Pro Glu Lys Thr Leu
                   565                 570                 575
   Thr Leu Gly Lys Gly Asn Gly Leu Leu Ser Gly Leu Leu Asn Ala Asp
               580                 585                 590
   Gly Asn Val Ser Leu Pro Lys Ala Gly Glu Thr Ile Lys Glu His Trp
           595                 600                 605
   Leu Pro Ile Ser Val Ile Val Gly Ala Met Gly Val Leu Met Ile Trp
       610                 615                 620
   Leu Ser Arg Arg Asn Lys Leu Lys Asn Lys Ala
   625                 630                 635
<210> 25
   <211> 2862
   <212> DNA
   <213> Staphylococcus sp.
<220>
   <221> CDS
   <222> (1)..(2862)
<400> 25
   atg aat aat aaa aag aca gca aca aat aga aaa ggc atg ata cca aat     48
   Met Asn Asn Lys Lys Thr Ala Thr Asn Arg Lys Gly Met Ile Pro Asn
     1               5                  10                  15
   cga tta aac aaa ttt tcg ata aga aag tat tct gta ggt act gct tca     96
   Arg Leu Asn Lys Phe Ser Ile Arg Lys Tyr Ser Val Gly Thr Ala Ser
                20                  25                  30
   att tta gta ggg aca aca ttg att ttt ggg tta agt ggt cat gaa gct    144
   Ile Leu Val Gly Thr Thr Leu Ile Phe Gly Leu Ser Gly His Glu Ala
            35                  40                  45
   aaa gcg gca gaa cat acg aat gga gaa tta aat caa tca aaa aat gaa    192
   Lys Ala Ala Glu His Thr Asn Gly Glu Leu Asn Gln Ser Lys Asn Glu
        50                  55                  60
   acg aca gcc cca agt gag aat aaa aca act gaa aaa gtt gat agt cgt    240
   Thr Thr Ala Pro Ser Glu Asn Lys Thr Thr Glu Lys Val Asp Ser Arg
    65                  70                  75                  80
   caa cta aaa gac aat acg caa act gca act gca gat cag cct aaa gtg    288
   Gln Leu Lys Asp Asn Thr Gln Thr Ala Thr Ala Asp Gln Pro Lys Val
                    85                  90                  95
   aca atg agt gat agt gca aca gtt aaa gaa act agt agt aac atg caa    336
   Thr Met Ser Asp Ser Ala Thr Val Lys Glu Thr Ser Ser Asn Met Gln
               100                 105                 110
   tca cca caa aac gct aca gct agt caa tct act aca caa act agc aat    384
   Ser Pro Gln Asn Ala Thr Ala Ser Gln Ser Thr Thr Gln Thr Ser Asn
           115                 120                 125
   gta aca aca aat gat aaa tca tca act aca tat agt aat gaa act gat    432
   Val Thr Thr Asn Asp Lys Ser Ser Thr Thr Tyr Ser Asn Glu Thr Asp
       130                 135                 140
   aaa agt aat tta aca caa gca aaa aac gtt tca act aca cct aaa aca    480
   Lys Ser Asn Leu Thr Gln Ala Lys Asn Val Ser Thr Thr Pro Lys Thr
   145                 150                 155                 160
   acg act att aaa caa aga gct tta aat cgc atg gca gtg aat act gtt    528
   Thr Thr Ile Lys Gln Arg Ala Leu Asn Arg Met Ala Val Asn Thr Val
                   165                 170                 175
   gca gct cca caa caa gga aca aat gtt aat gat aaa gta cat ttt acg    576
   Ala Ala Pro Gln Gln Gly Thr Asn Val Asn Asp Lys Val His Phe Thr
               180                 185                 190
   aac att gat att gcg att gat aaa gga cat gtt aat aaa aca aca gga    624
   Asn Ile Asp Ile Ala Ile Asp Lys Gly His Val Asn Lys Thr Thr Gly
           195                 200                 205
   aat act gaa ttt tgg gca act tca agt gat gtt tta aaa tta aaa gcg     672
   Asn Thr Glu Phe Trp Ala Thr Ser Ser Asp Val Leu Lys Leu Lys Ala
       210                 215                 220
   aat tac aca atc gat gat tct gtt aaa gag ggc gat aca ttt act ttt     720
   Asn Tyr Thr Ile Asp Asp Ser Val Lys Glu Gly Asp Thr Phe Thr Phe
   225                 230                 235                 240
   aaa tat ggt caa tat ttc cgt cca ggt tct gta aga tta cct tca caa     768
   Lys Tyr Gly Gln Tyr Phe Arg Pro Gly Ser Val Arg Leu Pro Ser Gln
                   245                 250                 255
   act caa aat tta tat aat gcc caa ggt aat att att gca aaa ggt att     816
   Thr Gln Asn Leu Tyr Asn Ala Gln Gly Asn Ile Ile Ala Lys Gly Ile
               260                 265                 270
   tac gat agt aaa aca aat aca aca acg tat act ttt acg aat tat gta     864
   Tyr Asp Ser Lys Thr Asn Thr Thr Thr Tyr Thr Phe Thr Asn Tyr Val
           275                 280                 285
   gat caa tac aca aat gtt agc ggt agc ttt gaa caa gtc gca ttt gcg     912
   Asp Gln Tyr Thr Asn Val Ser Gly Ser Phe Glu Gln Val Ala Phe Ala
       290                 295                 300
   aaa cgt gaa aat gca aca act gat aaa act gct tat aaa atg gaa gta     960
   Lys Arg Glu Asn Ala Thr Thr Asp Lys Thr Ala Tyr Lys Met Glu Val
   305                 310                 315                 320
   act tta ggt aat gat aca tat agt aaa gat gtc att gtc gat tat ggt     1008
   Thr Leu Gly Asn Asp Thr Tyr Ser Lys Asp Val Ile Val Asp Tyr Gly
                   325                 330                 335
   aat caa aaa ggt caa caa ctt att tcg agt aca aat tat att aat aat     1056
   Asn Gln Lys Gly Gln Gln Leu Ile Ser Ser Thr Asn Tyr Ile Asn Asn
               340                 345                 350
   gaa gat ttg tca cgt aat atg act gtt tat gta aat caa cct aaa aag     1104
   Glu Asp Leu Ser Arg Asn Met Thr Val Tyr Val Asn Gln Pro Lys Lys
           355                 360                 365
   acc tat aca aaa gaa aca ttt gta aca aat tta act ggt tat aaa ttt     1152
   Thr Tyr Thr Lys Glu Thr Phe Val Thr Asn Leu Thr Gly Tyr Lys Phe
       370                 375                 380
   aat cca gat gct aaa aac ttc aaa att tac gaa gtg aca gat caa aat     1200
   Asn Pro Asp Ala Lys Asn Phe Lys Ile Tyr Glu Val Thr Asp Gln Asn
   385                 390                 395                 400
   caa ttt gtg gat agt ttc acc cca gat act tca aaa ctt aaa gat gtt     1248
   Gln Phe Val Asp Ser Phe Thr Pro Asp Thr Ser Lys Leu Lys Asp Val
                   405                 410                 415
   act ggt caa ttc gat gtt att tat agt aat gat aat aag acg gcg aca     1296
   Thr Gly Gln Phe Asp Val Ile Tyr Ser Asn Asp Asn Lys Thr Ala Thr
               420                 425                 430
   gta gat tta ttg aat ggt caa tct agt agt gat aaa cag tac atc att     1344
   Val Asp Leu Leu Asn Gly Gln Ser Ser Ser Asp Lys Gln Tyr Ile Ile
           435                 440                 445
   caa caa gtt gct tat cca gat aat agt tca aca gat aat ggg aaa att     1392
   Gln Gln Val Ala Tyr Pro Asp Asn Ser Ser Thr Asp Asn Gly Lys Ile
       450                 455                 460
   gat tat act tta gaa aca caa aat gga aaa agt agt tgg tca aac agt    1440
   Asp Tyr Thr Leu Glu Thr Gln Asn Gly Lys Ser Ser Trp Ser Asn Ser
   465                 470                 475                 480
   tat tca aat gtg aat ggc tca tca act gca aat ggc gac caa aag aaa    1488
   Tyr Ser Asn Val Asn Gly Ser Ser Thr Ala Asn Gly Asp Gln Lys Lys
                   485                 490                 495
   tat aat cta ggt gac tat gta tgg gaa gat aca aat aaa gat ggt aaa    1536
   Tyr Asn Leu Gly Asp Tyr Val Trp Glu Asp Thr Asn Lys Asp Gly Lys
               500                 505                 510
   caa gat gcc aat gaa aaa ggg att aaa ggt gtt tat gtc att ctt aaa    1584
   Gln Asp Ala Asn Glu Lys Gly Ile Lys Gly Val Tyr Val Ile Leu Lys
           515                 520                 525
   gat agt aac ggt aaa gaa tta gat cgt acg aca aca gat gaa aat ggt    1632
   Asp Ser Asn Gly Lys Glu Leu Asp Arg Thr Thr Thr Asp Glu Asn Gly
       530                 535                 540
   aaa tat cag ttc act ggt tta agc aat gga act tat agt gta gag ttt    1680
   Lys Tyr Gln Phe Thr Gly Leu Ser Asn Gly Thr Tyr Ser Val Glu Phe
   545                 550                 555                 560
   tca aca cca gcc ggt tat aca ccg aca act gca aat gca ggt aca gat    1728
   Ser Thr Pro Ala Gly Tyr Thr Pro Thr Thr Ala Asn Ala Gly Thr Asp
                   565                 570                 575
   gat gct gta gat tct gat gga cta act aca aca ggt gtc att aaa gac    1776
   Asp Ala Val Asp Ser Asp Gly Leu Thr Thr Thr Gly Val Ile Lys Asp
               580                 585                 590
   gct gac aac atg aca tta gat agt gga ttc tac aaa aca cca aaa tat    1824
   Ala Asp Asn Met Thr Leu Asp Ser Gly Phe Tyr Lys Thr Pro Lys Tyr
           595                 600                 605
   agt tta ggt gat tat gtt tgg tac gac agt aat aaa gat ggt aaa caa    1872
   Ser Leu Gly Asp Tyr Val Trp Tyr Asp Ser Asn Lys Asp Gly Lys Gln
       610                 615                 620
   gat tcg act gaa aaa gga att aaa ggt gtt aaa gtt act ttg caa aac    1920
   Asp Ser Thr Glu Lys Gly Ile Lys Gly Val Lys Val Thr Leu Gln Asn
   625                 630                 635                 640
   gaa aaa ggc gaa gta att ggt aca act gaa aca gat gaa aat ggt aaa    1968
   Glu Lys Gly Glu Val Ile Gly Thr Thr Glu Thr Asp Glu Asn Gly Lys
                   645                 650                 655
   tac cgc ttt gat aat tta gat agt ggt aaa tac aaa gtt atc ttt gaa    2016
   Tyr Arg Phe Asp Asn Leu Asp Ser Gly Lys Tyr Lys Val Ile Phe Glu
               660                 665                 670
   aag cct gct ggt tta act caa aca ggt aca aat aca act gaa gat gat    2064
   Lys Pro Ala Gly Leu Thr Gln Thr Gly Thr Asn Thr Thr Glu Asp Asp
           675                 680                 685
   aaa gat gcc gat ggt ggc gaa gtt gat gta aca att acg gat cat gat    2112
   Lys Asp Ala Asp Gly Gly Glu Val Asp Val Thr Ile Thr Asp His Asp
       690                 695                 700
   gat ttc aca ctt gat aat ggc tac tac gaa gaa gaa aca tca gat agt     2160
   Asp Phe Thr Leu Asp Asn Gly Tyr Tyr Glu Glu Glu Thr Ser Asp Ser
   705                 710                 715                 720
   gac tca gat tcg gac agc gat tca gac tca gat agc gac tca gat tca     2208
   Asp Ser Asp Ser Asp Ser Asp Ser Asp Ser Asp Ser Asp Ser Asp Ser
                   725                 730                 735
   gat agt gac tca gac tca gat agc gac tca gac tca gat agc gac tca     2256
   Asp Ser Asp Ser Asp Ser Asp Ser Asp Ser Asp Ser Asp Ser Asp Ser
               740                 745                 750
   gac agc gac tca gac tca gat agt gat tca gat tcg gac agc gac tca     2304
   Asp Ser Asp Ser Asp Ser Asp Ser Asp Ser Asp Ser Asp Ser Asp Ser
           755                 760                 765
   gat tca gac agc gaa tca gat tcg gat agc gac tca gac tca gat agc     2352
   Asp Ser Asp Ser Glu Ser Asp Ser Asp Ser Asp Ser Asp Ser Asp Ser
       770                 775                 780
   gac tca gac agc gac tca gat tca gac agt gac tca gac tca gac agc     2400
   Asp Ser Asp Ser Asp Ser Asp Ser Asp Ser Asp Ser Asp Ser Asp Ser
   785                 790                 795                 800
   gac tca gat tca gac agc gat tca gat tcg gat agc gac tca gat tca     2448
   Asp Ser Asp Ser Asp Ser Asp Ser Asp Ser Asp Ser Asp Ser Asp Ser
                   805                 810                 815
   gat agc gat tcg gac tca gac aac gac tca gat tct gac agc gat tca     2496
   Asp Ser Asp Ser Asp Ser Asp Asn Asp Ser Asp Ser Asp Ser Asp Ser
               820                 825                 830
   gac tca gat agc gac tca gat tca gac agc gac tca gat tca gac agc     2544
   Asp Ser Asp Ser Asp Ser Asp Ser Asp Ser Asp Ser Asp Ser Asp Ser
           835                 840                 845
   gat tca gat tca gat agc gat tca gat tca gac agc gac tca gat tca     2592
   Asp Ser Asp Ser Asp Ser Asp Ser Asp Ser Asp Ser Asp Ser Asp Ser
       850                 855                 860
   gat agc gac tca gac tca gac agc gat tca gac tca gat agc gac tca     2640
   Asp Ser Asp Ser Asp Ser Asp Ser Asp Ser Asp Ser Asp Ser Asp Ser
   865                 870                 875                 880
   gac agc gat tca gat tcg gat agc gat tca gat tca gat gca ggt aaa     2688
   Asp Ser Asp Ser Asp Ser Asp Ser Asp Ser Asp Ser Asp Ala Gly Lys
                   885                 890                 895
   cat act ccg act aaa cca atg agt acg gtt aaa gat cag cat aaa aca     2736
   His Thr Pro Thr Lys Pro Met Ser Thr Val Lys Asp Gln His Lys Thr
               900                 905                 910
   gct aaa gca tta cca gaa aca ggt agt gaa aat aat aat tca aat aat     2784
   Ala Lys Ala Leu Pro Glu Thr Gly Ser Glu Asn Asn Asn Ser Asn Asn
           915                 920                 925
   ggc aca tta ttc ggt gga tta ttc gcg gca tta gga tca tta ttg tta     2832
   Gly Thr Leu Phe Gly Gly Leu Phe Ala Ala Leu Gly Ser Leu Leu Leu
       930                 935                 940
   ttc ggt cgt cgt aaa aaa caa aat aaa taa                             2862
   Phe Gly Arg Arg Lys Lys Gln Asn Lys
   945                 950
<210> 26
   <211> 953
   <212> PRT
   <213> Staphylococcus sp.
<400> 26
   Met Asn Asn Lys Lys Thr Ala Thr Asn Arg Lys Gly Met Ile Pro Asn
    1               5                  10                  15
   Arg Leu Asn Lys Phe Ser Ile Arg Lys Tyr Ser Val Gly Thr Ala Ser
               20                  25                  30
   Ile Leu Val Gly Thr Thr Leu Ile Phe Gly Leu Ser Gly His Glu Ala
           35                  40                  45
   Lys Ala Ala Glu His Thr Asn Gly Glu Leu Asn Gln Ser Lys Asn Glu
       50                  55                  60
   Thr Thr Ala Pro Ser Glu Asn Lys Thr Thr Glu Lys Val Asp Ser Arg
   65                  70                  75                  80
   Gln Leu Lys Asp Asn Thr Gln Thr Ala Thr Ala Asp Gln Pro Lys Val
                   85                  90                  95
   Thr Met Ser Asp Ser Ala Thr Val Lys Glu Thr Ser Ser Asn Met Gln
               100                 105                 110
   Ser Pro Gln Asn Ala Thr Ala Ser Gln Ser Thr Thr Gln Thr Ser Asn
           115                 120                 125
   Val Thr Thr Asn Asp Lys Ser Ser Thr Thr Tyr Ser Asn Glu Thr Asp
       130                 135                 140
   Lys Ser Asn Leu Thr Gln Ala Lys Asn Val Ser Thr Thr Pro Lys Thr
   145                 150                 155                 160
   Thr Thr Ile Lys Gln Arg Ala Leu Asn Arg Met Ala Val Asn Thr Val
                   165                 170                 175
   Ala Ala Pro Gln Gln Gly Thr Asn Val Asn Asp Lys Val His Phe Thr
               180                 185                 190
   Asn Ile Asp Ile Ala Ile Asp Lys Gly His Val Asn Lys Thr Thr Gly
           195                 200                 205
   Asn Thr Glu Phe Trp Ala Thr Ser Ser Asp Val Leu Lys Leu Lys Ala
       210                 215                 220
   Asn Tyr Thr Ile Asp Asp Ser Val Lys Glu Gly Asp Thr Phe Thr Phe
   225                 230                 235                 240
   Lys Tyr Gly Gln Tyr Phe Arg Pro Gly Ser Val Arg Leu Pro Ser Gln
                   245                 250                 255
   Thr Gln Asn Leu Tyr Asn Ala Gln Gly Asn Ile Ile Ala Lys Gly Ile
               260                 265                 270
   Tyr Asp Ser Lys Thr Asn Thr Thr Thr Tyr Thr Phe Thr Asn Tyr Val
           275                 280                 285
   Asp Gln Tyr Thr Asn Val Ser Gly Ser Phe Glu Gln Val Ala Phe Ala
       290                 295                 300
   Lys Arg Glu Asn Ala Thr Thr Asp Lys Thr Ala Tyr Lys Met Glu Val
   305                 310                 315                 320
   Thr Leu Gly Asn Asp Thr Tyr Ser Lys Asp Val Ile Val Asp Tyr Gly
                   325                 330                 335
   Asn Gln Lys Gly Gln Gln Leu Ile Ser Ser Thr Asn Tyr Ile Asn Asn
               340                 345                 350
   Glu Asp Leu Ser Arg Asn Met Thr Val Tyr Val Asn Gln Pro Lys Lys
           355                 360                 365
   Thr Tyr Thr Lys Glu Thr Phe Val Thr Asn Leu Thr Gly Tyr Lys Phe
       370                 375                 380
   Asn Pro Asp Ala Lys Asn Phe Lys Ile Tyr Glu Val Thr Asp Gln Asn
   385                 390                 395                 400
   Gln Phe Val Asp Ser Phe Thr Pro Asp Thr Ser Lys Leu Lys Asp Val
                   405                 410                 415
   Thr Gly Gln Phe Asp Val Ile Tyr Ser Asn Asp Asn Lys Thr Ala Thr
               420                 425                 430
   Val Asp Leu Leu Asn Gly Gln Ser Ser Ser Asp Lys Gln Tyr Ile Ile
           435                 440                 445
   Gln Gln Val Ala Tyr Pro Asp Asn Ser Ser Thr Asp Asn Gly Lys Ile
       450                 455                 460
   Asp Tyr Thr Leu Glu Thr Gln Asn Gly Lys Ser Ser Trp Ser Asn Ser
   465                 470                 475                 480
   Tyr Ser Asn Val Asn Gly Ser Ser Thr Ala Asn Gly Asp Gln Lys Lys
                   485                 490                 495
   Tyr Asn Leu Gly Asp Tyr Val Trp Glu Asp Thr Asn Lys Asp Gly Lys
               500                 505                 510
   Gln Asp Ala Asn Glu Lys Gly Ile Lys Gly Val Tyr Val Ile Leu Lys
           515                 520                 525
   Asp Ser Asn Gly Lys Glu Leu Asp Arg Thr Thr Thr Asp Glu Asn Gly
       530                 535                 540
   Lys Tyr Gln Phe Thr Gly Leu Ser Asn Gly Thr Tyr Ser Val Glu Phe
   545                 550                 555                 560
   Ser Thr Pro Ala Gly Tyr Thr Pro Thr Thr Ala Asn Ala Gly Thr Asp
                   565                 570                 575
   Asp Ala Val Asp Ser Asp Gly Leu Thr Thr Thr Gly Val Ile Lys Asp
               580                 585                 590
   Ala Asp Asn Met Thr Leu Asp Ser Gly Phe Tyr Lys Thr Pro Lys Tyr
           595                 600                 605
   Ser Leu Gly Asp Tyr Val Trp Tyr Asp Ser Asn Lys Asp Gly Lys Gln
       610                 615                 620
   Asp Ser Thr Glu Lys Gly Ile Lys Gly Val Lys Val Thr Leu Gln Asn
   625                 630                 635                 640
   Glu Lys Gly Glu Val Ile Gly Thr Thr Glu Thr Asp Glu Asn Gly Lys
                   645                 650                 655
   Tyr Arg Phe Asp Asn Leu Asp Ser Gly Lys Tyr Lys Val Ile Phe Glu
               660                 665                 670
   Lys Pro Ala Gly Leu Thr Gln Thr Gly Thr Asn Thr Thr Glu Asp Asp
           675                 680                 685
   Lys Asp Ala Asp Gly Gly Glu Val Asp Val Thr Ile Thr Asp His Asp
       690                 695                 700
   Asp Phe Thr Leu Asp Asn Gly Tyr Tyr Glu Glu Glu Thr Ser Asp Ser
   705                 710                 715                 720
   Asp Ser Asp Ser Asp Ser Asp Ser Asp Ser Asp Ser Asp Ser Asp Ser
                   725                 730                 735
   Asp Ser Asp Ser Asp Ser Asp Ser Asp Ser Asp Ser Asp Ser Asp Ser
               740                 745                 750
   Asp Ser Asp Ser Asp Ser Asp Ser Asp Ser Asp Ser Asp Ser Asp Ser
           755                 760                 765
   Asp Ser Asp Ser Glu Ser Asp Ser Asp Ser Asp Ser Asp Ser Asp Ser
       770                 775                 780
   Asp Ser Asp Ser Asp Ser Asp Ser Asp Ser Asp Ser Asp Ser Asp Ser
   785                 790                 795                 800
   Asp Ser Asp Ser Asp Ser Asp Ser Asp Ser Asp Ser Asp Ser Asp Ser
                   805                 810                 815
   Asp Ser Asp Ser Asp Ser Asp Asn Asp Ser Asp Ser Asp Ser Asp Ser
               820                 825                 830
   Asp Ser Asp Ser Asp Ser Asp Ser Asp Ser Asp Ser Asp Ser Asp Ser
           835                 840                 845
   Asp Ser Asp Ser Asp Ser Asp Ser Asp Ser Asp Ser Asp Ser Asp Ser
       850                 855                 860
   Asp Ser Asp Ser Asp Ser Asp Ser Asp Ser Asp Ser Asp Ser Asp Ser
   865                 870                 875                 880
   Asp Ser Asp Ser Asp Ser Asp Ser Asp Ser Asp Ser Asp Ala Gly Lys
                   885                 890                 895
   His Thr Pro Thr Lys Pro Met Ser Thr Val Lys Asp Gln His Lys Thr
               900                 905                 910
   Ala Lys Ala Leu Pro Glu Thr Gly Ser Glu Asn Asn Asn Ser Asn Asn
           915                 920                 925
   Gly Thr Leu Phe Gly Gly Leu Phe Ala Ala Leu Gly Ser Leu Leu Leu
       930                 935                 940
   Phe Gly Arg Arg Lys Lys Gln Asn Lys
   945                 950
<210> 27
   <211> 2970
   <212> DNA
   <213> Staphylococcus sp.
<220>
   <221> CDS
   <222> (1)..(2970)
<400> 27
   atg aat atg aag aaa aaa gaa aaa cac gca att cgg aaa aaa tcg att      48
   Met Asn Met Lys Lys Lys Glu Lys His Ala Ile Arg Lys Lys Ser Ile
   1               5                   10                  15
   ggc gtg gct tca gtg ctt gta ggt acg tta atc ggt ttt gga cta ctc      96
   Gly Val Ala Ser Val Leu Val Gly Thr Leu Ile Gly Phe Gly Leu Leu
               20                  25                  30
   agc agt aaa gaa gca gat gca agt gaa aat agt gtt acg caa tct gat      144
   Ser Ser Lys Glu Ala Asp Ala Ser Glu Asn Ser Val Thr Gln Ser Asp
           35                  40                  45
   agc gca agt aac gaa agc aaa agt aat gat tca agt agc gtt agt gct      192
   Ser Ala Ser Asn Glu Ser Lys Ser Asn Asp Ser Ser Ser Val Ser Ala
       50                  55                  60
   gca cct aaa aca gac gac aca aac gtg agt gat act aaa aca tcg tca      240
   Ala Pro Lys Thr Asp Asp Thr Asn Val Ser Asp Thr Lys Thr Ser Ser
   65                  70                  75                  80
   aac act aat aat ggc gaa acg agt gtg gcg caa aat cca gca caa cag      288
   Asn Thr Asn Asn Gly Glu Thr Ser Val Ala Gln Asn Pro Ala Gln Gln
                   85                  90                  95
   gaa acg aca caa tca tca tca aca aat gca act acg gaa gaa acg ccg      336
   Glu Thr Thr Gln Ser Ser Ser Thr Asn Ala Thr Thr Glu Glu Thr Pro
               100                 105                 110
   gta act ggt gaa gct act act acg aca acg aat caa gct aat aca ccg      384
   Val Thr Gly Glu Ala Thr Thr Thr Thr Thr Asn Gln Ala Asn Thr Pro
           115                 120                 125
   gca aca act caa tca agc aat aca aat gcg gag gaa tta gtg aat caa      432
   Ala Thr Thr Gln Ser Ser Asn Thr Asn Ala Glu Glu Leu Val Asn Gln
       130                 135                 140
   aca agt aat gaa acg act tct aat gat act aat aca gta tca tct gta      480
   Thr Ser Asn Glu Thr Thr Ser Asn Asp Thr Asn Thr Val Ser Ser Val
   145                 150                 155                 160
   aat tca cct caa aat tct aca aat gcg gaa aat gtt tca aca acg caa      528
   Asn Ser Pro Gln Asn Ser Thr Asn Ala Glu Asn Val Ser Thr Thr Gln
                   165                 170                 175
   gat act tca act gaa gca aca cct tca aac aat gaa tca gct cca cag      576
   Asp Thr Ser Thr Glu Ala Thr Pro Ser Asn Asn Glu Ser Ala Pro Gln
               180                 185                 190
   aat aca gat gca agt aat aaa gat gta gtt agt caa gcg gtt aat cca     624
   Asn Thr Asp Ala Ser Asn Lys Asp Val Val Ser Gln Ala Val Asn Pro
           195                 200                 205
   agt acg cct aga atg aga gca ttt agt tta gcg gca gta gct gca gat     672
   Ser Thr Pro Arg Met Arg Ala Phe Ser Leu Ala Ala Val Ala Ala Asp
       210                 215                 220
   gca ccg gca gct ggc aca gat att acg aat cag ttg aca gat gtg aaa     720
   Ala Pro Ala Ala Gly Thr Asp Ile Thr Asn Gln Leu Thr Asp Val Lys
   225                 230                 235                 240
   gtt act att gac tct ggt acg act gtg tat ccg cac caa gca ggt tat     768
   Val Thr Ile Asp Ser Gly Thr Thr Val Tyr Pro His Gln Ala Gly Tyr
                   245                 250                 255
   gtc aaa ctg aat tat ggt ttt tca gtg cct aat tct gct gtt aaa ggt     816
   Val Lys Leu Asn Tyr Gly Phe Ser Val Pro Asn Ser Ala Val Lys Gly
               260                 265                 270
   gac aca ttc aaa ata act gta cct aaa gaa tta aac tta aat ggt gta     864
   Asp Thr Phe Lys Ile Thr Val Pro Lys Glu Leu Asn Leu Asn Gly Val
           275                 280                 285
   act tca act gct aaa gtg cca cca att atg gct gga gat caa gta ttg     912
   Thr Ser Thr Ala Lys Val Pro Pro Ile Met Ala Gly Asp Gln Val Leu
       290                 295                 300
   gca aat ggt gta atc gat agt gat ggt aat gtt att tat aca ttt aca     960
   Ala Asn Gly Val Ile Asp Ser Asp Gly Asn Val Ile Tyr Thr Phe Thr
   305                 310                 315                 320
   gac tat gtt gat aat aaa gaa aat gta aca gct aat att act atg cca     1008
   Asp Tyr Val Asp Asn Lys Glu Asn Val Thr Ala Asn Ile Thr Met Pro
                   325                 330                 335
   gct tat att gac cct gaa aat gtt aca aag aca ggt aat gtg aca ttg     1056
   Ala Tyr Ile Asp Pro Glu Asn Val Thr Lys Thr Gly Asn Val Thr Leu
               340                 345                 350
   aca act ggc ata gga acc aat act gct agt aag aca gta tta atc gac     1104
   Thr Thr Gly Ile Gly Thr Asn Thr Ala Ser Lys Thr Val Leu Ile Asp
           355                 360                 365
   tat gag aaa tat gga caa ttc cat aat tta tca att aaa ggt acg att     1152
   Tyr Glu Lys Tyr Gly Gln Phe His Asn Leu Ser Ile Lys Gly Thr Ile
       370                 375                 380
   gat caa atc gat aaa aca aat aat acg tat cgc caa aca att tat gtc     1200
   Asp Gln Ile Asp Lys Thr Asn Asn Thr Tyr Arg Gln Thr Ile Tyr Val
   385                 390                 395                 400
   aat cca agc gga gat aac gtt gtg tta cct gcc tta aca ggt aat tta     1248
   Asn Pro Ser Gly Asp Asn Val Val Leu Pro Ala Leu Thr Gly Asn Leu
                   405                 410                 415
   att cct aat aca aag agt aat gcg tta ata gat gca aaa aac act gat     1296
   Ile Pro Asn Thr Lys Ser Asn Ala Leu Ile Asp Ala Lys Asn Thr Asp
               420                 425                 430
   att aaa gtt tat aga gtc gat aat gct aat gat tta tct gaa agt tat     1344
   Ile Lys Val Tyr Arg Val Asp Asn Ala Asn Asp Leu Ser Glu Ser Tyr
           435                 440                 445
   tat gtg aat cct agc gat ttt gaa gat gta act aat caa gtt aga att    1392
   Tyr Val Asn Pro Ser Asp Phe Glu Asp Val Thr Asn Gln Val Arg Ile
       450                 455                 460
   tca ttt cca aat gct aat caa tac aaa gta gaa ttt cct acg gac gat    1440
   Ser Phe Pro Asn Ala Asn Gln Tyr Lys Val Glu Phe Pro Thr Asp Asp
   465                 470                 475                 480
   gac caa att aca aca ccg tat att gta gtt gtt aat ggc cat att gat    1488
   Asp Gln Ile Thr Thr Pro Tyr Ile Val Val Val Asn Gly His Ile Asp
                   485                 490                 495
   cct gct agt aca ggt gat tta gca cta cgt tcg aca ttt tat ggt tat    1536
   Pro Ala Ser Thr Gly Asp Leu Ala Leu Arg Ser Thr Phe Tyr Gly Tyr
               500                 505                 510
   gat tct aat ttt ata tgg aga tct atg tca tgg gac aac gaa gta gca    1584
   Asp Ser Asn Phe Ile Trp Arg Ser Met Ser Trp Asp Asn Glu Val Ala
           515                 520                 525
   ttt aat aac gga tca ggt tct ggt gac ggt atc gat aaa cca gtt gtt    1632
   Phe Asn Asn Gly Ser Gly Ser Gly Asp Gly Ile Asp Lys Pro Val Val
       530                 535                 540
   cct gaa caa cct gat gag cct ggt gaa att gaa cca att cca gag gat    1680
   Pro Glu Gln Pro Asp Glu Pro Gly Glu Ile Glu Pro Ile Pro Glu Asp
   545                 550                 555                 560
   tca gat tct gac cca ggt tca gat tct ggc agc gat tct aat tca gat    1728
   Ser Asp Ser Asp Pro Gly Ser Asp Ser Gly Ser Asp Ser Asn Ser Asp
                   565                 570                 575
   agc ggt tca gat tct ggc agt gat tct aca tca gat agt ggt tca gat    1776
   Ser Gly Ser Asp Ser Gly Ser Asp Ser Thr Ser Asp Ser Gly Ser Asp
               580                 585                 590
   tca gcg agt gat tca gat tca gca agt gat tca gac tca gcg agt gat    1824
   Ser Ala Ser Asp Ser Asp Ser Ala Ser Asp Ser Asp Ser Ala Ser Asp
           595                 600                 605
   tca gat tca gca agt gat tca gat tca gca agt gat tca gat tca gca    1872
   Ser Asp Ser Ala Ser Asp Ser Asp Ser Ala Ser Asp Ser Asp Ser Ala
       610                 615                 620
   agt gat tca gac tca gca agt gat tca gat tca gca agt gat tca gat    1920
   Ser Asp Ser Asp Ser Ala Ser Asp Ser Asp Ser Ala Ser Asp Ser Asp
   625                 630                 635                 640
   tca gca agc gat tca gat tca gcg agc gat tca gat tca gcg agc gat    1968
   Ser Ala Ser Asp Ser Asp Ser Ala Ser Asp Ser Asp Ser Ala Ser Asp
                   645                 650                 655
   tca gat tca gcg agt gat tcc gac tca gcg agc gat tca gac tca gat    2016
   Ser Asp Ser Ala Ser Asp Ser Asp Ser Ala Ser Asp Ser Asp Ser Asp
               660                 665                 670
   agt gac tca gat tcc gat agc gat tcc gac tca gat agc gac tca gat    2064
   Ser Asp Ser Asp Ser Asp Ser Asp Ser Asp Ser Asp Ser Asp Ser Asp
           675                 680                 685
   tca gac agc gat tct gac tca gac agc gat tct gac tca gac agt gac    2112
   Ser Asp Ser Asp Ser Asp Ser Asp Ser Asp Ser Asp Ser Asp Ser Asp
       690                 695                 700
   tca gat tcc gat agc gat tcc gac tca gac agt gac tca gat tcc gat   2160
   Ser Asp Ser Asp Ser Asp Ser Asp Ser Asp Ser Asp Ser Asp Ser Asp
   705                 710                 715                 720
   agc gat tcc gac tca gac agt gac tca gat tcc gat agc gat tca gat   2208
   Ser Asp Ser Asp Ser Asp Ser Asp Ser Asp Ser Asp Ser Asp Ser Asp
                   725                 730                 735
   tcc gac agt gat tcc gac tca gat agc gat tcc gac tca gat agc gac   2256
   Ser Asp Ser Asp Ser Asp Ser Asp Ser Asp Ser Asp Ser Asp Ser Asp
               740                 745                 750
   tca gat tca gac agc gat tca gat tca gac agc gat tct gac tca gac   2304
   Ser Asp Ser Asp Ser Asp Ser Asp Ser Asp Ser Asp Ser Asp Ser Asp
           755                 760                 765
   agt gac tca gat tcc gat agc gat tca gat tca gac agt gat tca gac   2352
   Ser Asp Ser Asp Ser Asp Ser Asp Ser Asp Ser Asp Ser Asp Ser Asp
       770                 775                 780
   tca gat agc gat tca gat tcc gac agt gac tca gac tca gac agc gat   2400
   Ser Asp Ser Asp Ser Asp Ser Asp Ser Asp Ser Asp Ser Asp Ser Asp
   785                 790                 795                 800
   tca gat tcc gat agc gat tca gat tcc gac agt gac tca gat tcc gat   2448
   Ser Asp Ser Asp Ser Asp Ser Asp Ser Asp Ser Asp Ser Asp Ser Asp
                   805                 810                 815
   agt gac tcg gat tca gcg agt gat tca gat tca gat agc gat tca gaa   2496
   Ser Asp Ser Asp Ser Ala Ser Asp Ser Asp Ser Asp Ser Asp Ser Glu
               820                 825                 830
   tca gat agt gac tca gac tca gac agt gat tca gat tca gat agt gac   2544
   Ser Asp Ser Asp Ser Asp Ser Asp Ser Asp Ser Asp Ser Asp Ser Asp
           835                 840                 845
   tca gac tca gac agc gat tca gaa tca gat agt gac tcc gat tca gac   2592
   Ser Asp Ser Asp Ser Asp Ser Glu Ser Asp Ser Asp Ser Asp Ser Asp
       850                 855                 860
   agc gat tca gaa tca gat agt gac tcc gat tca gat agc gat tcg gat   2640
   Ser Asp Ser Glu Ser Asp Ser Asp Ser Asp Ser Asp Ser Asp Ser Asp
   865                 870                 875                 880
   tca gcg agt gat tca gac tca ggt agt gac tcc gat tca tca agt gat   2688
   Ser Ala Ser Asp Ser Asp Ser Gly Ser Asp Ser Asp Ser Ser Ser Asp
                   885                 890                 895
   tca gat tcc gat tca acg agt gac aca gga tca gac aac gac tca gac   2736
   Ser Asp Ser Asp Ser Thr Ser Asp Thr Gly Ser Asp Asn Asp Ser Asp
               900                 905                 910
   agt gat tca aat agc gat tcc gag tca ggt tct aac aat aat gta gtt   2784
   Ser Asp Ser Asn Ser Asp Ser Glu Ser Gly Ser Asn Asn Asn Val Val
           915                 920                 925
   ccg cct aat tca cct aaa aat ggt act aat gct tct aat aaa aat gag   2832
   Pro Pro Asn Ser Pro Lys Asn Gly Thr Asn Ala Ser Asn Lys Asn Glu
       930                 935                 940
   gct aaa gat agt aaa gaa cca tta cca gat aca ggt tct gaa gat gaa   2880
   Ala Lys Asp Ser Lys Glu Pro Leu Pro Asp Thr Gly Ser Glu Asp Glu
   945                 950                 955                 960
   gcg aat acg tca cta att tgg gga tta tta gca tca tta ggt tca tta    2928
   Ala Asn Thr Ser Leu Ile Trp Gly Leu Leu Ala Ser Leu Gly Ser Leu
                   965                 970                 975
   cta ctt ttc aga aga aaa aaa gaa aat aaa gat aag aaa taa            2970
   Leu Leu Phe Arg Arg Lys Lys Glu Asn Lys Asp Lys Lys
               980                 985
<210> 28
   <211> 989
   <212> PRT
   <213> Staphylococcus sp.
<400> 28
   Met Asn Met Lys Lys Lys Glu Lys His Ala Ile Arg Lys Lys Ser Ile
    1               5                  10                  15
   Gly Val Ala Ser Val Leu Val Gly Thr Leu Ile Gly Phe Gly Leu Leu
               20                  25                  30
   Ser Ser Lys Glu Ala Asp Ala Ser Glu Asn Ser Val Thr Gln Ser Asp
           35                  40                  45
   Ser Ala Ser Asn Glu Ser Lys Ser Asn Asp Ser Ser Ser Val Ser Ala
       50                  55                  60
   Ala Pro Lys Thr Asp Asp Thr Asn Val Ser Asp Thr Lys Thr Ser Ser
   65                  70                  75                  80
   Asn Thr Asn Asn Gly Glu Thr Ser Val Ala Gln Asn Pro Ala Gln Gln
                   85                  90                  95
   Glu Thr Thr Gln Ser Ser Ser Thr Asn Ala Thr Thr Glu Glu Thr Pro
               100                 105                 110
   Val Thr Gly Glu Ala Thr Thr Thr Thr Thr Asn Gln Ala Asn Thr Pro
           115                 120                 125
   Ala Thr Thr Gln Ser Ser Asn Thr Asn Ala Glu Glu Leu Val Asn Gln
       130                 135                 140
   Thr Ser Asn Glu Thr Thr Ser Asn Asp Thr Asn Thr Val Ser Ser Val
   145                 150                 155                 160
   Asn Ser Pro Gln Asn Ser Thr Asn Ala Glu Asn Val Ser Thr Thr Gln
                   165                 170                 175
   Asp Thr Ser Thr Glu Ala Thr Pro Ser Asn Asn Glu Ser Ala Pro Gln
               180                 185                 190
   Asn Thr Asp Ala Ser Asn Lys Asp Val Val Ser Gln Ala Val Asn Pro
           195                 200                 205
   Ser Thr Pro Arg Met Arg Ala Phe Ser Leu Ala Ala Val Ala Ala Asp
       210                 215                 220
   Ala Pro Ala Ala Gly Thr Asp Ile Thr Asn Gln Leu Thr Asp Val Lys
   225                 230                 235                 240
   Val Thr Ile Asp Ser Gly Thr Thr Val Tyr Pro His Gln Ala Gly Tyr
                   245                 250                 255
   Val Lys Leu Asn Tyr Gly Phe Ser Val Pro Asn Ser Ala Val Lys Gly
               260                 265                 270
   Asp Thr Phe Lys Ile Thr Val Pro Lys Glu Leu Asn Leu Asn Gly Val
           275                 280                 285
   Thr Ser Thr Ala Lys Val Pro Pro Ile Met Ala Gly Asp Gln Val Leu
       290                 295                 300
   Ala Asn Gly Val Ile Asp Ser Asp Gly Asn Val Ile Tyr Thr Phe Thr
   305                 310                 315                 320
   Asp Tyr Val Asp Asn Lys Glu Asn Val Thr Ala Asn Ile Thr Met Pro
                   325                 330                 335
   Ala Tyr Ile Asp Pro Glu Asn Val Thr Lys Thr Gly Asn Val Thr Leu
               340                 345                 350
   Thr Thr Gly Ile Gly Thr Asn Thr Ala Ser Lys Thr Val Leu Ile Asp
           355                 360                 365
   Tyr Glu Lys Tyr Gly Gln Phe His Asn Leu Ser Ile Lys Gly Thr Ile
       370                 375                 380
   Asp Gln Ile Asp Lys Thr Asn Asn Thr Tyr Arg Gln Thr Ile Tyr Val
   385                 390                 395                 400
   Asn Pro Ser Gly Asp Asn Val Val Leu Pro Ala Leu Thr Gly Asn Leu
                   405                 410                 415
   Ile Pro Asn Thr Lys Ser Asn Ala Leu Ile Asp Ala Lys Asn Thr Asp
               420                 425                 430
   Ile Lys Val Tyr Arg Val Asp Asn Ala Asn Asp Leu Ser Glu Ser Tyr
           435                 440                 445
   Tyr Val Asn Pro Ser Asp Phe Glu Asp Val Thr Asn Gln Val Arg Ile
       450                 455                 460
   Ser Phe Pro Asn Ala Asn Gln Tyr Lys Val Glu Phe Pro Thr Asp Asp
   465                 470                 475                 480
   Asp Gln Ile Thr Thr Pro Tyr Ile Val Val Val Asn Gly His Ile Asp
                   485                 490                 495
   Pro Ala Ser Thr Gly Asp Leu Ala Leu Arg Ser Thr Phe Tyr Gly Tyr
               500                 505                 510
   Asp Ser Asn Phe Ile Trp Arg Ser Met Ser Trp Asp Asn Glu Val Ala
           515                 520                 525
   Phe Asn Asn Gly Ser Gly Ser Gly Asp Gly Ile Asp Lys Pro Val Val
       530                 535                 540
   Pro Glu Gln Pro Asp Glu Pro Gly Glu Ile Glu Pro Ile Pro Glu Asp
   545                 550                 555                 560
   Ser Asp Ser Asp Pro Gly Ser Asp Ser Gly Ser Asp Ser Asn Ser Asp
                   565                 570                 575
   Ser Gly Ser Asp Ser Gly Ser Asp Ser Thr Ser Asp Ser Gly Ser Asp
               580                 585                 590
   Ser Ala Ser Asp Ser Asp Ser Ala Ser Asp Ser Asp Ser Ala Ser Asp
           595                 600                 605
   Ser Asp Ser Ala Ser Asp Ser Asp Ser Ala Ser Asp Ser Asp Ser Ala
       610                 615                 620
   Ser Asp Ser Asp Ser Ala Ser Asp Ser Asp Ser Ala Ser Asp Ser Asp
   625                 630                 635                 640
   Ser Ala Ser Asp Ser Asp Ser Ala Ser Asp Ser Asp Ser Ala Ser Asp
                   645                 650                 655
   Ser Asp Ser Ala Ser Asp Ser Asp Ser Ala Ser Asp Ser Asp Ser Asp
               660                 665                 670
   Ser Asp Ser Asp Ser Asp Ser Asp Ser Asp Ser Asp Ser Asp Ser Asp
           675                 680                 685
   Ser Asp Ser Asp Ser Asp Ser Asp Ser Asp Ser Asp Ser Asp Ser Asp
       690                 695                 700
   Ser Asp Ser Asp Ser Asp Ser Asp Ser Asp Ser Asp Ser Asp Ser Asp
   705                 710                 715                 720
   Ser Asp Ser Asp Ser Asp Ser Asp Ser Asp Ser Asp Ser Asp Ser Asp
                   725                 730                 735
   Ser Asp Ser Asp Ser Asp Ser Asp Ser Asp Ser Asp Ser Asp Ser Asp
               740                 745                 750
   Ser Asp Ser Asp Ser Asp Ser Asp Ser Asp Ser Asp Ser Asp Ser Asp
           755                 760                 765
   Ser Asp Ser Asp Ser Asp Ser Asp Ser Asp Ser Asp Ser Asp Ser Asp
       770                 775                 780
   Ser Asp Ser Asp Ser Asp Ser Asp Ser Asp Ser Asp Ser Asp Ser Asp
   785                 790                 795                 800
   Ser Asp Ser Asp Ser Asp Ser Asp Ser Asp Ser Asp Ser Asp Ser Asp
                   805                 810                 815
   Ser Asp Ser Asp Ser Ala Ser Asp Ser Asp Ser Asp Ser Asp Ser Glu
               820                 825                 830
   Ser Asp Ser Asp Ser Asp Ser Asp Ser Asp Ser Asp Ser Asp Ser Asp
           835                 840                 845
   Ser Asp Ser Asp Ser Asp Ser Glu Ser Asp Ser Asp Ser Asp Ser Asp
       850                 855                 860
   Ser Asp Ser Glu Ser Asp Ser Asp Ser Asp Ser Asp Ser Asp Ser Asp
   865                 870                 875                 880
   Ser Ala Ser Asp Ser Asp Ser Gly Ser Asp Ser Asp Ser Ser Ser Asp
                   885                 890                 895
   Ser Asp Ser Asp Ser Thr Ser Asp Thr Gly Ser Asp Asn Asp Ser Asp
               900                 905                 910
   Ser Asp Ser Asn Ser Asp Ser Glu Ser Gly Ser Asn Asn Asn Val Val
           915                 920                 925
   Pro Pro Asn Ser Pro Lys Asn Gly Thr Asn Ala Ser Asn Lys Asn Glu
       930                 935                 940
   Ala Lys Asp Ser Lys Glu Pro Leu Pro Asp Thr Gly Ser Glu Asp Glu
   945                 950                 955                 960
   Ala Asn Thr Ser Leu Ile Trp Gly Leu Leu Ala Ser Leu Gly Ser Leu
                   965                 970                 975
   Leu Leu Phe Arg Arg Lys Lys Glu Asn Lys Asp Lys Lys
               980                 985
<210> 29
   <211> 243
   <212> DNA
   <213> Staphylococcus sp.
<220>
   <221> CDS
   <222> (1)..(240)
<400> 29
   atg aat cag cac gta aaa gta aca ttt gat ttt act aat tat aat tac       48
   Met Asn Gln His Val Lys Val Thr Phe Asp Phe Thr Asn Tyr Asn Tyr
   1               5                   10                  15
   ggc aca tat gac tta gca gta cca gca tat tta ccg ata aaa aac tta       96
   Gly Thr Tyr Asp Leu Ala Val Pro Ala Tyr Leu Pro Ile Lys Asn Leu
               20                  25                  30
   ata gct tta gta ttg gat agt ttg gac att tca ata ttt gat gtc aat      144
   Ile Ala Leu Val Leu Asp Ser Leu Asp Ile Ser Ile Phe Asp Val Asn
           35                  40                  45
   aca caa att aaa gtg atg acg aaa ggt caa tta ctt gtt gaa aat gat      192
   Thr Gln Ile Lys Val Met Thr Lys Gly Gln Leu Leu Val Glu Asn Asp
       50                  55                  60
   cga ctc att gat tat caa atc gct gat gga gat att ttg aag tta cta      240
   Arg Leu Ile Asp Tyr Gln Ile Ala Asp Gly Asp Ile Leu Lys Leu Leu
   65                  70                  75                  80
   tag                                                                  243
<210> 30
   <211> 80
   <212> PRT
   <213> Staphylococcus sp.
<400> 30
   Met Asn Gln His Val Lys Val Thr Phe Asp Phe Thr Asn Tyr Asn Tyr
   1               5                   10                  15
   Gly Thr Tyr Asp Leu Ala Val Pro Ala Tyr Leu Pro Ile Lys Asn Leu
               20                  25                  30
   Ile Ala Leu Val Leu Asp Ser Leu Asp Ile Ser Ile Phe Asp Val Asn
           35                  40                  45
   Thr Gln Ile Lys Val Met Thr Lys Gly Gln Leu Leu Val Glu Asn Asp
       50                  55                  60
   Arg Leu Ile Asp Tyr Gln Ile Ala Asp Gly Asp Ile Leu Lys Leu Leu
   65                  70                  75                  80
<210> 31
   <211> 393
   <212> DNA
   <213> Staphylococcus sp.
<220>
   <221> CDS
   <222> (1)..(393)
<400> 31
   atg aat ttt aat gat att gaa aca atg gtt aag tcg aaa ttt aaa gat      48
   Met Asn Phe Asn Asp Ile Glu Thr Met Val Lys Ser Lys Phe Lys Asp
   1               5                   10                  15
   att aaa aag cat gct gaa gag att gcg cat gaa att gaa gtt cgt tct      96
   Ile Lys Lys His Ala Glu Glu Ile Ala His Glu Ile Glu Val Arg Ser
               20                  25                  30
   gga tat tta aga aaa gct gaa caa tat aag cga tta gaa ttt aat ttg     144
   Gly Tyr Leu Arg Lys Ala Glu Gln Tyr Lys Arg Leu Glu Phe Asn Leu
           35                  40                  45
   agt ttt gca cta gat gat att gaa agc aca gca aag gac gta caa act     192
   Ser Phe Ala Leu Asp Asp Ile Glu Ser Thr Ala Lys Asp Val Gln Thr
       50                  55                  60
   gca aaa tct agt gct aat aag gac agt gta act gtt aag gga aag gcg     240
   Ala Lys Ser Ser Ala Asn Lys Asp Ser Val Thr Val Lys Gly Lys Ala
   65                  70                  75                  80
   ccc aat acg tta tat att gaa aaa aga aat ttg atg aaa caa aag ctt     288
   Pro Asn Thr Leu Tyr Ile Glu Lys Arg Asn Leu Met Lys Gln Lys Leu
                   85                  90                  95
   gaa atg ttg ggt gaa gat atc gat aaa aat aaa gaa tcc ctc caa aaa     336
   Glu Met Leu Gly Glu Asp Ile Asp Lys Asn Lys Glu Ser Leu Gln Lys
               100                 105                 110
   gct aag gaa att gct ggc gaa aag gca agt gaa tat ttt aat aaa gca     384
   Ala Lys Glu Ile Ala Gly Glu Lys Ala Ser Glu Tyr Phe Asn Lys Ala
           115                 120                 125
   atg aat taa                                                          393
   Met Asn
       130
<210> 32
   <211> 130
   <212> PRT
   <213> Staphylococcus sp.
<400> 32
   Met Asn Phe Asn Asp Ile Glu Thr Met Val Lys Ser Lys Phe Lys Asp
   1               5                   10                  15
   Ile Lys Lys His Ala Glu Glu Ile Ala His Glu Ile Glu Val Arg Ser
               20                  25                  30
   Gly Tyr Leu Arg Lys Ala Glu Gln Tyr Lys Arg Leu Glu Phe Asn Leu
           35                  40                  45
   Ser Phe Ala Leu Asp Asp Ile Glu Ser Thr Ala Lys Asp Val Gln Thr
       50                  55                  60
   Ala Lys Ser Ser Ala Asn Lys Asp Ser Val Thr Val Lys Gly Lys Ala
   65                  70                  75                  80
   Pro Asn Thr Leu Tyr Ile Glu Lys Arg Asn Leu Met Lys Gln Lys Leu
                   85                  90                  95
   Glu Met Leu Gly Glu Asp Ile Asp Lys Asn Lys Glu Ser Leu Gln Lys
               100                 105                 110
   Ala Lys Glu Ile Ala Gly Glu Lys Ala Ser Glu Tyr Phe Asn Lys Ala
           115                 120                 125
   Met Asn
       130
<210> 33
   <211> 462
   <212> DNA
   <213> Staphylococcus aureus
<220>
   <221> CDS
   <222> (1)..(462)
<400> 33
   gca gaa att aat aaa caa aca aca tca caa ggt gtc aca act gaa aaa 48
   Ala Glu Ile Asn Lys Gln Thr Thr Ser Gln Gly Val Thr Thr Glu Lys
   1               5                   10                  15
   aat aat ggt atc gca gtg tta gaa caa gat gtg att aca cca aca gtt 96
   Asn Asn Gly Ile Ala Val Leu Glu Gln Asp Val Ile Thr Pro Thr Val
               20                  25                  30
   aaa cct caa gcg aaa caa gat att atc caa gca gtt aca act cgt aaa 144
   Lys Pro Gln Ala Lys Gln Asp Ile Ile Gln Ala Val Thr Thr Arg Lys
           35                  40                  45
   caa caa att aaa aag tca aat gca tca tta caa gat gaa aaa gat gta        192
   Gln Gln Ile Lys Lys Ser Asn Ala Ser Leu Gln Asp Glu Lys Asp Val
       50                  55                  60
   gca aat gat aaa att ggt aaa att gaa aca aag gca att aaa gat att        240
   Ala Asn Asp Lys Ile Gly Lys Ile Glu Thr Lys Ala Ile Lys Asp Ile
   65                  70                  75                  80
   gat gca gca aca aca aat gca caa gta gaa gcc att aaa aca aaa gca        288
   Asp Ala Ala Thr Thr Asn Ala Gln Val Glu Ala Ile Lys Thr Lys Ala
                   85                  90                  95
   atc aat gat att aat caa act aca cct gct aca aca gct aaa gca gca        336
   Ile Asn Asp Ile Asn Gln Thr Thr Pro Ala Thr Thr Ala Lys Ala Ala
               100                 105                 110
   gct ctt gaa gaa ttt gac gaa gtt gtt caa gca caa att gat caa gca        384
   Ala Leu Glu Glu Phe Asp Glu Val Val Gln Ala Gln Ile Asp Gln Ala
           115                 120                 125
   cct tta aat cct gat aca aca aat gaa gaa gta gcg gaa gct att gaa        432
   Pro Leu Asn Pro Asp Thr Thr Asn Glu Glu Val Ala Glu Ala Ile Glu
       130                 135                 140
   cgt att aat gca gct aaa gtt tct ggt gtt                                462
   Arg Ile Asn Ala Ala Lys Val Ser Gly Val
   145                 150
<210> 34
   <211> 154
   <212> PRT
   <213> Staphylococcus aureus
<400> 34
   Ala Glu Ile Asn Lys Gln Thr Thr Ser Gln Gly Val Thr Thr Glu Lys
   1               5                   10                  15
   Asn Asn Gly Ile Ala Val Leu Glu Gln Asp Val Ile Thr Pro Thr Val
               20                  25                  30
   Lys Pro Gln Ala Lys Gln Asp Ile Ile Gln Ala Val Thr Thr Arg Lys
           35                  40                  45
   Gln Gln Ile Lys Lys Ser Asn Ala Ser Leu Gln Asp Glu Lys Asp Val
       50                  55                  60
   Ala Asn Asp Lys Ile Gly Lys Ile Glu Thr Lys Ala Ile Lys Asp Ile
   65                  70                  75                  80
   Asp Ala Ala Thr Thr Asn Ala Gln Val Glu Ala Ile Lys Thr Lys Ala
                   85                  90                  95
   Ile Asn Asp Ile Asn Gln Thr Thr Pro Ala Thr Thr Ala Lys Ala Ala
               100                 105                 110
   Ala Leu Glu Glu Phe Asp Glu Val Val Gln Ala Gln Ile Asp Gln Ala
           115                 120                 125
   Pro Leu Asn Pro Asp Thr Thr Asn Glu Glu Val Ala Glu Ala Ile Glu
       130                 135                 140
   Arg Ile Asn Ala Ala Lys Val Ser Gly Val
   145                 150
<210> 35
   <211> 2319
   <212> DNA
   <213> Staphylococcus aureus
<400> 35
   atgaaagctt tattacttaa aacaagtgta tggctcgttt tgctttttag tgtaatggga      60
   ttatggcaag tctcgaacgc ggctgagcag catacaccaa tgaaagcaca tgcagtaaca     120
   acgatagaca aagcaacaac agataagcaa caagtaccgc caacaaagga agcggctcat     180
   cattctggca aagaagcggc aaccaacgta tcagcatcag cgcagggaac agctgatgat     240
   acaaacagca aagtaacatc caacgcacca tctaacaaac catctacagt agtttcaaca     300
   aaagtaaacg aaacacgcga cgtagataca caacaagcct caacacaaaa accaactcac     360
   acagcaacgt tcaaattatc aaatgctaaa acagcatcac tttcaccacg aatgtttgct     420
   gctaatgcac cacaaacaac aacacataaa atattacata caaatgatat ccatggccga     480
   ctagccgaag aaaaagggcg tgtcatcggt atggctaaat taaaaacagt aaaagaacaa     540
   gaaaagcctg atttaatgtt agacgcagga gacgccttcc aaggtttacc actttcaaac     600
   cagtctaaag gtgaagaaat ggctaaagca atgaatgcag taggttatga tgctatggca     660
   gtcggtaacc atgaatttga ctttggatac gatcagttga aaaagttaga gggtatgtta     720
   gacttcccga tgctaagtac taacgtttat aaagatggaa aacgcgcgtt taagccttca     780
   acgattgtaa caaaaaatgg tattcgttat ggaattattg gtgtaacgac accagaaaca     840
   aagacgaaaa caagacctga aggcattaaa ggcgttgaat ttagagatcc attacaaagt     900
   gtgacagcgg aaatgatgcg tatttataaa gacgtagata catttgttgt tatatcacat     960
   ttaggaattg atccttcaac acaagaaaca tggcgtggtg attacttagt gaaacaatta    1020
   agtcaaaatc cacaattgaa gaaacgtatt acagttattg atggtcattc acatacagta    1080
   cttcaaaatg gtcaaattta taacaatgat gcattggcac aaacaggtac agcacttgcg    1140
   aatatcggta agattacatt taattatcgc aatggagagg tatcgaatat taaaccgtca    1200
   ttgattaatg ttaaagacgt tgaaaatgta acaccgaaca aagcattagc tgaacaaatt    1260
   aatcaagctg atcaaacatt tagagcacaa actgcagagg taattattcc aaacaatacc   1320
   attgatttca aaggagaaag agatgacgtt agaacgcgtg aaacaaattt aggaaacgcg   1380
   attgcagatg ctatggaagc gtatggcgtt aagaatttct ctaaaaagac tgactttgcc   1440
   gtgacaaatg gtggaggtat tcgtgcctct atcgcaaaag gtaaggtgac acgctatgat   1500
   ttaatctcag tattaccatt tggaaatacg attgcgcaaa ttgatgtaaa aggttcagac   1560
   gtctggacgg ctttcgaaca tagtttaggc gcaccaacaa cacaaaagga cggtaagaca   1620
   gtgttaacag cgaatggcgg tttactacat atctctgatt caatccgtgt ttactatgat   1680
   ataaataaac cgtctggcaa acgaattaat gctattcaaa ttttaaataa agagacaggt   1740
   aagtttgaaa atattgattt aaaacgtgta tatcacgtaa cgatgaatga cttcacagca   1800
   tcaggtggcg acggatatag tatgttcggt ggtcctagag aagaaggtat ttcattagat   1860
   caagtactag caagttattt aaaaacagct aacttagcta agtatgatac gacagaacca   1920
   caacgtatgt tattaggtaa accagcagta agtgaacaac cagctaaagg acaacaaggt   1980
   agcaaaggta gtaagtctgg taaagataca caaccaattg gtgacgacaa agtgatggat   2040
   ccagcgaaaa aaccagctcc aggtaaagtt gtattgttgc tagcgcatag aggaactgtt   2100
   agtagcggta cagaaggttc tggtcgcaca atagaaggag ctactgtatc aagcaagagt   2160
   gggaaacaat tggctagaat gtcagtgcct aaaggtagcg cgcatgagaa acagttacca   2220
   aaaactggaa ctaatcaaag ttcaagccca gaagcgatgt ttgtattatt agcaggtata   2280
   ggtttaatcg cgactgtacg acgtagaaaa gctagctaa                          2319
<210> 36
   <211> 745
   <212> PRT
   <213> Staphylococcus aureus
<400> 36
   Ala Glu Gln His Thr Pro Met Lys Ala His Ala Val Thr Thr Ile Asp
   1               5                   10                  15
   Lys Ala Thr Thr Asp Lys Gln Gln Val Pro Pro Thr Lys Glu Ala Ala
               20                  25                  30
   His His Ser Gly Lys Glu Ala Ala Thr Asn Val Ser Ala Ser Ala Gln
           35                  40                  45
   Gly Thr Ala Asp Asp Thr Asn Ser Lys Val Thr Ser Asn Ala Pro Ser
       50                  55                  60
   Asn Lys Pro Ser Thr Val Val Ser Thr Lys Val Asn Glu Thr Arg Asp
   65                  70                  75                  80
   Val Asp Thr Gln Gln Ala Ser Thr Gln Lys Pro Thr His Thr Ala Thr
                   85                  90                  95
   Phe Lys Leu Ser Asn Ala Lys Thr Ala Ser Leu Ser Pro Arg Met Phe
               100                 105                 110
   Ala Ala Asn Ala Pro Gln Thr Thr Thr His Lys Ile Leu His Thr Asn
           115                 120                 125
   Asp Ile His Gly Arg Leu Ala Glu Glu Lys Gly Arg Val Ile Gly Met
       130                 135                 140
   Ala Lys Leu Lys Thr Val Lys Glu Gln Glu Lys Pro Asp Leu Met Leu
   145                 150                 155                 160
   Asp Ala Gly Asp Ala Phe Gln Gly Leu Pro Leu Ser Asn Gln Ser Lys
                   165                 170                 175
   Gly Glu Glu Met Ala Lys Ala Met Asn Ala Val Gly Tyr Asp Ala Met
               180                 185                 190
   Ala Val Gly Asn His Glu Phe Asp Phe Gly Tyr Asp Gln Leu Lys Lys
           195                 200                 205
   Leu Glu Gly Met Leu Asp Phe Pro Met Leu Ser Thr Asn Val Tyr Lys
       210                 215                 220
   Asp Gly Lys Arg Ala Phe Lys Pro Ser Thr Ile Val Thr Lys Asn Gly
   225                 230                 235                 240
   Ile Arg Tyr Gly Ile Ile Gly Val Thr Thr Pro Glu Thr Lys Thr Lys
                   245                 250                 255
   Thr Arg Pro Glu Gly Ile Lys Gly Val Glu Phe Arg Asp Pro Leu Gln
               260                 265                 270
   Ser Val Thr Ala Glu Met Met Arg Ile Tyr Lys Asp Val Asp Thr Phe
           275                 280                 285
   Val Val Ile Ser His Leu Gly Ile Asp Pro Ser Thr Gln Glu Thr Trp
       290                 295                 300
   Arg Gly Asp Tyr Leu Val Lys Gln Leu Ser Gln Asn Pro Gln Leu Lys
   305                 310                 315                 320
   Lys Arg Ile Thr Val Ile Asp Gly His Ser His Thr Val Leu Gln Asn
                   325                 330                 335
   Gly Gln Ile Tyr Asn Asn Asp Ala Leu Ala Gln Thr Gly Thr Ala Leu
               340                 345                 350
   Ala Asn Ile Gly Lys Ile Thr Phe Asn Tyr Arg Asn Gly Glu Val Ser
           355                 360                 365
   Asn Ile Lys Pro Ser Leu Ile Asn Val Lys Asp Val Glu Asn Val Thr
       370                 375                 380
   Pro Asn Lys Ala Leu Ala Glu Gln Ile Asn Gln Ala Asp Gln Thr Phe
   385                 390                 395                 400
   Arg Ala Gln Thr Ala Glu Val Ile Ile Pro Asn Asn Thr Ile Asp Phe
                   405                 410                 415
   Lys Gly Glu Arg Asp Asp Val Arg Thr Arg Glu Thr Asn Leu Gly Asn
               420                 425                 430
   Ala Ile Ala Asp Ala Met Glu Ala Tyr Gly Val Lys Asn Phe Ser Lys
           435                 440                 445
   Lys Thr Asp Phe Ala Val Thr Asn Gly Gly Gly Ile Arg Ala Ser Ile
       450                 455                 460
   Ala Lys Gly Lys Val Thr Arg Tyr Asp Leu Ile Ser Val Leu Pro Phe
   465                 470                 475                 480
   Gly Asn Thr Ile Ala Gln Ile Asp Val Lys Gly Ser Asp Val Trp Thr
                   485                 490                 495
   Ala Phe Glu His Ser Leu Gly Ala Pro Thr Thr Gln Lys Asp Gly Lys
               500                 505                 510
   Thr Val Leu Thr Ala Asn Gly Gly Leu Leu His Ile Ser Asp Ser Ile
           515                 520                 525
   Arg Val Tyr Tyr Asp Ile Asn Lys Pro Ser Gly Lys Arg Ile Asn Ala
       530                 535                 540
   Ile Gln Ile Leu Asn Lys Glu Thr Gly Lys Phe Glu Asn Ile Asp Leu
   545                 550                 555                 560
   Lys Arg Val Tyr His Val Thr Met Asn Asp Phe Thr Ala Ser Gly Gly
                   565                 570                 575
   Asp Gly Tyr Ser Met Phe Gly Gly Pro Arg Glu Glu Gly Ile Ser Leu
               580                 585                 590
   Asp Gln Val Leu Ala Ser Tyr Leu Lys Thr Ala Asn Leu Ala Lys Tyr
           595                 600                 605
   Asp Thr Thr Glu Pro Gln Arg Met Leu Leu Gly Lys Pro Ala Val Ser
       610                 615                 620
   Glu Gln Pro Ala Lys Gly Gln Gln Gly Ser Lys Gly Ser Lys Ser Gly
   625                 630                 635                 640
   Lys Asp Thr Gln Pro Ile Gly Asp Asp Lys Val Met Asp Pro Ala Lys
                   645                 650                 655
   Lys Pro Ala Pro Gly Lys Val Val Leu Leu Leu Ala His Arg Gly Thr
               660                 665                 670
   Val Ser Ser Gly Thr Glu Gly Ser Gly Arg Thr Ile Glu Gly Ala Thr
           675                 680                 685
   Val Ser Ser Lys Ser Gly Lys Gln Leu Ala Arg Met Ser Val Pro Lys
       690                 695                 700
   Gly Ser Ala His Glu Lys Gln Leu Pro Lys Thr Gly Thr Asn Gln Ser
   705                 710                 715                 720
   Ser Ser Pro Glu Ala Met Phe Val Leu Leu Ala Gly Ile Gly Leu Ile
                   725                 730                 735
   Ala Thr Val Arg Arg Arg Lys Ala Ser
               740                 745
<210> 37
   <211> 322
   <212> PRT
   <213> Staphylococcus sp.
<400> 37
   Met Met Lys Met Lys Thr Arg Ile Val Ser Ser Val Thr Thr Thr Leu
   1               5                   10                  15
   Leu Leu Gly Ser Ile Leu Met Asn Pro Val Ala Asn Ala Ala Asp Ser
               20                  25                  30
   Asp Ile Asn Ile Lys Thr Gly Thr Thr Asp Ile Gly Ser Asn Thr Thr
           35                  40                  45
   Val Lys Thr Gly Asp Leu Val Thr Tyr Asp Lys Glu Asn Gly Met His
       50                  55                  60
   Lys Lys Val Phe Tyr Ser Phe Ile Asp Asp Lys Asn His Asn Lys Lys
   65                  70                  75                  80
   Ile Leu Val Ile Arg Thr Lys Gly Thr Ile Ala Gly Gln Tyr Arg Val
                   85                  90                  95
   Tyr Ser Glu Glu Gly Ala Asn Lys Ser Gly Leu Ala Trp Pro Ser Ala
               100                 105                 110
   Phe Lys Val Gln Leu Gln Leu Pro Asp Asn Glu Val Ala Gln Ile Ser
           115                 120                 125
   Asp Tyr Tyr Pro Arg Asn Ser Ile Asp Thr Lys Glu Tyr Met Ser Thr
       130                 135                 140
   Leu Thr Tyr Gly Phe Asn Gly Asn Val Thr Gly Asp Asp Ser Gly Lys
   145                 150                 155                 160
   Ile Gly Gly Leu Ile Gly Ala Asn Val Ser Ile Gly His Thr Leu Lys
                   165                 170                 175
   Tyr Val Gln Pro Asp Phe Lys Thr Ile Leu Glu Ser Pro Thr Asp Lys
               180                 185                 190
   Lys Val Gly Trp Lys Val Ile Phe Asn Asn Met Val Asn Gln Asn Trp
           195                 200                 205
   Gly Pro Tyr Asp Arg Asp Ser Trp Asn Pro Val Tyr Gly Asn Gln Leu
       210                 215                 220
   Phe Met Lys Thr Arg Asn Gly Ser Met Lys Ala Ala Glu Asn Phe Leu
   225                 230                 235                 240
   Asp Pro Asn Lys Ala Ser Ser Leu Leu Ser Ser Gly Phe Ser Pro Asp
                   245                 250                 255
   Phe Ala Thr Val Ile Thr Met Asp Arg Lys Ala Thr Lys Gln Gln Thr
               260                 265                 270
   Asn Ile Asp Val Ile Tyr Glu Arg Val Arg Asp Asp Tyr Gln Leu His
           275                 280                 285
   Trp Thr Ser Thr Asn Trp Lys Gly Thr Asn Thr Lys Asp Lys Trp Thr
       290                 295                 300
   Asp Arg Ser Ser Glu Arg Tyr Lys Ile Asp Trp Glu Lys Glu Glu Met
   305 310 315 320
   Thr Asn
<210> 38
   <211> 10419
   <212> PRT
   <213> Staphylococcus sp.
<400> 38
   Met Asn Tyr Arg Asp Lys Ile Gln Lys Phe Ser Ile Arg Lys Tyr Thr
   1               5                   10                  15
   Val Gly Thr Phe Ser Thr Val Ile Ala Thr Leu Val Phe Leu Gly Phe
               20                  25                  30
   Asn Thr Ser Gln Ala His Ala Ala Glu Thr Asn Gln Pro Ala Ser Val
           35                  40                  45
   Val Lys Gln Lys Gln Gln Ser Asn Asn Glu Gln Thr Glu Asn Arg Glu
       50                  55                  60
   Ser Gln Val Gln Asn Ser Gln Asn Ser Gln Asn Gly Gln Ser Leu Ser
   65                  70                  75                  80
   Ala Thr His Glu Asn Glu Gln Pro Asn Ile Ser Gln Ala Asn Leu Val
                   85                  90                  95
   Asp Gln Lys Val Ala Gln Ser Ser Thr Thr Asn Asp Glu Gln Pro Ala
               100                 105                 110
   Ser Gln Asn Val Asn Thr Lys Lys Asp Ser Ala Thr Ala Ala Thr Thr
           115                 120                 125
   Gln Pro Asp Lys Glu Gln Ser Lys His Lys Gln Asn Glu Ser Gln Ser
       130                 135                 140
   Ala Asn Lys Asn Gly Asn Asp Asn Arg Ala Ala His Val Glu Asn His
   145                 150                 155                 160
   Glu Ala Asn Val Val Thr Ala Ser Asp Ser Ser Asp Asn Gly Asn Val
                   165                 170                 175
   Gln His Asp Arg Asn Glu Leu Gln Ala Phe Phe Asp Ala Asn Tyr His
               180                 185                 190
   Asp Tyr Arg Phe Ile Asp Arg Glu Asn Ala Asp Ser Gly Thr Phe Asn
           195                 200                 205
   Tyr Val Lys Gly Ile Phe Asp Lys Ile Asn Thr Leu Leu Gly Ser Asn
       210                 215                 220
   Asp Pro Ile Asn Asn Lys Asp Leu Gln Leu Ala Tyr Lys Glu Leu Glu
   225                 230                 235                 240
   Gln Ala Val Ala Leu Ile Arg Thr Met Pro Gln Arg Gln Gln Thr Ser
                   245                 250                 255
   Arg Arg Ser Asn Arg Ile Gln Thr Arg Ser Val Glu Ser Arg Ala Ala
               260                 265                 270
   Glu Pro Arg Ser Val Ser Asp Tyr Gln Asn Ala Asn Ser Ser Tyr Tyr
           275                 280                 285
   Val Glu Asn Ala Asn Asp Gly Ser Gly Tyr Pro Val Gly Thr Tyr Ile
       290                 295                 300
   Asn Ala Ser Ser Lys Gly Ala Pro Tyr Asn Leu Pro Thr Thr Pro Trp
   305                 310                 315                 320
   Asn Thr Leu Lys Ala Ser Asp Ser Lys Glu Ile Ala Leu Met Thr Ala
                   325                 330                 335
   Lys Gln Thr Gly Asp Gly Tyr Gln Trp Val Ile Lys Phe Asn Lys Gly
               340                 345                 350
   His Ala Pro His Gln Asn Met Ile Phe Trp Phe Ala Leu Pro Ala Asp
           355                 360                 365
   Gln Val Pro Val Gly Arg Thr Asp Phe Val Thr Val Asn Ser Asp Gly
       370                 375                 380
   Thr Asn Val Gln Trp Ser His Gly Ala Gly Ala Gly Ala Asn Lys Pro
   385                 390                 395                 400
   Leu Gln Gln Met Trp Glu Tyr Gly Val Asn Asp Pro His Arg Ser His
                   405                 410                 415
   Asp Phe Lys Ile Arg Asn Arg Ser Gly Gln Val Ile Tyr Asp Trp Pro
               420                 425                 430
   Thr Val His Ile Tyr Ser Leu Glu Asp Leu Ser Arg Ala Ser Asp Tyr
           435                 440                 445
   Phe Ser Glu Ala Gly Ala Thr Pro Ala Thr Lys Ala Phe Gly Arg Gln
       450                 455                 460
   Asn Phe Glu Tyr Ile Asn Gly Gln Lys Pro Ala Glu Ser Pro Gly Val
   465                 470                 475                 480
   Pro Lys Val Tyr Thr Phe Ile Gly Gln Gly Asp Ala Ser Tyr Thr Ile
                   485                 490                 495
   Ser Phe Lys Thr Gln Gly Pro Thr Val Asn Lys Leu Tyr Tyr Ala Ala
               500                 505                 510
   Gly Gly Arg Ala Leu Glu Tyr Asn Gln Leu Phe Met Tyr Ser Gln Leu
           515                 520                 525
   Tyr Val Glu Ser Thr Gln Asp His Gln Gln Arg Leu Asn Gly Leu Arg
       530                 535                 540
   Gln Val Val Asn Arg Thr Tyr Arg Ile Gly Thr Thr Lys Arg Val Glu
   545                 550                 555                 560
   Val Ser Gln Gly Asn Val Gln Thr Lys Lys Val Leu Glu Ser Thr Asn
                   565                 570                 575
   Leu Asn Ile Asp Asp Phe Val Asp Asp Pro Leu Ser Tyr Val Lys Thr
               580                 585                 590
   Pro Ser Asn Lys Val Leu Gly Phe Tyr Ser Asn Asn Ala Asn Thr Asn
           595                 600                 605
   Ala Phe Arg Pro Gly Gly Ala Gln Gln Leu Asn Glu Tyr Gln Leu Ser
       610                 615                 620
   Gln Leu Phe Thr Asp Gln Lys Leu Gln Glu Ala Ala Arg Thr Arg Asn
   625                 630                 635                 640
   Pro Ile Arg Leu Met Ile Gly Phe Asp Tyr Pro Asp Ala Tyr Gly Asn
                   645                 650                 655
   Ser Glu Thr Leu Val Pro Val Asn Leu Thr Val Leu Pro Glu Ile Gln
               660                 665                 670
   His Asn Ile Lys Phe Phe Lys Asn Asp Asp Thr Gln Asn Ile Ala Glu
           675                 680                 685
   Lys Pro Phe Ser Lys Gln Ala Gly His Pro Val Phe Tyr Val Tyr Ala
       690                 695                 700
   Gly Asn Gln Gly Asn Ala Ser Val Asn Leu Gly Gly Ser Val Thr Ser
   705                 710                 715                 720
   Ile Gln Pro Leu Arg Ile Asn Leu Thr Ser Asn Glu Asn Phe Thr Asp
                   725                 730                 735
   Lys Asp Trp Gln Ile Thr Gly Ile Pro Arg Thr Leu His Ile Glu Asn
               740                 745                 750
   Ser Thr Asn Arg Pro Asn Asn Ala Arg Glu Arg Asn Ile Glu Leu Val
           755                 760                 765
   Gly Asn Leu Leu Pro Gly Asp Tyr Phe Gly Thr Ile Arg Phe Gly Arg
       770                 775                 780
   Lys Glu Gln Leu Phe Glu Ile Arg Val Lys Pro His Thr Pro Thr Ile
   785                 790                 795                 800
   Thr Thr Thr Ala Glu Gln Leu Arg Gly Thr Ala Leu Gln Lys Val Pro
                   805                 810                 815
   Val Asn Ile Ser Gly Ile Pro Leu Asp Pro Ser Ala Leu Val Tyr Leu
               820                 825                 830
   Val Ala Pro Thr Asn Gln Thr Thr Asn Gly Gly Ser Glu Ala Asp Gln
           835                 840                 845
   Ile Pro Ser Gly Tyr Thr Ile Leu Ala Thr Gly Thr Pro Asp Gly Val
       850                 855                 860
   His Asn Thr Ile Thr Ile Arg Pro Gln Asp Tyr Val Val Phe Ile Pro
   865                 870                 875                 880
   Pro Val Gly Lys Gln Ile Arg Ala Val Val Tyr Tyr Asn Lys Val Val
                   885                 890                 895
   Ala Ser Asn Met Ser Asn Ala Val Thr Ile Leu Pro Asp Asp Ile Pro
               900                 905                 910
   Pro Thr Ile Asn Asn Pro Val Gly Ile Asn Ala Lys Tyr Tyr Arg Gly
           915                 920                 925
   Asp Glu Val Asn Phe Thr Met Gly Val Ser Asp Arg His Ser Gly Ile
       930                 935                 940
   Lys Asn Thr Thr Ile Thr Thr Leu Pro Asn Gly Trp Thr Ser Asn Leu
   945                 950                 955                 960
   Thr Lys Ala Asp Lys Asn Asn Gly Ser Leu Ser Ile Thr Gly Arg Val
                   965                 970                 975
   Ser Met Asn Gln Ala Phe Asn Ser Asp Ile Thr Phe Lys Val Ser Ala
               980                 985                 990
   Thr Asp Asn Val Asn Asn Thr Thr  Asn Asp Ser Gln Ser  Lys His Val
           995                 1000                 1005
   Ser Ile  His Val Gly Lys Ile  Ser Glu Asp Ala His  Pro Ile Val
       1010                 1015                 1020
   Leu Gly  Asn Thr Glu Lys Val  Val Val Val Asn Pro  Thr Ala Val
       1025                 1030                 1035
   Ser Asn  Asp Glu Lys Gln Ser  Ile Ile Thr Ala Phe  Met Asn Lys
       1040                 1045                 1050
   Asn Gln  Asn Ile Arg Gly Tyr  Leu Ala Ser Thr Asp  Pro Val Thr
       1055                 1060                 1065
   Val Asp  Asn Asn Gly Asn Val  Thr Leu His Tyr Arg  Asp Gly Ser
       1070                 1075                 1080
   Ser Thr  Thr Leu Asp Ala Thr  Asn Val Met Thr Tyr  Glu Pro Val
       1085                 1090                 1095
   Val Lys  Pro Glu Tyr Gln Thr  Val Asn Ala Ala Lys  Thr Ala Thr
       1100                 1105                 1110
   Val Thr  Ile Ala Lys Gly Gln  Ser Phe Ser Ile Gly  Asp Ile Lys
       1115                 1120                 1125
   Gln Tyr  Phe Thr Leu Ser Asn  Gly Gln Pro Ile Pro  Ser Gly Thr
       1130                 1135                 1140
   Phe Thr  Asn Ile Thr Ser Asp  Arg Thr Ile Pro Thr  Ala Gln Glu
       1145                 1150                 1155
   Val Ser  Gln Met Asn Ala Gly  Thr Gln Leu Tyr His  Ile Thr Ala
       1160                 1165                 1170
   Thr Asn  Ala Tyr His Lys Asp  Ser Glu Asp Phe Tyr  Ile Ser Leu
       1175                 1180                 1185
   Lys Ile  Ile Asp Val Lys Gln  Pro Glu Gly Asp Gln  Arg Val Tyr
   1190 1195 1200
   Arg Thr Ser Thr Tyr Asp Leu Thr Thr Asp Glu Ile Ser Lys Val
   1205 1210 1215
   Lys Gln Ala Phe Ile Asn Ala Asn Arg Asp Val Ile Thr Leu Ala
   1220 1225 1230
   Glu Gly Asp Ile Ser Val Thr Asn Thr Pro Asn Gly Ala Asn Val
   1235 1240 1245
   Ser Thr Ile Thr Val Asn Ile Asn Lys Gly Arg Leu Thr Lys Ser
   1250 1255 1260
   Phe Ala Ser Asn Leu Ala Asn Met Asn Phe Leu Arg Trp Val Asn
   1265 1270 1275
   Phe Pro Gln Asp Tyr Thr Val Thr Trp Thr Asn Ala Lys Ile Ala
   1280 1285 1290
   Asn Arg Pro Thr Asp Gly Gly Leu Ser Trp Ser Asp Asp His Lys
   1295 1300 1305
   Ser Leu Ile Tyr Arg Tyr Asp Ala Thr Leu Gly Thr Gln Ile Thr
   1310 1315 1320
   Thr Asn Asp Ile Leu Thr Met Leu Lys Ala Thr Thr Thr Val Pro
   1325 1330 1335
   Gly Leu Arg Asn Asn Ile Thr Gly Asn Glu Lys Ser Gln Ala Glu
   1340 1345 1350
   Ala Gly Gly Arg Pro Asn Phe Arg Thr Thr Gly Tyr Ser Gln Ser
   1355 1360 1365
   Asn Ala Thr Thr Asp Gly Gln Arg Gln Phe Thr Leu Asn Gly Gln
   1370 1375 1380
   Val Ile Gln Val Leu Asp Ile Ile Asn Pro Ser Asn Gly Tyr Gly
   1385 1390 1395
   Gly Gln Pro Val Thr Asn Ser Asn Thr Arg Ala Asn His Ser Asn
   1400 1405 1410
   Ser Thr Val Val Asn Val Asn Glu Pro Ala Ala Asn Gly Ala Gly
   1415 1420 1425
   Ala Phe Thr Ile Asp His Val Val Lys Ser Asn Ser Thr His Asn
   1430 1435 1440
   Ala Ser Asp Ala Val Tyr Lys Ala Gln Leu Tyr Leu Thr Pro Tyr
   1445 1450 1455
   Gly Pro Lys Gln Tyr Val Glu His Leu Asn Gln Asn Thr Gly Asn
   1460 1465 1470
   Thr Thr Asp Ala Ile Asn Ile Tyr Phe Val Pro Ser Asp Leu Val
   1475 1480 1485
   Asn Pro Thr Ile Ser Val Gly Asn Tyr Thr Asn His Gln Val Phe
   1490 1495 1500
   Ser Gly Glu Thr Phe Thr Asn Thr Ile Thr Ala Asn Asp Asn Phe
   1505 1510 1515
   Gly Val Gln Ser Val Thr Val Pro Asn Thr Ser Gln Ile Thr Gly
   1520 1525 1530
   Thr Val Asp Asn Asn His Gln His Val Ser Ala Thr Ala Pro Asn
   1535 1540 1545
   Val Thr Ser Ala Thr Asn Lys Thr Ile Asn Leu Leu Ala Thr Asp
   1550 1555 1560
   Thr Ser Gly Asn Thr Ala Thr Thr Ser Phe Asn Val Thr Val Lys
   1565 1570 1575
   Pro Leu Arg Asp Lys Tyr Arg Val Gly Thr Ser Ser Thr Ala Ala
   1580 1585 1590
   Asn Pro Val Arg Ile Ala Asn Ile Ser Asn Asn Ala Thr Val Ser
   1595 1600 1605
   Gln Ala Asp Gln Thr Thr Ile Ile Asn Ser Leu Thr Phe Thr Glu
   1610 1615 1620
   Thr Val Pro Asn Arg Ser Tyr Ala Arg Ala Ser Ala Asn Glu Ile
   1625 1630 1635
   Thr Ser Lys Thr Val Ser Asn Val Ser Arg Thr Gly Asn Asn Ala
   1640 1645 1650
   Asn Val Thr Val Thr Val Thr Tyr Gln Asp Gly Thr Thr Ser Thr
   1655 1660 1665
   Val Thr Val Pro Val Lys His Val Ile Pro Glu Ile Val Ala His
       1670                1675                1680
   Ser His Tyr Thr Val Gln Gly Gln Asp Phe Pro Ala Gly Asn Gly
       1685                1690                1695
   Ser Ser Ala Ser Asp Tyr Phe Lys Leu Ser Asn Gly Ser Asp Ile
       1700                1705                1710
   Ala Asp Ala Thr Ile Thr Trp Val Ser Gly Gln Ala Pro Asn Lys
       1715                1720                1725
   Asp Asn Thr Arg Ile Gly Glu Asp Ile Thr Val Thr Ala His Ile
       1730                1735                1740
   Leu Ile Asp Gly Glu Thr Thr Pro Ile Thr Lys Thr Ala Thr Tyr
       1745                1750                1755
   Lys Val Val Arg Thr Val Pro Lys His Val Phe Glu Thr Ala Arg
       1760                1765                1770
   Gly Val Leu Tyr Pro Gly Val Ser Asp Met Tyr Asp Ala Lys Gln
       1775                1780                1785
   Tyr Val Lys Pro Val Asn Asn Ser Trp Ser Thr Asn Ala Gln His
       1790                1795                1800
   Met Asn Phe Gln Phe Val Gly Thr Tyr Gly Pro Asn Lys Asp Val
       1805                1810                1815
   Val Gly Ile Ser Thr Arg Leu Ile Arg Val Thr Tyr Asp Asn Arg
       1820                1825                1830
   Gln Thr Glu Asp Leu Thr Ile Leu Ser Lys Val Lys Pro Asp Pro
       1835                1840                1845
   Pro Arg Ile Asp Ala Asn Ser Val Thr Tyr Lys Ala Gly Leu Thr
       1850                1855                1860
   Asn Gln Glu Ile Lys Val Asn Asn Val Leu Asn Asn Ser Ser Val
       1865                1870                1875
   Lys Leu Phe Lys Ala Asp Asn Thr Pro Leu Asn Val Thr Asn Ile
       1880                1885                1890
   Thr His Gly Ser Gly Phe Ser Ser Val Val Thr Val Ser Asp Ala
       1895                1900                1905
   Leu Pro Asn Gly Gly Ile Lys Ala Lys Ser Ser Ile Ser Met Asn
   1910 1915 1920
   Asn Val Thr Tyr Thr Thr Gln Asp Glu His Gly Gln Val Val Thr
   1925 1930 1935
   Val Thr Arg Asn Glu Ser Val Asp Ser Asn Asp Ser Ala Thr Val
   1940 1945 1950
   Thr Val Thr Pro Gln Leu Gln Ala Thr Thr Glu Gly Ala Val Phe
   1955 1960 1965
   Ile Lys Gly Gly Asp Gly Phe Asp Phe Gly His Val Glu Arg Phe
   1970 1975 1980
   Ile Gln Asn Pro Pro His Gly Ala Thr Val Ala Trp His Asp Ser
   1985 1990 1995
   Pro Asp Thr Trp Lys Asn Thr Val Gly Asn Thr His Lys Thr Ala
   2000 2005 2010
   Val Val Thr Leu Pro Asn Gly Gln Gly Thr Arg Asn Val Glu Val
   2015 2020 2025
   Pro Val Lys Val Tyr Pro Val Ala Asn Ala Lys Ala Pro Ser Arg
   2030 2035 2040
   Asp Val Lys Gly Gln Asn Leu Thr Asn Gly Thr Asp Ala Met Asn
   2045 2050 2055
   Tyr Ile Thr Phe Asp Pro Asn Thr Asn Thr Asn Gly Ile Thr Ala
   2060 2065 2070
   Ala Trp Ala Asn Arg Gln Gln Pro Asn Asn Gln Gln Ala Gly Val
   2075 2080 2085
   Gln His Leu Asn Val Asp Val Thr Tyr Pro Gly Ile Ser Ala Ala
   2090 2095 2100
   Lys Arg Val Pro Val Thr Val Asn Val Tyr Gln Phe Glu Phe Pro
   2105 2110 2115
   Gln Thr Thr Tyr Thr Thr Thr Val Gly Gly Thr Leu Ala Ser Gly
   2120 2125 2130
   Thr Gln Ala Ser Gly Tyr Ala His Met Gln Asn Ala Thr Gly Leu
   2135 2140 2145
   Pro Thr Asp Gly Phe Thr Tyr Lys Trp Asn Arg Asp Thr Thr Gly
   2150 2155 2160
   Thr Asn Asp Ala Asn Trp Ser Ala Met Asn Lys Pro Asn Val Ala
   2165 2170 2175
   Lys Val Val Asn Ala Lys Tyr Asp Val Ile Tyr Asn Gly His Thr
   2180 2185 2190
   Phe Ala Thr Ser Leu Pro Ala Lys Phe Val Val Lys Asp Val Gln
   2195 2200 2205
   Pro Ala Lys Pro Thr Val Thr Glu Thr Ala Ala Gly Ala Ile Thr
   2210 2215 2220
   Ile Ala Pro Gly Ala Asn Gln Thr Val Asn Thr His Ala Gly Asn
   2225 2230 2235
   Val Thr Thr Tyr Ala Asp Lys Leu Val Ile Lys Arg Asn Gly Asn
   2240 2245 2250
   Val Val Thr Thr Phe Thr Arg Arg Asn Asn Thr Ser Pro Trp Val
   2255 2260 2265
   Lys Glu Ala Ser Ala Ala Thr Val Ala Gly Ile Ala Gly Thr Asn
   2270 2275 2280
   Asn Gly Ile Thr Val Ala Ala Gly Thr Phe Asn Pro Ala Asp Thr
   2285 2290 2295
   Ile Gln Val Val Ala Thr Gln Gly Ser Gly Glu Thr Val Ser Asp
   2300 2305 2310
   Glu Gln Arg Ser Asp Asp Phe Thr Val Val Ala Pro Gln Pro Asn
   2315 2320 2325
   Gln Ala Thr Thr Lys Ile Trp Gln Asn Gly His Ile Asp Ile Thr
   2330 2335 2340
   Pro Asn Asn Pro Ser Gly His Leu Ile Asn Pro Thr Gln Ala Met
   2345 2350 2355
   Asp Ile Ala Tyr Thr Glu Lys Val Gly Asn Gly Ala Glu His Ser
   2360 2365 2370
   Lys Thr  Ile Asn Val Val Arg  Gly Gln Asn Asn Gln  Trp Thr Ile
       2375                 2380                 2385
   Ala Asn  Lys Pro Asp Tyr Val  Thr Leu Asp Ala Gln  Thr Gly Lys
       2390                 2395                 2400
   Val Thr  Phe Asn Ala Asn Thr  Ile Lys Pro Asn Ser  Ser Ile Thr
       2405                 2410                 2415
   Ile Thr  Pro Lys Ala Gly Thr  Gly His Ser Val Ser  Ser Asn Pro
       2420                 2425                 2430
   Ser Thr  Leu Thr Ala Pro Ala  Ala His Thr Val Asn  Thr Thr Glu
       2435                 2440                 2445
   Ile Val  Lys Asp Tyr Gly Ser  Asn Val Thr Ala Ala  Glu Ile Asn
       2450                 2455                 2460
   Asn Ala  Val Gln Val Ala Asn  Lys Arg Thr Ala Thr  Ile Lys Asn
       2465                 2470                 2475
   Gly Thr  Ala Met Pro Thr Asn  Leu Ala Gly Gly Ser  Thr Thr Thr
       2480                 2485                 2490
   Ile Pro  Val Thr Val Thr Tyr  Asn Asp Gly Ser Thr  Glu Glu Val
       2495                 2500                 2505
   Gln Glu  Ser Ile Phe Thr Lys  Ala Asp Lys Arg Glu  Leu Ile Thr
       2510                 2515                 2520
   Ala Lys  Asn His Leu Asp Asp  Pro Val Ser Thr Glu  Gly Lys Lys
       2525                 2530                 2535
   Pro Gly  Thr Ile Thr Gln Tyr  Asn Asn Ala Met His  Asn Ala Gln
       2540                 2545                 2550
   Gln Gln  Ile Asn Thr Ala Lys  Thr Glu Ala Gln Gln  Val Ile Asn
       2555                 2560                 2565
   Asn Glu  Arg Ala Thr Pro Gln  Gln Val Ser Asp Ala  Leu Thr Lys
       2570                 2575                 2580
   Val Arg  Ala Ala Gln Thr Lys  Ile Asp Gln Ala Lys  Ala Leu Leu
       2585                 2590                 2595
   Gln Asn  Lys Glu Asp Asn Ser  Gln Leu Val Thr Ser  Lys Asn Asn
       2600                 2605                 2610
   Leu Gln  Ser Ser Val Asn Gln  Val Pro Ser Thr Ala  Gly Met Thr
       2615                 2620                 2625
   Gln Gln  Ser Ile Asp Asn Tyr  Asn Ala Lys Lys Arg  Glu Ala Glu
       2630                 2635                 2640
   Thr Glu  Ile Thr Ala Ala Gln  Arg Val Ile Asp Asn  Gly Asp Ala
       2645                 2650                 2655
   Thr Ala  Gln Gln Ile Ser Asp  Glu Lys His Arg Val  Asp Asn Ala
       2660                 2665                 2670
   Leu Thr  Ala Leu Asn Gln Ala  Lys His Asp Leu Thr  Ala Asp Thr
       2675                 2680                 2685
   His Ala  Leu Glu Gln Ala Val  Gln Gln Leu Asn Arg  Thr Gly Thr
       2690                 2695                 2700
   Thr Thr  Gly Lys Lys Pro Ala  Ser Ile Thr Ala Tyr  Asn Asn Ser
       2705                 2710                 2715
   Ile Arg  Ala Leu Gln Ser Asp  Leu Thr Ser Ala Lys  Asn Ser Ala
       2720                 2725                 2730
   Asn Ala  Ile Ile Gln Lys Pro  Ile Arg Thr Val Gln  Glu Val Gln
       2735                 2740                 2745
   Ser Ala  Leu Thr Asn Val Asn  Arg Val Asn Glu Arg  Leu Thr Gln
       2750                 2755                 2760
   Ala Ile  Asn Gln Leu Val Pro  Leu Ala Asp Asn Ser  Ala Leu Lys
       2765                 2770                 2775
   Thr Ala  Lys Thr Lys Leu Asp  Glu Glu Ile Asn Lys  Ser Val Thr
       2780                 2785                 2790
   Thr Asp  Gly Met Thr Gln Ser  Ser Ile Gln Ala Tyr  Glu Asn Ala
       2795                 2800                 2805
   Lys Arg  Ala Gly Gln Thr Glu  Ser Thr Asn Ala Gln  Asn Val Ile
       2810                 2815                 2820
   Asn Asn  Gly Asp Ala Thr Asp  Gln Gln Ile Ala Ala  Glu Lys Thr
       2825                 2830                 2835
   Lys Val  Glu Glu Lys Tyr Asn  Ser Leu Lys Gln Ala  Ile Ala Gly
       2840                 2845                 2850
   Leu Thr Pro Asp Leu Ala Pro Leu Gln Thr Ala Lys Thr Gln Leu
   2855 2860 2865
   Gln Asn Asp Ile Asp Gln Pro Thr Ser Thr Thr Gly Met Thr Ser
   2870 2875 2880
   Ala Ser Ile Ala Ala Phe Asn Glu Lys Leu Ser Ala Ala Arg Thr
   2885 2890 2895
   Lys Ile Gln Glu Ile Asp Arg Val Leu Ala Ser His Pro Asp Val
   2900 2905 2910
   Ala Thr Ile Arg Gln Asn Val Thr Ala Ala Asn Ala Ala Lys Ser
   2915 2920 2925
   Ala Leu Asp Gln Ala Arg Asn Gly Leu Thr Val Asp Lys Ala Pro
   2930 2935 2940
   Leu Glu Asn Ala Lys Asn Gln Leu Gln His Ser Ile Asp Thr Gln
   2945 2950 2955
   Thr Ser Thr Thr Gly Met Thr Gln Asp Ser Ile Asn Ala Tyr Asn
   2960 2965 2970
   Ala Lys Leu Thr Ala Ala Arg Asn Lys Ile Gln Gln Ile Asn Gln
   2975 2980 2985
   Val Leu Ala Gly Ser Pro Thr Val Glu Gln Ile Asn Thr Asn Thr
   2990 2995 3000
   Ser Thr Ala Asn Gln Ala Lys Ser Asp Leu Asp His Ala Arg Gln
   3005 3010 3015
   Ala Leu Thr Pro Asp Lys Ala Pro Leu Gln Thr Ala Lys Thr Gln
   3020 3025 3030
   Leu Glu Gln Ser Ile Asn Gln Pro Thr Asp Thr Thr Gly Met Thr
   3035 3040 3045
   Thr Ala Ser Leu Asn Ala Tyr Asn Gln Lys Leu Gln Ala Ala Arg
   3050 3055 3060
   Gln Lys Leu Thr Glu Ile Asn Gln Val Leu Asn Gly Asn Pro Thr
   3065 3070 3075
   Val Gln Asn Ile Asn Asp Lys Val Thr Glu Ala Asn Gln Ala Lys
   3080 3085 3090
   Asp Gln Leu Asn Thr Ala Arg Gln Gly Leu Thr Leu Asp Arg Gln
   3095 3100 3105
   Pro Ala Leu Thr Thr Leu His Gly Ala Ser Asn Leu Asn Gln Ala
   3110 3115 3120
   Gln Gln Asn Asn Phe Thr Gln Gln Ile Asn Ala Ala Gln Asn His
   3125 3130 3135
   Ala Ala Leu Glu Thr Ile Lys Ser Asn Ile Thr Ala Leu Asn Thr
   3140 3145 3150
   Ala Met Thr Lys Leu Lys Asp Ser Val Ala Asp Asn Asn Thr Ile
   3155 3160 3165
   Lys Ser Asp Gln Asn Tyr Thr Asp Ala Thr Pro Ala Asn Lys Gln
   3170 3175 3180
   Ala Tyr Asp Asn Ala Val Asn Ala Ala Lys Gly Val Ile Gly Glu
   3185 3190 3195
   Thr Thr Asn Pro Thr Met Asp Val Asn Thr Val Asn Gln Lys Ala
   3200 3205 3210
   Ala Ser Val Lys Ser Thr Lys Asp Ala Leu Asp Gly Gln Gln Asn
   3215 3220 3225
   Leu Gln Arg Ala Lys Thr Glu Ala Thr Asn Ala Ile Thr His Ala
   3230 3235 3240
   Ser Asp Leu Asn Gln Ala Gln Lys Asn Ala Leu Thr Gln Gln Val
   3245 3250 3255
   Asn Ser Ala Gln Asn Val Gln Ala Val Asn Asp Ile Lys Gln Thr
   3260 3265 3270
   Thr Gln Ser Leu Asn Thr Ala Met Thr Gly Leu Lys Arg Gly Val
   3275 3280 3285
   Ala Asn His Asn Gln Val Val Gln Ser Asp Asn Tyr Val Asn Ala
   3290 3295 3300
   Asp Thr Asn Lys Lys Asn Asp Tyr Asn Asn Ala Tyr Asn His Ala
   3305 3310 3315
   Asn Asp  Ile Ile Asn Gly Asn  Ala Gln His Pro Val  Ile Thr Pro
       3320                 3325                 3330
   Ser Asp  Val Asn Asn Ala Leu  Ser Asn Val Thr Ser  Lys Glu His
       3335                 3340                 3345
   Ala Leu  Asn Gly Glu Ala Lys  Leu Asn Ala Ala Lys  Gln Glu Ala
       3350                 3355                 3360
   Asn Thr  Ala Leu Gly His Leu  Asn Asn Leu Asn Asn  Ala Gln Arg
       3365                 3370                 3375
   Gln Asn  Leu Gln Ser Gln Ile  Asn Gly Ala His Gln  Ile Asp Ala
       3380                 3385                 3390
   Val Asn  Thr Ile Lys Gln Asn  Ala Thr Asn Leu Asn  Ser Ala Met
       3395                 3400                 3405
   Gly Asn  Leu Arg Gln Ala Val  Ala Asp Lys Asp Gln  Val Lys Arg
       3410                 3415                 3420
   Thr Glu  Asp Tyr Ala Asp Ala  Asp Thr Ala Lys Gln  Asn Ala Tyr
       3425                 3430                 3435
   Asn Ser  Ala Val Ser Ser Ala  Glu Thr Ile Ile Asn  Gln Thr Thr
       3440                 3445                 3450
   Asn Pro  Thr Met Ser Val Asp  Asp Val Asn Arg Ala  Thr Ser Ala
       3455                 3460                 3465
   Val Thr  Ser Asn Lys Asn Ala  Leu Asn Gly Tyr Glu  Lys Leu Ala
       3470                 3475                 3480
   Gln Ser  Lys Thr Asp Ala Ala  Arg Ala Ile Asp Ala  Leu Pro His
       3485                 3490                 3495
   Leu Asn  Asn Ala Gln Lys Ala  Asp Val Lys Ser Lys  Ile Asn Ala
       3500                 3505                 3510
   Ala Ser  Asn Ile Ala Gly Val  Asn Thr Val Lys Gln  Gln Gly Thr
       3515                 3520                 3525
   Asp Leu  Asn Thr Ala Met Gly  Asn Leu Gln Gly Ala  Ile Asn Asp
       3530                 3535                 3540
   Glu Gln  Thr Thr Leu Asn Ser  Gln Asn Tyr Gln Asp  Ala Thr Pro
       3545                 3550                 3555
   Ser Lys Lys Thr Ala Tyr Thr Asn Ala Val Gln Ala Ala Lys Asp
   3560 3565 3570
   Ile Leu Asn Lys Ser Asn Gly Gln Asn Lys Thr Lys Asp Gln Val
   3575 3580 3585
   Thr Glu Ala Met Asn Gln Val Asn Ser Ala Lys Asn Asn Leu Asp
   3590 3595 3600
   Gly Thr Arg Leu Leu Asp Gln Ala Lys Gln Thr Ala Lys Gln Gln
   3605 3610 3615
   Leu Asn Asn Met Thr His Leu Thr Thr Ala Gln Lys Thr Asn Leu
   3620 3625 3630
   Thr Asn Gln Ile Asn Ser Gly Thr Thr Val Ala Gly Val Gln Thr
   3635 3640 3645
   Val Gln Ser Asn Ala Asn Thr Leu Asp Gln Ala Met Asn Thr Leu
   3650 3655 3660
   Arg Gln Ser Ile Ala Asn Lys Asp Ala Thr Lys Ala Ser Glu Asp
   3665 3670 3675
   Tyr Val Asp Ala Asn Asn Asp Lys Gln Thr Ala Tyr Asn Asn Ala
   3680 3685 3690
   Val Ala Ala Ala Glu Thr Ile Ile Asn Ala Asn Ser Asn Pro Glu
   3695 3700 3705
   Met Asn Pro Ser Thr Ile Thr Gln Lys Ala Glu Gln Val Asn Ser
   3710 3715 3720
   Ser Lys Thr Ala Leu Asn Gly Asp Glu Asn Leu Ala Ala Ala Lys
   3725 3730 3735
   Gln Asn Ala Lys Thr Tyr Leu Asn Thr Leu Thr Ser Ile Thr Asp
   3740 3745 3750
   Ala Gln Lys Asn Asn Leu Ile Ser Gln Ile Thr Ser Ala Thr Arg
   3755 3760 3765
   Val Ser Gly Val Asp Thr Val Lys Gln Asn Ala Gln His Leu Asp
   3770 3775 3780
   Gln Ala Met Ala Ser Leu Gln Asn Gly Ile Asn Asn Glu Ser Gln
   3785 3790 3795
   Val Lys Ser Ser Glu Lys Tyr Arg Asp Ala Asp Thr Asn Lys Gln
   3800 3805 3810
   Gln Glu Tyr Asp Asn Ala Ile Thr Ala Ala Lys Ala Ile Leu Asn
   3815 3820 3825
   Lys Ser Thr Gly Pro Asn Thr Ala Gln Asn Ala Val Glu Ala Ala
   3830 3835 3840
   Leu Gln Arg Val Asn Asn Ala Lys Asp Ala Leu Asn Gly Asp Ala
   3845 3850 3855
   Lys Leu Ile Ala Ala Gln Asn Ala Ala Lys Gln His Leu Gly Thr
   3860 3865 3870
   Leu Thr His Ile Thr Thr Ala Gln Arg Asn Asp Leu Thr Asn Gln
   3875 3880 3885
   Ile Ser Gln Ala Thr Asn Leu Ala Gly Val Glu Ser Val Lys Gln
   3890 3895 3900
   Asn Ala Asn Ser Leu Asp Gly Ala Met Gly Asn Leu Gln Thr Ala
   3905 3910 3915
   Ile Asn Asp Lys Ser Gly Thr Leu Ala Ser Gln Asn Phe Leu Asp
   3920 3925 3930
   Ala Asp Glu Gln Lys Arg Asn Ala Tyr Asn Gln Ala Val Ser Ala
   3935 3940 3945
   Ala Glu Thr Ile Leu Asn Lys Gln Thr Gly Pro Asn Thr Ala Lys
   3950 3955 3960
   Thr Ala Val Glu Gln Ala Leu Asn Asn Val Asn Asn Ala Lys His
   3965 3970 3975
   Ala Leu Asn Gly Thr Gln Asn Leu Asn Asn Ala Lys Gln Ala Ala
   3980 3985 3990
   Ile Thr Ala Ile Asn Gly Ala Ser Asp Leu Asn Gln Lys Gln Lys
   3995 4000 4005
   Asp Ala Leu Lys Ala Gln Ala Asn Gly Ala Gln Arg Val Ser Asn
   4010 4015 4020
   Ala Gln Asp Val Gln His Asn Ala Thr Glu Leu Asn Thr Ala Met
   4025 4030 4035
   Gly Thr Leu Lys His Ala Ile Ala Asp Lys Thr Asn Thr Leu Ala
   4040 4045 4050
   Ser Ser Lys Tyr Val Asn Ala Asp Ser Thr Lys Gln Asn Ala Tyr
   4055 4060 4065
   Thr Thr Lys Val Thr Asn Ala Glu His Ile Ile Ser Gly Thr Pro
   4070 4075 4080
   Thr Val Val Thr Thr Pro Ser Glu Val Thr Ala Ala Ala Asn Gln
   4085 4090 4095
   Val Asn Ser Ala Lys Gln Glu Leu Asn Gly Asp Glu Arg Leu Arg
   4100 4105 4110
   Glu Ala Lys Gln Asn Ala Asn Thr Ala Ile Asp Ala Leu Thr Gln
   4115 4120 4125
   Leu Asn Thr Pro Gln Lys Ala Lys Leu Lys Glu Gln Val Gly Gln
   4130 4135 4140
   Ala Asn Arg Leu Glu Asp Val Gln Thr Val Gln Thr Asn Gly Gln
   4145 4150 4155
   Ala Leu Asn Asn Ala Met Lys Gly Leu Arg Asp Ser Ile Ala Asn
   4160 4165 4170
   Glu Thr Thr Val Lys Thr Ser Gln Asn Tyr Thr Asp Ala Ser Pro
   4175 4180 4185
   Asn Asn Gln Ser Thr Tyr Asn Ser Ala Val Ser Asn Ala Lys Gly
   4190 4195 4200
   Ile Ile Asn Gln Thr Asn Asn Pro Thr Met Asp Thr Ser Ala Ile
   4205 4210 4215
   Thr Gln Ala Thr Thr Gln Val Asn Asn Ala Lys Asn Gly Leu Asn
   4220 4225 4230
   Gly Ala Glu Asn Leu Arg Asn Ala Gln Asn Thr Ala Lys Gln Asn
   4235 4240 4245
   Leu Asn Thr Leu Ser His Leu Thr Asn Asn Gln Lys Ser Ala Ile
   4250 4255 4260
   Ser Ser Gln Ile Asp Arg Ala Gly His Val Ser Glu Val Thr Ala
   4265 4270 4275
   Thr Lys Asn Ala Ala Thr Glu Leu Asn Thr Gln Met Gly Asn Leu
   4280 4285 4290
   Glu Gln Ala Ile His Asp Gln Asn Thr Val Lys Gln Ser Val Lys
   4295 4300 4305
   Phe Thr Asp Ala Asp Lys Ala Lys Arg Asp Ala Tyr Thr Asn Ala
   4310 4315 4320
   Val Ser Arg Ala Glu Ala Ile Leu Asn Lys Thr Gln Gly Ala Asn
   4325 4330 4335
   Thr Ser Lys Gln Asp Val Glu Ala Ala Ile Gln Asn Val Ser Ser
   4340 4345 4350
   Ala Lys Asn Ala Leu Asn Gly Asp Gln Asn Val Thr Asn Ala Lys
   4355 4360 4365
   Asn Ala Ala Lys Asn Ala Leu Asn Asn Leu Thr Ser Ile Asn Asn
   4370 4375 4380
   Ala Gln Lys Arg Asp Leu Thr Thr Lys Ile Asp Gln Ala Thr Thr
   4385 4390 4395
   Val Ala Gly Val Glu Ala Val Ser Asn Thr Ser Thr Gln Leu Asn
   4400 4405 4410
   Thr Ala Met Ala Asn Leu Gln Asn Gly Ile Asn Asp Lys Thr Asn
   4415 4420 4425
   Thr Leu Ala Ser Glu Asn Tyr His Asp Ala Asp Ser Asp Lys Lys
   4430 4435 4440
   Thr Ala Tyr Thr Gln Ala Val Thr Asn Ala Glu Asn Ile Leu Asn
   4445 4450 4455
   Lys Asn Ser Gly Ser Asn Leu Asp Lys Thr Ala Val Glu Asn Ala
   4460 4465 4470
   Leu Ser Gln Val Ala Asn Ala Lys Gly Ala Leu Asn Gly Asn His
   4475 4480 4485
   Asn Leu Glu Gln Ala Lys Ser Asn Ala Asn Thr Thr Ile Asn Gly
   4490 4495 4500
   Leu Gln His Leu Thr Thr Ala Gln Lys Asp Lys Leu Lys Gln Gln
   4505 4510 4515
   Val Gln Gln Ala Gln Asn Val Ala Gly Val Asp Thr Val Lys Ser
   4520 4525 4530
   Ser Ala Asn Thr Leu Asn Gly Ala Met Gly Thr Leu Arg Asn Ser
   4535 4540 4545
   Ile Gln Asp Asn Thr Ala Thr Lys Asn Gly Gln Asn Tyr Leu Asp
   4550 4555 4560
   Ala Thr Glu Arg Asn Lys Thr Asn Tyr Asn Asn Ala Val Asp Ser
   4565 4570 4575
   Ala Asn Gly Val Ile Asn Ala Thr Ser Asn Pro Asn Met Asp Ala
   4580 4585 4590
   Asn Ala Ile Asn Gln Ile Ala Thr Gln Val Thr Ser Thr Lys Asn
   4595 4600 4605
   Ala Leu Asp Gly Thr His Asn Leu Thr Gln Ala Lys Gln Thr Ala
   4610 4615 4620
   Thr Asn Ala Ile Asp Gly Ala Thr Asn Leu Asn Lys Ala Gln Lys
   4625 4630 4635
   Asp Ala Leu Lys Ala Gln Val Thr Ser Ala Gln Arg Val Ala Asn
   4640 4645 4650
   Val Thr Ser Ile Gln Gln Thr Ala Asn Glu Leu Asn Thr Ala Met
   4655 4660 4665
   Gly Gln Leu Gln His Gly Ile Asp Asp Glu Asn Ala Thr Lys Gln
   4670 4675 4680
   Thr Gln Lys Tyr Arg Asp Ala Glu Gln Ser Lys Lys Thr Ala Tyr
   4685 4690 4695
   Asp Gln Ala Val Ala Ala Ala Lys Ala Ile Leu Asn Lys Gln Thr
   4700 4705 4710
   Gly Ser Asn Ser Asp Lys Ala Ala Val Asp Arg Ala Leu Gln Gln
   4715 4720 4725
   Val Thr Ser Thr Lys Asp Ala Leu Asn Gly Asp Ala Lys Leu Ala
   4730 4735 4740
   Glu Ala  Lys Ala Ala Ala Lys  Gln Asn Leu Gly Thr  Leu Asn His
       4745                 4750                 4755
   Ile Thr  Asn Ala Gln Arg Thr  Asp Leu Glu Gly Gln  Ile Asn Gln
       4760                 4765                 4770
   Ala Thr  Thr Val Asp Gly Val  Asn Thr Val Lys Thr  Asn Ala Asn
       4775                 4780                 4785
   Thr Leu  Asp Gly Ala Met Asn  Ser Leu Gln Gly Ser  Ile Asn Asp
       4790                 4795                 4800
   Lys Asp  Ala Thr Leu Arg Asn  Gln Asn Tyr Leu Asp  Ala Asp Glu
       4805                 4810                 4815
   Ser Lys  Arg Asn Ala Tyr Thr  Gln Ala Val Thr Ala  Ala Glu Gly
       4820                 4825                 4830
   Ile Leu  Asn Lys Gln Thr Gly  Gly Asn Thr Ser Lys  Ala Asp Val
       4835                 4840                 4845
   Asp Asn  Ala Leu Asn Ala Val  Thr Arg Ala Lys Ala  Ala Leu Asn
       4850                 4855                 4860
   Gly Ala  Asp Asn Leu Arg Asn  Ala Lys Thr Ser Ala  Thr Asn Thr
       4865                 4870                 4875
   Ile Asp  Gly Leu Pro Asn Leu  Thr Gln Leu Gln Lys  Asp Asn Leu
       4880                 4885                 4890
   Lys His  Gln Val Glu Gln Ala  Gln Asn Val Ala Gly  Val Asn Gly
       4895                 4900                 4905
   Val Lys  Asp Lys Gly Asn Thr  Leu Asn Thr Ala Met  Gly Ala Leu
       4910                 4915                 4920
   Arg Thr  Ser Ile Gln Asn Asp  Asn Thr Thr Lys Thr  Ser Gln Asn
       4925                 4930                 4935
   Tyr Leu  Asp Ala Ser Asp Ser  Asn Lys Asn Asn Tyr  Asn Thr Ala
       4940                 4945                 4950
   Val Asn  Asn Ala Asn Gly Val  Ile Asn Ala Thr Asn  Asn Pro Asn
       4955                 4960                 4965
   Met Asp  Ala Asn Ala Ile Asn  Gly Met Ala Asn Gln  Val Asn Thr
   4970 4975 4980
   Thr Lys Ala Ala Leu Asn Gly Ala Gln Asn Leu Ala Gln Ala Lys
   4985 4990 4995
   Thr Asn Ala Thr Asn Thr Ile Asn Asn Ala His Asp Leu Asn Gln
   5000 5005 5010
   Lys Gln Lys Asp Ala Leu Lys Thr Gln Val Asn Asn Ala Gln Arg
   5015 5020 5025
   Val Ser Asp Ala Asn Asn Val Gln His Thr Ala Thr Glu Leu Asn
   5030 5035 5040
   Ser Ala Met Thr Ala Leu Lys Ala Ala Ile Ala Asp Lys Glu Arg
   5045 5050 5055
   Thr Lys Ala Ser Gly Asn Tyr Val Asn Ala Asp Gln Glu Lys Arg
   5060 5065 5070
   Gln Ala Tyr Asp Ser Lys Val Thr Asn Ala Glu Asn Ile Ile Ser
   5075 5080 5085
   Gly Thr Pro Asn Ala Thr Leu Thr Val Asn Asp Val Asn Ser Ala
   5090 5095 5100
   Ala Ser Gln Val Asn Ala Ala Lys Thr Ala Leu Asn Gly Asp Asn
   5105 5110 5115
   Asn Leu Arg Val Ala Lys Glu His Ala Asn Asn Thr Ile Asp Gly
   5120 5125 5130
   Leu Ala Gln Leu Asn Asn Ala Gln Lys Ala Lys Leu Lys Glu Gln
   5135 5140 5145
   Val Gln Ser Ala Thr Thr Leu Asp Gly Val Gln Thr Val Lys Asn
   5150 5155 5160
   Ser Ser Gln Thr Leu Asn Thr Ala Met Lys Gly Leu Arg Asp Ser
   5165 5170 5175
   Ile Ala Asn Glu Ala Thr Ile Lys Ala Gly Gln Asn Tyr Thr Asp
   5180 5185 5190
   Ala Ser Pro Asn Asn Arg Asn Glu Tyr Asp Ser Ala Val Thr Ala
   5195 5200 5205
   Ala Lys Ala Ile Ile Asn Gln Thr Ser Asn Pro Thr Met Glu Pro
   5210 5215 5220
   Asn Thr Ile Thr Gln Val Thr Ser Gln Val Thr Thr Lys Glu Gln
   5225 5230 5235
   Ala Leu Asn Gly Ala Arg Asn Leu Ala Gln Ala Lys Thr Thr Ala
   5240 5245 5250
   Lys Asn Asn Leu Asn Asn Leu Thr Ser Ile Asn Asn Ala Gln Lys
   5255 5260 5265
   Asp Ala Leu Thr Arg Ser Ile Asp Gly Ala Thr Thr Val Ala Gly
   5270 5275 5280
   Val Asn Gln Glu Thr Ala Lys Ala Thr Glu Leu Asn Asn Ala Met
   5285 5290 5295
   His Ser Leu Gln Asn Gly Ile Asn Asp Glu Thr Gln Thr Lys Gln
   5300 5305 5310
   Thr Gln Lys Tyr Leu Asp Ala Glu Pro Ser Lys Lys Ser Ala Tyr
   5315 5320 5325
   Asp Gln Ala Val Asn Ala Ala Lys Ala Ile Leu Thr Lys Ala Ser
   5330 5335 5340
   Gly Gln Asn Val Asp Lys Ala Ala Val Glu Gln Ala Leu Gln Asn
   5345 5350 5355
   Val Asn Ser Thr Lys Thr Ala Leu Asn Gly Asp Ala Lys Leu Asn
   5360 5365 5370
   Glu Ala Lys Ala Ala Ala Lys Gln Thr Leu Gly Thr Leu Thr His
   5375 5380 5385
   Ile Asn Asn Ala Gln Arg Thr Ala Leu Asp Asn Glu Ile Thr Gln
   5390 5395 5400
   Ala Thr Asn Val Glu Gly Val Asn Thr Val Lys Ala Lys Ala Gln
   5405 5410 5415
   Gln Leu Asp Gly Ala Met Gly Gln Leu Glu Thr Ser Ile Arg Asp
   5420 5425 5430
   Lys Asp Thr Thr Leu Gln Ser Gln Asn Tyr Gln Asp Ala Asp Asp
   5435 5440 5445
   Ala Lys Arg Thr Ala Tyr Ser Gln Ala Val Asn Ala Ala Ala Thr
   5450 5455 5460
   Ile Leu Asn Lys Thr Ala Gly Gly Asn Thr Pro Lys Ala Asp Val
   5465 5470 5475
   Glu Arg Ala Met Gln Ala Val Thr Gln Ala Asn Thr Ala Leu Asn
   5480 5485 5490
   Gly Ile Gln Asn Leu Asp Arg Ala Lys Gln Ala Ala Asn Thr Ala
   5495 5500 5505
   Ile Thr Asn Ala Ser Asp Leu Asn Thr Lys Gln Lys Glu Ala Leu
   5510 5515 5520
   Lys Ala Gln Val Thr Ser Ala Gly Arg Val Ser Ala Ala Asn Gly
   5525 5530 5535
   Val Glu His Thr Ala Thr Glu Leu Asn Thr Ala Met Thr Ala Leu
   5540 5545 5550
   Lys Arg Ala Ile Ala Asp Lys Ala Glu Thr Lys Ala Ser Gly Asn
   5555 5560 5565
   Tyr Val Asn Ala Asp Ala Asn Lys Arg Gln Ala Tyr Asp Glu Lys
   5570 5575 5580
   Val Thr Ala Ala Glu Asn Ile Val Ser Gly Thr Pro Thr Pro Thr
   5585 5590 5595
   Leu Thr Pro Ala Asp Val Thr Asn Ala Ala Thr Gln Val Thr Asn
   5600 5605 5610
   Ala Lys Thr Gln Leu Asn Gly Asn His Asn Leu Glu Val Ala Lys
   5615 5620 5625
   Gln Asn Ala Asn Thr Ala Ile Asp Gly Leu Thr Ser Leu Asn Gly
   5630 5635 5640
   Pro Gln Lys Ala Lys Leu Lys Glu Gln Val Gly Gln Ala Thr Thr
   5645 5650 5655
   Leu Pro Asn Val Gln Thr Val Arg Asp Asn Ala Gln Thr Leu Asn
   5660 5665 5670
   Thr Ala Met Lys Gly Leu Arg Asp Ser Ile Ala Asn Glu Ala Thr
   5675 5680 5685
   Ile Lys Ala Gly Gln Asn Tyr Thr Asp Ala Ser Gln Asn Lys Gln
   5690 5695 5700
   Thr Asp Tyr Asn Ser Ala Val Thr Ala Ala Lys Ala Ile Ile Gly
   5705 5710 5715
   Gln Thr Thr Ser Pro Ser Met Asn Ala Gln Glu Ile Asn Gln Ala
   5720 5725 5730
   Lys Asp Gln Val Thr Ala Lys Gln Gln Ala Leu Asn Gly Gln Glu
   5735 5740 5745
   Asn Leu Arg Thr Ala Gln Thr Asn Ala Lys Gln His Leu Asn Gly
   5750 5755 5760
   Leu Ser Asp Leu Thr Asp Ala Gln Lys Asp Ala Val Lys Arg Gln
   5765 5770 5775
   Ile Glu Gly Ala Thr His Val Asn Glu Val Thr Gln Ala Gln Asn
   5780 5785 5790
   Asn Ala Asp Ala Leu Asn Thr Ala Met Thr Asn Leu Lys Asn Gly
   5795 5800 5805
   Ile Gln Asp Gln Asn Thr Ile Lys Gln Gly Val Asn Phe Thr Asp
   5810 5815 5820
   Ala Asp Glu Ala Lys Arg Asn Ala Tyr Thr Asn Ala Val Thr Gln
   5825 5830 5835
   Ala Glu Gln Ile Leu Asn Lys Ala Gln Gly Pro Asn Thr Ser Lys
   5840 5845 5850
   Asp Gly Val Glu Thr Ala Leu Glu Asn Val Gln Arg Ala Lys Asn
   5855 5860 5865
   Glu Leu Asn Gly Asn Gln Asn Val Ala Asn Ala Lys Thr Thr Ala
   5870 5875 5880
   Lys Asn Ala Leu Asn Asn Leu Thr Ser Ile Asn Asn Ala Gln Lys
   5885 5890 5895
   Glu Ala Leu Lys Ser Gln Ile Glu Gly Ala Thr Thr Val Ala Gly
   5900 5905 5910
   Val Asn Gln Val Ser Thr Thr Ala Ser Glu Leu Asn Thr Ala Met
   5915 5920 5925
   Ser Asn Leu Gln Asn Gly Ile Asn Asp Glu Ala Ala Thr Lys Ala
   5930 5935 5940
   Ala Gln Lys Tyr Thr Asp Ala Asp Arg Glu Lys Gln Thr Ala Tyr
   5945 5950 5955
   Asn Asp Ala Val Thr Ala Ala Lys Thr Leu Leu Asp Lys Thr Ala
   5960 5965 5970
   Gly Ser Asn Asp Asn Lys Ala Ala Val Glu Gln Ala Leu Gln Arg
   5975 5980 5985
   Val Asn Thr Ala Lys Thr Ala Leu Asn Gly Asp Glu Arg Leu Asn
   5990 5995 6000
   Glu Ala Lys Asn Thr Ala Lys Gln Gln Val Ala Thr Met Ser His
   6005 6010 6015
   Leu Thr Asp Ala Gln Lys Ala Asn Leu Thr Ser Gln Ile Glu Ser
   6020 6025 6030
   Gly Thr Thr Val Ala Gly Val Gln Gly Ile Gln Ala Asn Ala Gly
   6035 6040 6045
   Thr Leu Asp Gln Ala Met Asn Gln Leu Arg Gln Ser Ile Ala Ser
   6050 6055 6060
   Lys Asp Ala Thr Lys Ser Ser Glu Asp Tyr Gln Asp Ala Asn Ala
   6065 6070 6075
   Asp Leu Gln Asn Ala Tyr Asn Asp Ala Val Thr Asn Ala Glu Gly
   6080 6085 6090
   Ile Ile Ser Ala Thr Asn Asn Pro Glu Met Asn Pro Asp Thr Ile
   6095 6100 6105
   Asn Gln Lys Ala Ser Gln Val Asn Ser Ala Lys Ser Ala Leu Asn
   6110 6115 6120
   Gly Asp Glu Lys Leu Ala Ala Ala Lys Gln Thr Ala Lys Ser Asp
   6125 6130 6135
   Ile Gly Arg Leu Thr Asp Leu Asn Asn Ala Gln Arg Thr Ala Ala
   6140 6145 6150
   Asn Ala Glu Val Asp Gln Ala Pro Asn Leu Ala Ala Val Thr Ala
       6155                6160                6165
   Ala Lys Asn Lys Ala Thr Ser Leu Asn Thr Ala Met Gly Asn Leu
       6170                6175                6180
   Lys His Ala Leu Ala Glu Lys Asp Asn Thr Lys Arg Ser Val Asn
       6185                6190                6195
   Tyr Thr Asp Ala Asp Gln Pro Lys Gln Gln Ala Tyr Asp Thr Ala
       6200                6205                6210
   Val Thr Gln Ala Glu Ala Ile Thr Asn Ala Asn Gly Ser Asn Ala
       6215                6220                6225
   Asn Glu Thr Gln Val Gln Ala Ala Leu Asn Gln Leu Asn Gln Ala
       6230                6235                6240
   Lys Asn Asp Leu Asn Gly Asp Asn Lys Val Ala Gln Ala Lys Glu
       6245                6250                6255
   Ser Ala Lys Arg Ala Leu Ala Ser Tyr Ser Asn Leu Asn Asn Ala
       6260                6265                6270
   Gln Ser Thr Ala Ala Ile Ser Gln Ile Asp Asn Ala Thr Thr Val
       6275                6280                6285
   Ala Gly Val Thr Ala Ala Gln Asn Thr Ala Asn Glu Leu Asn Thr
       6290                6295                6300
   Ala Met Gly Gln Leu Gln Asn Gly Ile Asn Asp Gln Asn Thr Val
       6305                6310                6315
   Lys Gln Gln Val Asn Phe Thr Asp Ala Asp Gln Gly Lys Lys Asp
       6320                6325                6330
   Ala Tyr Thr Asn Ala Val Thr Asn Ala Gln Gly Ile Leu Asp Lys
       6335                6340                6345
   Ala His Gly Gln Asn Met Thr Lys Ala Gln Val Glu Ala Ala Leu
       6350                6355                6360
   Asn Gln Val Thr Thr Ala Lys Asn Ala Leu Asn Gly Asp Ala Asn
       6365                6370                6375
   Val Arg Gln Ala Lys Ser Asp Ala Lys Ala Asn Leu Gly Thr Leu
       6380                6385                6390
   Thr His Leu Asn Asn Ala Gln Lys Gln Asp Leu Thr Ser Gln Ile
   6395 6400 6405
   Glu Gly Ala Thr Thr Val Asn Gly Val Asn Gly Val Lys Thr Lys
   6410 6415 6420
   Ala Gln Asp Leu Asp Gly Ala Met Gln Arg Leu Gln Ser Ala Ile
   6425 6430 6435
   Ala Asn Lys Asp Gln Thr Lys Ala Ser Glu Asn Tyr Ile Asp Ala
   6440 6445 6450
   Asp Pro Thr Lys Lys Thr Ala Phe Asp Asn Ala Ile Thr Gln Ala
   6455 6460 6465
   Glu Ser Tyr Leu Asn Lys Asp His Gly Ala Asn Lys Asp Lys Gln
   6470 6475 6480
   Ala Val Glu Gln Ala Ile Gln Ser Val Thr Ser Thr Glu Asn Ala
   6485 6490 6495
   Leu Asn Gly Asp Ala Asn Leu Gln Arg Ala Lys Thr Glu Ala Ile
   6500 6505 6510
   Gln Ala Ile Asp Asn Leu Thr His Leu Asn Thr Pro Gln Lys Thr
   6515 6520 6525
   Ala Leu Lys Gln Gln Val Asn Ala Ala Gln Arg Val Ser Gly Val
   6530 6535 6540
   Thr Asp Leu Lys Asn Ser Ala Thr Ser Leu Asn Asn Ala Met Asp
   6545 6550 6555
   Gln Leu Lys Gln Ala Ile Ala Asp His Asp Thr Ile Val Ala Ser
   6560 6565 6570
   Gly Asn Tyr Thr Asn Ala Ser Pro Asp Lys Gln Gly Ala Tyr Thr
   6575 6580 6585
   Asp Ala Tyr Asn Ala Ala Lys Asn Ile Val Asn Gly Ser Pro Asn
   6590 6595 6600
   Val Ile Thr Asn Ala Ala Asp Val Thr Ala Ala Thr Gln Arg Val
   6605 6610 6615
   Asn Asn Ala Glu Thr Gly Leu Asn Gly Asp Thr Asn Leu Ala Thr
   6620 6625 6630
   Ala Lys Gln Gln Ala Lys Asp Ala Leu Arg Gln Met Thr His Leu
       6635                6640                6645
   Ser Asp Ala Gln Lys Gln Ser Ile Thr Gly Gln Ile Asp Ser Ala
       6650                6655                6660
   Thr Gln Val Thr Gly Val Gln Ser Val Lys Asp Asn Ala Thr Asn
       6665                6670                6675
   Leu Asp Asn Ala Met Asn Gln Leu Arg Asn Ser Ile Ala Asn Lys
       6680                6685                6690
   Asp Asp Val Lys Ala Ser Gln Pro Tyr Val Asp Ala Asp Arg Asp
       6695                6700                6705
   Lys Gln Asn Ala Tyr Asn Thr Ala Val Thr Asn Ala Glu Asn Ile
       6710                6715                6720
   Ile Asn Ala Thr Ser Gln Pro Thr Leu Asp Pro Ser Ala Val Thr
       6725                6730                6735
   Gln Ala Ala Asn Gln Val Ser Thr Asn Lys Thr Ala Leu Asn Gly
       6740                6745                6750
   Ala Gln Asn Leu Ala Asn Lys Lys Gln Glu Thr Thr Ala Asn Ile
       6755                6760                6765
   Asn Gln Leu Ser His Leu Asn Asn Ala Gln Lys Gln Asp Leu Asn
       6770                6775                6780
   Thr Gln Val Thr Asn Ala Pro Asn Ile Ser Thr Val Asn Gln Val
       6785                6790                6795
   Lys Thr Lys Ala Glu Gln Leu Asp Gln Ala Met Glu Arg Leu Ile
       6800                6805                6810
   Asn Gly Ile Gln Asp Lys Asp Gln Val Lys Gln Ser Val Asn Phe
       6815                6820                6825
   Thr Asp Ala Asp Pro Glu Lys Gln Thr Ala Tyr Asn Asn Ala Val
       6830                6835                6840
   Thr Ala Ala Glu Asn Ile Ile Asn Gln Ala Asn Gly Thr Asn Ala
       6845                6850                6855
   Asn Gln Ser Gln Val Glu Ala Ala Leu Ser Thr Val Thr Thr Thr
       6860                6865                6870
   Lys Gln  Ala Leu Asn Gly Asp  Arg Lys Val Thr Asp  Ala Lys Asn
       6875                6880                6885
   Asn Ala  Asn Gln Thr Leu Ser  Thr Leu Asp Asn Leu  Asn Asn Ala
       6890                6895                6900
   Gln Lys  Gly Ala Val Thr Gly  Asn Ile Asn Gln Ala  His Thr Val
       6905                6910                6915
   Ala Glu  Val Thr Gln Ala Ile  Gln Thr Ala Gln Glu  Leu Asn Thr
       6920                6925                6930
   Ala Met  Gly Asn Leu Lys Asn  Ser Leu Asn Asp Lys  Asp Thr Thr
       6935                6940                6945
   Leu Gly  Ser Gln Asn Phe Ala  Asp Ala Asp Pro Glu  Lys Lys Asn
       6950                6955                6960
   Ala Tyr  Asn Glu Ala Val His  Asn Ala Glu Asn Ile  Leu Asn Lys
       6965                6970                6975
   Ser Thr  Gly Thr Asn Val Pro  Lys Asp Gln Val Glu  Ala Ala Met
       6980                6985                6990
   Asn Gln  Val Asn Ala Thr Lys  Ala Ala Leu Asn Gly  Thr Gln Asn
       6995                7000                7005
   Leu Glu  Lys Ala Lys Gln His  Ala Asn Thr Ala Ile  Asp Gly Leu
       7010                7015                7020
   Ser His  Leu Thr Asn Ala Gln  Lys Glu Ala Leu Lys  Gln Leu Val
       7025                7030                7035
   Gln Gln  Ser Thr Thr Val Ala  Glu Ala Gln Gly Asn  Glu Gln Lys
       7040                7045                7050
   Ala Asn  Asn Val Asp Ala Ala  Met Asp Lys Leu Arg  Gln Ser Ile
       7055                7060                7065
   Ala Asp  Asn Ala Thr Thr Lys  Gln Asn Gln Asn Tyr  Thr Asp Ala
       7070                7075                7080
   Ser Gln  Asn Lys Lys Asp Ala  Tyr Asn Asn Ala Val  Thr Thr Ala
       7085                7090                7095
   Gln Gly Ile Ile Asp Gln Thr Thr Ser Pro Thr Leu Asp Pro Thr
   7100 7105 7110
   Val Ile Asn Gln Ala Ala Gly Gln Val Ser Thr Thr Lys Asn Ala
   7115 7120 7125
   Leu Asn Gly Asn Glu Asn Leu Glu Ala Ala Lys Gln Gln Ala Ser
   7130 7135 7140
   Gln Ser Leu Gly Ser Leu Asp Asn Leu Asn Asn Ala Gln Lys Gln
   7145 7150 7155
   Thr Val Thr Asp Gln Ile Asn Gly Ala His Thr Val Asp Glu Ala
   7160 7165 7170
   Asn Gln Ile Lys Gln Asn Ala Gln Asn Leu Asn Thr Ala Met Gly
   7175 7180 7185
   Asn Leu Lys Gln Ala Ile Ala Asp Lys Asp Ala Thr Lys Ala Thr
   7190 7195 7200
   Val Asn Phe Thr Asp Ala Asp Gln Ala Lys Gln Gln Ala Tyr Asn
   7205 7210 7215
   Thr Ala Val Thr Asn Ala Glu Asn Ile Ser Lys Ala Asn Gly Asn
   7220 7225 7230
   Ala Thr Gln Ala Glu Val Glu Gln Ala Ile Lys Gln Val Asn Ala
   7235 7240 7245
   Ala Lys Gln Ala Leu Asn Gly Asn Ala Asn Val Gln His Ala Lys
   7250 7255 7260
   Asp Glu Ala Thr Ala Leu Ile Asn Ser Ser Asn Asp Leu Asn Gln
   7265 7270 7275
   Ala Gln Lys Asp Ala Leu Lys Gln Gln Val Gln Asn Ala Thr Thr
   7280 7285 7290
   Val Ala Gly Val Asn Asn Val Lys Gln Thr Ala Gln Glu Leu Asn
   7295 7300 7305
   Asn Ala Met Thr Gln Leu Lys Gln Gly Ile Ala Asp Lys Glu Gln
   7310 7315 7320
   Thr Lys Ala Asp Gly Asn Phe Val Asn Ala Asp Pro Asp Lys Gln
   7325 7330 7335
   Asn Ala  Tyr Asn Gln Ala Val  Ala Lys Ala Glu Ala  Leu Ile Ser
       7340                 7345                 7350
   Ala Thr  Pro Asp Val Val Val  Thr Pro Ser Glu Ile  Thr Ala Ala
       7355                 7360                 7365
   Leu Asn  Lys Val Thr Gln Ala  Lys Asn Asp Leu Asn  Gly Asn Thr
       7370                 7375                 7380
   Asn Leu  Ala Thr Ala Lys Gln  Asn Val Gln His Ala  Ile Asp Gln
       7385                 7390                 7395
   Leu Pro  Asn Leu Asn Gln Ala  Gln Arg Asp Glu Tyr  Ser Lys Gln
       7400                 7405                 7410
   Ile Thr  Gln Ala Thr Leu Val  Pro Asn Val Asn Ala  Ile Gln Gln
       7415                 7420                 7425
   Ala Ala  Thr Thr Leu Asn Asp  Ala Met Thr Gln Leu  Lys Gln Gly
       7430                 7435                 7440
   Ile Ala  Asn Lys Ala Gln Ile  Lys Gly Ser Glu Asn  Tyr His Asp
       7445                 7450                 7455
   Ala Asp  Thr Asp Lys Gln Thr  Ala Tyr Asp Asn Ala  Val Thr Lys
       7460                 7465                 7470
   Ala Glu  Glu Leu Leu Lys Gln  Thr Thr Asn Pro Thr  Met Asp Pro
       7475                 7480                 7485
   Asn Thr  Ile Gln Gln Ala Leu  Thr Lys Val Asn Asp  Thr Asn Gln
       7490                 7495                 7500
   Ala Leu  Asn Gly Asn Gln Lys  Leu Ala Asp Ala Lys  Gln Asp Ala
       7505                 7510                 7515
   Lys Thr  Thr Leu Gly Thr Leu  Asp His Leu Asn Asp  Ala Gln Lys
       7520                 7525                 7530
   Gln Ala  Leu Thr Thr Gln Val  Glu Gln Ala Pro Asp  Ile Ala Thr
       7535                 7540                 7545
   Val Asn  Asn Val Lys Gln Asn  Ala Gln Asn Leu Asn  Asn Ala Met
       7550                 7555                 7560
   Thr Asn  Leu Asn Asn Ala Leu  Gln Asp Lys Thr Glu  Thr Leu Asn
       7565                 7570                 7575
   Ser Ile Asn Phe Thr Asp Ala Asp Gln Ala Lys Lys Asp Ala Tyr
       7580                7585                7590
   Thr Asn Ala Val Ser His Ala Glu Gly Ile Leu Ser Lys Ala Asn
       7595                7600                7605
   Gly Ser Asn Ala Ser Gln Thr Glu Val Glu Gln Ala Met Gln Arg
       7610                7615                7620
   Val Asn Glu Ala Lys Gln Ala Leu Asn Gly Asn Asp Asn Val Gln
       7625                7630                7635
   Arg Ala Lys Asp Ala Ala Lys Gln Val Ile Thr Asn Ala Asn Asp
       7640                7645                7650
   Leu Asn Gln Ala Gln Lys Asp Ala Leu Lys Gln Gln Val Asp Ala
       7655                7660                7665
   Ala Gln Thr Val Ala Asn Val Asn Thr Ile Lys Gln Thr Ala Gln
       7670                7675                7680
   Asp Leu Asn Gln Ala Met Thr Gln Leu Lys Gln Gly Ile Ala Asp
       7685                7690                7695
   Lys Asp Gln Thr Lys Ala Asn Gly Asn Phe Val Asn Ala Asp Thr
       7700                7705                7710
   Asp Lys Gln Asn Ala Tyr Asn Asn Ala Val Ala His Ala Glu Gln
       7715                7720                7725
   Ile Ile Ser Gly Thr Pro Asn Ala Asn Val Asp Pro Gln Gln Val
       7730                7735                7740
   Ala Gln Ala Leu Gln Gln Val Asn Gln Ala Lys Gly Asp Leu Asn
       7745                7750                7755
   Gly Asn His Asn Leu Gln Val Ala Lys Asp Asn Ala Asn Thr Ala
       7760                7765                7770
   Ile Asp Gln Leu Pro Asn Leu Asn Gln Pro Gln Lys Thr Ala Leu
       7775                7780                7785
   Lys Asp Gln Val Ser His Ala Glu Leu Val Thr Gly Val Asn Ala
       7790                7795                7800
   Ile Lys Gln Asn Ala Asp Ala Leu Asn Asn Ala Met Gly Thr Leu
   7805 7810 7815
   Lys Gln Gln Ile Gln Ala Asn Ser Gln Val Pro Gln Ser Val Asp
   7820 7825 7830
   Phe Thr Gln Ala Asp Gln Asp Lys Gln Gln Ala Tyr Asn Asn Ala
   7835 7840 7845
   Ala Asn Gln Ala Gln Gln Ile Ala Asn Gly Ile Pro Thr Pro Val
   7850 7855 7860
   Leu Thr Pro Asp Thr Val Thr Gln Ala Val Thr Thr Met Asn Gln
   7865 7870 7875
   Ala Lys Asp Ala Leu Asn Gly Asp Glu Lys Leu Ala Gln Ala Lys
   7880 7885 7890
   Gln Glu Ala Leu Ala Asn Leu Asp Thr Leu Arg Asp Leu Asn Gln
   7895 7900 7905
   Pro Gln Arg Asp Ala Leu Arg Asn Gln Ile Asn Gln Ala Gln Ala
   7910 7915 7920
   Leu Ala Thr Val Glu Gln Thr Lys Gln Asn Ala Gln Asn Val Asn
   7925 7930 7935
   Thr Ala Met Ser Asn Leu Lys Gln Gly Ile Ala Asn Lys Asp Thr
   7940 7945 7950
   Val Lys Ala Ser Glu Asn Tyr His Asp Ala Asp Ala Asp Lys Gln
   7955 7960 7965
   Thr Ala Tyr Thr Asn Ala Val Ser Gln Ala Glu Gly Ile Ile Asn
   7970 7975 7980
   Gln Thr Thr Asn Pro Thr Leu Asn Pro Asp Glu Ile Thr Arg Ala
   7985 7990 7995
   Leu Thr Gln Val Thr Asp Ala Lys Asn Gly Leu Asn Gly Glu Ala
   8000 8005 8010
   Lys Leu Ala Thr Glu Lys Gln Asn Ala Lys Asp Ala Val Ser Gly
   8015 8020 8025
   Met Thr His Leu Asn Asp Ala Gln Lys Gln Ala Leu Lys Gly Gln
   8030 8035 8040
   Ile Asp Gln Ser Pro Glu Ile Ala Thr Val Asn Gln Val Lys Gln
   8045 8050 8055
   Thr Ala Thr Ser Leu Asp Gln Ala Met Asp Gln Leu Ser Gln Ala
   8060 8065 8070
   Ile Asn Asp Lys Ala Gln Thr Leu Ala Asp Gly Asn Tyr Leu Asn
   8075 8080 8085
   Ala Asp Pro Asp Lys Gln Asn Ala Tyr Lys Gln Ala Val Ala Lys
   8090 8095 8100
   Ala Glu Ala Leu Leu Asn Lys Gln Ser Gly Thr Asn Glu Val Gln
   8105 8110 8115
   Ala Gln Val Glu Ser Ile Thr Asn Glu Val Asn Ala Ala Lys Gln
   8120 8125 8130
   Ala Leu Asn Gly Asn Asp Asn Leu Ala Asn Ala Lys Gln Gln Ala
   8135 8140 8145
   Lys Gln Gln Leu Ala Asn Leu Thr His Leu Asn Asp Ala Gln Lys
   8150 8155 8160
   Gln Ser Phe Glu Ser Gln Ile Thr Gln Ala Pro Leu Val Thr Asp
   8165 8170 8175
   Val Thr Thr Ile Asn Gln Lys Ala Gln Thr Leu Asp His Ala Met
   8180 8185 8190
   Glu Leu Leu Arg Asn Ser Val Ala Asp Asn Gln Thr Thr Leu Ala
   8195 8200 8205
   Ser Glu Asp Tyr His Asp Ala Thr Ala Gln Arg Gln Asn Asp Tyr
   8210 8215 8220
   Asn Gln Ala Val Thr Ala Ala Asn Asn Ile Ile Asn Gln Thr Thr
   8225 8230 8235
   Ser Pro Thr Met Asn Pro Asp Asp Val Asn Gly Ala Thr Thr Gln
   8240 8245 8250
   Val Asn Asn Thr Lys Val Ala Leu Asp Gly Asp Glu Asn Leu Ala
   8255 8260 8265
   Ala Ala Lys Gln Gln Ala Asn Asn Arg Leu Asp Gln Leu Asp His
   8270 8275 8280
   Leu Asn Asn Ala Gln Lys Gln Gln Leu Gln Ser Gln Ile Thr Gln
   8285 8290 8295
   Ser Ser Asp Ile Ala Ala Val Asn Gly His Lys Gln Thr Ala Glu
   8300 8305 8310
   Ser Leu Asn Thr Ala Met Gly Asn Leu Ile Asn Ala Ile Ala Asp
   8315 8320 8325
   His Gln Ala Val Glu Gln Arg Gly Asn Phe Ile Asn Ala Asp Thr
   8330 8335 8340
   Asp Lys Gln Thr Ala Tyr Asn Thr Ala Val Asn Glu Ala Ala Ala
   8345 8350 8355
   Met Ile Asn Lys Gln Thr Gly Gln Asn Ala Asn Gln Thr Glu Val
   8360 8365 8370
   Glu Gln Ala Ile Thr Lys Val Gln Thr Thr Leu Gln Ala Leu Asn
   8375 8380 8385
   Gly Asp His Asn Leu Gln Val Ala Lys Thr Asn Ala Thr Gln Ala
   8390 8395 8400
   Ile Asp Ala Leu Thr Ser Leu Asn Asp Pro Gln Lys Thr Ala Leu
   8405 8410 8415
   Lys Asp Gln Val Thr Ala Ala Thr Leu Val Thr Ala Val His Gln
   8420 8425 8430
   Ile Glu Gln Asn Ala Asn Thr Leu Asn Gln Ala Met His Gly Leu
   8435 8440 8445
   Arg Gln Ser Ile Gln Asp Asn Ala Ala Thr Lys Ala Asn Ser Lys
   8450 8455 8460
   Tyr Ile Asn Glu Asp Gln Pro Glu Gln Gln Asn Tyr Asp Gln Ala
   8465 8470 8475
   Val Gln Ala Ala Asn Asn Ile Ile Asn Glu Gln Thr Ala Thr Leu
   8480 8485 8490
   Asp Asn Asn Ala Ile Asn Gln Ala Ala Thr Thr Val Asn Thr Thr
   8495 8500 8505
   Lys Ala Ala Leu His Gly Asp Val Lys Leu Gln Asn Asp Lys Asp
   8510 8515 8520
   His Ala  Lys Gln Thr Val Ser  Gln Leu Ala His Leu  Asn Asn Ala
       8525                 8530                 8535
   Gln Lys  His Met Glu Asp Thr  Leu Ile Asp Ser Glu  Thr Thr Arg
       8540                 8545                 8550
   Thr Ala  Val Lys Gln Asp Leu  Thr Glu Ala Gln Ala  Leu Asp Gln
       8555                 8560                 8565
   Leu Met  Asp Ala Leu Gln Gln  Ser Ile Ala Asp Lys  Asp Ala Thr
       8570                 8575                 8580
   Arg Ala  Ser Ser Ala Tyr Val  Asn Ala Glu Pro Asn  Lys Lys Gln
       8585                 8590                 8595
   Ser Tyr  Asp Glu Ala Val Gln  Asn Ala Glu Ser Ile  Ile Ala Gly
       8600                 8605                 8610
   Leu Asn  Asn Pro Thr Ile Asn  Lys Gly Asn Val Ser  Ser Ala Thr
       8615                 8620                 8625
   Gln Ala  Val Ile Ser Ser Lys  Asn Ala Leu Asp Gly  Val Glu Arg
       8630                 8635                 8640
   Leu Ala  Gln Asp Lys Gln Thr  Ala Gly Asn Ser Leu  Asn His Leu
       8645                 8650                 8655
   Asp Gln  Leu Thr Pro Ala Gln  Gln Gln Ala Leu Glu  Asn Gln Ile
       8660                 8665                 8670
   Asn Asn  Ala Thr Thr Arg Gly  Glu Val Ala Gln Lys  Leu Thr Glu
       8675                 8680                 8685
   Ala Gln  Ala Leu Asn Gln Ala  Met Glu Ala Leu Arg  Asn Ser Ile
       8690                 8695                 8700
   Gln Asp  Gln Gln Gln Thr Glu  Ala Gly Ser Lys Phe  Ile Asn Glu
       8705                 8710                 8715
   Asp Lys  Pro Gln Lys Asp Ala  Tyr Gln Ala Ala Val  Gln Asn Ala
       8720                 8725                 8730
   Lys Asp  Leu Ile Asn Gln Thr  Asn Asn Pro Thr Leu  Asp Lys Ala
       8735                 8740                 8745
   Gln Val  Glu Gln Leu Thr Gln  Ala Val Asn Gln Ala  Lys Asp Asn
       8750                8755                8760
   Leu His  Gly Asp Gln Lys Leu  Ala Asp Asp Lys Gln  His Ala Val
       8765                8770                8775
   Thr Asp  Leu Asn Gln Leu Asn  Gly Leu Asn Asn Pro  Gln Arg Gln
       8780                8785                8790
   Ala Leu  Glu Ser Gln Ile Asn  Asn Ala Ala Thr Arg  Gly Glu Val
       8795                8800                8805
   Ala Gln  Lys Leu Ala Glu Ala  Lys Ala Leu Asp Gln  Ala Met Gln
       8810                8815                8820
   Ala Leu  Arg Asn Ser Ile Gln  Asp Gln Gln Gln Thr  Glu Ser Gly
       8825                8830                8835
   Ser Lys  Phe Ile Asn Glu Asp  Lys Pro Gln Lys Asp  Ala Tyr Gln
       8840                8845                8850
   Ala Ala  Val Gln Asn Ala Lys  Asp Leu Ile Asn Gln  Thr Gly Asn
       8855                8860                8865
   Pro Thr  Leu Asp Lys Ser Gln  Val Glu Gln Leu Thr  Gln Ala Val
       8870                8875                8880
   Thr Thr  Ala Lys Asp Asn Leu  His Gly Asp Gln Lys  Leu Ala Arg
       8885                8890                8895
   Asp Gln  Gln Gln Ala Val Thr  Thr Val Asn Ala Leu  Pro Asn Leu
       8900                8905                8910
   Asn His  Ala Gln Gln Gln Ala  Leu Thr Asp Ala Ile  Asn Ala Ala
       8915                8920                8925
   Pro Thr  Arg Thr Glu Val Ala  Gln His Val Gln Thr  Ala Thr Glu
       8930                8935                8940
   Leu Asp  His Ala Met Glu Thr  Leu Lys Asn Lys Val  Asp Gln Val
       8945                8950                8955
   Asn Thr  Asp Lys Ala Gln Pro  Asn Tyr Thr Glu Ala  Ser Thr Asp
       8960                8965                8970
   Lys Lys  Glu Ala Val Asp Gln  Ala Leu Gln Ala Ala  Glu Ser Ile
       8975                8980                8985
   Thr Asp Pro Thr Asn Gly Ser Asn Ala Asn Lys Asp Ala Val Asp
   8990 8995 9000
   Gln Val Leu Thr Lys Leu Gln Glu Lys Glu Asn Glu Leu Asn Gly
   9005 9010 9015
   Asn Glu Arg Val Ala Glu Ala Lys Thr Gln Ala Lys Gln Thr Ile
   9020 9025 9030
   Asp Gln Leu Thr His Leu Asn Ala Asp Gln Ile Ala Thr Ala Lys
   9035 9040 9045
   Gln Asn Ile Asp Gln Ala Thr Lys Leu Gln Pro Ile Ala Glu Leu
   9050 9055 9060
   Val Asp Gln Ala Thr Gln Leu Asn Gln Ser Met Asp Gln Leu Gln
   9065 9070 9075
   Gln Ala Val Asn Glu His Ala Asn Val Glu Gln Thr Val Asp Tyr
   9080 9085 9090
   Thr Gln Ala Asp Ser Asp Lys Gln Asn Ala Tyr Lys Gln Ala Ile
   9095 9100 9105
   Ala Asp Ala Glu Asn Val Leu Lys Gln Asn Ala Asn Lys Gln Gln
   9110 9115 9120
   Val Asp Gln Ala Leu Gln Asn Ile Leu Asn Ala Lys Gln Ala Leu
   9125 9130 9135
   Asn Gly Asp Glu Arg Val Ala Leu Ala Lys Thr Asn Gly Lys His
   9140 9145 9150
   Asp Ile Asp Gln Leu Asn Ala Leu Asn Asn Ala Gln Gln Asp Gly
   9155 9160 9165
   Phe Lys Gly Arg Ile Asp Gln Ser Asn Asp Leu Asn Gln Ile Gln
   9170 9175 9180
   Gln Ile Val Asp Glu Ala Lys Ala Leu Asn Arg Ala Met Asp Gln
   9185 9190 9195
   Leu Ser Gln Glu Ile Thr Asp Asn Glu Gly Arg Thr Lys Gly Ser
   9200 9205 9210
   Thr Asn Tyr Val Asn Ala Asp Thr Gln Val Lys Gln Val Tyr Asp
   9215 9220 9225
   Glu Thr Val Asp Lys Ala Lys Gln Ala Leu Asp Lys Ser Thr Gly
   9230 9235 9240
   Gln Asn Leu Thr Ala Lys Gln Val Ile Lys Leu Asn Asp Ala Val
   9245 9250 9255
   Thr Ala Ala Lys Lys Ala Leu Asn Gly Glu Glu Arg Leu Asn Asn
   9260 9265 9270
   Arg Lys Ala Glu Ala Leu Gln Arg Leu Asp Gln Leu Thr His Leu
   9275 9280 9285
   Asn Asn Ala Gln Arg Gln Leu Ala Ile Gln Gln Ile Asn Asn Ala
   9290 9295 9300
   Glu Thr Leu Asn Lys Ala Ser Arg Ala Ile Asn Arg Ala Thr Lys
   9305 9310 9315
   Leu Asp Asn Ala Met Gly Ala Val Gln Gln Tyr Ile Asp Glu Gln
   9320 9325 9330
   His Leu Gly Val Ile Ser Ser Thr Asn Tyr Ile Asn Ala Asp Asp
   9335 9340 9345
   Asn Leu Lys Ala Asn Tyr Asp Asn Ala Ile Ala Asn Ala Ala His
   9350 9355 9360
   Glu Leu Asp Lys Val Gln Gly Asn Ala Ile Ala Lys Ala Glu Ala
   9365 9370 9375
   Glu Gln Leu Lys Gln Asn Ile Ile Asp Ala Gln Asn Ala Leu Asn
   9380 9385 9390
   Gly Asp Gln Asn Leu Ala Asn Ala Lys Asp Lys Ala Asn Ala Phe
   9395 9400 9405
   Val Asn Ser Leu Asn Gly Leu Asn Gln Gln Gln Gln Asp Leu Ala
   9410 9415 9420
   His Lys Ala Ile Asn Asn Ala Asp Thr Val Ser Asp Val Thr Asp
   9425 9430 9435
   Ile Val Asn Asn Gln Ile Asp Leu Asn Asp Ala Met Glu Thr Leu
   9440 9445 9450
   Lys His Leu Val Asp Asn Glu Ile Pro Asn Ala Glu Gln Thr Val
   9455 9460 9465
   Asn Tyr  Gln Asn Ala Asp Asp  Asn Ala Lys Thr Asn  Phe Asp Asp
       9470                 9475                 9480
   Ala Lys  Arg Leu Ala Asn Thr  Leu Leu Asn Ser Asp  Asn Thr Asn
       9485                 9490                 9495
   Val Asn  Asp Ile Asn Gly Ala  Ile Gln Ala Val Asn  Asp Ala Ile
       9500                 9505                 9510
   His Asn  Leu Asn Gly Asp Gln  Arg Leu Gln Asp Ala  Lys Asp Lys
       9515                 9520                 9525
   Ala Ile  Gln Ser Ile Asn Gln  Ala Leu Ala Asn Lys  Leu Lys Glu
       9530                 9535                 9540
   Ile Glu  Ala Ser Asn Ala Thr  Asp Gln Asp Lys Leu  Ile Ala Lys
       9545                 9550                 9555
   Asn Lys  Ala Glu Glu Leu Ala  Asn Ser Ile Ile Asn  Asn Ile Asn
       9560                 9565                 9570
   Lys Ala  Thr Ser Asn Gln Ala  Val Ser Gln Val Gln  Thr Ala Gly
       9575                 9580                 9585
   Asn His  Ala Ile Glu Gln Val  His Ala Asn Glu Ile  Pro Lys Ala
       9590                 9595                 9600
   Lys Ile  Asp Ala Asn Lys Asp  Val Asp Lys Gln Val  Gln Ala Leu
       9605                 9610                 9615
   Ile Asp  Glu Ile Asp Arg Asn  Pro Asn Leu Thr Asp  Lys Glu Lys
       9620                 9625                 9630
   Gln Ala  Leu Lys Asp Arg Ile  Asn Gln Ile Leu Gln  Gln Gly His
       9635                 9640                 9645
   Asn Gly  Ile Asn Asn Ala Met  Thr Lys Glu Glu Ile  Glu Gln Ala
       9650                 9655                 9660
   Lys Ala  Gln Leu Ala Gln Ala  Leu Gln Asp Ile Lys  Asp Leu Val
       9665                 9670                 9675
   Lys Ala  Lys Glu Asp Ala Lys  Gln Asp Val Asp Lys  Gln Val Gln
       9680                 9685                 9690
   Ala Leu  Ile Asp Glu Ile Asp  Gln Asn Pro Asn Leu  Thr Asp Lys
   9695 9700 9705
   Glu Lys Gln Ala Leu Lys Tyr Arg Ile Asn Gln Ile Leu Gln Gln
   9710 9715 9720
   Gly His Asn Asp Ile Asn Asn Ala Leu Thr Lys Glu Glu Ile Glu
   9725 9730 9735
   Gln Ala Lys Ala Gln Leu Ala Gln Ala Leu Gln Asp Ile Lys Asp
   9740 9745 9750
   Leu Val Lys Ala Lys Glu Asp Ala Lys Asn Ala Ile Lys Ala Leu
   9755 9760 9765
   Ala Asn Ala Lys Arg Asp Gln Ile Asn Ser Asn Pro Asp Leu Thr
   9770 9775 9780
   Pro Glu Gln Lys Ala Lys Ala Leu Lys Glu Ile Asp Glu Ala Glu
   9785 9790 9795
   Lys Arg Ala Leu Gln Asn Val Glu Asn Ala Gln Thr Ile Asp Gln
   9800 9805 9810
   Leu Asn Arg Gly Leu Asn Leu Gly Leu Asp Asp Ile Arg Asn Thr
   9815 9820 9825
   His Val Trp Glu Val Asp Glu Gln Pro Ala Val Asn Glu Ile Phe
   9830 9835 9840
   Glu Ala Thr Pro Glu Gln Ile Leu Val Asn Gly Glu Leu Ile Val
   9845 9850 9855
   His Arg Asp Asp Ile Ile Thr Glu Gln Asp Ile Leu Ala His Ile
   9860 9865 9870
   Asn Leu Ile Asp Gln Leu Ser Ala Glu Val Ile Asp Thr Pro Ser
   9875 9880 9885
   Thr Ala Thr Ile Ser Asp Ser Leu Thr Ala Lys Val Glu Val Thr
   9890 9895 9900
   Leu Leu Asp Gly Ser Lys Val Ile Val Asn Val Pro Val Lys Val
   9905 9910 9915
   Val Glu Lys Glu Leu Ser Val Val Lys Gln Gln Ala Ile Glu Ser
   9920 9925 9930
   Ile Glu Asn Ala Ala Gln Gln Lys Ile Asn Glu Ile Asn Asn Ser
   9935 9940 9945
   Val Thr Leu Thr Leu Glu Gln Lys Glu Ala Ala Ile Ala Glu Val
   9950 9955 9960
   Asn Lys Leu Lys Gln Gln Ala Ile Asp His Val Asn Asn Ala Pro
   9965 9970 9975
   Asp Val His Ser Val Glu Glu Ile Gln Gln Gln Glu Gln Ala His
   9980 9985 9990
   Ile Glu Gln Phe Asn Pro Glu Gln Phe Thr Ile Glu Gln Ala Lys
   9995 10000 10005
   Ser Asn Ala Ile Lys Ser Ile Glu Asp Ala Ile Gln His Met Ile
   10010 10015 10020
   Asp Glu Ile Lys Ala Arg Thr Asp Leu Thr Asp Lys Glu Lys Gln
   10025 10030 10035
   Glu Ala Ile Ala Lys Leu Asn Gln Leu Lys Glu Gln Ala Ile Gln
   10040 10045 10050
   Ala Ile Gln Arg Ala Gln Ser Ile Asp Glu Ile Ser Glu Gln Leu
   10055 10060 10065
   Glu Gln Phe Lys Ala Gln Met Lys Ala Ala Asn Pro Thr Ala Lys
   10070 10075 10080
   Glu Leu Ala Lys Arg Lys Gln Glu Ala Ile Ser Arg Ile Lys Asp
   10085 10090 10095
   Phe Ser Asn Glu Lys Ile Asn Ser Ile Arg Asn Ser Glu Ile Gly
   10100 10105 10110
   Thr Ala Asp Glu Lys Gln Ala Ala Met Asn Gln Ile Asn Glu Ile
   10115 10120 10125
   Val Leu Glu Thr Ile Arg Asp Ile Asn Asn Ala His Thr Leu Gln
   10130 10135 10140
   Gln Val Glu Ala Ala Leu Asn Asn Gly Ile Ala Arg Ile Ser Ala
   10145 10150 10155
   Val Gln Ile Val Thr Ser Asp Arg Ala Lys Gln Ser Ser Ser Thr
   10160 10165 10170
   Gly Asn   Glu Ser Asn Ser His   Leu Thr Ile Gly Tyr   Gly Thr Ala
       10175                 10180                 10185
   Asn His   Pro Phe Asn Ser Ser   Thr Ile Gly His Lys   Lys Lys Leu
       10190                 10195                 10200
   Asp Glu   Asp Asp Asp Ile Asp   Pro Leu His Met Arg   His Phe Ser
       10205                 10210                 10215
   Asn Asn   Phe Gly Asn Val Ile   Lys Asn Ala Ile Gly   Val Val Gly
       10220                 10225                 10230
   Ile Ser   Gly Leu Leu Ala Ser   Phe Trp Phe Phe Ile   Ala Lys Arg
       10235                 10240                 10245
   Arg Arg   Lys Glu Asp Glu Glu   Glu Glu Leu Glu Ile   Arg Asp Asn
       10250                 10255                 10260
   Asn Lys   Asp Ser Ile Lys Glu   Thr Leu Asp Asp Thr   Lys His Leu
       10265                 10270                 10275
   Pro Leu   Leu Phe Ala Lys Arg   Arg Arg Lys Glu Asp   Glu Glu Asp
       10280                 10285                 10290
   Val Thr   Val Glu Glu Lys Asp   Ser Leu Asn Asn Gly   Glu Ser Leu
       10295                 10300                 10305
   Asp Lys   Val Lys His Thr Pro   Phe Phe Leu Pro Lys   Arg Arg Arg
       10310                 10315                 10320
   Lys Glu   Asp Glu Glu Asp Val   Glu Val Thr Asn Glu   Asn Thr Asp
       10325                 10330                 10335
   Glu Lys   Val Leu Lys Asp Asn   Glu His Ser Pro Leu   Leu Phe Ala
       10340                 10345                 10350
   Lys Arg   Arg Lys Asp Lys Glu   Glu Asp Val Glu Thr   Thr Thr Ser
       10355                 10360                 10365
   Ile Glu   Ser Lys Asp Glu Asp   Val Pro Leu Leu Leu   Ala Lys Lys
       10370                 10375                 10380
   Lys Asn   Gln Lys Asp Asn Gln   Ser Lys Asp Lys Lys   Ser Ala Ser
       10385                 10390                 10395
   Lys Asn   Thr Ser Lys Lys Val   Ala Ala Lys Lys Lys   Lys Lys Lys
       10400                 10405                 10410
   Ala Lys Lys Asn Lys Lys
   10415
<210> 39
   <211> 636
   <212> PRT
   <213> Staphylococcus sp.
<400> 39
   Met Lys Lys Gln Ile Ile Ser Leu Gly Ala Leu Ala Val Ala Ser Ser
   1 5 10 15
   Leu Phe Thr Trp Asp Asn Lys Ala Asp Ala Ile Val Thr Lys Asp Tyr
   20 25 30
   Ser Gly Lys Ser Gln Val Asn Ala Gly Ser Lys Asn Gly Thr Leu Ile
   35 40 45
   Asp Ser Arg Tyr Leu Asn Ser Ala Leu Tyr Tyr Leu Glu Asp Tyr Ile
   50 55 60
   Ile Tyr Ala Ile Gly Leu Thr Asn Lys Tyr Glu Tyr Gly Asp Asn Ile
   65 70 75 80
   Tyr Lys Glu Ala Lys Asp Arg Leu Leu Glu Lys Val Leu Arg Glu Asp
   85 90 95
   Gln Tyr Leu Leu Glu Arg Lys Lys Ser Gln Tyr Glu Asp Tyr Lys Gln
   100 105 110
   Trp Tyr Ala Asn Tyr Lys Lys Glu Asn Pro Arg Thr Asp Leu Lys Met
   115 120 125
   Ala Asn Phe His Lys Tyr Asn Leu Glu Glu Leu Ser Met Lys Glu Tyr
   130 135 140
   Asn Glu Leu Gln Asp Ala Leu Lys Arg Ala Leu Asp Asp Phe His Arg
   145 150 155 160
   Glu Val Lys Asp Ile Lys Asp Lys Asn Ser Asp Leu Lys Thr Phe Asn
   165 170 175
   Ala Ala Glu Glu Asp Lys Ala Thr Lys Glu Val Tyr Asp Leu Val Ser
   180 185 190
   Glu Ile Asp Thr Leu Val Val Ser Tyr Tyr Gly Asp Lys Asp Tyr Gly
   195 200 205
   Glu His Ala Lys Glu Leu Arg Ala Lys Leu Asp Leu Ile Leu Gly Asp
       210                 215                 220
   Thr Asp Asn Pro His Lys Ile Thr Asn Glu Arg Ile Lys Lys Glu Met
   225                 230                 235                 240
   Ile Asp Asp Leu Asn Ser Ile Ile Asp Asp Phe Phe Met Glu Thr Lys
                   245                 250                 255
   Gln Asn Arg Pro Lys Ser Ile Thr Lys Tyr Asn Pro Thr Thr His Asn
               260                 265                 270
   Tyr Lys Thr Asn Ser Asp Asn Lys Pro Asn Phe Asp Lys Leu Val Glu
           275                 280                 285
   Glu Thr Lys Lys Ala Val Lys Glu Ala Asp Asp Ser Trp Lys Lys Lys
       290                 295                 300
   Thr Val Lys Lys Tyr Gly Glu Thr Glu Thr Lys Ser Pro Val Val Lys
   305                 310                 315                 320
   Glu Glu Lys Lys Val Glu Glu Pro Gln Ala Pro Lys Val Asp Asn Gln
                   325                 330                 335
   Gln Glu Val Lys Thr Thr Ala Gly Lys Ala Glu Glu Thr Thr Gln Pro
               340                 345                 350
   Val Ala Gln Pro Leu Val Lys Ile Pro Gln Gly Thr Ile Thr Gly Glu
           355                 360                 365
   Ile Val Lys Gly Pro Glu Tyr Pro Thr Met Glu Asn Lys Thr Val Gln
       370                 375                 380
   Gly Glu Ile Val Gln Gly Pro Asp Phe Leu Thr Met Glu Gln Ser Gly
   385                 390                 395                 400
   Pro Ser Leu Ser Asn Asn Tyr Thr Asn Pro Pro Leu Thr Asn Pro Ile
                   405                 410                 415
   Leu Glu Gly Leu Glu Gly Ser Ser Ser Lys Leu Glu Ile Lys Pro Gln
               420                 425                 430
   Gly Thr Glu Ser Thr Leu Lys Gly Thr Gln Gly Glu Ser Ser Asp Ile
           435                 440                 445
   Glu Val Lys Pro Gln Ala Thr Glu Thr Thr Glu Ala Ser Gln Tyr Gly
   450 455 460
   Pro Arg Pro Gln Phe Asn Lys Thr Pro Lys Tyr Val Lys Tyr Arg Asp
   465 470 475 480
   Ala Gly Thr Gly Ile Arg Glu Tyr Asn Asp Gly Thr Phe Gly Tyr Glu
   485 490 495
   Ala Arg Pro Arg Phe Asn Lys Pro Ser Glu Thr Asn Ala Tyr Asn Val
   500 505 510
   Thr Thr His Ala Asn Gly Gln Val Ser Tyr Gly Ala Arg Pro Thr Tyr
   515 520 525
   Lys Lys Pro Ser Glu Thr Asn Ala Tyr Asn Val Thr Thr His Ala Asn
   530 535 540
   Gly Gln Val Ser Tyr Gly Ala Arg Pro Thr Gln Asn Lys Pro Ser Lys
   545 550 555 560
   Thr Asn Ala Tyr Asn Val Thr Thr His Gly Asn Gly Gln Val Ser Tyr
   565 570 575
   Gly Ala Arg Pro Thr Gln Asn Lys Pro Ser Lys Thr Asn Ala Tyr Asn
   580 585 590
   Val Thr Thr His Ala Asn Gly Gln Val Ser Tyr Gly Ala Arg Pro Thr
   595 600 605
   Tyr Lys Lys Pro Ser Lys Thr Asn Ala Tyr Asn Val Thr Thr His Ala
   610 615 620
   Asp Gly Thr Ala Thr Tyr Gly Pro Arg Val Thr Lys
   625 630 635
<210> 40
   <211> 628
   <212> PRT
   <213> Staphylococcus sp.
<400> 40
   Met Ser Asp Arg Phe Ile Lys Phe Asn Asp Glu Gln Leu Asp Ala Lys
   1               5                   10                  15
   Gln Val Met Met Leu Gln Asp Leu Ala Arg Leu Leu Leu Lys Asn Glu
               20                  25                  30
   Gln Thr Gln Val Lys Ile Gln Lys Phe Pro Tyr Tyr Asn Pro Val Gln
           35                  40                  45
   Asn Val Leu Ile Thr Ser Trp Phe Trp Ser His Arg Pro Ser His Ile
       50                  55                  60
   Glu Met Ala Gly Leu Lys Thr Asp Val Met Leu Ala Ala Tyr Gly Tyr
   65                  70                  75                  80
   His Met Met Asp Val Gln Ile Val Asn Glu Val Val Gln Asp Lys Thr
                   85                  90                  95
   Phe Lys His Pro Lys Phe Tyr Gln Gln Leu Phe Lys Leu Leu Glu Asp
               100                 105                 110
   Met Arg Val Leu Asn Ser Ile Lys Val Glu Arg Pro Ser Thr Ala Lys
           115                 120                 125
   Leu Ile Asp Leu Arg Leu Asp Thr Arg Ile Ser Tyr Thr Glu Ser Gln
       130                 135                 140
   Ile Lys Val Tyr Arg Thr Lys Thr Gln Tyr Thr Asp Leu Leu Phe Leu
   145                 150                 155                 160
   Tyr Leu Glu His Ala Phe Leu Ser Gln Asp Phe Phe Asp Ile Pro Ser
                   165                 170                 175
   Ile His Ser Asp Leu Asp Asp Ile Leu Val Asn Met Phe Leu Tyr Leu
               180                 185                 190
   Pro Asn Phe Phe Gln Asn Gln Asn Ser Glu Asp Asn Met Tyr Leu Ala
           195                 200                 205
   Gln Arg Ile Met Tyr Gln Val Asp Asp Ile Leu Lys Glu Asp Met Leu
       210                 215                 220
   Asn Glu Tyr Tyr Tyr Leu Pro Lys Thr Leu Tyr Asn Thr Leu Ala Ser
   225                 230                 235                 240
   Pro Glu Phe Asp Asp Leu Lys Arg Thr Asp Ala Ser Gln Val Asp Gly
                   245                 250                 255
   Gln Asp Asp Thr Ser Glu Asp Asp Asp Asn Glu Ser Glu Lys Ala Asp
               260                 265                 270
   Ser Lys Ser Ala Asp Ser Glu Ser Lys Gly Gly Ala Tyr Leu Glu Met
           275                 280                 285
   Glu Leu His Glu Gly Gln Asn Ser Glu Thr Leu Gly Asn Asp Glu Ala
       290                 295                 300
   Arg Glu Gly Asp Ala Thr Asp Asp Met Thr Asp Met Met Thr Lys Lys
   305                 310                 315                 320
   Gly Lys Gly Ser Asn Asp Thr Leu Asn Arg Glu Glu Gly Asp Ala Val
                   325                 330                 335
   Gly Gln Ser Gln Ala Phe Gln Leu Asp Gly Val Asn Lys Asn Val Glu
               340                 345                 350
   Ile Lys Trp Gln Ile Pro Glu Ile Glu Pro Gln Tyr Val Leu Glu Tyr
           355                 360                 365
   Gln Glu Ser Lys Gln Asp Val Gln Tyr Glu Ile Lys Asp Leu Ile Gln
       370                 375                 380
   Ile Ile Lys Lys Thr Ile Glu Arg Glu Gln Arg Asp Ala Arg Phe Asn
   385                 390                 395                 400
   Leu Thr Lys Gly Arg Leu Gln Lys Asp Leu Ile Asn Trp Phe Ile Asp
                   405                 410                 415
   Asp Gln Tyr Lys Leu Phe Tyr Lys Lys Gln Asp Leu Ser Lys Ser Phe
               420                 425                 430
   Asp Ala Thr Phe Thr Leu Leu Ile Asp Ala Ser Ala Ser Met His Asp
           435                 440                 445
   Lys Met Ala Glu Thr Lys Lys Gly Val Val Leu Phe His Glu Thr Leu
       450                 455                 460
   Lys Ala Leu Asn Ile Lys His Glu Ile Leu Ser Phe Ser Glu Asp Ala
   465                 470                 475                 480
   Phe Asp Ser Asp Glu His Ala Gln Pro Asn Ile Ile Asn Glu Ile Ile
                   485                 490                 495
   Asn Tyr Asp Tyr Ser Thr Phe Glu Lys Asp Gly Pro Arg Ile Met Ala
               500                 505                 510
   Leu Glu Pro Gln Asp Asp Asn Arg Asp Gly Val Ala Ile Arg Val Ala
           515                 520                 525
   Ser Glu Arg Leu Met Arg Arg Asn Gln His Gln Arg Phe Leu Ile Val
       530                 535                 540
   Phe Ser Asp Gly Glu Pro Ser Ala Phe Asn Tyr Ser Gln Asp Gly Ile
   545 550 555 560
   Ile Asp Thr Tyr Glu Ala Val Glu Met Ser Arg Lys Phe Gly Ile Glu
   565 570 575
   Val Phe Asn Val Phe Leu Ser Gln Asp Pro Ile Thr Glu Asp Val Glu
   580 585 590
   Gln Thr Ile His Asn Ile Tyr Gly Gln Tyr Ala Ile Phe Val Glu Gly
   595 600 605
   Val Ala His Leu Pro Gly His Leu Ser Pro Leu Leu Lys Lys Leu Leu
   610 615 620
   Leu Lys Ser Leu
   625
<210> 41
   <211> 780
   <212> PRT
   <213> Bacillus anthracis
<400> 41
   Met Lys Lys Ser Lys Lys Met Leu Ala Gly Ala Thr Leu Ala Ile Gly
   1 5 10 15
   Val Met Ala Pro Gln Val Leu Pro Ala Thr Ala His Ala Asp Glu Lys
   20 25 30
   Thr Gly Glu Ser Thr Val Asn Leu Arg Ile Leu Glu Thr Ser Asp Ile
   35 40 45
   His Val Asn Leu Met Asn Tyr Asp Tyr Tyr Gln Thr Lys Thr Asp Asn
   50 55 60
   Lys Val Gly Leu Val Gln Thr Ala Thr Leu Val Asn Lys Ala Arg Glu
   65 70 75 80
   Glu Ala Lys Asn Ser Val Leu Phe Asp Asp Gly Asp Ala Leu Gln Gly
   85 90 95
   Thr Pro Leu Gly Asp Tyr Val Ala Asn Lys Ile Asn Asp Pro Lys Lys
   100 105 110
   Pro Val Asp Pro Ser Tyr Thr His Pro Leu Tyr Arg Leu Met Asn Leu
   115 120 125
   Met Lys Tyr Asp Val Ile Ser Leu Gly Asn His Glu Phe Asn Tyr Gly
   130 135 140
   Leu Asp Tyr Leu Asn Lys Val Ile Ser Lys Thr Glu Phe Pro Val Ile
   145 150 155 160
   Asn Ser Asn Val Tyr Lys Asp Asp Lys Asp Asn Asn Glu Glu Asn Asp
   165 170 175
   Gln Asn Tyr Phe Lys Pro Tyr His Val Phe Glu Lys Glu Val Glu Asp
   180 185 190
   Glu Ser Gly Gln Lys Gln Lys Val Lys Ile Gly Val Met Gly Phe Val
   195 200 205
   Pro Pro Gln Val Met Asn Trp Asp Lys Ala Asn Leu Glu Gly Lys Val
   210 215 220
   Lys Ala Lys Asp Ile Val Glu Thr Ala Lys Lys Met Val Pro Lys Met
   225 230 235 240
   Lys Ala Glu Gly Ala Asp Val Ile Val Ala Leu Ala His Ser Gly Val
   245 250 255
   Asp Lys Ser Gly Tyr Asn Val Gly Met Glu Asn Ala Ser Tyr Tyr Leu
   260 265 270
   Thr Glu Val Pro Gly Val Asp Ala Val Leu Met Gly His Ser His Thr
   275 280 285
   Glu Val Lys Asp Val Phe Asn Gly Val Pro Val Val Met Pro Gly Val
   290 295 300
   Phe Gly Ser Asn Leu Gly Ile Ile Asp Met Gln Leu Lys Lys Val Asn
   305 310 315 320
   Gly Lys Trp Glu Val Gln Lys Glu Gln Ser Lys Pro Gln Leu Arg Pro
   325 330 335
   Ile Ala Asp Ser Lys Gly Asn Pro Leu Val Gln Ser Asp Gln Asn Leu
   340 345 350
   Val Asn Glu Ile Lys Asp Asp His Gln Ala Thr Ile Asp Tyr Val Asn
   355 360 365
   Thr Ala Val Gly Lys Thr Thr Ala Pro Ile Asn Ser Tyr Phe Ser Leu
       370                 375                 380
   Val Gln Asp Asp Pro Ser Val Gln Leu Val Thr Asn Ala Gln Lys Trp
   385                 390                 395                 400
   Tyr Val Glu Lys Leu Phe Ala Glu Asn Gly Gln Tyr Ser Lys Tyr Lys
                   405                 410                 415
   Gly Ile Pro Val Leu Ser Ala Gly Ala Pro Phe Lys Ala Gly Gly Arg
               420                 425                 430
   Asn Gly Ala Thr Tyr Tyr Thr Asp Ile Pro Ala Gly Thr Leu Ala Ile
           435                 440                 445
   Lys Asn Val Ala Asp Leu Tyr Val Tyr Pro Asn Thr Leu Tyr Ala Val
       450                 455                 460
   Lys Val Asn Gly Ala Gln Val Lys Glu Trp Leu Glu Met Ser Ala Gly
   465                 470                 475                 480
   Gln Phe Asn Gln Ile Asp Pro Lys Lys Thr Glu Glu Gln Pro Leu Val
                   485                 490                 495
   Asn Ile Gly Tyr Pro Thr Tyr Asn Phe Asp Ile Leu Asp Gly Leu Lys
               500                 505                 510
   Tyr Glu Ile Asp Val Thr Gln Pro Ala Lys Tyr Asp Lys Asp Gly Lys
           515                 520                 525
   Val Val Asn Ala Asn Thr Asn Arg Ile Ile Asn Met Thr Tyr Glu Gly
       530                 535                 540
   Lys Pro Val Ala Asp Asn Gln Glu Phe Ile Val Ala Thr Asn Asn Tyr
   545                 550                 555                 560
   Arg Gly Ser Ser Gln Thr Phe Pro Gly Val Ser Lys Gly Glu Val Val
                   565                 570                 575
   Tyr Gln Ser Gln Asp Glu Thr Arg Gln Ile Ile Val Lys Tyr Met Gln
               580                 585                 590
   Glu Thr Pro Val Ile Asp Pro Ala Ala Asp Lys Asn Trp Ala Phe Lys
           595                 600                 605
   Pro Ile Val Ala Asp Lys Leu Asn Thr Thr Phe Asp Ser Ser Pro Asn
       610                 615                 620
   Ala Gln Lys Tyr Ile Lys Lys Asp Gly Asn Ile Ser Tyr Val Gly Pro
   625                 630                 635                 640
   Ser Glu Asn Glu Phe Ala Lys Tyr Ala Ile Asp Ile Thr Lys Lys Asn
                   645                 650                 655
   Asp Asp Asp Lys Glu Thr Gly Gly Glu Asn Pro Thr Ile Pro Pro Thr
               660                 665                 670
   Gly Glu Gly Asp Asn Gly Glu Asn Pro Thr Thr Pro Pro Thr Gly Glu
           675                 680                 685
   Gly Asn Asn Gly Glu Asn Pro Thr Thr Pro Pro Thr Gly Glu Gly Asn
       690                 695                 700
   Asn Gly Gly Asn Pro Thr Thr Pro Ser Thr Asp Glu Gly Asn Asn Ala
   705                 710                 715                 720
   Gly Ser Gly Gln Thr Thr Thr Asp Asn Gln Asn Ser Lys Glu Thr Thr
                   725                 730                 735
   Thr Val Ser Glu Asn Lys Glu Glu Arg Asp Leu Pro Lys Thr Gly Thr
               740                 745                 750
   Asn Val Ala Ser Thr Ile Gly Ala Gly Leu Ala Phe Ile Gly Ala Gly
           755                 760                 765
   Phe Leu Leu Leu Phe Arg Arg Lys Lys Ala Asn Arg
       770                 775                 780
<210> 42
   <211> 30
   <212> DNA
   <213> Artificial
<220>
   <223> Synthetic primer
<400> 42
   tttcccggga cgatccagct ctaatcgctg 30
<210> 43
   <211> 41
   <212> DNA
   <213> Artificial
<220>
   <223> Synthetic primer
<400> 43
   tttgagctca aagcaaatag ataatcgaga aatataaaaa g 41
<210> 44
   <211> 27
   <212> DNA
   <213> Artificial
<220>
   <223> Synthetic primer
<400> 44
   tttgagctca gttgctccag ccagcat 27
<210> 45
   <211> 28
   <212> DNA
   <213> Artificial
<220>
   <223> Synthetic primer
<400> 45
   tttgaattca aacggattca ttccagcc 28
<210> 46
   <211> 30
   <212> DNA
   <213> Artificial
<220>
   <223> Synthetic primer
<400> 46
   tacgaattcg acttggcagg caattgaaaa 30
<210> 47
   <211> 29
   <212> DNA
   <213> Artificial
<220>
   <223> Synthetic primer
<400> 47
   tgtgaattct tagctagctt ttctacgtc 29
<210> 48
   <211> 30
   <212> DNA
   <213> Artificial
<220>
   <223> Synthetic primer
<400> 48
   tcgggatccg ctgagcagca tacaccaatg 30
<210> 49
   <211> 36
   <212> DNA
   <213> Artificial
<220>
   <223> Synthetic primer
<400> 49
   tgtggatcct tattgattaa tttgttcagc taatgc 36
<210> 50
   <211> 340
   <212> PRT
   <213> Staphylococcus aureus
<400> 50
   Met Lys Lys Lys Leu Leu Val Leu Thr Met Ser Thr Leu Phe Ala Thr
   1               5                   10                  15
   Gln Leu Ile Asn Ser Asn His Ala Asn Ala Ser Val Thr Glu Ser Val
               20                  25                  30
   Asp Lys Lys Phe Val Val Pro Glu Ser Gly Ile Asn Lys Ile Ile Pro
           35                  40                  45
   Thr Tyr Asn Glu Phe Lys Lys Ala Pro Lys Val Asn Val Ser Asn Leu
       50                  55                  60
   Thr Asp Asn Lys Asn Phe Val Ala Ser Glu Asp Lys Leu Lys Lys Ile
   65                  70                  75                  80
   Ser Asp Pro Ser Ala Ala Ser Lys Ile Val Asp Lys Asn Phe Val Val
                   85                  90                  95
   Pro Glu Ser Lys Leu Gly Asn Ile Val Pro Glu Tyr Lys Glu Ile Asn
               100                 105                 110
   Asn Arg Val Asn Val Ala Thr Asn Asn Pro Ala Ser Gln Gln Val Asp
           115                 120                 125
   Lys His Phe Val Ala Lys Gly Pro Glu Val Asn Arg Phe Ile Thr Gln
       130                 135                 140
   Asn Lys Val Asn His His Phe Ile Thr Thr Gln Thr His Tyr Lys Lys
   145                 150                 155                 160
   Val Ile Thr Ser Tyr Lys Ser Thr His Val His Lys His Val Asn His
                   165                 170                 175
   Ala Thr Asp Ser Ile Asn Lys His Phe Ile Val Lys Pro Ser Glu Ala
               180                 185                 190
   Pro Arg Tyr Thr His Pro Ser Gln Ser Leu Met Ile Asn His Tyr Phe
           195                 200                 205
   Ala Val Pro Gly Tyr His Ala His Lys Phe Val Thr Pro Gly His Ala
       210                 215                 220
   Ser Ile Lys Ile Asn His Phe Cys Val Val Pro Gln Ile Asn Ser Phe
   225                 230                 235                 240
   Lys Val Ile Pro Pro Tyr Gly His Asn Ser His Arg Met His Val Pro
                   245                 250                 255
   Ser Phe Gln Asn Asn Thr Thr Ala Thr His Gln Asn Ala Lys Val Asn
               260                 265                 270
   Lys Ala Tyr Asp Tyr Lys Tyr Phe Tyr Ser Tyr Lys Val Val Lys Gly
           275                 280                 285
   Val Lys Lys Tyr Phe Ser Phe Ser Gln Ser Asn Gly Tyr Lys Ile Gly
       290                 295                 300
   Lys Pro Ser Leu Asn Ile Lys Asn Val Asn Tyr Gln Tyr Ala Val Pro
   305                 310                 315                 320
   Ser Tyr Ser Pro Thr Asn Tyr Val Pro Glu Phe Lys Gly Ser Leu Pro
                   325                 330                 335
   Ala Pro Arg Val
               340
<210> 51
   <211> 339
   <212> PRT
   <213> Staphylococcus aureus
<400> 51
   Met Lys Lys Lys Leu Phe Val Leu Thr Met Ser Thr Leu Phe Ala Thr
   1               5                   10                  15
   Gln Leu Ile Asn Ser Asn His Ala Asn Ala Ser Thr Glu Ser Val Asp
               20                  25                  30
   Lys Asn Phe Val Val Pro Glu Ser Gly Ile Asn Lys Ile Ile Pro Thr
           35                  40                  45
   Tyr Asp Glu Phe Lys Lys Ala Pro Lys Val Asn Val Gly Ser Leu Ala
       50                  55                  60
   Asp Asn Lys Asn Phe Val Ala Ser Glu Asp Lys Leu Ser Lys Ile Ala
   65                  70                  75                  80
   Asp Pro Ser Ala Ala Ser Lys Ile Val Asp Lys Asn Phe Val Val Pro
                   85                  90                  95
   Glu Ser Lys Leu Gly Asn Ile Val Pro Glu Tyr Lys Glu Ile Asn Asn
               100                 105                 110
   Arg Val Asn Val Ala Thr Asn Asn Pro Ala Ser Gln Gln Val Asp Lys
           115                 120                 125
   His Phe Val Ala Lys Gly Pro Glu Val Asn Arg Phe Ile Thr Gln Asn
       130                 135                 140
   Lys Val Asn His Pro Phe Ile Thr Thr Gln Thr His Tyr Lys Lys Val
   145                 150                 155                 160
   Ile Thr Ser Tyr Lys Ser Thr His Val His Lys His Val Asn His Ala
                   165                 170                 175
   Thr Gly Ser Ile Asn Lys His Phe Ile Val Lys Pro Ser Glu Ala Pro
               180                 185                 190
   Arg Tyr Thr Gln Pro Ser Gln Ser Leu Met Ile Asn His Tyr Phe Ala
           195                 200                 205
   Val Pro Gly Tyr His Ala His Lys Phe Val Thr Pro Gly His Ala Ser
       210                 215                 220
   Ile Lys Ile Asn His Phe Cys Val Val Pro Gln Ile Asn Ser Phe Lys
   225                 230                 235                 240
   Val Ile Pro Pro Tyr Gly His Asn Ser His Arg Met His Val Pro Ser
                   245                 250                 255
   Phe Gln Asn Asn Thr Thr Ala Thr His Gln Asn Ala Lys Val Lys Lys
               260                 265                 270
   Ala Tyr Asp Tyr Lys Tyr Phe Tyr Ser Tyr Lys Val Val Lys Gly Val
           275                 280                 285
   Lys Lys Tyr Phe Ser Phe Ser Gln Ser Asn Gly Tyr Lys Ile Gly Glu
       290                 295                 300
   Pro Ser Leu Asn Ile Lys Asn Val Asn Tyr Gln Tyr Ala Val Pro Ser
   305 310 315 320
   Tyr Ser Pro Thr His Tyr Val Pro Glu Phe Lys Gly Ser Ile Pro Ala
   325 330 335
   Pro Arg Val
<210> 52
   <211> 340
   <212> PRT
   <213> Staphylococcus aureus
<400> 52
   Met Lys Lys Lys Leu Leu Val Leu Thr Met Ser Thr Leu Phe Ala Thr
   1 5 10 15
   Gln Leu Ile Asn Ser Asn His Ala Lys Ala Ser Val Thr Glu Ser Val
   20 25 30
   Asp Lys Lys Phe Val Val Pro Glu Ser Gly Ile Asn Lys Ile Ile Pro
   35 40 45
   Ala Tyr Asp Glu Phe Lys Asn Ser Pro Lys Val Asn Val Ser Asn Leu
   50 55 60
   Thr Asp Asn Lys Asn Phe Val Val Ser Glu Asp Lys Leu Asn Lys Ile
   65 70 75 80
   Val Asp Ser Ser Ala Ala Ser Lys Ile Val Asp Lys Asn Phe Ala Val
   85 90 95
   Pro Glu Ser Lys Leu Gly Asn Ile Val Pro Glu Tyr Lys Glu Ile Asn
   100 105 110
   Asn Arg Val Asn Val Ala Thr Asn Asn Pro Ala Ser Gln Gln Val Asp
   115 120 125
   Lys His Phe Val Ala Lys Gly Pro Glu Val Asn Arg Phe Ile Thr Gln
   130 135 140
   Asn Lys Val Asn His His Phe Ile Thr Thr Gln Thr His Tyr Lys Lys
   145 150 155 160
   Val Ile Thr Ser Tyr Lys Ser Thr His Val His Lys His Val Asn His
   165 170 175
   Ala Lys Asp Ser Ile Asn Lys His Phe Ile Val Lys Pro Ser Glu Ser
   180 185 190
   Pro Arg Tyr Thr His Pro Ser Gln Ser Leu Ile Ile Lys His His Phe
   195 200 205
   Ala Val Pro Gly Tyr His Ala His Lys Phe Val Thr Pro Gly His Ala
   210 215 220
   Ser Ile Lys Ile Asn His Phe Cys Val Val Pro Gln Ile Asn Ser Phe
   225 230 235 240
   Lys Val Ile Pro Pro Tyr Gly His Asn Ser His Arg Met His Val Pro
   245 250 255
   Ser Phe Gln Asn Asn Thr Thr Ala Thr His Gln Asn Ala Lys Val Asn
   260 265 270
   Lys Ala Tyr Asp Tyr Lys Tyr Phe Tyr Ser Tyr Lys Val Val Lys Gly
   275 280 285
   Val Lys Lys Tyr Phe Ser Phe Ser Gln Ser Asn Gly Tyr Lys Ile Gly
   290 295 300
   Lys Pro Ser Leu Asn Ile Lys Asn Val Asn Tyr Gln Tyr Ala Val Pro
   305 310 315 320
   Ser Tyr Ser Pro Thr His Tyr Val Pro Glu Phe Lys Gly Ser Leu Pro
   325 330 335
   Ala Pro Arg Val
   340
<210> 53
   <211> 340
   <212> PRT
   <213> Staphylococcus aureus
<400> 53
   Met Lys Lys Lys Leu Leu Val Leu Thr Met Ser Thr Leu Phe Ala Thr
   1 5 10 15
   Gln Ile Met Asn Ser Asn His Ala Lys Ala Ser Val Thr Glu Ser Val
   20 25 30
   Asp Lys Lys Phe Val Val Pro Glu Ser Gly Ile Asn Lys Ile Ile Pro
   35 40 45
   Thr Tyr Asn Glu Phe Lys Lys Ala Pro Lys Val Asn Val Gly Asn Leu
   50 55 60
   Ala Asp Asn Lys Asn Phe Val Ala Ser Glu Asp Lys Leu Asn Lys Ile
   65 70 75 80
   Val Asp Ser Ser Ala Ala Ser Lys Ile Val Asp Lys Asn Phe Ala Val
   85 90 95
   Pro Glu Ser Lys Leu Gly Asn Ile Val Pro Glu Tyr Lys Glu Ile Asn
   100 105 110
   Asn Arg Val Asn Val Ala Thr Asn Asn Pro Ala Ser Gln Gln Val Asp
   115 120 125
   Lys His Phe Val Ala Lys Gly Pro Glu Val Asn Arg Phe Ile Thr Gln
   130 135 140
   Asn Lys Val Asn His His Phe Ile Thr Thr Gln Thr His Tyr Lys Lys
   145 150 155 160
   Val Ile Thr Ser Tyr Lys Ser Thr His Val His Lys His Val Asn His
   165 170 175
   Ala Lys Asp Ser Ile Asn Lys His Phe Ile Val Lys Pro Ser Glu Ser
   180 185 190
   Pro Arg Tyr Thr His Pro Ser Gln Ser Leu Ile Ile Lys His His Phe
   195 200 205
   Ala Val Pro Gly Tyr His Ala His Lys Phe Val Thr Pro Gly His Ala
   210 215 220
   Ser Ile Lys Ile Asn His Phe Cys Val Val Pro Gln Ile Asn Ser Phe
   225 230 235 240
   Lys Val Ile Pro Pro Tyr Gly His Asn Ser His Arg Met His Val Pro
   245 250 255
   Ser Phe Gln Asn Asn Thr Thr Ala Thr His Gln Asn Ala Lys Val Asn
   260 265 270
   Lys Ala Tyr Asp Tyr Lys Tyr Phe Tyr Ser Tyr Lys Val Val Lys Gly
           275                 280                 285
   Val Lys Lys Tyr Phe Ser Phe Ser Gln Ser Asn Gly Tyr Lys Ile Gly
       290                 295                 300
   Lys Pro Ser Leu Asn Ile Lys Asn Val Asn Tyr Gln Tyr Ala Val Pro
   305                 310                 315                 320
   Ser Tyr Ser Pro Thr His Tyr Val Pro Glu Phe Lys Gly Ser Leu Pro
                   325                 330                 335
   Ala Pro Arg Val
               340

## Description

### BACKGROUND OF THE INVENTION

### I. FIELD OF THE INVENTION

The present invention relates generally to the fields of immunology, microbiology, and pathology. More particularly, it concerns methods and compositions involving the bacterial protein Emp, which can be used to invoke an immune response against the bacteria or provide passive immunotherapy. The protein includes peptides or proteins comprising Emp amino acid sequences and antibodies that bind the same.

### II. BACKGROUND

The number of both community acquired and hospital acquired infections have increased over recent years. Hospital acquired (nosocomial) infections are a major cause of morbidity and mortality. In the United States, hospital acquired infections affect more than 2 million patients annually. The most frequent infections are urinary tract infections (33% of the infections), followed by pneumonia (15.5%), surgical site infections (14.8%) and primary bloodstream infections (13%) (Emorl and Gaynes, 1993).

*Staphylococcus aureus,* Coagulase-negative Staphylococci (mostly *Staphylococcus epidermidis*), *enterococcus* spp., *Escherichia coli* and *Pseudomonas aeruginosa* are the major nosocomial pathogens. Although those pathogens cause approximately the same number of infections, the severity of the disorders they can produce combined with the frequency of antibiotic resistant isolates balance this ranking towards *S. aureus* and *S. epidermidis* as being the most significant nosocomial pathogens.

*Staphylococcus epidermidis* is a normal skin commensal which is also an important opportunistic pathogen responsible for infections of impaired medical devices and infections at sites of surgery. Medical devices infected by *S. epidermidis* include cardiac pacemakers, cerebrospinal fluid shunts, continuous ambulatory peritoneal dialysis catheters, orthopedic devices and prosthetic heart valves.

*Staphylococcus aureus* is the most common cause of nosocomial infections with significant morbidity and mortality. It can cause osteomyelitis, endocarditis, septic arthritis, pneumonia, abscesses and toxic shock syndrome. *S. aureus* can survive on dry surfaces, increasing the chance of transmission. Any *S. aureus* infection can cause the staphylococcal scalded skin syndrome, a cutaneous reaction to exotoxin absorbed into the bloodstream. It can also cause a type of septicemia called pyaemia that can be life-threatening. Problematically, methicillin-resistant Staphylococcus aureus (MRSA) has become a major cause of hospital-acquired infections.

*S. aureus* and *S. epidermidis* infections are typically treated with antibiotics, with penicillin being the drug of choice, whereas vancomycin is used for methicillin resistant isolates. The percentage of staphylococcal strains exhibiting wide-spectrum resistance to antibiotics has become increasingly prevalent, posing a threat for effective antimicrobial therapy. In addition, the recent emergence of vancomycin resistant *S. aureus* strain has aroused fears that MRSA strains are emerging and spreading for which no effective therapy is available. Zhou et al (Vaccine (2006): 24:4830-4837) discloses an immunogenicity study of a new fusion protein Cna-FnBP.

The first generation of vaccines targeted against *S. aureus* or against the exoproteins it produces have met with limited success (Lee, 1996). There remains a need to develop effective vaccines against staphylococcus infections. Additional compositions for treating staphylococcal infections are also needed.

### SUMMARY OF THE INVENTION

*Staphylococcus aureus* is the single most frequent cause of bacteremia and soft tissue infection in hospitalized or healthy individuals, and dramatic increases in mortality are attributed to the spread of methicillin-resistant *S. aureus* (MRSA) strains that are often not susceptible to antibiotic therapy (Klevens *et al.,* 2007; Klevens *et al.,* 2006). Abscesses with characteristic fibrin deposits and massive immune cell infiltrates represent the pathological substrate of staphylococcal infection (Lowy, 1998). Scanning electron microscopy was used to observe biofilm-like structures at the center of staphylococcal abscesses. Genetic analyses revealed that *in vitro* biofilm formation was correlated with the ability of staphylococci to form abscesses, and the inventors identified envelope proteins that are essential for both processes. When purified and used for immunization of mice, Emp and Eap confers protective immunity to staphylococcal infection. Passive immunization using antibodies that bind Eap or antibodies that bind Emp also demonstrates therapeutic effects.

This application describes in one embodiment the use of Emp, or antibodies that bind all or part of Emp, in methods and compositions for the treatment of bacterial and/or staphylococcal infection. This application also provides an immunogenic composition comprising an Emp antigen or immunogenic fragment thereof. Furthermore, the present invention provides methods and compositions that can be used to treat (*e.g*., limiting staphylococcal abscess formation and/or persistence in a subject) or prevent bacterial infection. Methods for stimulating an immune response involve administering to the subject an effective amount of a composition including or encoding all or part of the Emp polypeptide or antigen, and other bacterial proteins. Other bacterial proteins include, but are not limited to (i) a secreted virulence factor, and/or a cell surface protein or peptide, or (ii) a recombinant nucleic acid molecule encoding a secreted virulence factor, and/or a cell surface protein or peptide, and/or (iii) polysaccharides and the like. Thus, the invention provides an antigen composition comprising:
(a) an isolated Emp antigen that is at least 80% identical to SEQ ID NO:2, or an immunogenic fragment thereof capable of conferring protective immunity to staphylococcal infection; and
(b) at least one additional staphylococcal antigen selected from a group consisting of an isolated ClfA, ClfB, Eap, EsaB, EsxA, EsxB, EsaC, IsdA, IsdB, IsdC, SasF, SasB, SdrC, SdrD, SdrE, SasH, Ebh, Coa, vWa, Hla and SpA antigen, or an immunogenic fragment thereof,
wherein the antigens are comprised in a pharmaceutically acceptable composition and are capable of stimulating an immune response in a subject in need thereof. In other aspects the subject can be administered an Emp modulator, such as an antibody (e.g., a polyclonal, monoclonal, or single chain antibody or fragment thereof) that binds Emp. An Emp modulator can bind Emp directly. The Emp modulator is an antibody that binds Emp. An antibody can be an antibody fragment, a humanized antibody, a human antibody, and/or a monoclonal antibody or the like. In certain aspects, the Emp modulator is elicited by providing an Emp peptide that results in the production of an antibody that binds Emp in the subject or a source subject (*e.g*., donor). The Emp modulator is typically formulated in a pharmaceutically acceptable composition. The Emp modulator composition further comprises at least one staphylococcal antigen or immunogenic fragment thereof, or antibody that binds such (*e.g*., Eap, EsxA, EsxB, EsaB, EsaC, SdrC, SdrD, Hla, SdrE, IsdA, IsdB, SpA, ClfA, ClfB, IsdC, SasB, SasH (AdsA, Ebh, Coa, vWa, or SasF). Additional staphylococcal antigens that can be used in combination with a Emp modulator include, but are not limited to 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK, SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein. The staphylococcal antigen or antibody can be administered concurrently with the Emp modulator. The staphylococcal antigen or antibody and the Emp modulator can be administered in the same composition.

Certain embodiments are directed to a therapeutic composition comprising an isolated antibody, or fragment thereof, that binds an Emp antigen, or an immunogenic fragment thereof, in a pharmaceutically acceptable composition for use in a method of treating a staphylococcus bacterial infection. The antibody can be a human or humanized antibody. In certain aspects the antibody is a polyclonal antibody, or monoclonal antibody, or single chain antibody, or fragment thereof. An antibody composition can further comprise at least one additional isolated antibody that binds an antigen selected from one or more of a group consisting of an isolated ClfA, ClfB, Eap, EsaB, EsaC, EsxA, EsxB, Hla, IsdA, IsdB, IsdC, SasB, SasF, SasH (AdsA), Ebh, Coa, vWa, SdrC, SdrD, SdrE, and SpA antigen, or a fragment thereof. Additional antibodies to a staphylococcal antigen that can be used in combination with a Emp modulator include, but are not limited to antibodies against 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein.

The Emp modulator can also be a recombinant nucleic acid molecule encoding an Emp peptide. A recombinant nucleic acid molecule can encode the Emp peptide and at least one staphylococcal antigen or immunogenic fragment. A nucleic acid can encode or a polypeptide can comprise a number of antigens including 2, 3, 4, 5, 6, 7, 8, 9, 10 or more of one or more of all or part of Emp, Eap, EsaB, EsaC, EsxA, EsxB, SdrC, SdrD, SdrE, Hla, IsdA, IsdB, IsdC, ClfA, ClfB, SasB, SasF, SasH (AdsA), Ebh, Coa, vWa, SpA.

Nucleic acids can encode additional staphylococcal antigens including, but not limited to 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, ibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein.

In certain embodiments the methods and compositions use or include or encode all or part of the Emp polypeptide, peptide, or antigen, as well as antibodies that bind the same. Emp is used in combination with other staphylococcal or bacterial factors such as all or part of Eap, EsaB, EsaC, EsxA, EsxB, SdrC, SdrD, SdrE, Hla IsdA, IsdB, ClfA, ClfB, IsdC, SasB, SasF, SasH (AdsA), Ebh, Coa, vWa, SpA, or immunogenic fragment thereof or combinations thereof. Additional staphylococcal antigens that can be used in combination with a Emp modulator include, but are not limited to 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein. In certain embodiments, 1, 2, 3, 4, 5, 6, 7, 8, 9, 10 or more of EsaB, EsaC, EsxA, EsxB, SdrC, SdrD, SdrE, Hla, IsdA, IsdB, IsdC, ClfA, ClfB, SasB, SasF, SasH (AdsA), Ebh, Coa, vWa, or SpA can be specifically excluded or included from a method, a composition, or a formulation of the invention. Additional staphylococcal antigens that can be explicitly excluded include, but are not limited to 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein.

Embodiments include compositions that contain or do not contain a bacterium. A composition may or may not include an attenuated or viable or intact staphylococcal or other bacterium. In certain aspects, the composition comprises a bacterium that is not a Staphylococcal bacterium or does not contain Staphylococci bacteria. In certain embodiments a bacterial composition comprises an isolated or recombinantly expressed Emp polypeptide or a nucleotide encoding the same. In still further aspects, the isolated Emp polypeptide is multimerized, *e.g*., dimerized. In certain aspects, a composition comprises multimers of 1, 2, 3, 4, 5, 6, 7, 8, 9, 10 or more isolated cell surface proteins or segments thereof. In a further aspect the other polypeptides or peptides can be expressed or included in a bacterial composition including, but not limited to Eap, EsaB, EsaC, EsxA, EsxB, SdrC, SdrD, SdrE, Hla, IsdA, IsdB, ClfA, ClfB, IsdC, SasB, SasF, SasH (AdsA), Ebh, Coa, vWa, or SpA or immunogenic fragments thereof. Additional staphylococcal polypeptides that can be expressed or included in a bacterial composition include, but are not limited to 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein. Alternatively, the composition may be or include a recombinantly engineered Staphylococcus bacterium that has been altered in a way that comprises specifically altering the bacterium with respect to a secreted virulence factor or cell surface protein. For example, the bacteria may be recombinantly modified to express more of the virulence factor or cell surface protein than it would express if unmodified.

The term "Emp polypeptide" refers to polypeptides that include isolated wild-type Emp proteins from staphylococcus bacteria, as well as as well as isolated Emp antigens at least 80% identical to SEQ ID NO: 2 and segments or fragments that stimulate an immune response against staphylococcus bacteria Emp or Eap proteins. Similarly, the term Eap, EsaB, EsaC, EsxA, EsxB, SdrC, SdrD, SdrE, Hla, IsdA, IsdB, ClfA, ClfB, IsdC, SasB, SasF, SasH (AdsA), Ebh, Coa, vWa, or SpA protein refers to a protein that includes isolated wild-type Eap, EsaB, EsaC, EsxA, EsxB, SdrC, SdrD, SdrE, Hla, IsdA, IsdB, ClfA, ClfB, IsdC, SasB, SasF, SasH (AdsA), or SpA polypeptides from staphylococcus bacteria, as well as immunogenic fragments that stimulate an immune response against staphylococcus bacteria Eap, EsaB, EsaC, EsxA, EsxB, SdrC, SdrD, SdrE, Hla, IsdA, IsdB, ClfA, ClfB, IsdC, SasB, SasF, SasH (AdsA), Ebh, Coa, vWa, or SpA proteins. Additionally, the terms 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein refers to a protein that includes isolated wild type 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein from staphylococcus bacteria, as well as variants, segments, or fragments that stimulate an immune response against staphylococcal 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein. An immune response refers to a humoral response, a cellular response, or both a humoral and cellular response in an organism. An immune response can be measured by assays that include, but are not limited to, assays measuring the presence or amount of antibodies that specifically recognize a protein or cell surface protein, assays measuring T-cell activation or proliferation, and/or assays that measure modulation in terms of activity or expression of one or more cytokines.

Embodiments include methods for eliciting an immune response against a staphylococcus bacterium or staphylococci in a subject comprising providing to the subject an effective amount of 1, 2, 3, 4, 5, 6, 7, 8, 9, 10 or more antigens or segments/fragments thereof. Staphylococcal antigens include, but are not limited to an Eap, Emp, EsaB, EsaC, EsxA, EsxB, SdrC, SdrD, SdrE, Hla, IsdA, IsdA, IsdB, ClfA, ClfB, IsdC, SasB, SasF, SasH (AdsA), Ebh, Coa, vWa, or SpA, or immunogenic fragment thereof. Additional Staphylococcal antigens include, but are not limited to 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein.

Emp polypeptides that are at least 80% identical to SEQ ID NO: 2 or immunogenic fragments thereof are provided in combination with one or more antigens or immunogenic fragments of one or more of Eap, EsaB, EsaC, EsxA, EsxB, SdrC, SdrD, SdrE, Hla or a variant thereof, IsdA, IsdB, ClfA, ClfB, IsdC, SasB, SasF, SasH (AdsA), Ebh, Coa, vWa, or SpA. Additional antigens or immunogenic fragments of 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein can also be used.

Embodiments include compositions that may include a polypeptide, peptide, or protein that has or has at least 70%, 75%, 80%, 85%, 90%, 95%, 96%, 97%, 98%, or 99% identity or similarity to Emp, Eap EsaB, EsaC, EsxA, EsxB, SdrC, SdrD, SdrE, Hla or a variant thereof, IsdA, IsdB, ClfA, ClfB, IsdC, SasB, SasF, SasH (AdsA), Ebh, Coa, vWa, SpA, 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein over 5, 10, 15, 20, 50, 100, 200, or more consecutive amino acids including all values and ranges there between. In a further embodiment of the invention a composition may include a polypeptide, peptide, or protein that is or is at least 70%, 75%, 80%, 85%, 90%, 95%, 96%, 97%, 98%, or 99% identical or similar to an Emp polypeptide (SEQ ID NO:2, 50-53) and/or Eap polypeptide (SEQ ID NO:4) or Emp nucleic acid (SEQ ID NO:1) and/or Eap nucleic acid (SEQ ID NO:3). In certain aspects the Emp polypeptide or Eap polypeptide will have an amino acid sequence of (SEQ ID NO:2) or (SEQ ID NO:4), respectively. Similarity or identity, with identity being preferred, is known in the art and a number of different programs can be used to identify whether a protein (or nucleic acid) has sequence identity or similarity to a known sequence. Sequence identity and/or similarity is determined using standard techniques known in the art, including, but not limited to, the local sequence identity algorithm of Smith & Waterman (1981), by the sequence identity alignment algorithm of Needleman & Wunsch (1970), by the search for similarity method of Pearson & Lipman (1988), by computerized implementations of these algorithms (GAP, BESTFIT, FASTA, and TFASTA in the Wisconsin Genetics Software Package, Genetics Computer Group, 575 Science Drive, Madison, Wis.), the Best Fit sequence program described by Devereux *et al.* (1984), preferably using the default settings, or by inspection. Preferably, percent identity is calculated by using alignment tools known to and readily ascertainable to those of skill in the art.

The term "AdsA polypeptide" refers to polypeptides that include isolated wild-type bacterial AdsA proteins, *e.g*., staphylococcus (*S. aureus* SEQ ID NO:36) or bacillus (*B. anthracis* SEQ ID NO:41) bacteria, as well as variants and segments or fragments of AdsA proteins. In certain aspects, the AdsA polypeptide stimulates an immune response against bacterial AdsA proteins.

In still further embodiments of the invention a composition may include a polypeptide, peptide, or protein that is or is at least 70%, 75%, 80%, 85%, 90%, 95%, 96%, 97%, 98%, or 99% identical or similar to an EsxA protein. In certain aspects the EsxA protein will have the amino acid sequence of SEQ ID NO:6.

In still further embodiments of the invention a composition may include a polypeptide, peptide, or protein that is or is at least 70%, 75%, 80%, 85%, 90%, 95%, 96%, 97%, 98%, or 99% identical or similar to an EsxB protein. In certain aspects the EsxB protein will have the amino acid sequence of SEQ ID NO:8.

In yet still further embodiments of the invention a composition may include a polypeptide, peptide, or protein that is or is at least 70%, 75%, 80%, 85%, 90%, 95%, 96%, 97%, 98%, or 99% identical or similar to an SdrD protein. In certain aspects the SdrD protein will have the amino acid sequence of SEQ ID NO:10.

In further embodiments of the invention a composition may include a polypeptide, peptide, or protein that is or is at least 70%, 75%, 80%, 85%, 90%, 95%, 96%, 97%, 98%, or 99% identical or similar to an SdrE protein. In certain aspects the SdrE protein will have the amino acid sequence of SEQ ID NO:12.

In still further embodiments of the invention a composition may include a polypeptide, peptide, or protein that is or is at least 70%, 75%, 80%, 85%, 90%, 95%, 96%, 97%, 98%, or 99% identical or similar to an IsdA protein. In certain aspects the IsdA protein will have the amino acid sequence of SEQ ID NO:14.

In yet still further embodiments of the invention a composition may include a polypeptide, peptide, or protein that is or is at least 70%, 75%, 80%, 85%, 90%, 95%, 96%, 97%, 98%, or 99% identical or similar to an IsdB protein. In certain aspects the IsdB protein will have the amino acid sequence of SEQ ID NO:16.

Embodiments of the invention include compositions that include a polypeptide, peptide, or protein that is or is at least 70%, 75%, 80%, 85%, 90%, 95%, 96%, 97%, 98%, or 99% identical or similar to a SpA protein. In certain aspects the SpA protein will have the amino acid sequence of SEQ ID NO:18.

In a further embodiments of the invention a composition may include a polypeptide, peptide, or protein that is or is at least 70%, 75%, 80%, 85%, 90%, 95%, 96%, 97%, 98%, or 99% identical or similar to a ClfB protein. In certain aspects the ClfB protein will have the amino acid sequence of SEQ ID NO:20.

In still further embodiments of the invention a composition may include a polypeptide, peptide, or protein that is or is at least 70%, 75%, 80%, 85%, 90%, 95%, 96%, 97%, 98%, or 99% identical or similar to an IsdC protein. In certain aspects the IsdC protein will have the amino acid sequence of SEQ ID NO:22.

In yet further embodiments of the invention a composition may include a polypeptide, peptide, or protein that is or is at least 70%, 75%, 80%, 85%, 90%, 95%, 96%, 97%, 98%, or 99% identical or similar to a SasF protein. In certain aspects the SasF protein will have the amino acid sequence of SEQ ID NO:24.

In yet still further embodiments of the invention a composition may include a polypeptide, peptide, or protein that is or is at least 70%, 75%, 80%, 85%, 90%, 95%, 96%, 97%, 98%, or 99% identical or similar to an SdrC protein. In certain aspects the SdrC protein will have the amino acid sequence of SEQ ID NO:26.

In yet still further embodiments of the invention a composition may include a polypeptide, peptide, or protein that is or is at least 70%, 75%, 80%, 85%, 90%, 95%, 96%, 97%, 98%, or 99% identical or similar to an ClfA protein. In certain aspects the ClfA protein will have the amino acid sequence of SEQ ID NO: 28.

In yet still further embodiments of the invention a composition may include a polypeptide, peptide, or protein that is or is at least 70%, 75%, 80%, 85%, 90%, 95%, 96%, 97%, 98%, or 99% identical or similar to an EsaB protein. In certain aspects the EsaB protein will have the amino acid sequence of SEQ ID NO: 30.

In yet still further embodiments of the invention a composition may include a polypeptide, peptide, or protein that is or is at least 70%, 75%, 80%, 85%, 90%, 95%, 96%, 97%, 98%, or 99% identical or similar to an EsaC protein. In certain aspects the EsaC protein will have the amino acid sequence of SEQ ID NO: 32.

In yet still further embodiments of the invention a composition may include a polypeptide, peptide, or protein that is or is at least 70%, 75%, 80%, 85%, 90%, 95%, 96%, 97%, 98%, or 99% identical or similar to an SasB protein. In certain aspects the SasB protein will have the amino acid sequence of SEQ ID NO: 34.

In yet still further embodiments of the invention a composition may include a polypeptide, peptide, or protein that is or is at least 70%, 75%, 80%, 85%, 90%, 95%, 96%, 97%, 98%, or 99% identical or similar to an SasH (AdsA) protein. In certain aspects the SasH (AdsA) protein will have the amino acid sequence of SEQ ID NO: 36 or SEQ ID NO:41.

In yet still further embodiments of the invention a composition may include a polypeptide, peptide, or protein that is or is at least 70%, 75%, 80%, 85%, 90%, 95%, 96%, 97%, 98%, or 99% identical or similar to an Hla protein. In certain aspects the Hla protein will have the amino acid sequence of SEQ ID NO: 37. In certain aspects, a variant Hla includes amino acid substitutions or D24C, H35C, H35K, H35L, R66C, E70C, or K110C, or any combination thereof (amino acids referred to using single letter code).

In yet still further embodiments of the invention a composition may include a polypeptide, peptide, or protein that is or is at least 70%, 75%, 80%, 85%, 90%, 95%, 96%, 97%, 98%, or 99% identical or similar to an Ebh protein. In certain aspects the Ebh protein will have the amino acid sequence of SEQ ID NO:38.

In yet still further embodiments of the invention a composition may include a polypeptide, peptide, or protein that is or is at least 70%, 75%, 80%, 85%, 90%, 95%, 96%, 97%, 98%, or 99% identical or similar to an Coa protein. In certain aspects the Coa protein will have the amino acid sequence of SEQ ID NO:39.

In yet still further embodiments of the invention a composition may include a polypeptide, peptide, or protein that is or is at least 70%, 75%, 80%, 85%, 90%, 95%, 96%, 97%, 98%, or 99% identical or similar to an vWa protein. In certain aspects the vWa protein will have the amino acid sequence of SEQ ID NO: 40. In certain aspects, a polypeptide or segment/fragment can have a sequence that is at least 85%, at least 90%, at least 95%, at least 98% or at least 99% or more identical to the amino acid sequence of the reference polypeptide. The term "similarity" refers to a polypeptide that has a sequence that has a certain percentage of amino acids that are either identical with the reference polypeptide or constitute conservative substitutions with the reference polypeptides.

The polypeptides or segments or fragments described herein may include the following, or at least, or at most 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 300, 350, 400, 450, 500, 550 or more contiguous amino acids, or any range derivable therein, of SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO:26, SEQ ID NO:28, SEQ ID NO:30, SEQ ID NO:32, SEQ ID NO:34, SEQ ID NO:36, SEQ ID NO:37, SEQ ID NO:38, SEQ ID NO:39, SEQ ID NO:40, SEQ ID NO:41,or SEQ ID NO:41.

In further embodiments a composition comprises a recombinant nucleic acid molecule encoding 1, 2, 3, 4, or more of Eap, Emp, EsaB, EsaC, EsxA, EsxB, SdrC, SdrD, SdrE, Hla, IsdA, IsdB, ClfA, ClfB, IsdC, SasB, SasF, SasH (AdsA), SpA, Ebh, Coa, vWa, 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein.

Still further embodiments include methods for stimulating in a subject a protective or therapeutic immune response against a staphylococcus bacterium comprising administering to the subject an effective amount of a composition including (i) an Emp polypeptide or peptide thereof; or, (ii) a nucleic acid molecule encoding an Emp polypeptide or peptide thereof, or (iii) administering an Emp polypeptide with any combination or permutation of bacterial proteins or polysaccharides described herein. Yet still further embodiments include vaccines comprising a pharmaceutically acceptable composition having an isolated Emp polypeptide, or segment or fragment thereof, or any other combination or permutation of protein(s) or peptide(s) or polysaccharide(s) described, wherein the composition is capable of stimulating an immune response against a staphylococcus bacterium. The vaccine may comprise an isolated Emp polypeptide, and/or any other combination or permutation of protein(s) or peptide(s) or polysaccharide(s) described. In certain aspects of the invention the isolated Emp polypeptide, or any other combination or permutation of protein(s) or peptide(s) or polysaccharide(s) described are multimerized, e.g., dimerized.

In a further aspect, the vaccine composition is contaminated by less than about 10, 9, 8, 7, 6, 5, 4, 3, 2, 1, 0.5, 0.25, 0.05% (or any range derivable therein) of other Staphylococcal proteins. A composition may further comprise an isolated non-Emp polypeptide. Typically the vaccine comprises an adjuvant. In certain aspects a protein or peptide described herein is linked (covalently or non-covalently coupled) to the adjuvant, preferably the adjuvant is chemically conjugated to the protein.

In still yet further embodiments, a vaccine composition is a pharmaceutically acceptable composition having a recombinant nucleic acid encoding all or part of an Emp polypeptide, and/or any other combination or permutation of protein(s) or peptide(s) described, wherein the composition is capable of stimulating an immune response against a staphylococcus bacteria. The vaccine composition may comprise a recombinant nucleic acid encoding all or part of an Emp polypeptide, and/or any other combination or permutation of protein(s) or peptide(s) described. In certain embodiments the recombinant nucleic acid contains a heterologous promoter. Preferably the recombinant nucleic acid is a vector. More preferably the vector is a plasmid or a viral vector.

Still further embodiments include methods for stimulating in a subject a protective or therapeutic immune response against a staphylococcus bacterium comprising administering to the subject an effective amount of a composition of an Emp polypeptide or segment/fragment thereof comprising one or more of (i) a Eap, EsaB, EsaC, EsxA, EsxB, SdrC, SdrD, SdrE, Hla, IsdA, IsdB, ClfA, ClfB, IsdC, SasB, SasF, SasH (AdsA), SpA, Ebh, Coa, vWa, 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein polypeptide or segment or fragment thereof; or, (ii) a nucleic acid molecule encoding the same. Methods described herein also include Emp compositions that contain 1, 2, 3, 4, 5, 6, 7, 8, 9, 10 or more of Eap, EsaB, EsaC, EsxA, EsxB, SdrC, SdrD, SdrE, Hla, IsdA, IsdB, ClfA, ClfB, IsdC, SasB, SasF, SasH (AdsA), SpA, Ebh, Coa, vWa, 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein in various combinations. In certain aspects a vaccine formulation includes an IsdA polypeptide or immunogenic fragment thereof.

A further aspect includes a staphylococcal bacterium lacking an Emp and/or Eap polypeptide and/or EsaB polypeptide. Such a bacterium will be limited or attenuated with respect to prolonged or persistent abscess formation and/or biofilm formation. This characteristic can be used to provide bacterial strains for the production of attenuated bacteria for use in the preparation of vaccines or treatments for staphylococcal infections or related diseases. In yet a further aspect, Emp and/or Eap can be overexpressed in an attenuated bacterium to further enhance or supplement an immune response or vaccine formulation.

Any embodiment discussed with respect to one aspect described herein applies to other aspects described herein as well. In particular, any embodiment discussed in the context of an Emp peptide or nucleic acid may be implemented with respect to Eap, EsaB, EsaC, EsxA, EsxB, SdrC, SdrD, SdrE, Hla, IsdA, IsdB, ClfA, ClfB, IsdC, SasB, SasF, SasH (AdsA), SpA, Ebh, Coa, vWa, 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding proteins or nucleic acids or antibodies that bind the same, and vice versa. The term "providing" or "administering" is used according to its ordinary meaning to indicate "to supply or furnish for use." In some embodiments, the protein is provided directly by administering the protein, while in other embodiments, the protein is effectively provided or administered by administering a nucleic acid that encodes the protein. In certain aspects contemplated are compositions comprising various combinations of antibodies, nucleic acid, antigens, peptides, epitopes, and/or polysaccharides and the like.

The subject typically will have (e.g., diagnosed with a persistent staphylococcal infection), will be suspected of having, or will be at risk of developing a staphylococcal infection. Compositions of the present invention include immunogenic compositions wherein the antigen(s) or epitope(s) are contained in an amount effective to achieve the intended purpose (e.g., treating or preventing infection). More specifically, an effective amount means an amount of active ingredients necessary to stimulate or elicit an immune response, or provide resistance to, amelioration of, or mitigation of infection. In more specific aspects, an effective amount prevents, alleviates, or ameliorates symptoms of disease or infection, or prolongs the survival of the subject being treated. Determination of the effective amount is well within the capability of those skilled in the art, especially in light of the detailed disclosure provided herein. For any preparation used in the methods of the invention, an effective amount or dose can be estimated initially from *in vitro,* cell culture, and/or animal model assays. For example, a dose can be formulated in animal models to achieve a desired immune response or circulating antibody concentration or titer. Such information can be used to more accurately determine useful doses in humans.

As used herein, the term "modulate" or "modulation" encompasses the meanings of the words "enhance," or "inhibit." "Modulation" of activity may be either an increase or a decrease in activity. As used herein, the term "modulator" refers to compounds that effect the function of a moiety, including up-regulation, induction, stimulation, potentiation, inhibition, down-regulation, or suppression of a protein, nucleic acid, gene, organism or the like.

The term "isolated" can refer to a nucleic acid or polypeptide or peptide that is substantially free of cellular material, bacterial material, viral material, or culture medium (when produced by recombinant DNA techniques or isolated from naturally occurring organism(s)) of their source of origin, or chemical precursors or other chemicals (when chemically synthesized). Moreover, an isolated compound refers to one that can be administered to a subject as an isolated compound; in other words, the compound may not simply be considered "isolated" if it is adhered to a column or embedded in an agarose gel. Moreover, an "isolated nucleic acid fragment" or "isolated peptide" is a nucleic acid or protein fragment that does not naturally occur and/or function as a fragment and/or is not typically in the functional state.

Moieties of the invention, such as antibodies, polypeptides, peptides, antigens or immunogens, may be conjugated or linked covalently or noncovalently to other moieties such as adjuvants, proteins, peptides, supports, fluorescence moieties, or labels. The term "conjugate" or "immunoconjugate" is broadly used to define the operative association of one moiety with another agent and is not intended to refer solely to any type of operative association, and is particularly not limited to chemical "conjugation." Recombinant fusion proteins are particularly contemplated. Compositions of the invention may further comprise an adjuvant or a pharmaceutically acceptable excipient. An adjuvant may be covalently or non-covalently coupled to a polypeptide or peptide of the invention. In certain aspects, the adjuvant is chemically conjugated to a protein, polypeptide, or peptide. In still a further aspect the adjuvant is part of a recombinant protein and is comprised in a fusion protein comprising one or more antigens of interest.

The compositions may be formulated in a pharmaceutically acceptable composition. In certain aspects of the invention the staphylococcus bacterium is an *S. aureus* bacterium.

In further aspects a composition may be administered more than one time to the subject, and may be administered 1, 2, 3, 4, 5, 6, 7, 8, 9, 10, 15, 20 or more times. The administration of the compositions include, but is not limited to nasal, pleural, oral, parenteral, subcutaneous, intramuscular, intravenous administration, or various combinations thereof, including inhalation or aspiration.

In still further embodiments, a composition comprises a recombinant nucleic acid molecule encoding an Emp polypeptide or immunogenic fragment thereof. Typically a recombinant nucleic acid molecule encoding an Emp polypeptide contains a heterologous promoter.

In certain aspects, a recombinant nucleic acid molecule is a vector, in still other aspects the vector is a plasmid. In certain embodiments the vector is a viral vector. Aspects include compositions that further comprise a nucleic acid encoding an additional 1, 2, 3, 4, 5, 6, 7, 8, or more polypeptides or peptides.

In certain aspects a composition includes a recombinant, non-staphylococcus bacterium containing or expressing one or more polypeptides described herein in, *e.g*., an Emp polypeptide. In particular aspects the recombinant non-staphylococcus bacteria is *Salmonella* or another gram-positive bacteria. A composition is typically administered to mammals, such as human subjects, but administration to other animals capable of eliciting an immune response is contemplated. In further aspects the staphylococcus bacterium containing or expressing the Emp polypeptide is a *Staphylococcus aureus.* In further embodiments the immune response is a protective immune response. Compositions of the invention are typically administered to human subjects, but administration to other animals that are capable of eliciting an immune response to a staphylococcus bacterium is contemplated, particularly mice, dogs, cats, cattle, horses, goats, sheep and other domestic animals, *i.e*., mammals, including transgenic animals (e.g., animal manipulated to express human antibodies).

In still further aspects, the methods and compositions of the invention can be used to prevent, ameliorate, reduce, or treat infection of tissues or glands, e.g., mammary glands, particularly mastitis and other infections. Other methods include, but are not limited to prophylatically reducing bacterial burden in a subject not exhibiting signs of infection, particularly those subjects suspected of or at risk of being colonized by a target bacteria, e.g., patients that are or will be at risk or susceptible to infection during a hospital stay, treatment, and/or recovery.

### DESCRIPTION OF THE DRAWINGS

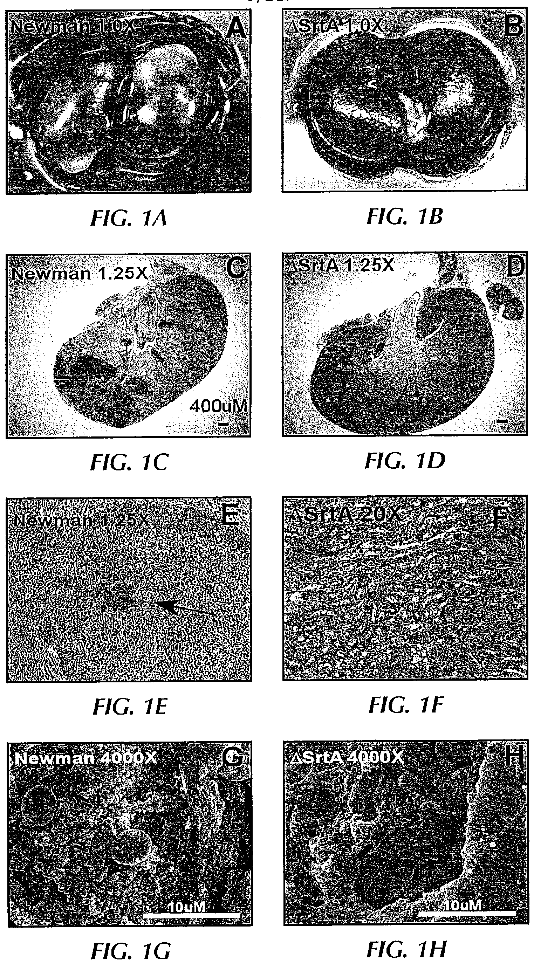

**FIGs**. **1A**-**1H** Examination of abscess formation in staphylococcus aureus. (FIG. 1A) Photograph of Newman infected kidneys. (FIG. 1B) Photograph of srtA mutant infected kidneys. (FIG. 1C) H&E histological section of Newman infected kidney. (FIG. 1D) Histological section of ΔSrtA infected kidney. (FIG. 1E) Closeup of Newman infected kidney. (FIG. 1F) Closeup of ΔSrtA infected kidney. (FIG. 1G) Scanning Electron Microscopy of Newman abscess. (FIG. 1H) SEM of ΔSrtA infected kidney.

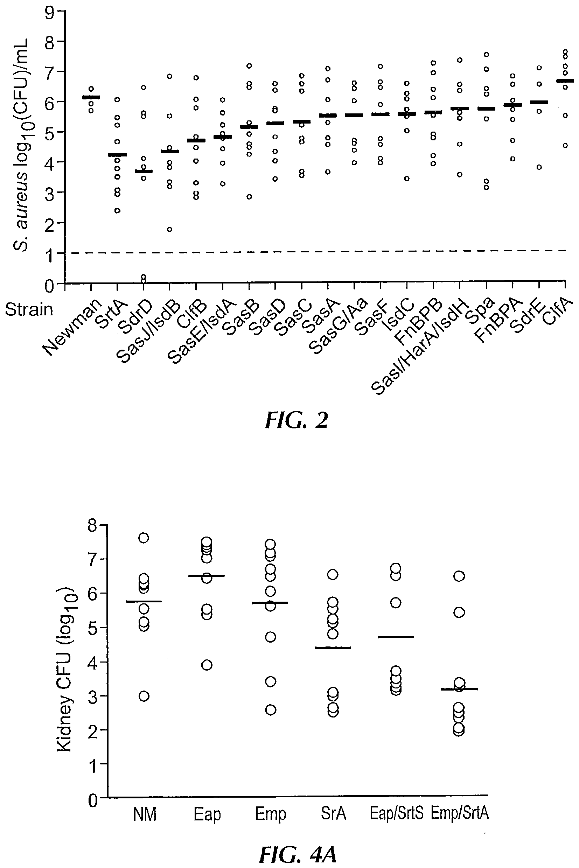

**FIG. 2** Murine renal abscess screen. Recovered colony forming units (CFU) from kidneys infected with respective mutant strains.

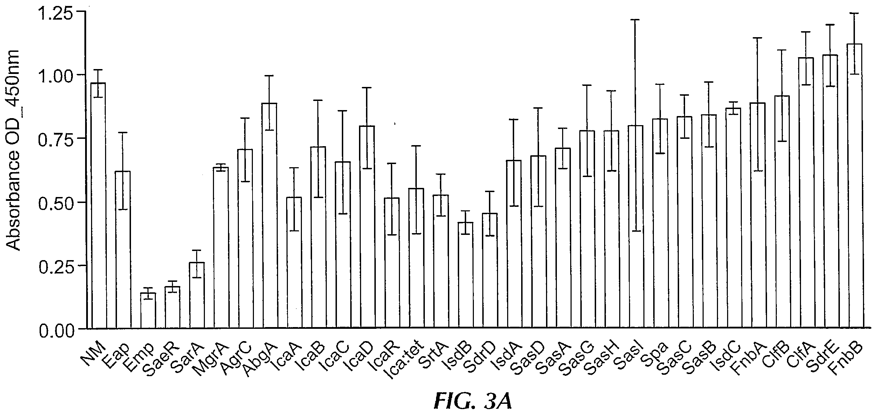

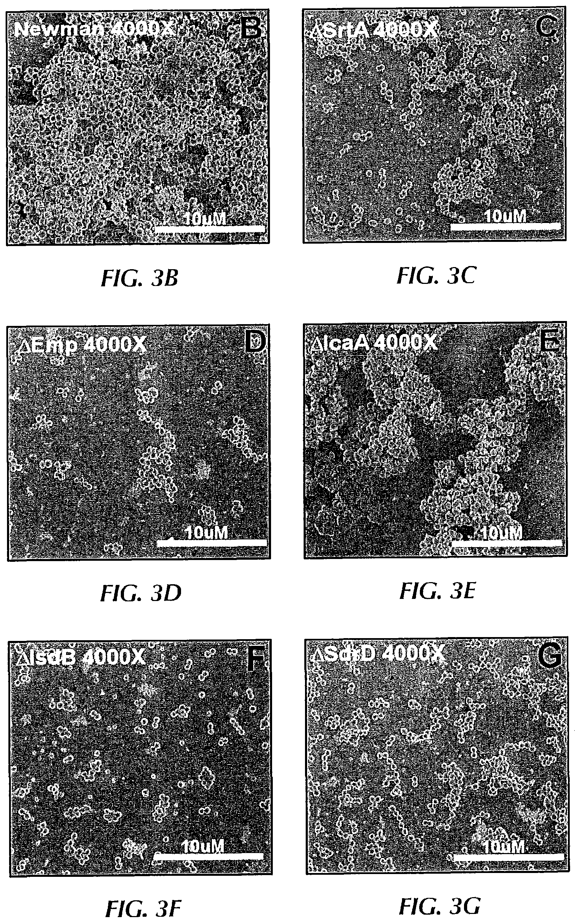

**FIGs. 3A-3G** Biofilm screen. (FIG. 3A) 96 well plate assay for *in vitro* biofilm growth. (FIG. 3B) SEM Newman *in vitro* biofilm (FIG. 3C) ΔsrtA biofilm. (FIG. 3D) ΔEmp biofilm. (FIG. 3E) ΔIcaA biofilm. (FIG. 3F) ΔIsdB biofilm. (FIG. 3G) ΔSdrD biofilm.

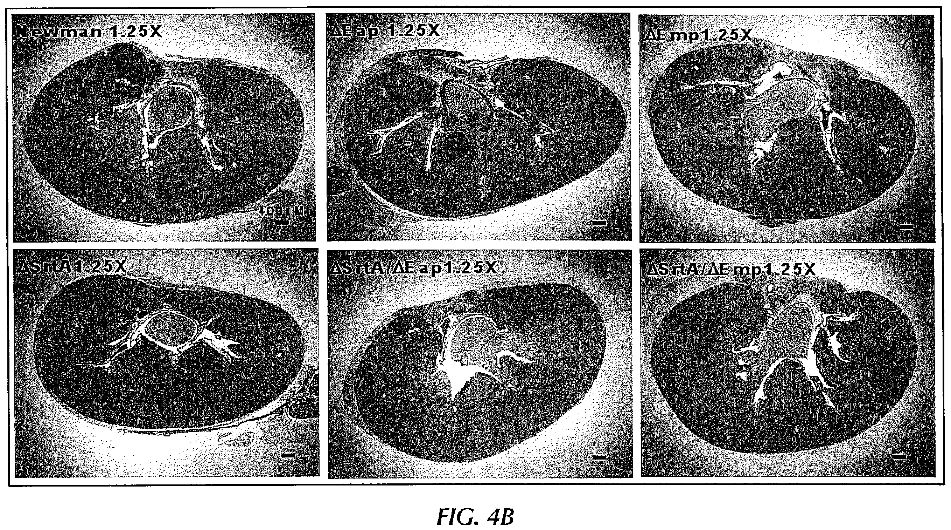

**FIGS. 4A-4B** Emp virulence. (FIG. 4A) Recovered CFUs for Newman, ΔEap, ΔEmp, ΔSrtA, ΔEap/ΔSrtA, ΔEmp/ΔSrtA. (FIG. 4B) Histopathology for respective strains.

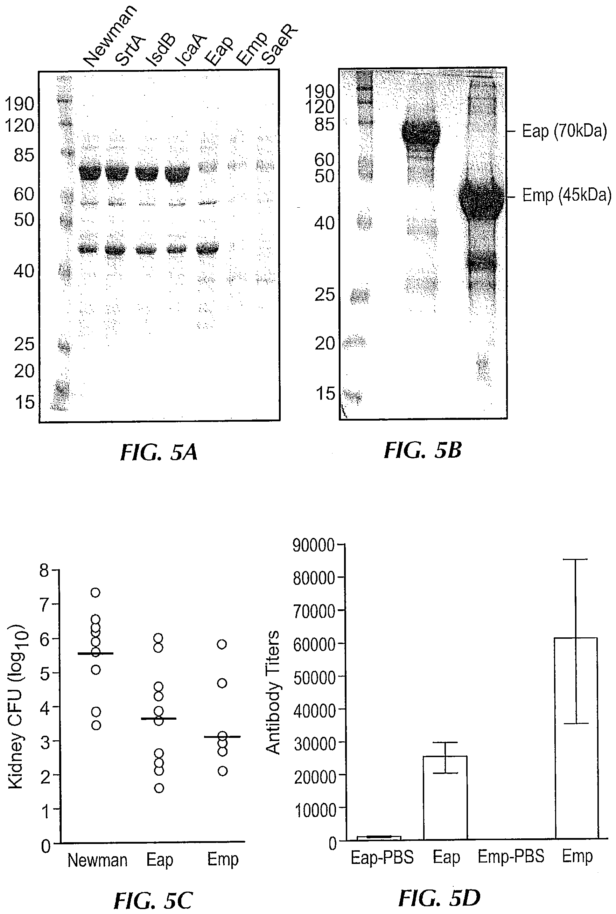

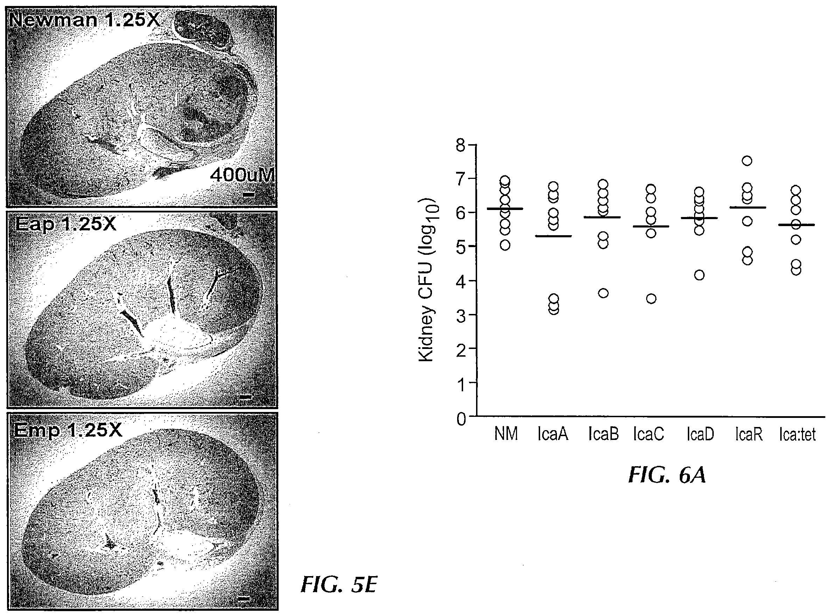

**FIGs. 5A**-**5E** Eap, Emp vaccination. (FIG. 5A) SDS extraction of Newman, ΔSrtA, ΔIsdB, ΔIcaA, ΔEap, ΔEmp, ΔSaeR. (FIG. 5B) Protein purification of Eap (70kDa) and Emp (45kDa). (FIG. 5C) Recovered CFUs from mice vaccinated with PBS, Eap, or Emp and challenged with Newman. (FIG. 5D) ELISA IgG titers from vaccinated mice. (FIG. 5E) Histopathology from vaccinated mice.

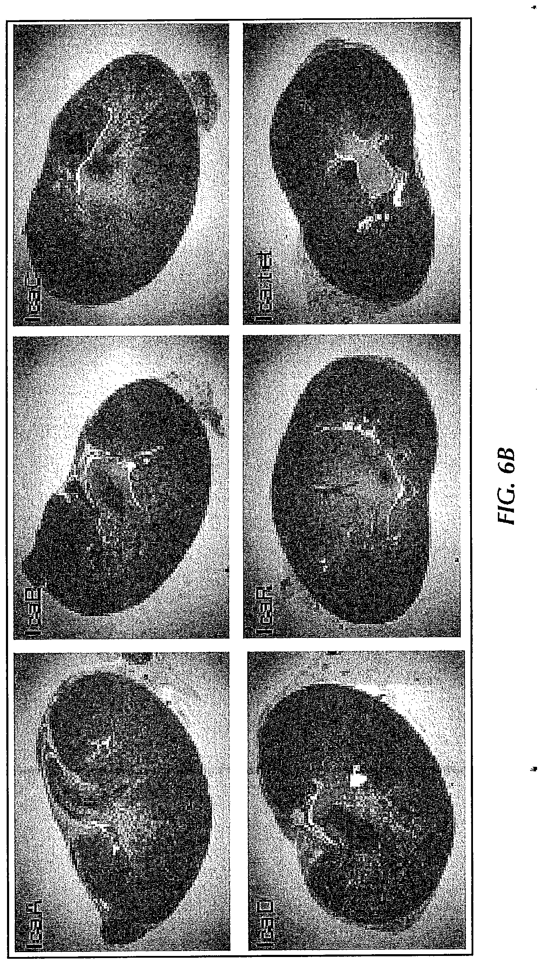

**FIGs. 6A-6B** Ica virulence. (FIG. 6A) Recovered CFUs from mice infected with Newman, DIcaA, DIcaB, DIcaC, DIcaD, DIcaR, DIca:tet (entire operon deletion). (FIG. 6B) Histopathology from infected mice.

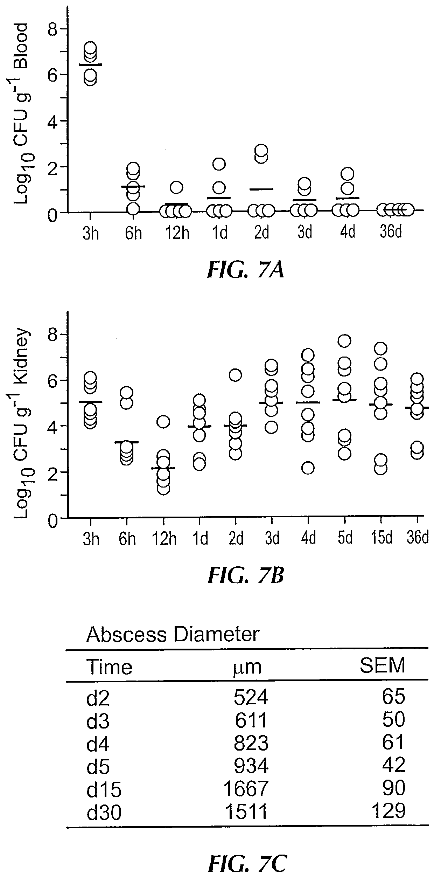

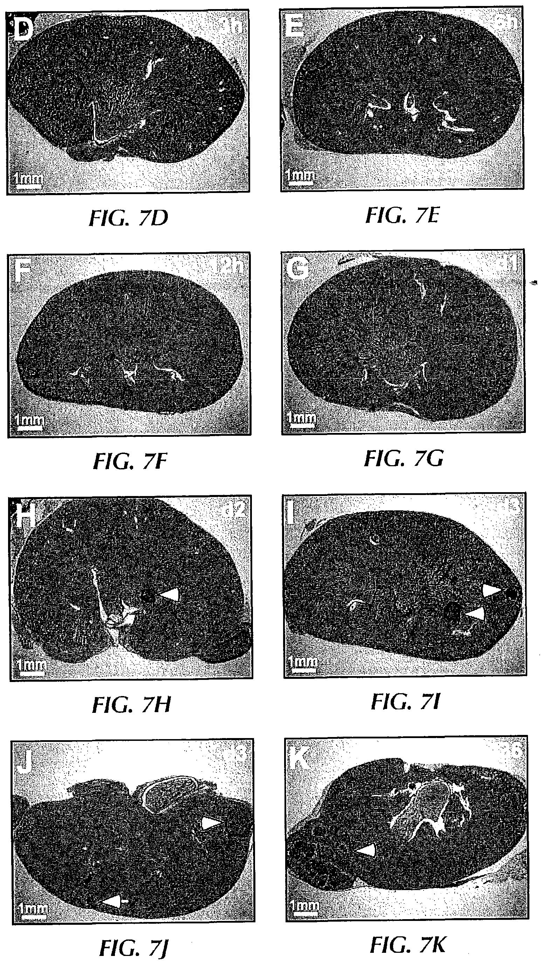

**FIGs. 7A-7K** Staphylococcal abscess formation following intravenous infection of mice. (A) BALB/c mice were infected with 1 × 10₇ colony forming units (CFU) of *S. aureus* Newman by retro-orbital injection. Cohorts of five mice were examined by cardiac puncture at timed intervals for bacterial load in blood; sample aliquots were plated on agar medium and CFU per ml of blood were enumerated. The means of these observations is indicated by a black bar. (B) Dissemination of *S. aureus* Newman into peripheral organ tissues and replication of the pathogen was measured at timed intervals in the kidneys of mice (cohorts of ten animals), which were homogenized and plated on agar medium for CFU. (C) Diameter of abscess lesions were measured in thin-sectioned hematoxylin-eosin stained tissues of infected kidneys at timed intervals. (D-K) Images of infected kidneys at timed intervals analyzed in thin-sectioned hematoxylin-eosin stained tissues. Arrowheads point to abscess lesions.

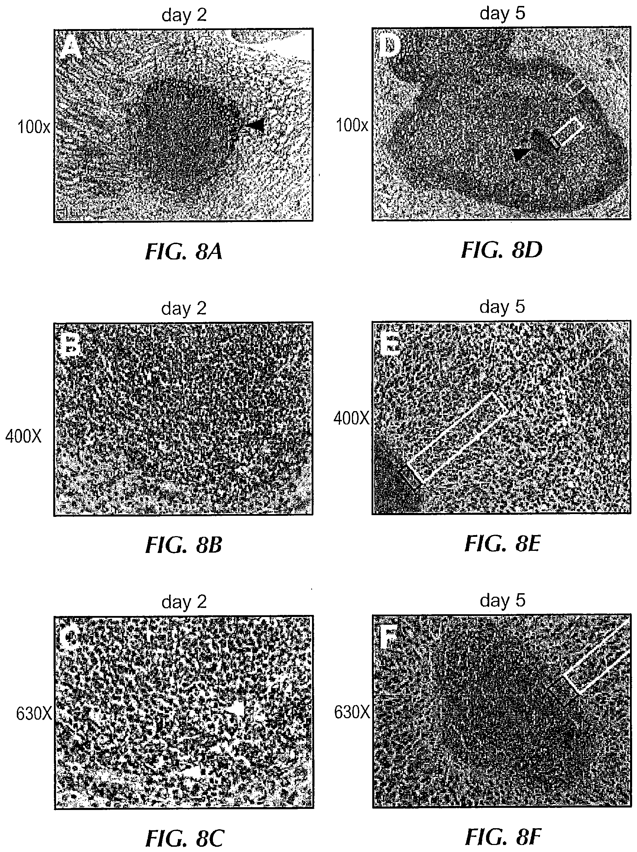

**FIGs. 8A-8F** Histopathology of staphylococcal abscess communities. BALB/c mice were infected with *S. aureus* Newman via retro-orbital injection. Thin-sectioned, hematoxylineosin stained tissues of infected kidneys on day 2 (ABC) and day 5 following infection (DEF) were analyzed by light microscopy and images captured. On day 2, a massive infiltrate (blue arrow in A) of polymorphonuclear granulocytes (PMNs) with occasional intracellular staphylococci (yellow arrows in C) are characteristic of early infectious lesions. By day 5, staphylococcal abscess communities developed as a central nidus (D, black arrow). Staphylococci were enclosed by an amorphous, eosinophilic pseudocapsule (boxed in black) and surrounded by a zone of dead PMNs (boxed in white), a zone of apparently healthy PMNs (boxed in red) and a rim of necrotic PMNs (boxed in green), separated through an eosinophilic layer from healthy kidney tissue.

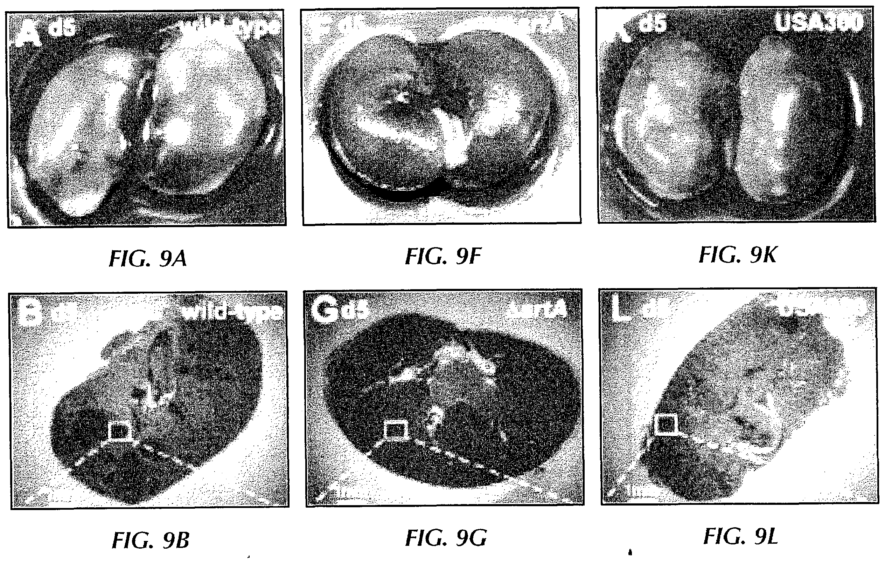

**FIGs. 9A-9P** Sortase A is required for abscess formation and staphylococcal persistence in host tissues. Kidneys of BALB/c mice (cohorts of ten animals) infected with *S. aureus* Newman, its isogenic sortase A mutant (Δ*srtA*) or methicillin-resistant *S. aureus* USA300 were removed during necropsy of animals 5 (d5) and 15 days (d15) following inoculation. Kidneys were inspected for surface abscesses (A, F, K) or fixed in formalin, embedded, thin sectioned and stained with hematoxylin-eosin. Histopathology images were acquired with light microscopy at 10 × (B, G, L, D, I, N) and 100 × fold magnification (C, H, M, E, J, O). (P) Staphylococcal replication and persistence in kidney tissue was measured 5 and 15 days following infection. Kidneys were removed from infected mice during necropsy, tissue was homogenized and plated on agar medium for colony formation and enumeration.

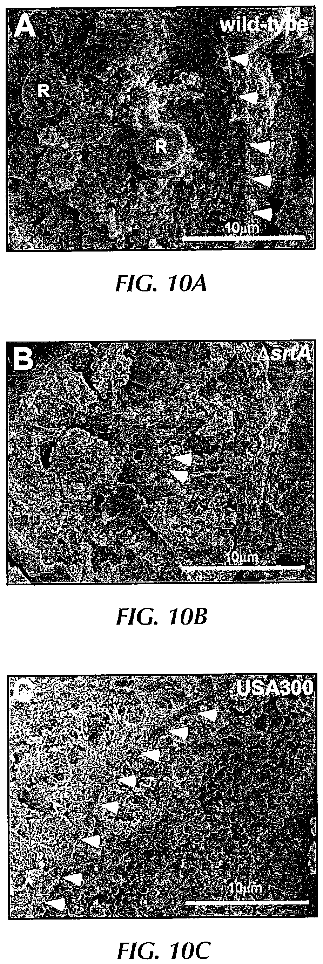

**FIGs. 10A-10C** Staphylococcal communities at the center of abscess lesions. Kidney tissue from mice infected with *S. aureus* Newman (wild-type), its isogenic sortase A mutant (Δ*srtA*)*,* or MRSA strain USA300 was sectioned, fixed, dehydrated and sputter coated with 80% platinum / 20% palladium for scanning electron microscopy. (A) The wild-type pathogen is organized as a tightly associated lawn, the *staphylococcal abscess community* (SAC), at the abscess center that is contained within an amorphous pseudocapsule (white arrow heads), separating SACs from the cuff of leukocytes. Red blood cells were located among staphylococci (R). (B) The sortase mutant (*ΔsrtA,* arrows) did not form SACs and isolated staphylococci were found in healthy kidney tissue. (C) Similar to *S. aureus* Newman, MRSA strain USA300 also formed SACs contained within a pseudocapsule (white arrow heads).

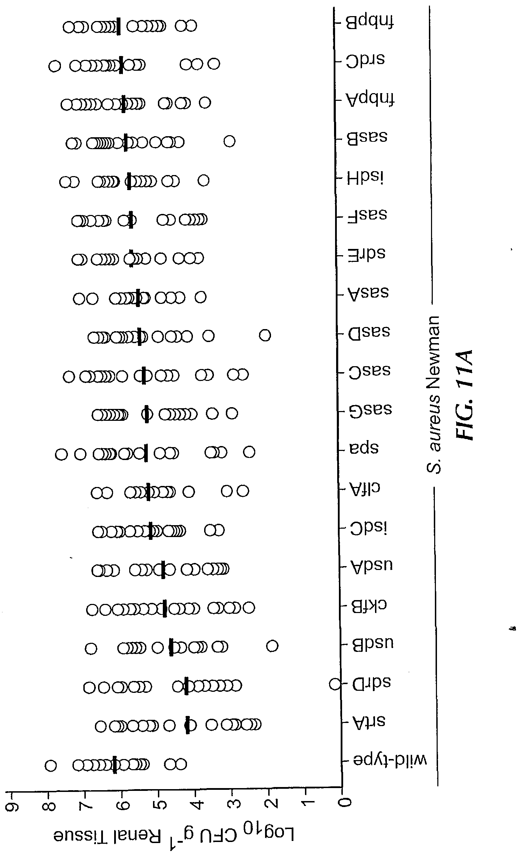

**FIGs. 11A-11B** Formation of staphylococcal abscess communities requires specific surface proteins. (A) *S. aureus* Newman variants with *bursa aurealis* insertions in surface protein genes were examined five days following infection of BALB/c mice (cohorts of 20 animals) for bacterial load in homogenized kidney tissues. (B) Hematoxylin-eosin stained thin sections of infected kidneys were examined by light microscopy and 10 × fold magnification for abscess lesions (white arrows).
**FIGs**. **12A-12L** Emp and Eap in staphylococcal abscess lesions. Kidneys of BALB/c mice infected with *S. aureus* Newman variants carrying *bursa aurealis* insertions in *emp* or *eap* were removed 5 (d5) and 15 days (d15) following inoculation. Kidneys were stained with hematoxylin-eosin and histopathology images acquired with light microscopy at 10 × (A, C, E, H) and 100 × fold magnification (B, D, F, I). Expression of Eap (J) and Emp (K) in abscess lesions of wild-type *S. aureus* Newman were detected with rabbit anti-Emp or anti-Eap and secondary Alexafluor-647 labeled antibodies (red) in renal tissue stained with Hoechst-dye (blue) to detect nuclei of polymorphonuclear leukocytes, and with BODIPY-vancomycin (green) to reveal staphylococcal abscess communities. (L) Staphylococcal replication and persistence in kidney tissue was measured 5, 15 and 30 days following intravenous inoculation. Kidneys were removed from infected mice (cohorts of 10 animals), tissue was homogenized and plated on agar medium for colony formation and enumeration.

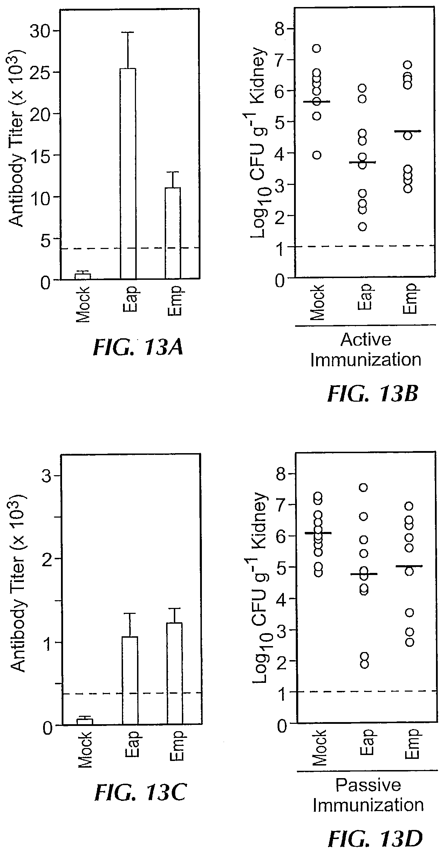

**FIGs. 13A-13E** Active and passive immunization with Eap generates protection from staphylococcal challenge. (A) BALB/c mice were immunized with purified Eap or Emp or mock treated with adjuvant alone and serum IgG titers analyzed by ELISA. (B) Three weeks following immunization, animals were challenged via intravenous inoculation of staphylococci. Five days following infection, kidneys were removed during necropsy and renal tissue analyzed for staphylococcal load or histopathology. (C) Rabbit antibodies directed against Eap or Emp were purified by affinity chromatography and passively transferred by intraperitoneal injection into mice. Twenty-four hours later, serum IgG titers of passively immunized animals were analyzed by ELISA. (D) Animals passively immunized with purified antibodies against Eap or Emp as well as mock immunized animals subsequently challenged with *S. aureus* Newman and bacterial load enumerated on day 4. (E) Abscess formation in kidneys was detected in thin-sectioned, hematoxylin-eosin stained tissues.
**FIG**. **14** A working model for staphylococcal abscess formation and persistence in host tissues. Stage I - following intravenous inoculation, *S. aureus* survives in the blood stream and disseminates via the vasculature to peripheral organ tissues. Stage II - in renal tissues, staphylococci attract a massive infiltrate of polymorphonuclear leukocytes and other immune cells. Stage III - abscesses mature with a central accumulation of the pathogen (staphylococcal abscess communities - SAC), enclosed by an eosinophilic pseudocapsule. The SAC is surrounded by a zone of dead PMNs, apparently healthy PMNs and finally an outer zone of dead PMNs with a rim of eosinophilic material. Stage 4 - abscesses mature and rupture on the organ surface, thereby releasing staphylococci into circulation and initiating new rounds of abscess development. Genes for bacterial envelope components that are required for specific stages of staphylococcal abscess development are printed in red underneath the corresponding stage during which these genes function.

### DETAILED DESCRIPTION OF THE INVENTION

Biofilms are microbial communities embedded in a secreted extracellular matrix (Hall-Stoodley *et al*., 2004; Kolter and Greenberg, 2006). Many bacterial species are capable of switching from planktonic growth to the formation of biofilms and thereby display increased antibiotic resistance (Drenkard and Ausubel, 2002), evasion from host immune defenses (Singh *et al*., 2002), and are more adept at establishing chronic infections in humans (Brady *et al*., 2008). Biofilms of staphylococcal species have been associated with a number of diseases including endocarditis (Xiong *et al*., 2005), osteomyelitis (Brady *et al.,* 2006), and various implant-mediated infections including urinary catheters, prosthetic heart valves, and artificial joints (Cassat *et al*., 2007). This applies in particular to *Staphylococcus epidermidis*, an opportunistic pathogen that avidly forms biofilms *in vitro* and *in vivo* (Mack *et al*., 1996). While there is clear association between the ability to form biofilms and virulence in *S. epidermidis,* such correlation has not yet been demonstrated for *S. aureus* (Cassat *et al*., 2007).

*S. aureus* is a commensal of human skin and nares and the leading cause of bloodstream and skin/soft tissue infections (Klevens *et al*., 2007). The pathogenesis of staphylococcal infections is initiated as bacteria invade skin or blood stream via trauma, surgical wounds, or medical devices (Lowy, 1998). Some staphylococci are cleared from the blood stream by phagocytic killing, however staphylococci that escape immune defenses seed infections in organ tissues and induce a proinflammatory response mediated by the release of cytokines and chemokines from macrophages, neutrophils, and other phagocytes (Lowy, 1998). The resulting invasion of immune cells to the site of infection is accompanied by central liquefaction necrosis and formation of peripheral fibrin walls in an effort to prevent microbial spread and allow for removal of necrotic tissue debris (Lowy, 1998). Such lesions can be observed by microscopy as hypercellular areas containing necrotic tissue, leukocytes, and a central nidus of bacteria. Organ abscesses occur within two days of infection (unpublished data) and represent a hallmark of staphylococcal disease.

### I. STAPHYLOCOCCAL ANTIGENS

The *Staphylococcus aureus* Ess pathway can be viewed as a secretion module equipped with specialized transport components (Ess), accessory factors (Esa), and cognate secretion substrates (Esx). EssA, EssB and EssC are required for EsxA and EsxB secretion. Because EssA, EssB and EssC are predicted to be transmembrane proteins, it is contemplated that these proteins form a secretion apparatus. Some of the proteins in the *ess* gene cluster may actively transport secreted substrates (acting as motor) while others may regulate transport (regulator). Regulation may be achieved, but need not be limited to, transcriptional or post-translational mechanisms for secreted polypeptides, sorting of specific substrates to defined locations (e.g., extracellular medium or host cells), or timing of secretion events during infection. At this point, it is unclear whether all secreted Esx proteins function as toxins or contribute indirectly to pathogenesis.

Staphylococci rely on surface protein mediated-adhesion to host cells or invasion of tissues as a strategy for escape from immune defenses. Furthermore, *S. aureus* utilize surface proteins to sequester iron from the host during infection. The majority of surface proteins involved in staphylococcal pathogenesis carry C-terminal sorting signals, *i.e*., they are covalently linked to the cell wall envelope by sortase. Further, staphylococcal strains lacking the genes required for surface protein anchoring, *i.e*., sortase A and B, display a dramatic defect in the virulence in several different mouse models of disease. Thus, surface protein antigens represent a validated vaccine target as the corresponding genes are essential for the development of staphylococcal disease and can be exploited in various embodiments of the invention. The sortase enzyme superfamily are Gram-positive transpeptidases responsible for anchoring surface protein virulence factors to the peptidoglycan cell wall layer. Two sortase isoforms have been identified in *Staphylococcus aureus*, SrtA and SrtB. These enzymes have been shown to recognize a LPXTG motif in substrate proteins. The SrtB isoform appears to be important in heme iron acquisition and iron homeostasis, whereas the SrtA isoform plays a critical role in the pathogenesis of Gram-positive bacteria by modulating the ability of the bacterium to adhere to host tissue via the covalent anchoring of adhesions and other proteins to the cell wall peptidoglycan.

Embodiments of the invention include compositions and methods related to Emp used in combination with other staphylococcal proteins such as Eap, EsaB, EsaC, EsxA, EsxB, SdrC, SdrD, SdrE, Hla, IsdA, IsdB, ClfA, ClfB, IsdC, SasB, SasF, SasH (AdsA), Ebh, Coa, vWa, and/or SpA proteins. Emp (SEQ ID NO:2) or Eap (SEQ ID NO:4) are staphylococcal polypeptides. Sequence of other Emp polypeptides can be found in the protein databases and include, but are not limited to accession numbers YP_185731, NP_371337, NP_645584, CAB75985, YP_416239, YP_040269, and NM0758. Additional Staphyloccal antigens include 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein.

In mammals, adenosine assumes an essential role in regulating innate and acquired immune responses (Thiel *et al*., 2003). Strong or excessive host inflammatory responses, for example in response to bacterial infection, exacerbate the tissue damage inflicted by invading pathogens (Thiel *et al*., 2003). Successful immune clearance of microbes therefore involves the balancing of pro- and anti-inflammatory mediators. Cytokines IL-4, IL-10, IL-13 and TGF-β restrict excessive inflammation, however only adenosine is able to completely suppress immune responses (Nemeth *et al*., 2006). The immunoregulatory attributes of adenosine are mediated via four transmembrane adenosine receptors: A₁, A_{2A}, A_{2B}, and A₃ (Hasko and Pacher, 2008). T lymphocytes express the high affinity A_{2A} receptor as well as the low affinity A_{2B} receptor (Thiel *et al*., 2003). Depending on their activation state, macrophages and neutrophils express all four adenosine receptors, whereas B cells harbor only A_{2A} (Thiel *et al*., 2003). Engagement of A_{2A} inhibits IL-12 production, increases IL-10 in monocytes (Khoa *et al.,* 2001) and dendritic cells (Panther *et al*., 2001), and decreases cytotoxic attributes and chemokine production in neutrophils (McColl *et al*., 2006; Cronstein *et al*., 1986). Generation of adenosine at sites of inflammation, hypoxia, organ injury, and traumatic shock is mediated by two sequential enzymes. Ecto-ATP diphosphohydrolase (CD39) converts circulating adenosine triphosphate (ATP) and adenosine diphosphate (ADP) to 5'-adenosine monophosphate (AMP) (Eltzshig *et al*., 2003). CD73, expressed on the surface of endothelial cells (Deussen *et al*., 1993) and subsets of T cells (Thompson *et al*., 1989; Thompson *et al.,* 1987; Yang *et al*., 2005), then converts 5'-AMP to adenosine (Zimmermann, 1992).

Although extracellular adenosine is essential for the suppression of inflammation, build-up of excess adenosine is also detrimental. This is exemplified in patients with a deficiency in adenosine deaminase (ADA), an enzyme that converts adenosine into inosine (Giblett, *et al*., 1972). ADA deficiency causes the severe compromised immunodeficiency syndrome (SCID) with impaired cellular immunity and severely decreased production of immunoglobulins (Buckley *et al*., 1997). As the regulation of extracellular adenosine is critical in maintaining immune homeostasis, perturbation of adenosine levels is likely to impact host immune responses during infection. The inventors describe herein that bacteria, *e.g*., *S. aureus* and *Bacillus anthracis*, use adenosine synthesis to escape host immune responses and provide methods and composition for utilizing this information for the treatment and/or prevention of bacterial infection, e.g., bacteremia.

Certain aspects of the invention include methods and compositions concerning proteinaceous compositions including polypeptides, peptides, antibodies that bind such polypeptides and peptides, or nucleic acids encoding Emp and other staphylococcal antigens such as Eap, EsaB, EsaC, EsxA, EsxB, SdrC, SdrD, SdrE, Hla, IsdA, IsdB, ClfA, ClfB, IsdC, SasB, SasF, SasH (AdsA), Ebh, Coa, vWa, SpA, 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein or combinations thereof.

These polypeptides include the amino acid sequence of proteins from bacteria in the Staphylococcus genus. The sequence may be from a particular staphylococcus species, such as *Staphylococcus aureus,* and may be from a particular strain, such as Newman. The sortase substrate polypeptides include, but are not limited to the amino acid sequence of SdrC, SdrD, SdrE, IsdA, IsdB, SpA, ClfA, ClfB, IsdC or SasF proteins from bacteria in the Staphylococcus genus. The sortase substrate polypeptide sequence may be from a particular staphylococcus species, such as Staphylococcus aureus, and may be from a particular strain, such as Newman. In certain embodiments, the SdrD sequence is from strain N315 and can be accessed using GenBank Accession Number NP_373773.1 (gi|15926240), which is incorporated by reference. In other embodiments, the SdrE sequence is from strain N315 and can be accessed using GenBank Accession Number NP_373774.1 (gi| 15926241). In other embodiments, the IsdA sequence is SAV1130 from strain Mu50 (which is the same amino acid sequence for Newman) and can be accessed using Genbank Accession Number NP_371654.1 (gi|15924120). In other embodiments, the IsdB sequence is SAV1129 from strain Mu50 (which is the same amino acid sequence for Newman) and can be accessed using Genbank Accession Number NP_371653.1 (gi|15924119). In further embodiments, other polypeptides transported by the Ess pathway or processed by sortase may be used, the sequences of which may be identified by one of skill in the art using databases and internet accessible resources.

Examples of various proteins that can be used in the context of the present invention can be identified by analysis of database submissions of bacterial genomes, including but not limited to accession numbers NC_002951 (GI:57650036 and GenBank CP000046), NC_002758 (GI:57634611 and GenBank BA000017), NC_002745 (GI:29165615 and GenBank BA000018), NC_003923 (GI:21281729 and GenBank BA000033), NC_002952 (GI:49482253 and GenBank BX571856), NC_002953 (GI:49484912 and GenBank BX571857), NC_007793 (GI:87125858 and GenBank CP000255), NC_007795 (GI:87201381 and GenBank CP000253).

As used herein, a "protein" or "polypeptide" refers to a molecule comprising at least ten amino acid residues. In some embodiments, a wild-type version of a protein or polypeptide are employed, however, in many embodiments of the invention, a modified protein or polypeptide is employed to generate an immune response. The terms described above may be used interchangeably. A "modified protein" or "modified polypeptide" refers to a protein or polypeptide whose chemical structure, particularly its amino acid sequence, is altered with respect to the wild-type protein or polypeptide. In some embodiments, a modified protein or polypeptide has at least one modified activity or function (recognizing that proteins or polypeptides may have multiple activities or functions). It is specifically contemplated that a modified protein or polypeptide may be altered with respect to one activity or function yet retain a wild-type activity or function in other respects, such as immunogenicity.

In certain embodiments the size of a protein or polypeptide (wild-type or modified) may comprise, but is not limited to, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 275, 300, 325, 350, 375, 400, 425, 450, 475, 500, 525, 550, 575, 600, 625, 650, 675, 700, 725, 750, 775, 800, 825, 850, 875, 900, 925, 950, 975, 1000, 1100, 1200, 1300, 1400, 1500, 1750, 2000, 2250, 2500 amino molecules or greater, and any range derivable therein, or derivative of a corresponding amino sequence described or referenced herein. It is contemplated that polypeptides may be mutated by truncation, rendering them shorter than their corresponding wild-type form, but also they might be altered by fusing or conjugating a heterologous protein sequence with a particular function (*e.g*., for targeting or localization, for enhanced immunogenicity, for purification purposes, *etc*.). As used herein, an "amino molecule" refers to any amino acid, amino acid derivative, or amino acid mimic known in the art. In certain embodiments, the residues of the proteinaceous molecule are sequential, without any non-amino molecule interrupting the sequence of amino molecule residues. In other embodiments, the sequence may comprise one or more non-amino molecule moieties. In particular embodiments, the sequence of residues of the proteinaceous molecule may be interrupted by one or more non-amino molecule moieties.

Accordingly, the term "proteinaceous composition" encompasses amino molecule sequences comprising at least one of the 20 common amino acids in naturally synthesized proteins, or at least one modified or unusual amino acid.

Proteinaceous compositions may be made by any technique known to those of skill in the art, including (i) the expression of proteins, polypeptides, or peptides through standard molecular biological techniques, (ii) the isolation of proteinaceous compounds from natural sources, or (iii) the chemical synthesis of proteinaceous materials. The nucleotide as well as the protein, polypeptide, and peptide sequences for various genes have been previously disclosed, and may be found in the recognized computerized databases. One such database is the National Center for Biotechnology Information's Genbank and GenPept databases (on the World Wide Web at ncbi.nlm.nih.gov/). The coding regions for these genes may be amplified and/or expressed using the techniques disclosed herein or as would be known to those of ordinary skill in the art.

Amino acid sequence variants of Emp and other polypeptides, Eap, AdsA, EsaB, EsaC, EsxA, EsxB, SdrC, SdrD, SdrE, Hla, IsdA, IsdB, ClfA, ClfB, IsdC, SasB, SasF, Ebh, Coa, vWa, SpA, 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein can be substitutional, insertional, or deletion variants. A modification in a polypeptide of the invention may affect 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500 or more non-contiguous or contiguous amino acids of the polypeptide, as compared to wild-type. An antigen of the invention can comprise a segment or fragment of an antigen (AdsA, Emp, Eap, EsaB, EsaC, EsxA, EsxB, SdrC, SdrD, SdrE, Hla, IsdA, IsdB, ClfA, ClfB, IsdC, SasB, SasF, Ebh, Coa, vWa, SpA, 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein) described herein comprising amino acid 1, 2, 3, 4, 5, 6, 7, 8, 9, 10, 11, 12, 13, 14, 15, 16, 17,18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509, 510, 511, 512, 513, 514, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, or more (including all values and ranges there between) to amino acid 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509, 510, 511, 512, 513, 514, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, or more (including all values and ranges there between) of the antigen, including all fragments there between. Certain aspects are directed to an antibody that binds one or more of the above described fragments or segments.

Deletion variants typically lack one or more residues of the native or wild-type protein. Individual residues can be deleted or a number of contiguous amino acids can be deleted. A stop codon may be introduced (by substitution or insertion) into an encoding nucleic acid sequence to generate a truncated protein. Insertional mutants typically involve the addition of material at a non-terminal point in the polypeptide. This may include the insertion of one or more residues. Terminal additions, called fusion proteins, may also be generated.

Substitutional variants typically contain the exchange of one amino acid for another at one or more sites within the protein, and may be designed to modulate one or more properties of the polypeptide, with or without the loss of other functions or properties. Substitutions may be conservative, that is, one amino acid is replaced with one of similar shape and charge. Conservative substitutions are well known in the art and include, for example, the changes of: alanine to serine; arginine to lysine; asparagine to glutamine or histidine; aspartate to glutamate; cysteine to serine; glutamine to asparagine; glutamate to aspartate; glycine to proline; histidine to asparagine or glutamine; isoleucine to leucine or valine; leucine to valine or isoleucine; lysine to arginine; methionine to leucine or isoleucine; phenylalanine to tyrosine, leucine or methionine; serine to threonine; threonine to serine; tryptophan to tyrosine; tyrosine to tryptophan or phenylalanine; and valine to isoleucine or leucine. Alternatively, substitutions may be non-conservative such that a function or activity of the polypeptide is affected. Non-conservative changes typically involve substituting a residue with one that is chemically dissimilar, such as a polar or charged amino acid for a nonpolar or uncharged amino acid, and vice versa.

**Table 1. Exemplary surface proteins of S. aureus strains.**

| SAV # | SA# | Surface | MW2 | Mu50 | N315 | Newman | MRSA252* | MSSA476* |
|---|---|---|---|---|---|---|---|---|
| SAV0111 | SA0107 | SpA | 492 | 450 | 450 | | 516 | 492 |
| SAV2503 | SA2291 | FnBPA | 1015 | 1038 | 1038 | | - | 1015 |
| SAV2502 | SA2290 | FnBPB | 943 | 961 | 961 | | 965 | 957 |
| SAV0811 | SA0742 | ClfA | 946 | 935 | 989 | 933 | 1029 | 928 |
| SAV2630 | SA2423 | ClfB | 907 | 877 | 877 | 913 | 873 | 905 |
| Np | np | Can | 1183 | | | | 1183 | 1183 |
| SAV0561 | SA0519 | SdrC | 955 | 953 | 953 | 947 | 906 | 957 |
| SAV0562 | SA0520 | SdrD | 1347 | 1385 | 1385 | 1315 | - | 1365 |
| SAV0563 | SA0521 | SdrE | 1141 | 1141 | 1141 | 1166 | 1137 | 1141 |
| Np | np | Pls | - | | | | - | |
| SAV2654 | SA2447 | SasA | 2275 | 2271 | 2271 | | 1351 | 2275 |
| SAV2160 | SA1964 | SasB | 686 | 2481 | 2481 | | 2222 | 685 |
| | SA1577 | SasC | 2186 | 213 | 2186 | | 2189 | 2186 |
| SAV0134 | SA0129 | SasD | 241 | 241 | 241 | | 221 | 241 |
| SAV1130 | SA0977 | SasE/IsdA | 350 | 350 | 350 | | 354 | 350 |
| SAV2646 | SA2439 | SasF | 635 | 635 | 635 | | 627 | 635 |
| SAV2496 | | SasG | 1371 | 525 | 927 | | - | 1371 |
| SAV0023 | SA0022 | SasH | 772 | | 772 | | 786 | 786 |
| SAV1731 | SA1552 | SasI | 895 | 891 | 891 | | 534 | 895 |
| SAV1129 | SA0976 | SasJ/IsdB | 645 | 645 | 645 | | 652 | 645 |
| | SA2381 | SasK | 198 | 211 | 211 | | - | 197 |
| | Np | SasL | - | 232 | | | - | |
| SAV1131 | SA0978 | IsdC | 227 | 227 | 227 | | 227 | 227 |

Proteins described herein may be recombinant, or synthesized *in vitro.* Alternatively, a non-recombinant or recombinant protein may be isolated from bacteria. It is also contemplated that a bacteria containing such a variant may be implemented in compositions and methods described herein. Consequently, a protein need not be isolated. The term "functionally equivalent codon" is used herein to refer to codons that encode the same amino acid, such as the six codons for arginine or serine, and also refers to codons that encode biologically equivalent amino acids (see Table 2, below).

**Table 2 Codon Table**

| Amino Acids | | | Codons | | | | | |
|---|---|---|---|---|---|---|---|---|
| Alanine | Ala | A | GCA | GCC | GCG | GCU | | |
| Cysteine | Cys | C | UGC | UGU | | | | |
| Aspartic acid | Asp | D | GAC | GAU | | | | |
| Glutamic acid | Glu | E | GAA | GAG | | | | |
| Phenylalanine | Phe | F | UUC | UUU | | | | |
| Glycine | Gly | G | GGA | GGC | GGG | GGU | | |
| Histidine | His | H | CAC | CAU | | | | |
| Isoleucine | Ile | I | AUA | AUC | AUU | | | |
| Lysine | Lys | K | AAA | AAG | | | | |
| Leucine | Leu | L | UUA | UUG | CUA | CUC | CUG | CUU |
| Methionine | Met | M | AUG | | | | | |
| Asparagine | Asn | N | AAC | AAU | | | | |
| Proline | Pro | P | CCA | CCC | CCG | CCU | | |
| Glutamine | Gln | Q | CAA | CAG | | | | |
| Arginine | Arg | R | AGA | AGG | CGA | CGC | CGG | CGU |
| Serine | Ser | S | AGC | AGU | UCA | UCC | UCG | UCU |
| Threonine | Thr | T | ACA | ACC | ACG | ACU | | |
| Valine | Val | V | GUA | GUC | GUG | GUU | | |
| Tryptophan | Trp | W | UGG | | | | | |
| Tyrosine | Tyr | Y | UAC | UAU | | | | |

It also will be understood that amino acid and nucleic acid sequences may include additional residues, such as additional N- or C-terminal amino acids, or 5' or 3' sequences, respectively, and yet still be essentially as set forth in one of the sequences disclosed herein, so long as the sequence meets the criteria set forth above, including the maintenance of biological protein activity where protein expression is concerned. The addition of terminal sequences particularly applies to nucleic acid sequences that may, for example, include various non-coding sequences flanking either of the 5' or 3' portions of the coding region.

The following is a discussion based upon changing of the amino acids of a protein to create an equivalent, or even an improved, second-generation molecule. For example, certain amino acids may be substituted for other amino acids in a protein structure without appreciable loss of interactive binding capacity with structures such as, for example, antigen-binding regions of antibodies or binding sites on substrate molecules. Since it is the interactive capacity and nature of a protein that defines that protein's biological functional activity, certain amino acid substitutions can be made in a protein sequence, and in its underlying DNA coding sequence, and nevertheless produce a protein with like properties. It is thus contemplated by the inventors that various changes may be made in the DNA sequences of genes without appreciable loss of their biological utility or activity, e.g., immunogenicity.

In making such changes, the hydropathic index of amino acids may be considered. The importance of the hydropathic amino acid index in conferring interactive biologic function on a protein is generally understood in the art (Kyte and Doolittle, 1982). It is accepted that the relative hydropathic character of the amino acid contributes to the secondary structure of the resultant protein, which in turn defines the interaction of the protein with other molecules, for example, enzymes, substrates, receptors, DNA, antibodies, antigens, and the like.

It also is understood in the art that the substitution of like amino acids can be made effectively on the basis of hydrophilicity. U.S. Patent 4,554,101, states that the greatest local average hydrophilicity of a protein, as governed by the hydrophilicity of its adjacent amino acids, correlates with a biological property of the protein. It is understood that an amino acid can be substituted for another having a similar hydrophilicity value and still produce a biologically equivalent and immunologically equivalent protein.

As outlined above, amino acid substitutions generally are based on the relative similarity of the amino acid side-chain substituents, for example, their hydrophobicity, hydrophilicity, charge, size, and the like. Exemplary substitutions that take into consideration the various foregoing characteristics are well known and include: arginine and lysine; glutamate and aspartate; serine and threonine; glutamine and asparagine; and valine, leucine and isoleucine.

It is contemplated that in compositions of the invention, there may be between about 0.001 mg and about 10 mg of total polypeptide, peptide, and/or protein per ml. Thus, the concentration of protein in a composition can be about, at least about or at most about 0.001, 0.010, 0.050, 0.1, 0.2, 0.3, 0.4, 0.5, 0.6, 0.7, 0.8, 0.9, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0 µg or mg/ml or more (or any range derivable therein). Of this, about, at least about, or at most about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100% may be Emp and may be used in combination with other polypeptide sequences described herein.

The present invention also discloses combinations of staphylococcal antigens which when combined, lead to the production of an immunogenic composition that is effective at treating or preventing staphylococcal infection. Staphylococcal infections progress through several different stages. For example, the staphylococcal life cycle involves commensal colonization, initiation of infection by accessing adjoining tissues or the bloodstream, anaerobic multiplication in the blood, interplay between *S. aureus* virulence determinants and the host defense mechanisms and induction of complications including endocarditis, metastatic abscess formation and sepsis syndrome. Different molecules on the surface of the bacterium will be involved in different steps of the infection cycle. Combinations of certain antigens can elicit an immune response which protects against multiple stages of staphylococcal infection. The effectiveness of the immune response can be measured either in animal model assays and/or using an opsonophagocytic assay.

### A. Polypeptides and Polypeptide Production

The present invention describes polypeptides, peptides, and proteins and immunogenic fragments thereof for use in various embodiments of the present invention. For example, specific polypeptides are assayed for or used to elicit an immune response. In specific embodiments, all or part of the proteins of the invention can also be synthesized in solution or on a solid support in accordance with conventional techniques. Various automatic synthesizers are commercially available and can be used in accordance with known protocols. See, for example, Stewart and Young, (1984); Tam *et al*., (1983); Merrifield, (1986); and Barany and Merrifield (1979). Alternatively, recombinant DNA technology may be employed wherein a nucleotide sequence which encodes a peptide of the invention is inserted into an expression vector, transformed or transfected into an appropriate host cell and cultivated under conditions suitable for expression.

One embodiment includes the use of gene transfer to cells, including microorganisms, for the production and/or presentation of proteins. The gene for the protein of interest may be transferred into appropriate host cells followed by culture of cells under the appropriate conditions. A nucleic acid encoding virtually any polypeptide may be employed. The generation of recombinant expression vectors, and the elements included therein, are discussed herein. Alternatively, the protein to be produced may be an endogenous protein normally synthesized by the cell used for protein production.

Another embodiment uses autologous B lymphocyte cell lines, which are transfected with a viral vector that expresses an immunogen product, and more specifically, a protein having immunogenic activity. Other examples of mammalian host cell lines include, but are not limited to Vero and HeLa cells, other B- and T- cell lines, such as CEM, 721.221, H9, Jurkat, Raji, as well as cell lines of Chinese hamster ovary, W138, BHK, COS-7, 293, HepG2, 3T3, RIN and MDCK cells. In addition, a host cell strain may be chosen that modulates the expression of the inserted sequences, or that modifies and processes the gene product in the manner desired. Such modifications (e.g., glycosylation) and processing (e.g., cleavage) of protein products may be important for the function of the protein. Different host cells have characteristic and specific mechanisms for the post-translational processing and modification of proteins. Appropriate cell lines or host systems can be chosen to ensure the correct modification and processing of the foreign protein expressed.

A number of selection systems may be used including, but not limited to HSV thymidine kinase, hypoxanthine-guanine phosphoribosyltransferase, and adenine phosphoribosyltransferase genes, in tk-, hgprt- or aprt- cells, respectively. Also, antimetabolite resistance can be used as the basis of selection: for dhfr, which confers resistance to trimethoprim and methotrexate; gpt, which confers resistance to mycophenolic acid; neo, which confers resistance to the aminoglycoside G418; and hygro, which confers resistance to hygromycin. Animal cells can be propagated *in vitro* in two modes: as non-anchorage-dependent cells growing in suspension throughout the bulk of the culture or as anchorage-dependent cells requiring attachment to a solid substrate for their propagation (*i.e*., a monolayer type of cell growth).

Non-anchorage dependent or suspension cultures from continuous established cell lines are the most widely used means of large scale production of cells and cell products. However, suspension cultured cells have limitations, such as tumorigenic potential and lower protein production than adherent cells.

Where a protein is specifically mentioned herein, it is preferably a reference to a native or recombinant protein or optionally a protein in which any signal sequence has been removed. The protein may be isolated directly from the staphylococcal strain or produced by recombinant DNA techniques. Immunogenic fragments of the protein may be incorporated into the immunogenic composition of the invention. These are fragments comprising at least 10 amino acids, 20 amino acids, 30 amino acids, 40 amino acids, 50 amino acids, or 100 amino acids, including all values and ranges there between, taken contiguously from the amino acid sequence of the protein. In addition, such immunogenic fragments are immunologically reactive with antibodies generated against the Staphylococcal proteins or with antibodies generated by infection of a mammalian host with Staphylococci. Immunogenic fragments also includes fragments that when administered at an effective dose, (either alone or as a hapten bound to a carrier), elicit a protective immune response against Staphylococcal infection, in certain aspects it is protective against *S. aureus* and/or *S. epidermidis* infection. Such an immunogenic fragment may include, for example, the protein lacking an N-terminal leader sequence, and/or a transmembrane domain and/or a C-terminal anchor domain. In a preferred aspect the immunogenic fragment according to the invention comprises substantially all of the extracellular domain of a protein which has at least 85% identity, at least 90% identity, at least 95% identity, or at least 97-99% identity, including all values and ranges there between, to that a sequence selected over the length of the fragment sequence.

Also included in immunogenic compositions of the invention are fusion proteins composed of Staphylococcal proteins, or immunogenic fragments of staphylococcal proteins. Such fusion proteins may be made recombinantly and may comprise one portion of at least 2, 3, 4, 5 or 6 staphylococcal proteins. Alternatively, a fusion protein may comprise multiple portions of at least 1, 2, 3, 4 or 5 staphylococcal proteins. These may combine different Staphylococcal proteins and/or multiples of the same protein or protein fragment, or immunogenic fragments thereof in the same protein. Alternatively, the invention also includes individual fusion proteins of Staphylococcal proteins or immunogenic fragments thereof, as a fusion protein with heterologous sequences such as a provider of T-cell epitopes or purification tags, for example: β- galactosidase, glutathione-S-transferase, green fluorescent proteins (GFP), epitope tags such as FLAG, myc tag, poly histidine, or viral surface proteins such as influenza virus haemagglutinin, or bacterial proteins such as tetanus toxoid, diphtheria toxoid, and CRM197.

### II. THERAPEUTIC METHODS

Active immunization with vaccines and passive immunization with immunoglobulins are promising alternatives to classical small molecule (e.g., antibiotic) therapy. A few bacterial diseases that once caused widespread illness, disability and death can now be prevented through the use of vaccines. The vaccines are based on weakened (attenuated) or dead bacteria, components of the bacterial surface or inactivated toxins. The immune response raised by a vaccine is mainly directed to immunogenic structures; a limited number of proteins or sugar structures on the bacteria that are actively processed by the immune system.

A method described herein includes treatment for a disease or condition caused by or related to a bacterial pathogen, *e.g*., staphylococcus or bacillus. An immunogenic polypeptide, and/or antibody that binds the same, can be given to induce or provide a therapeutic response in a person infected with a bacteria or suspected of having been exposed to a bacteria. Methods may be employed with respect to individuals who have tested positive for exposure to staphylococcus or bacillus or who are deemed to be at risk for infection based on possible exposure.

The invention encompasses use of the claimed compositions in methods of treatment of staphylococcal infection, particularly hospital acquired nosocomial infections. In particular, the invention encompasses use of the claimed compositions in methods of treatment for bacterial infection, particularly bacteremia. The therapeutic compositions and vaccines of the invention are particularly advantageous in cases of elective surgery. Such patients will know the date of surgery in advance and could be inoculated or treated in advance. The immunogenic compositions and vaccines of the invention are also advantageous in inoculating health care workers, first responders, and the like.

In some embodiments, the treatment is administered in the presence of adjuvants or carriers or other staphylococcal antigens. Furthermore, in some examples, treatment comprises administration of other agents commonly used against bacterial infection, such as one or more antibiotics.

### A. Vaccines

Described herein are methods for preventing or ameliorating staphylococcal infections, particularly hospital acquired nosocomial infections. As such contemplated are vaccines for use in both active and passive immunization embodiments. Immunogenic compositions, proposed to be suitable for use as a vaccine, may be prepared from immunogenic Emp polypeptide(s), such as the full-length Emp antigen or immunogenic fragments thereof. In other embodiments Emp can be used in combination with other secreted virulence proteins, surface proteins or immunogenic fragments thereof, including Eap, AdsA, EsaB, EsaC, EsxA, EsxB, SdrC, SdrD, SdrE, Hla, IsdA, IsdB, ClfA, ClfB, IsdC, SasB, SasF, Ebh, Coa, vWa, SpA, 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein or other staphylococcal antigen, peptide, or protein known to one of skill in the art. Preferably the antigenic material is extensively dialyzed to remove undesired small molecular weight molecules and/or lyophilized for more ready formulation into a desired vehicle.

Other viable and important options for a protein/peptide-based vaccine involve introducing nucleic acids encoding the antigen(s) as DNA vaccines. In this regard, recent reports described construction of recombinant vaccinia viruses expressing 10 contiguous minimal CTL epitopes (Thomson, 1996) or a combination of B cell, cytotoxic T-lymphocyte (CTL), and T-helper (Th) epitopes from several microbes (An, 1997), and successful use of such constructs to immunize mice for priming protective immune responses. Thus, there is ample evidence in the literature for successful utilization of peptides, peptide-pulsed antigen presenting cells (APCs), and peptide-encoding constructs for efficient *in vivo* priming of protective immune responses. The use of nucleic acid sequences as vaccines is exemplified in U.S. Patents 5,958,895 and 5,620,896.

The preparation of vaccines that contain polypeptide or peptide sequence(s) as active ingredients is generally well understood in the art, as exemplified by U.S. Patents 4,608,251; 4,601,903; 4,599,231; 4,599,230; 4,596,792; and 4,578,770. Typically, such vaccines are prepared as injectables either as liquid solutions or suspensions: solid forms suitable for solution in or suspension in liquid prior to injection may also be prepared. The preparation may also be emulsified. The active immunogenic ingredient is often mixed with excipients that are pharmaceutically acceptable and compatible with the active ingredient. Suitable excipients are, for example, water, saline, dextrose, glycerol, ethanol, or the like and combinations thereof. In addition, if desired, the vaccine may contain amounts of auxiliary substances such as wetting or emulsifying agents, pH buffering agents, or adjuvants that enhance the effectiveness of the vaccines. In specific embodiments, vaccines are formulated with a combination of substances, as described in U.S. Patents 6,793,923 and 6,733,754.

Vaccines may be conventionally administered by inhalation or parenterally by injection, e.g., subcutaneously or intramuscularly. Additional formulations which are suitable for other modes of administration include suppositories and, in some cases, oral formulations. For suppositories, traditional binders and carriers may include, for example, polyalkalene glycols or triglycerides: such suppositories may be formed from mixtures containing the active ingredient in the range of about 0.5% to about 10%, preferably about 1% to about 2%. Oral formulations include such normally employed excipients as, for example, pharmaceutical grades of mannitol, lactose, starch, magnesium stearate, sodium saccharine, cellulose, magnesium carbonate and the like. These compositions take the form of solutions, suspensions, tablets, pills, capsules, sustained release formulations or powders and contain about 10% to about 95% of active ingredient, preferably about 25% to about 70%.

The polypeptide, peptides and peptide-encoding DNA constructs may be formulated into a vaccine as neutral or salt forms. Pharmaceutically-acceptable salts include the acid addition salts (formed with the free amino groups of the peptide) and those that are formed with inorganic acids such as, for example, hydrochloric or phosphoric acids, or such organic acids as acetic, oxalic, tartaric, mandelic, and the like. Salts formed with the free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

Typically, vaccines are administered in a manner compatible with the dosage formulation, and in such amount as will be therapeutically effective and immunogenic. The quantity to be administered depends on the subject to be treated, including the capacity of the individual's immune system to synthesize antibodies and the degree of protection desired. Precise amounts of active ingredient required to be administered depend on the judgment of the practitioner. However, suitable dosage ranges are of the order of several hundred micrograms active ingredient per vaccination. Suitable regimes for initial administration and booster shots are also variable, but are typified by an initial administration followed by subsequent inoculations or other administrations.

The manner of application may vary widely. Any of the conventional methods for administration of a vaccine are applicable. These are believed to include oral application on a solid physiologically acceptable base or in a physiologically acceptable dispersion, parenterally, by injection and the like. The dosage of the vaccine will depend on the route of administration and will vary according to the size and health of the subject.

In many instances, it will be desirable to have multiple administrations of the vaccine, usually at most, at least, or not exceeding six vaccinations, more usually four vaccinations, and typically one or more, usually at least about three vaccinations. The vaccinations will normally be at 1, 2, 3, 4, 5, 6, to 5, 6, 7, 8, 9, 10, 11, to 12 week intervals, including all values and ranges there between, more usually from three to five week intervals. Typically, periodic boosters at intervals of 1-5 years, usually three years, will be desirable to maintain protective levels of the antibodies. The course of the immunization may be followed by assays for antibodies against the antigens, as described supra, U.S. Patents 3,791,932; 4,174,384 and 3,949,064, are illustrative of these types of assays.

The use of peptides for vaccination typically requires conjugation of the peptide to an immunogenic carrier protein, such as hepatitis B surface antigen, keyhole limpet hemocyanin, or bovine serum albumin, or an adjuvant. Methods for performing this conjugation are well known in the art.

### 1. Carriers

A given composition may vary in its immunogenicity. It is often necessary therefore to boost the host immune system, as may be achieved by coupling a peptide or polypeptide to a carrier. Carriers include, but are not limited to keyhole limpet hemocyanin (KLH) and bovine serum albumin (BSA). Other albumins such as ovalbumin, mouse serum albumin, or rabbit serum albumin can also be used as carriers. Means for conjugating a polypeptide to a carrier protein are well known in the art and include glutaraldehyde, m-maleimidobencoyl-N-hydroxysuccinimide ester, carbodiimyde, and bis-biazotized benzidine.

### 2. Adjuvants

The immunogenicity of polypeptide or peptide compositions can be enhanced by the use of non-specific stimulators of the immune response, known as adjuvants. Suitable adjuvants include all acceptable immunostimulatory compounds, such as cytokines, toxins, or synthetic compositions. A number of adjuvants can be used to enhance an antibody response against an Emp peptide or any other antigen described herein. In other embodiments Emp can be used in combination with Eap, AdsA, EsaB, EsaC, EsxA, EsxB, SdrC, SdrD, SdrE, Hla, IsdA, IsdB, ClfA, ClfB, IsdC, SasB, SasF, Ebh, Coa, vWa, and/or SpA peptide or protein. Adjuvants can (1) trap the antigen in the body to cause a slow release; (2) attract cells involved in the immune response to the site of administration; (3) induce proliferation or activation of immune system cells; or (4) improve the spread of the antigen throughout the subject's body.

Adjuvants include, but are not limited to, oil-in-water emulsions, water-in-oil emulsions, mineral salts, polynucleotides, and natural substances. Specific adjuvants that may be used include IL-1, IL-2, IL-4, IL-7, IL-12, γ-interferon, GMCSP, BCG, aluminum salts, such as aluminum hydroxide or other aluminum compound, MDP compounds, such as thur-MDP and nor-MDP, CGP (MTP-PE), lipid A, and monophosphoryl lipid A (MPL). RIBI, which contains three components extracted from bacteria, MPL, trehalose dimycolate (TDM), and cell wall skeleton (CWS) in a 2% squalene/Tween 80 emulsion may also be used. MHC antigens may even be used.

Others adjuvants or methods are exemplified in U.S. Patents 6,814,971, 5,084,269, 6,656,462.

Various methods of achieving adjuvant affect for the vaccine includes use of agents such as aluminum hydroxide or phosphate (alum), commonly used as about 0.05 to about 0.1% solution in phosphate buffered saline, admixture with synthetic polymers of sugars (Carbopol®) used as an about 0.25% solution, aggregation of the protein in the vaccine by heat treatment with temperatures ranging between about 70° to about 101°C for a 30-second to 2-minute period, respectively. Aggregation by reactivating with pepsin-treated (Fab) antibodies to albumin; mixture with bacterial cells (*e.g*., C. *parvum*), endotoxins or lipopolysaccharide components of Gram-negative bacteria; emulsion in physiologically acceptable oil vehicles (e.g., mannide mono-oleate (Aracel A)); or emulsion with a 20% solution of a perfluorocarbon (Fluosol-DA®) used as a block substitute may also be employed to produce an adjuvant effect. A typical adjuvant is complete Freund's adjuvant (a non-specific stimulator of the immune response containing killed *Mycobacterium tuberculosis*), incomplete Freund's adjuvants, and aluminum hydroxide.

In some aspects, it is preferred that the adjuvant be selected to be a preferential inducer of either a Th1 or a Th2-type of response. High levels of Th1-type cytokines tend to favor the induction of cell mediated immune responses to a given antigen, while high levels of Th2-type cytokines tend to favor the induction of humoral immune responses to the antigen.

The distinction between Th1 and Th2-type immune response is not absolute. In reality an individual will support an immune response which is described as being predominantly Th1 or predominantly Th2. However, it is often convenient to consider the families of cytokines in terms of that described in murine CD4+ T cell clones by Mosmann and Coffman (Mosmann, and Coffman, 1989). Traditionally, Th1-type responses are associated with the production of the INF-γ and IL-2 cytokines by T-lymphocytes. Other cytokines often directly associated with the induction of Th1-type immune responses are not produced by T-cells, such as IL-12. In contrast, Th2-type responses are associated with the secretion of IL- 4, IL-5, IL-6, IL-10.

In addition to adjuvants, it may be desirable to co-administer biologic response modifiers (BRM) to enhance immune responses. BRMs have been shown to upregulate T cell immunity or downregulate suppresser cell activity. Such BRMs include, but are not limited to, Cimetidine (CIM; 1200 mg/d) (Smith/Kline, PA); or low-dose Cyclophosphamide (CYP; 300 mg/m²) (Johnson/ Mead, NJ) and cytokines such as γ-interferon, IL-2, or IL-12 or genes encoding proteins involved in immune helper functions, such as B-7.

### B. Antibodies And Passive Immunization

Direct administration of therapeutic immunoglobulins, also referred to as passive immunization, does not require an immune response from the patient and, therefore, gives immediate protection. In addition, passive immunization can be directed to bacterial structures that are not immunogenic and that are less specific to the organism. Passive immunization against pathogenic organisms has been based on immunoglobulins derived from sera of human or non-human donors.

One aspect is a method of preparing an immunoglobulin for use in prevention or treatment of staphylococcal infection comprising the steps of immunizing a recipient with the vaccine of the invention and isolating immunoglobulin or antibodies from the recipient. An immunoglobulin prepared by this method is a further aspect. A pharmaceutical composition comprising the immunoglobulin of the invention and a pharmaceutically acceptable carrier is a further aspect which could be used in the manufacture of a medicament for the treatment or prevention of staphylococcal disease. A method for treatment or prevention of staphylococcal infection comprising a step of administering to a patient an effective amount of the pharmaceutical preparation of the invention is a further aspect.

Inocula for polyclonal antibody production are typically prepared by dispersing the antigenic composition in a physiologically tolerable diluent such as saline or other adjuvants suitable for human use to form an aqueous composition. An immunostimulatory amount of inoculum is administered to a mammal and the inoculated mammal is then maintained for a time sufficient for the antigenic composition to induce protective antibodies. The antibodies can be isolated to the extent desired by well known techniques such as affinity chromatography (Harlow and Lane, 1988).

Antibodies can include antiserum preparations from a variety of commonly used animals, e.g., goats, primates, donkeys, swine, horses, guinea pigs, rats, or man. The animals are bled and serum recovered.

An immunoglobulin produced in accordance with the present disclosure can include whole antibodies, antibody fragments or sub fragments. Antibodies can be whole immunoglobulins, chimeric antibodies or hybrid antibodies with dual specificity to two or more antigens described herein. They may also be fragments, e.g., F(ab')2, Fab', Fab, Fv and the like including hybrid fragments. An immunoglobulin also includes natural, synthetic or genetically engineered proteins that act like an antibody by binding to specific antigens to form a complex. The term "immunoglobulin," as used herein, includes all immunoglobulin classes and subclasses known in the art including IgA, IgD, IgE, IgG, and IgM, and their subclasses (isotypes), e.g., IgA1, IgA2, IgG1, IgG2, IgG3 and IgG4. Preferably, the immunoglobulins described herein are human immunoglobulins. Also, an antigen-binding and/or variable domain comprising fragment of an immunoglobulin is meant. Antigen-binding fragments include, inter alia, Fab, F(ab'), F(ab')2, Fv, dAb, Fd, complementarity-determining region (CDR) fragments, single-chain antibodies (scFv), bivalent single-chain antibodies, single-chain phage antibodies, diabodies, triabodies, tetrabodies, (poly)peptides that contain at least a fragment of an immunoglobulin that is sufficient to confer specific antigen binding to the (poly)peptide, *etc.*

An antigen composition or vaccine as described herein can be administered to a recipient who then acts as a source of immunoglobulin, produced in response to challenge from the specific vaccine. A subject thus treated would donate plasma from which hyperimmune globulin would be obtained via conventional plasma fractionation methodology. The hyperimmune globulin would be administered to another subject in order to impart resistance against or treat staphylococcal infection. Hyperimmune globulins of the invention are particularly useful for treatment or prevention of staphylococcal disease in infants, immune compromised individuals or where treatment is required and there is no time for the individual to produce antibodies in response to vaccination.

An additional aspect is a pharmaceutical composition comprising one or more monoclonal antibodies (or fragments thereof; preferably human or humanized) reactive against constituents of the immunogenic composition of the invention, which could be used to treat or prevent infection by Gram positive bacteria, preferably staphylococci, more preferably *S. aureus* or *S. epidermidis.* Such pharmaceutical compositions comprise monoclonal antibodies that can be whole immunoglobulins of any class *e.g.* IgG, IgM, IgA, IgD or IgE, chimeric antibodies or hybrid antibodies with specificity to antigens described herein. They may also be fragments, e.g., F(ab')2, Fab', Fab, Fv and the like including hybrid fragments. Methods of making monoclonal antibodies are well known in the art and can include the fusion of splenocytes with myeloma cells (Kohler and Milstein, 1975; Harlow and Lane, 1988). Alternatively, monoclonal Fv fragments can be obtained by screening a suitable phage display library (Vaughan *et al*., 1998). Monoclonal antibodies may be human, humanized, or partly humanized by known methods.

### C. Combination Therapy

The compositions and related methods described herein, particularly administration of a staphylococcal antigen, including a polypeptide or peptide of Emp in combination with one or more of Eap, Adsa, EsaB, EsaC, EsxA, EsxB, SdrC, SdrD, SdrE, Hla, IsdA, IsdB, ClfA, ClfB, IsdC, SasB, SasF, Ebh, Coa, vWa, SpA, 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein peptide or protein to a patient/subject, may also be used in combination with the administration of traditional therapies. These include, but are not limited to, the administration of antibiotics such as streptomycin, ciprofloxacin, doxycycline, gentamycin, chloramphenicol, trimethoprim, sulfamethoxazole, ampicillin, tetracycline or various combinations of antibiotics. In one aspect, it is contemplated that a polypeptide vaccine and/or therapy is used in conjunction with a small molecule or non-peptide inhibitor of AdsA activity.

In one aspect, it is contemplated that a polypeptide vaccine and/or therapy is used in conjunction with antibacterial treatment. Alternatively, the therapy may precede or follow the treatment with the other agent by intervals ranging from minutes to weeks. In embodiments where the other agents and/or a proteins or polynucleotides are administered separately, one would generally ensure that a significant period of time did not expire between the time of each delivery, such that the agent and antigenic composition would still be able to exert an advantageously combined effect on the subject. In such instances, it is contemplated that one may administer both modalities within about 12-24 h of each other and, more preferably, within about 6-12 h of each other. In some situations, it may be desirable to extend the time period for administration significantly, however, where several days (2, 3, 4, 5, 6 or 7) to several weeks (1, 2, 3, 4, 5, 6, 7 or 8) lapse between the respective administrations.

Various combinations may be employed, for example antibiotic therapy is "A" and an immunogenic molecule or antibody given as part of an immune therapy regime, such as an antigen or an AdsA modulator, is "B":

| | | | | | | | | | |
|---|---|---|---|---|---|---|---|---|---|
| A/B/A | B/A/B | B/B/A | A/A/B | A/B/B | | B/A/A | | A/B/B/B | B/A/B/B |
| B/B/B/A | B/B/A/B | | A/A/B/B | | A/B/A/B | | A/B/B/A | | B/B/A/A |
| B/A/B/A | B/A/A/B | | A/A/A/B | | B/A/A/A | | A/B/A/A | | A/A/B/A |

Administration of the immunogenic compositions described herein to a patient/subject will follow general protocols for the administration of such compounds, taking into account the toxicity, if any, of the Emp composition, or composition of any other antigen or antigen combination described herein. It is expected that the treatment cycles would be repeated as necessary. It also is contemplated that various standard therapies, such as hydration, may be applied in combination with the described therapy.

### III. PHARMACEUTICAL COMPOSITIONS

In some embodiments, pharmaceutical compositions are administered to a subject. Different aspects described herein involve administering an effective amount of a composition to a subject. In some embodiments, Empantigens in combination with Eap and/or AdsA and/or members of the Ess pathway and including polypeptides or peptides of the Esa or Esx class, and/or members of sortase substrates, and/or secreted virulence factor and or polysaccharides may be administered to the patient to protect against or treat infection by one or more staphylococcus pathogens. Alternatively, an expression vector encoding one or more such polypeptides or peptides may be given to a patient as a preventative treatment. Additionally, such compounds can be administered in combination with an antibiotic. Such compositions will generally be dissolved or dispersed in a pharmaceutically acceptable carrier or aqueous medium.

As used herein, the term "pharmaceutically acceptable" or "pharmacologically acceptable" refer to those compounds, materials, compositions, and/or dosage forms which are, within the scope of sound medical judgment, suitable for contact with the tissues of human beings and animals without excessive toxicity, irritation, allergic response, or other problem complications commensurate with a reasonable benefit/risk ratio. The term "pharmaceutically acceptable carrier," means a pharmaceutically acceptable material, composition or vehicle, such as a liquid or solid filler, diluent, excipient, solvent or encapsulating material, involved in carrying or transporting a chemical agent. Pharmaceutically acceptable carrier includes any and all solvents, dispersion media, coatings, antibacterial and antifungal agents, isotonic and absorption delaying agents, and the like. The use of such media and agents for pharmaceutical active substances is well known in the art. Except insofar as any conventional media or agent is incompatible with the active ingredients, its use in immunogenic and therapeutic compositions is contemplated. Supplementary active ingredients, such as other anti-bacterial agents, can also be incorporated into the compositions.

In addition to the compounds formulated for parenteral administration, such as those for intravenous or intramuscular injection, other pharmaceutically acceptable forms include, e.g., tablets or other solids for oral administration; time release capsules; and any other form currently used, including creams, lotions, mouthwashes, inhalants and the like.

The active compounds described herein can be formulated for parenteral administration, *e*.*g*., formulated for injection via the intravenous, intramuscular, subcutaneous, or even intraperitoneal routes.

Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions; formulations including sesame oil, peanut oil, or aqueous propylene glycol; and sterile powders for the extemporaneous preparation of sterile injectable solutions or dispersions. In all cases the form must be sterile and must be fluid to the extent that it may be easily injected. It also should be stable under the conditions of manufacture and storage and must be preserved against the contaminating action of microorganisms, such as bacteria and fungi.

The proteinaceous compositions may be formulated into a neutral or salt form. Pharmaceutically acceptable salts, include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier also can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion, and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin. Sterile injectable solutions are prepared by incorporating the active compounds in the required amount in the appropriate solvent with various ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques, which yield a powder of the active ingredient, plus any additional desired ingredient from a previously sterile-filtered solution thereof.

Administration of the compositions according to the present disclosure will typically be via any common route. This includes, but is not limited to oral, nasal, or buccal administration. Alternatively, administration may be by orthotopic, intradermal, subcutaneous, intramuscular, intraperitoneal, intranasal, or intravenous administration. In certain embodiments, a vaccine composition may be inhaled (*e*.*g*., U.S. Patent 6,651,655). Such compositions would normally be administered as pharmaceutically acceptable compositions that include physiologically acceptable carriers, buffers or other excipients.

For parenteral administration in an aqueous solution, for example, the solution should be suitably buffered, if necessary, and the liquid diluent first rendered isotonic with sufficient saline or glucose. These particular aqueous solutions are especially suitable for intravenous, intramuscular, subcutaneous, and intraperitoneal administration. In this connection, sterile aqueous media which can be employed will be known to those of skill in the art in light of the present disclosure. For example, one dosage could be dissolved in isotonic NaCl solution and either added to hypodermoclysis fluid or injected at the proposed site of infusion, (see for example, Remington's Pharmaceutical Sciences, 1990). Some variation in dosage will necessarily occur depending on the condition of the subject. The person responsible for administration will, in any event, determine the appropriate dose for the individual subject.

An effective amount of therapeutic or prophylactic composition is determined based on the intended goal. The term "unit dose" or "dosage" refers to physically discrete units suitable for use in a subject, each unit containing a predetermined quantity of the composition calculated to produce the desired responses discussed above in association with its administration, *i.e*., the appropriate route and regimen. The quantity to be administered, both according to number of treatments and unit dose, depends on the protection desired.

Precise amounts of the composition also depend on the judgment of the practitioner and are peculiar to each individual. Factors affecting dose include physical and clinical state of the subject, route of administration, intended goal of treatment (alleviation of symptoms versus cure), and potency, stability, and toxicity of the particular composition.

Upon formulation, solutions will be administered in a manner compatible with the dosage formulation and in such amount as is therapeutically or prophylactically effective. The formulations are easily administered in a variety of dosage forms, such as the type of injectable solutions described above.

### A. In Vitro, Ex Vivo, or In Vivo Administration

As used herein, the term *in vitro* administration refers to manipulations performed on cells removed from or outside of an animal, including, but not limited to cells in culture. The term *ex vivo* administration refers to cells which have been manipulated *in vitro*, and are subsequently administered to a living animal. The term *in vivo* administration includes all manipulations performed within an animal.

In certain aspects, the compositions may be administered either *in vitro*, *ex vivo*, or *in vivo*. In certain *in vitro* embodiments, autologous B-lymphocyte cell lines are incubated with a virus vector of the instant invention for 24 to 48 hours or with Emp, and/or Eap, AdsA, EsaB, EsaC, EsxA, EsxB, SdrC, SdrD, SdrE, Hla, IsdA, IsdB, ClfA, ClfB, IsdC, SasB, SasF, SpA, vWa, Coa, Ebh, 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein (or any combination thereof). The transduced cells can then be used for *in vitro* analysis, or alternatively for *ex vivo* administration.

U.S. Patents 4,690,915 and 5,199,942disclose methods for *ex vivo* manipulation of blood mononuclear cells and bone marrow cells for use in therapeutic applications.

### B. Lipid Components and Moieties

In certain embodiments, the present disclosure concerns compositions comprising one or more lipids associated with a nucleic acid or a polypeptide/peptide. A lipid is a substance that is insoluble in water and extractable with an organic solvent. Compounds other than those specifically described herein are understood by one of skill in the art as lipids, and are encompassed by the compositions and methods of the present invention. A lipid component and a non-lipid may be attached to one another, either covalently or non-covalently.

A lipid may be a naturally occurring lipid or a synthetic lipid. However, a lipid is usually a biological substance. Biological lipids are well known in the art, and include for example, neutral fats, phospholipids, phosphoglycerides, steroids, terpenes, lysolipids, glycosphingolipids, glucolipids, sulphatides, lipids with ether and ester-linked fatty acids and polymerizable lipids, and combinations thereof.

A nucleic acid molecule or a polypeptide/peptide associated with a lipid may be dispersed in a solution containing a lipid, dissolved with a lipid, emulsified with a lipid, mixed with a lipid, combined with a lipid, covalently bonded to a lipid, contained as a suspension in a lipid or otherwise associated with a lipid. A lipid-associated composition of the present invention is not limited to any particular structure. For example, they may also simply be interspersed in a solution, possibly forming aggregates which are not uniform in either size or shape. In another example, they may be present in a bilayer structure, as micelles, or with a "collapsed" structure. In another non-limiting example, a lipofectamine(Gibco BRL) or Superfect (Qiagen) complex is also contemplated.

In certain embodiments, a composition may comprise about 1%, about 2%, about 3%, about 4% about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, or any range therebetween, of a particular lipid, lipid type, or non-lipid component such as an adjuvant, antigen, peptide, polypeptide, sugar, nucleic acid or other material disclosed herein or as would be known to one of skill in the art. In a non-limiting example, a composition may comprise about 10% to about 20% neutral lipids, and about 33% to about 34% of a cerebroside, and about 1% cholesterol. In another non-limiting example, a liposome may comprise about 4% to about 12% terpenes, wherein about 1% of the micelle is specifically lycopene, leaving about 3% to about 11% of the liposome as comprising other terpenes; and about 10% to about 35% phosphatidyl choline, and about 1% of a non-lipid component. Thus, it is contemplated that compositions of the present invention may comprise any of the lipids, lipid types or other components in any combination or percentage range.

### IV. POLYSACCHARIDES

The immunogenic compositions of the invention may further comprise capsular polysaccharides including one or more of PIA (also known as PNAG) and/or *S.aureus* Type V and/or type VIII capsular polysaccharide and/or *S. epidermidis* Type I, and/or Type II and/or Type III capsular polysaccharide.

### A. PIA (PNAG)

It is now clear that the various forms of staphylococcal surface polysaccharides identified as PS/A, PIA, and SAA are the same chemical entity - PNAG (Maira-Litran *et al*., 2004). Therefore the term PIA or PNAG encompasses all these polysaccharides or oligosaccharides derived from them.

PIA is a polysaccharide intercellular adhesin and is composed of a polymer of β-(1→6)-linked glucosamine substituted with N-acetyl and O-succinyl constituents. This polysaccharide is present in both *S. aureus* and *S. epidermidis* and can be isolated from either source (Joyce *et al*., 2003; Maira-Litran *et al*., 2002). For example, PNAG may be isolated from *S*. *aureus* strain MN8m (WO04/43407). PIA isolated from *S*. *epidermidis* is a integral constituent of biofilm. It is responsible for mediating cell-cell adhesion and probably also functions to shield the growing colony from the host's immune response.

The polysaccharide previously known as poly-N-succinyl-β-(1→6)-glucosamine (PNSG) was recently shown not to have the expected structure since the identification of N- succinylation was incorrect (Maira-Litran *et al*., 2002). Therefore the polysaccharide formally known as PNSG and now found to be PNAG is also encompassed by the term PIA. PIA (or PNAG) may be of different sizes varying from over 400kDa to between 75 and 400kDa to between 10 and 75kDa to oligosaccharides composed of up to 30 repeat units (of β-(1→6)-linked glucosamine substituted with N-acetyl and O-succinyl constituents). Any size of PIA polysaccharide or oligosaccharide may be use in an immunogenic composition of the invention, however a size of over 40kDa is preferred. Sizing may be achieved by any method known in the art, for instance by micro fluidization, ultrasonic irradiation or by chemical cleavage (WO 03/53462, EP497524, EP497525). Preferred size ranges of PIA (PNAG) are 40-400kDa, 40-300kDa, 50-350kDa, 60-300kDa, 50-250kDa and 60-200kDa.

PIA (PNAG) can have different degrees of acetylation due to substitution on the amino groups by acetate. PIA produced *in vitro* is almost fully substituted on amino groups (95- 100%). Alternatively, a deacetylated PIA (PNAG) can be used having less than 60%, preferably less than 50%, 40%, 30%, 20%, 10% acetylation. Use of a deacetylated PIA (PNAG) is preferred since non-acetylated epitopes of PNAG are efficient at mediating opsonic killing of Gram positive bacteria, preferably *S. aureus* and/or *S. epidermidis.* Most preferably, the PIA (PNAG) has a size between 40kDa and 300kDa and is deacetylated so that less than 60%, 50%, 40%, 30% or 20% of amino groups are acetylated. The term deacetylated PNAG (dPNAG) refers to a PNAG polysaccharide or oligosaccharide in which less than 60%, 50%, 40%, 30%, 20% or 10% of the amino groups are acetylated.

In an embodiment, PNAG is a deaceylated to form dPNAG by chemically treating the native polysaccharide. For example, the native PNAG is treated with a basic solution such that the pH rises to above 10. For instance the PNAG is treated with 0.1-5 M, 0.2-4 M, 0.3-3 M, 0.5-2 M, 0.75-1.5 M or 1 M NaOH , KOH or NH₄OH. Treatment is for at least 10 to 30 minutes, or 1, 2, 3, 4, 5, 10, 15 or 20 hours at a temperature of 20-100, 25-80, 30-60 or 30-50 or 35-45 °C. dPNAG may be prepared as described in WO 04/43405.

The polysaccharide(s) included in the immunogenic composition of the invention are preferably conjugated to a carrier protein as described below or alternatively unconjugated.

### B. Type 5 and Type 8 polysaccharides from Staphylococcus

Most strains *of S. aureus* that cause infection in man contain either Type 5 or Type 8 polysaccharides. Approximately 60% of human strains are Type 8 and approximately 30% are Type 5. The structures of Type 5 and Type 8 capsular polysaccharide antigens are described in Moreau *et al*., 1990 and Fournier *et al*., 1984). Both have FucNAcp in their repeat unit as well as ManNAcA which can be used to introduce a sulfhydryl group. The structures were reported as:
Type 5 →4)-β-D-ManNAcA(3OAc)-(1→4)-α-L-FucNAc(1→3)-β-D-FucNAc-(1→ Type 8 →3)-β-D-ManNAcA(4OAc)-(1→3)-α-L-FucNAc(1→3)-β-D-FucNAc-(1→ Recently (Jones, 2005) NMR spectroscopy revised the structures to:Type 5 →4)-β-D-ManNAcA-(1→4)-α-L-FucNAc(3OAc)-(1→3)-β-D-FucNAc-(1→ Type 8 →3)-β-D-ManNAcA(4OAc)-(1→3)-α-L-FucNAc(1→3)-α-D-FucNAc-(1→ Polysaccharides may be extracted from the appropriate strain of *S. aureus* using known methods, U.S. Patent 6,294,177. ATCC 12902 is a Type 5 *S*. *aureus* strain and ATCC 12605 is a Type 8 *S*. *aureus* strain.

Polysaccharides are of native size or alternatively may be sized, for instance by microfluidisation, ultrasonic irradiation or by chemical treatment. The type 5 and 8 polysaccharides included in the immunogenic composition of the invention are preferably conjugated to a carrier protein as described below or are alternatively unconjugated. The immunogenic compositions of the invention may alternatively contain either type 5 or type 8 polysaccharide.

### C. S. atreus 336 antigen

In an embodiment, the immunogenic composition of the invention may comprise the *S*. *aureus* 336 antigen described in U.S. Patent 6,294,177. The 336 antigen comprises β-linked hexosamine, contains no O-acetyl groups and specifically binds to antibodies to *S. aureus* Type 336 deposited under ATCC 55804. In an embodiment, the 336 antigen is a polysaccharide which is of native size or alternatively may be sized, for instance by microfluidisation, ultrasonic irradiation or by chemical treatment. The invention also covers oligosaccharides derived from the 336 antigen. The 336 antigen, where included in the immunogenic composition of the invention is preferably conjugated to a carrier protein as described below or are alternatively unconjugated.

### D. Type I, II and III polysaccharides from S. epidermidis

Strains ATCC-31432, SE-360 and SE-10 *of S. epidermidis* are characteristic of three different capsular types, I, II and III respectively (Ichiman and Yoshida, 1981). Capsular polysaccharides extracted from each serotype of *S. epidermidis* constitute Type I, II, and III polysaccharides. Polysaccharides may be extracted by several methods including the method described in U.S. Patent 4,197,290 or as described in Ichiman *et al*., 1991. In one embodiment of the invention, the immunogenic composition comprises type I and/or II and/or III polysaccharides or oligosaccharides *from S. epidermidis.*

Polysaccharides are of native size or alternatively may be sized, for instance by microfluidisation, ultrasonic irradiation or chemical cleavage. The invention also covers oligosaccharides extracted from *S. epidermidis* strains. These polysaccharides are unconjugated or are preferably conjugated as described below.

### E. Conjugation of polysaccharides

Amongst the problems associated with the use of polysaccharides in vaccination, is the fact that polysaccharides *per se* are poor immunogens. It is preferred that the polysaccharides utilized in the invention are linked to such a protein carrier to improve immunogenicity. Examples of such carriers which may be conjugated to polysaccharide immunogens include the Diphtheria and Tetanus toxoids (DT, DT CRM197 and TT respectively), Keyhole Limpet Haemocyanin (KLH), and the purified protein derivative of Tuberculin (PPD), *Pseudomonas aeruginosa* exoprotein A (rEPA), protein D from *Haemophilus influenzae*, pneumolysin or fragments of any of the above. Fragments suitable for use include fragments encompassing T-helper epitopes. In particular the protein D fragment from *H. influenza* will preferably contain the N-terminal 1/3 of the protein. Protein D is an IgD-binding protein from *Haemophilus influenzae* (EP 0 594 610 B1) and is a potential immunogen. In addition, staphylococcal proteins/antigens may be used as carrier protein in the polysaccharide conjugates of the invention.

A carrier protein that would be particularly advantageous to use in the context of a staphylococcal vaccine is staphylococcal alpha toxoid. The native form may be conjugated to a polysaccharide since the process of conjugation reduces toxicity. Preferably a genetically detoxified alpha toxins such as the His35Leu or His35Arg variants are used as carriers since residual toxicity is lower. Alternatively the alpha toxin is chemically detoxified by treatment with a cross-linking reagent, formaldehyde or glutaraldehyde.

The polysaccharides may be linked to the carrier protein(s) by any known method (for example, U.S. Patents 4,372,945, 4,474,757, and 4,356,170). Preferably, CDAP conjugation chemistry is carried out (see WO95/08348). In CDAP, the cyanylating reagent 1-cyano-dimethylaminopyridinium tetrafluoroborate (CDAP) is preferably used for the synthesis of polysaccharide-protein conjugates. The cyanilation reaction can be performed under relatively mild conditions, which avoids hydrolysis of the alkaline sensitive polysaccharides. This synthesis allows direct coupling to a carrier protein.

The polysaccharide is solubilized in water or a saline solution. CDAP is dissolved in acetonitrile and added immediately to the polysaccharide solution. The CDAP reacts with the hydroxyl groups of the polysaccharide to form a cyanate ester. After the activation step, the carrier protein is added. Amino groups of lysine react with the activated polysaccharide to form an isourea covalent link. After the coupling reaction, a large excess of glycine is then added to quench residual activated functional groups. The product is then passed through a gel permeation column to remove unreacted carrier protein and residual reagents.

Conjugation preferably involves producing a direct linkage between the carrier protein and polysaccharide. Optionally a spacer (such as adipic dihydride (ADH)) may be introduced between the carrier protein and the polysaccharide.

### V. IMMUNE RESPONSE AND ASSAYS

As discussed above, the invention concerns evoking or inducing an immune response in a subject against an Emp polypeptide. In other embodiments an immune response to other peptides or antigens can be evoked or induced, including Eap, AdsA, EsaB, EsaC, EsxA, EsxB, SdrC, SdrD, SdrE, Hla or a variant thereof, IsdA, IsdB, ClfA, ClfB, IsdC, SasB, SasF, SpA, Ebh, Coa, vWa, 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein or any other Staphylococcal peptide or protein. In one embodiment, the immune response can protect against or treat a subject (e.g., limiting abscess persistence) having, suspected of having, or at risk of developing an infection or related disease, particularly those related to staphylococci. One use of the immunogenic compositions of the invention is to prevent nosocomial infections by inoculating or treating a subject prior to hospital treatment.

### A. Immunoassays

The present disclosure includes the implementation of serological assays to evaluate if an immune response is induced or evoked by Emp and any other polypeptide or peptide agent described herein. There are many types of immunoassays that can be implemented. Immunoassays encompassed by the present invention include, but are not limited to, those described in U.S. Patent 4,367,110 (double monoclonal antibody sandwich assay) and U.S. Patent 4,452,901 (western blot). Other assays include immunoprecipitation of labeled ligands and immunocytochemistry, both *in vitro* and *in vivo.*

Immunoassays generally are binding assays. Certain preferred immunoassays are the various types of enzyme linked immunosorbent assays (ELISAs) and radioimmunoassays (RIA) known in the art. Immunohistochemical detection using tissue sections is also particularly useful. In one example, the antibodies or antigens are immobilized on a selected surface, such as a well in a polystyrene microtiter plate, dipstick, or column support. Then, a test composition suspected of containing the desired antigen or antibody, such as a clinical sample, is added to the wells. After binding and washing to remove nonspecifically bound immune complexes, the bound antigen or antibody may be detected. Detection is generally achieved by the addition of another antibody, specific for the desired antigen or antibody, that is linked to a detectable label. This type of ELISA is known as a "sandwich ELISA". Detection also may be achieved by the addition of a second antibody specific for the desired antigen, followed by the addition of a third antibody that has binding affinity for the second antibody, with the third antibody being linked to a detectable label.

Variations on ELISA techniques are known to those of skill in the art. In one such variation, the samples suspected of containing a target antigen or antibody are immobilized onto the well surface and then contacted with the antibodies or antigens of the invention. After binding and appropriate washing, the bound immune complexes are detected. Where the initial antigen specific antibodies are linked to a detectable label, the immune complexes may be detected directly. Again, the immune complexes may be detected using a second antibody that has binding affinity for the first antigen specific antibody, with the second antibody being linked to a detectable label.

Competition ELISAs are also possible implementations in which test samples compete for binding with known amounts of labeled antigens or antibodies. The amount of reactive species in the unknown sample is determined by mixing the sample with the known labeled species before or during incubation with coated wells. The presence of reactive species in the sample acts to reduce the amount of labeled species available for binding to the well and thus reduces the ultimate signal.

Irrespective of the format employed, ELISAs have certain features in common, such as coating, incubating or binding, washing to remove non specifically bound species, and detecting the bound immune complexes.

In ELISAs, it is more customary to use a secondary or tertiary detection means rather than a direct procedure. Thus, after binding of the antigen or antibody to the well, coating with a non reactive material to reduce background, and washing to remove unbound material, the immobilizing surface is contacted with the clinical or biological sample to be tested under conditions effective to allow immune complex (antigen/antibody) formation. Detection of the immune complex then requires a labeled secondary binding ligand or antibody, or a secondary binding ligand or antibody in conjunction with a labeled tertiary antibody or third binding ligand.

After all incubation steps in an ELISA are followed, the contacted surface is washed so as to remove non complexed material. Washing often includes washing with a solution of PBS/Tween, or borate buffer. Following the formation of specific immune complexes between the test sample and the originally bound material, and subsequent washing, the occurrence of even minute amounts of immune complexes may be determined.

To provide a detecting means, the second or third antibody can have an associated label to allow detection. In certain aspects, this label will be an enzyme that will generate color development upon incubating with an appropriate chromogenic substrate. Thus, for example, one will desire to contact and incubate the first or second immune complex with a urease, glucose oxidase, alkaline phosphatase, or hydrogen peroxidase conjugated antibody for a period of time and under conditions that favor the development of further immune complex formation, e.g., incubation for 2 hours at room temperature in a PBS containing solution such as PBS Tween.

After incubation with the labeled antibody, and subsequent to washing to remove unbound material, the amount of label is quantified, e.g., by incubation with a chromogenic substrate such as urea and bromocresol purple or 2,2' azino-di(3-ethyl benzthiazoline-6-sulfonic acid [ABTS] and H₂O₂, in the case of peroxidase as the enzyme label. Quantification is then achieved by measuring the degree of color generation, e.g., using a visible spectra spectrophotometer. Alternatively, the label may be a chemiluminescent label (see, U.S. Patents 5,310,687, 5,238,808 and 5,221,605).

### B. Diagnosis of Bacterial Infection

In addition to the use of proteins, polypeptides, and/or peptides, as well as antibodies binding these polypeptides, proteins, and/or peptides to treat or prevent infection as described above, the present disclosure contemplates the use of these polypeptides, proteins, peptides, and/or antibodies in a variety of ways, including the detection of the presence of Staphylococci and diagnosing an infection, whether in a patient or on medical equipment which may also become infected. A preferred method of detecting the presence of infections involves the steps of obtaining a sample suspected of being infected by one or more staphylococcal bacteria species or strains, such as a sample taken from an individual, for example, from one's blood, saliva, tissues, bone, muscle, cartilage, or skin. Following isolation of the sample, diagnostic assays utilizing the polypeptides, proteins, peptides, and/or antibodies of the present invention may be carried out to detect the presence of staphylococci, and such assay techniques for determining such presence in a sample are well known to those skilled in the art and include methods such as radioimmunoassay, western blot analysis and ELISA assays. In general, a method of diagnosing an infection is contemplated wherein a sample suspected of being infected with staphylococci has added to it the polypeptide, protein, peptide, antibody, or monoclonal antibody in accordance with the present invention, and staphylococci are indicated by antibody binding to the polypeptides, proteins, and/or peptides, or polypeptides, proteins, and/or peptides binding to the antibodies in the sample.

Accordingly, antibodies in accordance with the invention may be used for the prevention of infection from staphylococcal bacteria, for the treatment of an ongoing infection, or for use as research tools. The term "antibodies" as used herein includes monoclonal, polyclonal, chimeric, single chain, bispecific, human, simianized, and humanized or primatized antibodies as well as Fab fragments, such as those fragments which maintain the binding specificity of the antibodies, including the products of an Fab immunoglobulin expression library. Accordingly, the invention contemplates the use of single chains such as the variable heavy and light chains of the antibodies. Generation of any of these types of antibodies or antibody fragments is well known to those skilled in the art. Specific examples of the generation of an antibody to a bacterial protein can be found in U.S. Patent Publication 20030153022.

Any of the above described polypeptides, proteins, peptides, and/or antibodies may be labeled directly with a detectable label for identification and quantification of staphylococcal bacteria. Labels for use in immunoassays are generally known to those skilled in the art and include enzymes, radioisotopes, and fluorescent, luminescent and chromogenic substances, including colored particles such as colloidal gold or latex beads. Suitable immunoassays include ELISAs.

### C. Protective Immunity

In some embodimentsof the invention, proteinaceous compositions confer protective immunity on a subject. Protective immunity refers to a body's ability to mount a specific immune response that protects the subject from developing a particular disease or condition that involves the agent against which there is an immune response. An immunogenically effective amount is capable of conferring protective immunity to the subject. As used herein, the term polypeptide and peptide refers to a stretch of amino acids covalently linked there amongst via peptide bonds. Different polypeptides have different functionalities according to the present invention. While according to one aspect, a polypeptide is derived from an immunogen designed to induce an active immune response in a recipient, according to another aspect of the invention, a polypeptide is derived from an antibody which results following the elicitation of an active immune response, in, for example, an animal, and which can serve to induce a passive immune response in the recipient. In both cases, however, the polypeptide can be encoded by a polynucleotide according to any possible codon usage.

As used herein the phrase "immune response" or its equivalent "immunological response" refers to the development of a humoral (antibody mediated), cellular (mediated by antigen-specific T cells or their secretion products) or both humoral and cellular response directed against a protein, peptide, carbohydrate or polypeptide of the invention in a recipient patient. Such a response can be an active response induced by administration of immunogen or a passive response induced by administration of antibody, antibody containing material, or primed T-cells.

A cellular immune response is elicited by the presentation of polypeptide antigens or epitopes in association with Class I or Class II MHC molecules, to activate antigen-specific CD4 (+) T helper cells and/or CD8 (+) cytotoxic T cells. The response may also involve activation ofmonocytes, macrophages, NK cells, basophils, dendritic cells, astrocytes, microglia cells, eosinophils or other components of innate immunity.

As used herein "active immunity" refers to any immunity conferred upon a subject by administration of an antigen.

As used herein "passive immunity" refers to any immunity conferred upon a subject without administration of an antigen to the subject. "Passive immunity" therefore includes, but is not limited to, administration of activated immune effectors including cellular mediators or protein mediators (e.g., monoclonal and/or polyclonal antibodies) of an immune response.

A monoclonal or polyclonal antibody composition may be used in passive immunization for the prevention or treatment of infection by organisms that carry or may be exposed to the antigen recognized by the antibody. An antibody composition may include antibodies that bind to a variety of antigens that may in turn be associated with various organisms. The antibody component can be a polyclonal antiserum. In certain aspects the antibody or antibodies are affinity purified from an animal or second subject that has been challenged with an antigen(s). Alternatively, an antibody mixture may be used, which is a mixture of monoclonal and/or polyclonal antibodies to antigens present in the same, related, or different microbes or organisms, such as gram-positive bacteria, gram-negative bacteria, including but not limited to staphylococcus bacteria.

Passive immunity may be imparted to a patient or subject by administering to the patient immunoglobulins (Ig) and/or other immune factors obtained from a donor or other non-patient source having a known immunoreactivity. In other aspects, an antigenic composition of the present invention can be administered to a subject who then acts as a source or donor for globulin, produced in response to challenge with the antigenic composition ("hyperimmune globulin"), that contains antibodies directed against Staphylococcus or other organism. A subject thus treated would donate plasma from which hyperimmune globulin would then be obtained, *via* conventional plasma-fractionation methodology, and administered to another subject in order to impart resistance against or to treat staphylococcus infection. Hyperimmune globulins according to the invention are particularly useful for immune-compromised individuals, for individuals undergoing invasive procedures or where time does not permit the individual to produce their own antibodies in response to vaccination. See U.S. Patents 6,936,258, 6,770,278, 6,756,361, 5,548,066, 5,512,282, 4,338,298, and 4,748,018, for exemplary methods and compositions related to passive immunity.

In certain aspects, methods include treating or preventing infection by administering the antibody compositions, such as antibodies that bind the above-described antigens, to a subject in need thereof. A target patient population for the treatment and prevention of infection includes mammals, such as humans, who are infected with or at risk of being infected by bacterial pathogens. In one embodiment, the infection to be treated or prevented is an *S. aureus* infection, including an infection of methicillin-resistant *S*. *aureus* or *S. aureus* producing alpha-toxin, or an *S. epidermidis* infection. In accordance with one embodiment, provided is a method for treating or preventing an *S*. *aureus* infection using compositions comprising one or more *S. aureus* Emp, Eap, AdsA, EsaB, EsaC, EsxA, EsxB, SdrC, SdrD, SdrE, Hla, IsdA, IsdB, ClfA, ClfB, IsdC, SasB, SasF, SpA, Ebh, vWa, Coa, 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein antibodies and a pharmaceutically acceptable carrier. The *S. aureus* antibody can bind to any of those antigens described above. In one embodiment, the antibody composition is a antibody composition or a hyperimmune composition. In another embodiment, the antibodies are recombinant, human, or humanized antibodies. In yet another embodiment, the antibodies are monoclonal antibodies, or fragments thereof.

A therapeutically or prophylactically effective amount of the antibody compositions can be determined by methods that are routine in the art. Skilled artisans will recognize that the amount may vary according to the particular antibodies within the composition, the concentration of antibodies in the composition, the frequency of administration, the severity of infection to be treated or prevented, and subject details, such as age, weight and immune condition. In some embodiments, the dosage will be at least 1, 5, 10, 25, 50, or 100 µg or mg of antibody composition per kilogram of body weight (mg/kg), including at least 100 mg/kg, at least 150 mg/kg, at least 200 mg/kg, at least 250 mg/kg, at least 500 mg/kg, at least 750 mg/kg and at least 1000 mg/kg. Dosages for monoclonal antibody compositions typically may be lower, such as 1/10 of the dosage of an antibody composition, such as at least about 1, 5, 10, 25, or 50 µg or mg/kg, at least about 10 mg/kg, at least about 15 mg/kg, at least about 20 mg/kg, or at least about 25 mg/kg. The route of administration may be any of those appropriate for a passive vaccine. Thus, intravenous, subcutaneous, intramuscular, intraperitoneal, inhalation, and other routes of administration are envisioned. As noted above, a therapeutically or prophylactically effective amount of antibody is an amount sufficient to achieve a therapeutically or prophylactically beneficial effect.

A protective antibody composition may neutralize and/or prevent infection. A protective antibody composition may comprise amounts of Emp, Eap, AdsA, EsaB, EsaC, EsxA, EsxB, SdrC, SdrD, SdrE, Hla, IsdA, IsdB, ClfA, ClfB, IsdC, SasB, SasF, SpA, Ebh, Coa, vWa, 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein antibodies that are not protective on their own, but which, in combination, yield a protective antibody composition.

The antibody composition may be administered in conjunction with an anti-infective agent, an antibiotic agent, and/or an antimicrobial agent, in a combination therapy. Anti-infective agents include, but are not limited to vancomycin and lysostaphin. Antibiotic agents and antimicrobial agents include, but are not limited to penicillinase-resistant penicillins, cephalosporins and carbapenems, including vancomycin, lysostaphin, penicillin G, ampicillin, oxacillin, nafcillin, cloxacillin, dicloxacillin, cephalothin, cefazolin, cephalexin, cephradine, cefamandole, cefoxitin, imipenem, meropenem, gentamycin, teicoplanin, lincomycin and clindamycin. The dosages of these antibiotics are well known in the art. See for example, Merck Manual Of Diagnosis And Therapy, § 13, Ch. 157, 100^{th} Ed. (Beers & Berkow, eds., 2004). The anti-infective, antibiotic and/or antimicrobial agents may be combined prior to administration, or administered concurrently or sequentially with active or passive immunotherapies described herein.

In some embodiments, relatively few doses of antibody composition are administered, such as one or two doses, and conventional antibiotic therapy is employed, which generally involves multiple doses over a period of days or weeks. Thus, the antibiotics can be taken one, two or three or more times daily for a period of time, such as for at least 5 days, 10 days or even 14 or more days, while the antibody composition is usually administered only once or twice. However, the different dosages, timing of dosages and relative amounts of antibody composition and antibiotics can be selected and adjusted by one of ordinary skill in the art.

For purposes of this specification and the accompanying claims the terms "epitope" and "antigenic determinant" are used interchangeably to refer to a site on an antigen to which B and/or T cells respond or recognize. B-cell epitopes can be formed both from contiguous amino acids or noncontiguous amino acids juxtaposed by tertiary folding of a protein. Epitopes formed from contiguous amino acids are typically retained on exposure to denaturing solvents whereas epitopes formed by tertiary folding are typically lost on treatment with denaturing solvents. An epitope typically includes at least 3, and more usually, at least 5 or 8-10 amino acids in a unique spatial conformation. Methods of determining spatial conformation of epitopes include, for example, x-ray crystallography and 2-dimensional nuclear magnetic resonance. See, *e*.*g*., Epitope Mapping Protocols (1996). Antibodies that recognize the same epitope can be identified in a simple immunoassay showing the ability of one antibody to block the binding of another antibody to a target antigen. T- cells recognize continuous epitopes of about nine amino acids for CD8 cells or about 13-15 amino acids for CD4 cells. T cells that recognize the epitope can be identified by *in vitro* assays that measure antigen-dependent proliferation, as determined by ³H-thymidine incorporation by primed T cells in response to an epitope (Burke *et al*., 1994), by antigen-dependent killing (cytotoxic T lymphocyte assay, Tigges *et al*., 1996) or by cytokine secretion.

The presence of a cell-mediated immunological response can be determined by proliferation assays (CD4 (+) T cells) or CTL (cytotoxic T lymphocyte) assays. The relative contributions of humoral and cellular responses to the protective or therapeutic effect of an immunogen can be distinguished by separately isolating IgG and T-cells from an immunized syngeneic animal and measuring protective or therapeutic effect in a second subject.

As used herein and in the claims, the terms "antibody" or "immunoglobulin" are used interchangeably and refer to any of several classes of structurally related proteins that function as part of the immune response of an animal or recipient, which proteins include IgG, IgD, IgE, IgA, IgM and related proteins.

Under normal physiological conditions antibodies are found in plasma and other body fluids and in the membrane of certain cells and are produced by lymphocytes of the type denoted B cells or their functional equivalent. Antibodies of the IgG class are made up of four polypeptide chains linked together by disulfide bonds. The four chains of intact IgG molecules are two identical heavy chains referred to as H-chains and two identical light chains referred to as L-chains.

In order to produce polyclonal antibodies, a host, such as a rabbit, goat, sheep or human, is immunized with the antigen or antigen fragment, generally with an adjuvant and, if necessary, coupled to a carrier. Antibodies to the antigen are subsequently collected from the sera of the host. The polyclonal antibody can be affinity purified against the antigen rendering it monospecific.

In order to produce monoclonal antibodies, hyperimmunization of an appropriate donor, generally a mouse, with the antigen is undertaken. Isolation of splenic antibody producing cells is then carried out. These cells are fused to a cell characterized by immortality, such as a myeloma cell, to provide a fused cell hybrid (hybridoma) which can be maintained in culture and which secretes the required monoclonal antibody. The cells are then cultured, in bulk, and the monoclonal antibodies harvested from the culture media for use. By definition, monoclonal antibodies are specific to a single epitope. Monoclonal antibodies often have lower affinity constants than polyclonal antibodies raised against similar antigens for this reason.

Monoclonal antibodies may also be produced *ex-vivo* by use of primary cultures of splenic cells or cell lines derived from spleen (Anavi, 1998). In order to produce recombinant antibody (see generally Huston *et al*., 1991; Johnson *et al*., 1991; Mernaugh *et al*., 1995), messenger RNAs from antibody producing B-lymphocytes of animals, or hybridoma are reverse-transcribed to obtain complementary DNAs (cDNAs). Antibody cDNA, which can be full length or partial length, is amplified and cloned into a phage or a plasmid. The cDNA can be a partial length of heavy and light chain cDNA, separated or connected by a linker. The antibody, or antibody fragment, is expressed using a suitable expression system to obtain recombinant antibody. Antibody cDNA can also be obtained by screening pertinent expression libraries.

The antibody can be bound to a solid support substrate or conjugated with a detectable moiety or be both bound and conjugated as is well known in the art. For a general discussion of conjugation of fluorescent or enzymatic moieties see Johnstone *et al.* (1982). The binding of antibodies to a solid support substrate is also well known in the art (Harlow *et al*., 1988; Borrebaeck, 1992).

As used herein and in the claims, the phrase "an immunological portion of an antibody" include a Fab fragment of an antibody, a Fv fragment of an antibody, a heavy chain of an antibody, a light chain of an antibody, an unassociated mixture of a heavy chain and a light chain of an antibody, a heterodimer consisting of a heavy chain and a light chain of an antibody, a catalytic domain of a heavy chain of an antibody, a catalytic domain of a light chain of an antibody, a variable fragment of a light chain of an antibody, a variable fragment of a heavy chain of an antibody, and a single chain variant of an antibody, which is also known as scFv. In addition, the term includes chimeric immunoglobulins, which are the expression products of fused genes derived from different species. One of the species can be a human, in which case a chimeric immunoglobulin is said to be humanized. Typically, an immunological portion of an antibody competes with the intact antibody from which it was derived for specific binding to an antigen.

Optionally, an antibody or preferably an immunological portion of an antibody, can be chemically conjugated to, or expressed as, a fusion protein with other proteins. For purposes of this specification and the accompanying claims, all such fused proteins are included in the definition of antibodies or an immunological portion of an antibody. As used herein the terms "immunogenic agent" or "immunogen" or "antigen" are used interchangeably to describe a molecule capable of inducing an immunological response against itself on administration to a recipient, either alone, in conjunction with an adjuvant, or presented on a display vehicle.

### VI. NUCLEIC ACIDS

In certain embodiments, the present disclosure concerns recombinant polynucleotides encoding the proteins, polypeptides and peptides described herein. The nucleic acid sequences for Empand other bacterial proteins including, but not limited to Eap, AdsA, EsaB, EsaC, EsxA, EsxB, SdrC, SdrD, SdrE, Hla, IsdA, IsdB, ClfB, ClfB, IsdC, SasB, SasF, Ebh, Coa, vWa, SpA, Coa, vWa, Ebh, 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein are included.

As used in this application, the term "polynucleotide" refers to a nucleic acid molecule that either is recombinant or has been isolated free of total genomic nucleic acid. Included within the term "polynucleotide" are oligonucleotides (nucleic acids 100 residues or less in length), recombinant vectors, including, for example, plasmids, cosmids, phage, viruses, and the like. Polynucleotides include, in certain aspects, regulatory sequences, isolated substantially away from their naturally occurring genes or protein encoding sequences. Polynucleotides may be single-stranded (coding or antisense) or double-stranded, and may be RNA, DNA (genomic, cDNA or synthetic), analogs thereof, or a combination thereof. Additional coding or non-coding sequences may, but need not, be present within a polynucleotide. In this respect, the term "gene," "polynucleotide," or "nucleic acid" is used to refer to a nucleic acid that encodes a protein, polypeptide, or peptide (including any sequences required for proper transcription, post-translational modification, or localization). As will be understood by those in the art, this term encompasses genomic sequences, expression cassettes, cDNA sequences, mRNA sequences, and smaller engineered nucleic acid segments that express, or may be adapted to express, proteins, polypeptides, domains, peptides, fusion proteins, and mutants. A nucleic acid encoding all or part of a polypeptide may contain a contiguous nucleic acid sequence encoding all or a portion of such a polypeptide of the following lengths: 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 441, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, 1010, 1020, 1030, 1040, 1050, 1060, 1070, 1080, 1090, 1095, 1100, 1500, 2000, 2500, 3000, 3500, 4000, 4500, 5000, 5500, 6000, 6500, 7000, 7500, 8000, 9000, 10000, or more nucleotides, nucleosides, or base pairs of a polypeptide of the invention. It also is contemplated that a particular polypeptide may be encoded by nucleic acids containing variations having slightly different nucleic acid sequences but, nonetheless, encode the same or substantially similar protein (see Table 2 above).

Particular embodiments concern isolated nucleic acid segments and recombinant vectors incorporating nucleic acid sequences that encode Emp, that may also be in combination with Eap, Adsa, EsaB, EsaC, EsxA, EsxB, SdrC, SdrD, SdrE, Hla , IsdA, IsdB, ClfA, ClfB, IsdC, SasB, SasF, Ebh, Coa, vWa, SpA, 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein. Thus, an isolated nucleic acid segment or vector containing a nucleic acid segment may encode, for example, Emp and Eap, Adsa, EsaB, EsaC, EsxA, EsxB, SdrC, SdrD, SdrE, Hla, IsdA, IsdB, ClfA, ClfB, IsdC, SasB, SasF, Ebh, Coa, vWa, SpA, 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein that is/are immunogenic. The term "recombinant" may be used in conjunction with a polypeptide or the name of a specific polypeptide, and this generally refers to a polypeptide produced from a nucleic acid molecule that has been manipulated *in vitro* or that is a replication product of such a molecule.

Other embodiments concerns isolated nucleic acid segments and recombinant vectors incorporating nucleic acid sequences that encode a peptide or polypeptide to generate an immune response in a subject. In various embodiments the nucleic acids of the invention may be used in genetic vaccines.

The nucleic acid segments used herein, regardless of the length of the coding sequence itself, may be combined with other nucleic acid sequences, such as promoters, polyadenylation signals, additional restriction enzyme sites, multiple cloning sites, other coding segments, and the like, such that their overall length may vary considerably. It is therefore contemplated that a nucleic acid fragment of almost any length may be employed, with the total length preferably being limited by the ease of preparation and use in the intended recombinant nucleic acid protocol. In some cases, a nucleic acid sequence may encode a polypeptide sequence with additional heterologous coding sequences, for example to allow for purification of the polypeptide, transport, secretion, post-translational modification, or for therapeutic benefits such as targeting or efficacy. As discussed above, a tag or other heterologous polypeptide may be added to the modified polypeptide-encoding sequence, wherein "heterologous" refers to a polypeptide that is not the same as the modified polypeptide.

Certain other embodiments concernisolated nucleic acid segments and recombinant vectors that include within their sequence a contiguous nucleic acid sequence from SEQ ID NO:1 (Emp), SEQ ID NO:3 (Eap), SEQ ID NO:5 (EsxA), SEQ ID NO:7 (EsxB), SEQ ID NO:9 (SdrD), SEQ ID NO:11 (SdrE), SEQ ID NO:13 (IsdA), SEQ ID NO:15 (IsdB), SEQ ID NO:17 (SpA), SEQ ID NO:19 (ClfB), SEQ ID NO:21 (IsdC), SEQ ID NO:23 (SasF), SEQ ID NO:25 (SdrC), SEQ ID NO:27 (ClfA),SEQ ID NO:29 (EsaB), SEQ ID NO:31 (EsaC), SEQ ID NO:33 (SasB), or SEQ ID NO:35 (Sas) or any other nucleic acid sequences encoding secreted virulence factors and/or surface proteins.

Certain embodiments provide polynucleotide variants having substantial identity to the sequences disclosed herein; those comprising at least 70%, 75%, 80%, 85%, 90%, 95%, 96%, 97%, 98%, or 99% or higher sequence identity, including all values and ranges there between, compared to a polynucleotide sequence of this invention using the methods described herein (*e*.*g*., BLAST analysis using standard parameters). In certain aspects, the isolated polynucleotide described herein will comprise a nucleotide sequence encoding a polypeptide that has at least 90%, preferably 95% and above, identity to an amino acid sequence of the invention, over the entire length of the sequence; or a nucleotide sequence complementary to said isolated polynucleotide.

Also contemplated is the use of polynucleotides which are complementary to all the above described polynucleotides.

The disclosure also provides for the use of a fragment of a polynucleotide of the invention which when administered to a subject has the same immunogenic properties as a polynucleotide. The disclosure also provides for the use of a polynucleotide encoding an immunological fragment of a protein described herein.

### A. Vectors

Polypeptides described herein may be encoded by a nucleic acid molecule comprised in a vector. The term "vector" is used to refer to a carrier nucleic acid molecule into which a heterologous nucleic acid sequence can be inserted for introduction into a cell where it can be replicated and expressed. A nucleic acid sequence can be "heterologous," which means that it is in a context foreign to the cell in which the vector is being introduced or to the nucleic acid in which is incorporated, which includes a sequence homologous to a sequence in the cell or nucleic acid but in a position within the host cell or nucleic acid where it is ordinarily not found. Vectors include DNAs, RNAs, plasmids, cosmids, viruses (bacteriophage, animal viruses, and plant viruses), and artificial chromosomes (*e*.*g*., YACs). One of skill in the art would be well equipped to construct a vector through standard recombinant techniques (for example Sambrook *et al*., 2001; Ausubel *et al*., 1996). In addition to encoding an Emp polypeptide the vector can encode an Eap, AdsA, EsaB, EsaC, EsxA, EsxB, SdrC, SdrD, SdrE, Hla or a variant thereof, IsdA, IsdB, ClfA, ClfB, IsdC, SasB, SasF, , Ebh, Coa, vWa, SpA, 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein or any other Staphylococcal peptides or proteins, a vector may encode polypeptide sequences such as a tag or immunogenicity enhancing peptide. Useful vectors encoding such fusion proteins include pIN vectors (Inouye *et al*., 1985), vectors encoding a stretch of histidines, and pGEX vectors, for use in generating glutathione S-transferase (GST) soluble fusion proteins for later purification and separation or cleavage.

Vectors described herein may be used in a host cell to produce an Emp = polypeptide. In certain aspects the vectors may also produce Eap, AdsA, EsaB, EsaC, EsxA, EsxB, SdrC, SdrD, SdrE, Hla or a variant thereof, IsdA, IsdB, ClfA, ClfB, IsdC, SasB, SasF, Ebh, Coa, vWa, SpA, 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein or any other Staphylococcal peptides or proteins that may subsequently be purified for administration to a subject or the vector may be purified for direct administration to a subject for expression of the protein in the subject.

The term "expression vector" refers to a vector containing a nucleic acid sequence coding for at least part of a gene product capable of being transcribed. In some cases, RNA molecules are then translated into a protein, polypeptide, or peptide. Expression vectors can contain a variety of "control sequences," which refer to nucleic acid sequences necessary for the transcription and possibly translation of an operably linked coding sequence in a particular host organism. In addition to control sequences that govern transcription and translation, vectors and expression vectors may contain nucleic acid sequences that serve other functions as well and are described herein.

### 1. Promoters and Enhancers

A "promoter" is a control sequence. The promoter is typically a region of a nucleic acid sequence at which initiation and rate of transcription are controlled. It may contain genetic elements at which regulatory proteins and molecules may bind such as RNA polymerase and other transcription factors. The phrases "operatively positioned," "operatively linked," "under control," and "under transcriptional control" mean that a promoter is in a correct functional location and/or orientation in relation to a nucleic acid sequence to control transcriptional initiation and expression of that sequence. A promoter may or may not be used in conjunction with an "enhancer," which refers to a cis-acting regulatory sequence involved in the transcriptional activation of a nucleic acid sequence.

A promoter may be one naturally associated with a gene or sequence, as may be obtained by isolating the 5' non-coding sequences located upstream of the coding segment or exon. Such a promoter can be referred to as "endogenous." Similarly, an enhancer may be one naturally associated with a nucleic acid sequence, located either downstream or upstream of that sequence. Alternatively, certain advantages will be gained by positioning the coding nucleic acid segment under the control of a recombinant or heterologous promoter, which refers to a promoter that is not normally associated with a nucleic acid sequence in its natural environment. A recombinant or heterologous enhancer refers also to an enhancer not normally associated with a nucleic acid sequence in its natural state. Such promoters or enhancers may include promoters or enhancers of other genes, and promoters or enhancers isolated from any other prokaryotic, viral, or eukaryotic cell, and promoters or enhancers not "naturally occurring," *i.e*., containing different elements of different transcriptional regulatory regions, and/or mutations that alter expression. In addition to producing nucleic acid sequences of promoters and enhancers synthetically, sequences may be produced using recombinant cloning and/or nucleic acid amplification technology, including PCR™, in connection with the compositions disclosed herein (see U.S. Patent 4,683,202, U.S. Patent 5,928,906).

Naturally, it may be important to employ a promoter and/or enhancer that effectively directs the expression of the DNA segment in the cell type or organism chosen for expression. Those of skill in the art of molecular biology generally know the use of promoters, enhancers, and cell type combinations for protein expression (see Sambrook *et al*., 2001). The promoters employed may be constitutive, tissue-specific, or inducible and in certain embodiments may direct high level expression of the introduced DNA segment under specified conditions, such as large-scale production of recombinant proteins or peptides. Various elements/promoters may be employed in the context of the present invention to regulate the expression of a gene. Examples of such inducible elements, which are regions of a nucleic acid sequence that can be activated in response to a specific stimulus, include but are not limited to Immunoglobulin Heavy Chain (Banerji *et al*., 1983; Gilles *et al*., 1983; Grosschedl *et al*., 1985; Atchinson *et al*., 1986, 1987; Imler *et al*., 1987; Weinberger *et al*., 1984; Kiledjian *et al*., 1988; Porton *et al*.; 1990), Immunoglobulin Light Chain (Queen *et al*., 1983; Picard *et al*., 1984), T Cell Receptor (Luria *et al*., 1987; Winoto *et al*., 1989; Redondo *et al*.; 1990), HLA DQ α and/or DQ β (Sullivan *et al*., 1987), β Interferon (Goodbourn *et al*., 1986; Fujita *et al*., 1987; Goodbourn *et al*., 1988), Interleukin-2 (Greene *et al*., 1989), Interleukin-2 Receptor (Greene *et al*., 1989; Lin *et al*., 1990), MHC Class II 5 (Koch *et al*., 1989), MHC Class II HLA-DRα (Sherman *et al*., 1989), β-Actin (Kawamoto *et al*., 1988; Ng *et al*.; 1989), Muscle Creatine Kinase (MCK) (Jaynes *et al*., 1988; Horlick *et al*., 1989; Johnson *et al*., 1989), Prealbumin (Transthyretin) (Costa *et al.,* 1988), Elastase I (Ornitz *et al*., 1987), Metallothionein (MTII) (Karin *et al*., 1987; Culotta *et al*., 1989), Collagenase (Pinkert *et al*., 1987; Angel *et al*., 1987), Albumin (Pinkert *et al*., 1987; Tronche *et al*., 1989, 1990), α-Fetoprotein (Godbout *et al.,* 1988; Campere *et al*., 1989), γ-Globin (Bodine *et al*., 1987; Perez-Stable *et al*., 1990), β-Globin (Trudel *et al*., 1987), c-fos (Cohen *et al*., 1987), c-Ha-Ras (Triesman, 1986; Deschamps *et al*., 1985), Insulin (Edlund *et al*., 1985), Neural Cell Adhesion Molecule (NCAM) (Hirsh *et al*., 1990), α1-Antitrypain (Latimer *et al*., 1990), H2B (TH2B) Histone (Hwang *et al*., 1990), Mouse and/or Type I Collagen (Ripe *et al*., 1989), Glucose-Regulated Proteins (GRP94 and GRP78) (Chang *et al*., 1989), Rat Growth Hormone (Larsen *et al*., 1986), Human Serum Amyloid A (SAA) (Edbrooke *et al*., 1989), Troponin I (TN I) (Yutzey *et al*., 1989), Platelet-Derived Growth Factor (PDGF) (Pech *et al*., 1989), Duchenne Muscular Dystrophy (Klamut *et al*., 1990), SV40 (Banerji *et al*., 1981; Moreau *et al*., 1981; Sleigh *et al*., 1985; Firak *et al*., 1986; Herr *et al*., 1986; Imbra *et al*., 1986; Kadesch *et al*., 1986; Wang *et al*., 1986; Ondek *et al*., 1987; Kuhl *et al*., 1987; Schaffner *et al*., 1988), Polyoma (Swartzendruber *et al*., 1975; Vasseur *et al*., 1980; Katinka *et al*., 1980, 1981; Tyndell *et al*., 1981; Dandolo *et al*., 1983; de Villiers *et al*., 1984; Hen *et al*., 1986; Satake *et al*., 1988; Campbell *et al*., 1988), Retroviruses (Kriegler *et al*., 1982, 1983; Levinson *et al*., 1982; Kriegler *et al*., 1983, 1984a, b, 1988; Bosze *et al*., 1986; Miksicek *et al*., 1986; Celander *et al*., 1987; Thiesen *et al*., 1988; Celander *et al*., 1988; Choi *et al*., 1988; Reisman *et al*., 1989), Papilloma Virus (Campo *et al*., 1983; Lusky *et al*., 1983; Spandidos and Wilkie, 1983; Spalholz *et al*., 1985; Lusky *et al*., 1986; Cripe *et al*., 1987; Gloss *et al*., 1987; Hirochika *et al*., 1987; Stephens *et al*., 1987), Hepatitis B Virus (Bulla *et al*., 1986; Jameel *et al*., 1986; Shaul *et al*., 1987; Spandau *et al*., 1988; Vannice *et al*., 1988), Human Immunodeficiency Virus (Muesing *et al*., 1987; Hauber *et al*., 1988; Jakobovits *et al*., 1988; Feng *et al*., 1988; Takebe *et al*., 1988; Rosen *et al*., 1988; Berkhout *et al*., 1989; Laspia *et al*., 1989; Sharp *et al*., 1989; Braddock *et al*., 1989), Cytomegalovirus (CMV) IE (Weber *et al.,* 1984; Boshart *et al.,* 1985; Foecking *et al*., 1986), Gibbon Ape Leukemia Virus (Holbrook *et al*., 1987; Quinn *et al*., 1989).

Inducible elements include, but are not limited to MT II - Phorbol Ester (TFA)/Heavy metals (Palmiter *et al*., 1982; Haslinger *et al*., 1985; Searle *et al*., 1985; Stuart *et al*., 1985; Imagawa *et al*., 1987, Karin *et al*., 1987; Angel *et al*., 1987b; McNeall *et al*., 1989); MMTV (mouse mammary tumor virus) - Glucocorticoids (Huang *et al*., 1981; Lee *et al*., 1981; Majors *et al*., 1983; Chandler *et al*., 1983; Lee *et al*., 1984; Ponta *et al*., 1985; Sakai *et al*., 1988); β-Interferon - poly(rI)x/poly(rc) (Tavernier *et al*., 1983); Adenovirus 5 E2 - E1A (Imperiale *et al*., 1984); Collagenase - Phorbol Ester (TPA) (Angel *et al*., 1987a); Stromelysin - Phorbol Ester (TPA) (Angel *et al*., 1987b); SV40 - Phorbol Ester (TPA) (Angel *et al*., 1987b); Murine MX Gene - Interferon, Newcastle Disease Virus (Hug *et al*., 1988); GRP78 Gene - A23187 (Resendez *et al*., 1988); α-2-Macroglobulin - IL-6 (Kunz *et al*., 1989); Vimentin - Serum (Rittling *et al*., 1989); MHC Class I Gene H-2κb - Interferon (Blanar *et al*., 1989); HSP70 - ElA/SV40 Large T Antigen (Taylor *et al*., 1989, 1990a, 1990b); Proliferin - Phorbol Ester/TPA (Mordacq *et al*., 1989); Tumor Necrosis Factor - PMA (Hensel *et al*., 1989); and Thyroid Stimulating Hormone α Gene - Thyroid Hormone (Chatterjee *et al*., 1989).

The particular promoter that is employed to control the expression of a peptide or protein encoding a polynucleotide described herein is not believed to be critical, so long as it is capable of expressing the polynucleotide in a targeted cell, preferably a bacterial cell. Where a human cell is targeted, it is preferable to position the polynucleotide coding region adjacent to and under the control of a promoter that is capable of being expressed in a human cell. Generally speaking, such a promoter might include either a bacterial, human, or viral promoter.

In various embodiments, the human cytomegalovirus (CMV) immediate early gene promoter, the SV40 early promoter, or the Rous sarcoma virus long terminal repeat can be used to obtain high level expression of an Emp, AdsA and/or Eap polynucleotide. In other embodiments Emp, Eap and/or AdsA can be used expressed in combination with EsaB, EsaC, EsxA, EsxB, SdrC, SdrD, SdrE, Hla or a variant thereof, IsdA, IsdB, ClfA, ClfB, IsdC, SasB, SasF, Spa, vWa, Coa, Ebh, 52kDa vitronectin binding protein (WO 01/60852), Aaa, Aap, Ant, autolysin glucosaminidase, autolysin amidase, Cna, collagen binding protein (US6288214), EFB (FIB), Elastin binding protein (EbpS), EPB, FbpA, fibrinogen binding protein (US6008341), Fibronectin binding protein (US5840846), FnbA, FnbB, GehD (US 2002/0169288), HarA, HBP, Immunodominant ABC transporter, IsaA/PisA, laminin receptor, Lipase GehD, MAP, Mg2+ transporter, MHC II analogue (US5648240), MRPII, Npase, RNA III activating protein (RAP), SasA, SasB, SasC, SasD, SasK,SBI, SdrF(WO 00/12689), SdrG / Fig (WO 00/12689), SdrH (WO 00/12689), SEA exotoxins (WO 00/02523), SEB exotoxins (WO 00/02523), SitC and Ni ABC transporter, SitC/MntC/saliva binding protein (US5,801,234), SsaA, SSP-1, SSP-2, and/or Vitronectin binding protein or any other bacterial polypeptide. The use of other viral or mammalian cellular or bacterial phage promoters, which are well known in the art, to achieve expression of polynucleotides is contemplated as well.

In embodiments in which a vector is administered to a subject for expression of the protein, it is contemplated that a desirable promoter for use with the vector is one that is not down-regulated by cytokines or one that is strong enough that even if down-regulated, it produces an effective amount of an Emp,Eap and/or AdsA polypeptide for eliciting an immune response. Non-limiting examples of these are CMV IE and RSV LTR. In other embodiments, a promoter that is up-regulated in the presence of cytokines is employed. The MHC I promoter increases expression in the presence of IFN-γ.

Tissue specific promoters can be used, particularly if expression is in cells in which expression of an antigen is desirable, such as dendritic cells or macrophages. The mammalian MHC I and MHC II promoters are examples of such tissue-specific promoters.

### 2. Initiation Signals and Internal Ribosome Binding Sites (IRES)

A specific initiation signal also may be required for efficient translation of coding sequences. These signals include the ATG initiation codon or adjacent sequences. Exogenous translational control signals, including the ATG initiation codon, may need to be provided. One of ordinary skill in the art would readily be capable of determining this and providing the necessary signals. It is well known that the initiation codon must be "in-frame" with the reading frame of the desired coding sequence to ensure translation of the entire insert. The exogenous translational control signals and initiation codons can be either natural or synthetic and may be operable in bacteria or mammalian cells. The efficiency of expression may be enhanced by the inclusion of appropriate transcription enhancer elements.

In certain embodiments, the use of internal ribosome entry sites (IRES) elements are used to create multigene, or polycistronic, messages. IRES elements are able to bypass the ribosome scanning model of 5'□ methylated Cap dependent translation and begin translation at internal sites (Pelletier and Sonenberg, 1988). IRES elements from two members of the picornavirus family (polio and encephalomyocarditis) have been described (Pelletier and Sonenberg, 1988), as well an IRES from a mammalian message (Macejak and Sarnow, 1991). IRES elements can be linked to heterologous open reading frames. Multiple open reading frames can be transcribed together, each separated by an IRES, creating polycistronic messages. By virtue of the IRES element, each open reading frame is accessible to ribosomes for efficient translation. Multiple genes can be efficiently expressed using a single promoter/enhancer to transcribe a single message (see U.S. Patents 5,925,565 and 5,935,819).

### 3. Multiple Cloning Sites

Vectors can include a multiple cloning site (MCS), which is a nucleic acid region that contains multiple restriction enzyme sites, any of which can be used in conjunction with standard recombinant technology to digest the vector. (See Carbonelli *et al*., 1999, Levenson *et al*., 1998, and Cocea, 1997.) Frequently, a vector is linearized or fragmented using a restriction enzyme that cuts within the MCS to enable exogenous sequences to be ligated to the vector. Techniques involving restriction enzymes and ligation reactions are well known to those of skill in the art of recombinant technology.

### 4. Splicing Sites

Most transcribed eukaryotic RNA molecules will undergo RNA splicing to remove introns from the primary transcripts. Vectors containing genomic eukaryotic sequences may require donor and/or acceptor splicing sites to ensure proper processing of the transcript for protein expression. (See Chandler *et al*., 1997.)

### 5. Termination Signals

The vectors or constructs described herein will generally comprise at least one termination signal. A "termination signal" or "terminator" is comprised of the DNA sequences involved in specific termination of an RNA transcript by an RNA polymerase. Thus, in certain embodiments a termination signal that ends the production of an RNA transcript is contemplated. A terminator may be necessary *in vivo* to achieve desirable message levels.

In eukaryotic systems, the terminator region may also comprise specific DNA sequences that permit site-specific cleavage of the new transcript so as to expose a polyadenylation site. This signals a specialized endogenous polymerase to add a stretch of about 200 A residues (polyA) to the 3' end of the transcript. RNA molecules modified with this polyA tail appear to more stable and are translated more efficiently. Thus, in other embodiments involving eukaryotes, it is preferred that that terminator comprises a signal for the cleavage of the RNA, and it is more preferred that the terminator signal promotes polyadenylation of the message.

Terminators contemplated for use herein include any known terminator of transcription described herein or known to one of ordinary skill in the art, including but not limited to, for example, the bovine growth hormone terminator or viral termination sequences, such as the SV40 terminator. In certain embodiments, the termination signal may be a lack of transcribable or translatable sequence, such as due to a sequence truncation. Polyadenylation Signals

In expression, particularly eukaryotic expression, one will typically include a polyadenylation signal to effect proper polyadenylation of the transcript. The nature of the polyadenylation signal is not believed to be crucial to the successful practice of the invention, and/or any such sequence may be employed. Preferred embodiments include the SV40 polyadenylation signal and/or the bovine growth hormone polyadenylation signal, convenient and/or known to function well in various target cells. Polyadenylation may increase the stability of the transcript or may facilitate cytoplasmic transport.

### 6. Origins of Replication

In order to propagate a vector in a host cell, it may contain one or more origins of replication sites (often termed "ori"), which is a specific nucleic acid sequence at which replication is initiated. Alternatively an autonomously replicating sequence (ARS) can be employed if the host cell is yeast.

### 7. Selectable and Screenable Markers

In certain embodiments, cells containing a nucleic acid construct of the present disclosure may be identified *in vitro* or *in vivo* by encoding a screenable or selectable marker in the expression vector. When transcribed and translated, a marker confers an identifiable change to the cell permitting easy identification of cells containing the expression vector. Generally, a selectable marker is one that confers a property that allows for selection. A positive selectable marker is one in which the presence of the marker allows for its selection, while a negative selectable marker is one in which its presence prevents its selection. An example of a positive selectable marker is a drug resistance marker.

Usually the inclusion of a drug selection marker aids in the cloning and identification of transformants, for example, markers that confer resistance to neomycin, puromycin, hygromycin, DHFR, GPT, zeocin or histidinol are useful selectable markers. In addition to markers conferring a phenotype that allows for the discrimination of transformants based on the implementation of conditions, other types of markers including screenable markers such as GFP for colorimetric analysis. Alternatively, screenable enzymes such as herpes simplex virus thymidine kinase (tk) or chloramphenicol acetyltransferase (CAT) may be utilized. One of skill in the art would also know how to employ immunologic markers that can be used in conjunction with FACS analysis. The marker used is not believed to be important, so long as it is capable of being expressed simultaneously with the nucleic acid encoding a protein described herein. Further examples of selectable and screenable markers are well known to one of skill in the art.

### B. Host Cells

As used herein, the terms "cell," "cell line," and "cell culture" may be used interchangeably. All of these terms also include their progeny, which is any and all subsequent generations. It is understood that all progeny may not be identical due to deliberate or inadvertent mutations. In the context of expressing a heterologous nucleic acid sequence, "host cell" refers to a prokaryotic or eukaryotic cell, and it includes any transformable organism that is capable of replicating a vector or expressing a heterologous gene encoded by a vector. A host cell can, and has been, used as a recipient for vectors or viruses. A host cell may be "transfected" or "transformed," which refers to a process by which exogenous nucleic acid, such as a recombinant protein-encoding sequence, is transferred or introduced into the host cell. A transformed cell includes the primary subject cell and its progeny.

Host cells may be derived from prokaryotes or eukaryotes, including bacteria, yeast cells, insect cells, and mammalian cells for replication of the vector or expression of part or all of the nucleic acid sequence(s). Numerous cell lines and cultures are available for use as a host cell, and they can be obtained through the American Type Culture Collection (ATCC), which is an organization that serves as an archive for living cultures and genetic materials (www.atcc.org). An appropriate host can be determined by one of skill in the art based on the vector backbone and the desired result. A plasmid or cosmid, for example, can be introduced into a prokaryote host cell for replication of many vectors or expression of encoded proteins. Bacterial cells used as host cells for vector replication and/or expression include Staphylococcus strains, DH5α, JM109, and KC8, as well as a number of commercially available bacterial hosts such as SURE® Competent Cells and SOLOPACK™ Gold Cells (STRATAGENE®, La Jolla, CA). Alternatively, bacterial cells such as E. coli LE392 could be used as host cells for phage viruses. Appropriate yeast cells include *Saccharomyces cerevisiae*, *Saccharomyces pombe*, and *Pichia pastoris.*

Examples of eukaryotic host cells for replication and/or expression of a vector include HeLa, NIH3T3, Jurkat, 293, Cos, CHO, Saos, and PC12. Many host cells from various cell types and organisms are available and would be known to one of skill in the art. Similarly, a viral vector may be used in conjunction with either a eukaryotic or prokaryotic host cell, particularly one that is permissive for replication or expression of the vector.

Some vectors may employ control sequences that allow it to be replicated and/or expressed in both prokaryotic and eukaryotic cells. One of skill in the art would further understand the conditions under which to incubate all of the above described host cells to maintain them and to permit replication of a vector. Also understood and known are techniques and conditions that would allow large-scale production of vectors, as well as production of the nucleic acids encoded by vectors and their cognate polypeptides, proteins, or peptides.

### C. Expression Systems

Numerous expression systems exist that comprise at least a part or all of the compositions discussed above. Prokaryote- and/or eukaryote-based systems can be employed for uses described herein to produce nucleic acid sequences, or their cognate polypeptides, proteins and peptides. Many such systems are commercially and widely available.

The insect cell/baculovirus system can produce a high level of protein expression of a heterologous nucleic acid segment, such as described in U.S. Patents 5,871,986, 4,879,236, both herein incorporated by reference, and which can be bought, for example, under the name MAXBAC® 2.0 from INVITROGEN® and BACPACK™ BACULOVIRUS EXPRESSION SYSTEM FROM CLONTECH®.

In addition to the disclosed expression systems of the invention, other examples of expression systems include STRATAGENE®'s COMPLETE CONTROL™ Inducible Mammalian Expression System, which involves a synthetic ecdysone-inducible receptor, or its pET Expression System, an *E. coli* expression system. Another example of an inducible expression system is available from INVITROGEN®, which carries the T-REX™ (tetracycline-regulated expression) System, an inducible mammalian expression system that uses the full-length CMV promoter. INVITROGEN® also provides a yeast expression system called the *Pichia methanolica* Expression System, which is designed for high-level production of recombinant proteins in the methylotrophic yeast Pichia methanolica. One of skill in the art would know how to express a vector, such as an expression construct, to produce a nucleic acid sequence or its cognate polypeptide, protein, or peptide.

### D. Amplification of Nucleic Acids

Nucleic acids used as a template for amplification may be isolated from cells, tissues or other samples according to standard methodologies (Sambrook *et al*., 2001). In certain embodiments, analysis is performed on samples without substantial purification of the template nucleic acid. The nucleic acid may be genomic DNA. Where RNA is used, it may be desired to first convert the RNA to a complementary DNA.

The term "primer," as used herein, is meant to encompass any nucleic acid that is capable of priming the synthesis of a nascent nucleic acid in a template-dependent process. Typically, primers are oligonucleotides from ten to twenty and/or thirty base pairs in length, but longer sequences can be employed. Primers may be provided in double-stranded and/or single-stranded form, although the single-stranded form is preferred.

Pairs of primers designed to selectively hybridize to nucleic acids corresponding to sequences of genes identified herein are contacted with the template nucleic acid under conditions that permit selective hybridization. Depending upon the desired application, high stringency hybridization conditions may be selected that will only allow hybridization to sequences that are completely complementary to the primers. In other embodiments, hybridization may occur under reduced stringency to allow for amplification of nucleic acids containing one or more mismatches with the primer sequences. Once hybridized, the template-primer complex is contacted with one or more enzymes that facilitate template-dependent nucleic acid synthesis. Multiple rounds of amplification, also referred to as "cycles," are conducted until a sufficient amount of amplification product is produced. A number of template dependent processes are available to amplify the oligonucleotide sequences present in a given template sample. One of the best known amplification methods is the polymerase chain reaction (referred to as PCR™) which is described in detail in U.S. Patents 4,683,195, 4,683,202 and 4,800,159, and in Innis *et al*., 1988.

Alternative methods for amplification of target nucleic acid sequences that may be used in the practice of the present invention are disclosed in U.S. Patents 5,843,650, 5,846,709, 5,846,783, 5,849,546, 5,849,497, 5,849,547, 5,858,652, 5,866,366, 5,916,776, 5,922,574, 5,928,905, 5,928,906, 5,932,451, 5,935,825, 5,939,291 and 5,942,391, GB Application No. 2 202 328, and in PCT Application No. PCT/US89/01025.

### E. Methods of Gene Transfer

Suitable methods for nucleic acid delivery to effect expression of compositions of the present invention are believed to include virtually any method by which a nucleic acid (*e*.*g*., DNA, including viral and nonviral vectors) can be introduced into a cell, a tissue or an organism, as described herein or as would be known to one of ordinary skill in the art. Such methods include, but are not limited to, direct delivery of DNA such as by injection (U.S. Patents 5,994,624, 5,981,274, 5,945,100, 5,780,448, 5,736,524, 5,702,932, 5,656,610, 5,589,466 and 5,580,859), including microinjection (Harland and Weintraub, 1985; U.S. Patent 5,789,215); by electroporation (U.S. Patent No. 5,384,253); by calcium phosphate precipitation (Graham and Van Der Eb, 1973; Chen and Okayama, 1987; Rippe *et al*., 1990); by using DEAE dextran followed by polyethylene glycol (Gopal, 1985); by direct sonic loading (Fechheimer *et al*., 1987); by liposome mediated transfection (Nicolau and Sene, 1982; Fraley *et al*., 1979; Nicolau *et al*., 1987; Wong *et al*., 1980; Kaneda *et al*., 1989; Kato *et al*., 1991); or by microprojectile bombardment (PCT Application Nos. WO 94/09699 and 95/06128; U.S. Patents 5,610,042; 5,322,783, 5,563,055, 5,550,318, 5,538,877 and 5,538,880). Through the application of techniques such as these, organelle(s), cell(s), tissue(s) or organism(s) may be stably or transiently transformed.

### EXAMPLES

The following examples are given for the purpose of illustrating various embodiments of the invention and are not meant to limit the present invention in any fashion. One skilled in the art will appreciate readily that the present invention is well adapted to carry out the objects and obtain the ends and advantages mentioned, as well as those objects, ends and advantages inherent herein. The present examples, along with the methods described herein are presently representative of preferred embodiments, are exemplary, and are not intended as limitations on the scope of the invention.

### EXAMPLE 1

### ENVELOPE PROTEINS ASSOCIATED WITH ABSCESS FORMATION AND VACCINE PROTECTION

### F. RESULTS

The inventors sought to study the pathogenesis of staphylococcal abscess formation and identify the bacterial factors that enable staphylococcal survival and proliferation within this lesion. The studies used the murine renal abscess model, wherein mice are infected with a sub-lethal dose *of S. aureus* to develop a sustained infection (Burts *et al*., 2005). Mice were killed on the fifth day post-infection, their kidneys excised and subjected to histopathology of thin-sectioned hemotoxylin-eosin stained tissue or to enumeration of staphylococcal load by plating tissue homogenate for colony forming units (CFU). In comparison to the wild-type clinical isolated *S. aureus* Newman (Baba *et al*., 2008), an isogenic variant with a transposon insertion in the structural gene for sortase A failed to form abscesses (FIGS. 1B, ID, and 1F). Sortase A, which anchors a large spectrum of surface proteins with LPXTG motif sorting signals to the cell wall envelope, is responsible for the surface display of many different virulence factors (Mazmanian *et al*., 2000; Mazmanian *et al*., 1999). To quantify abscess formation, kidneys were visually inspected, and each individual organ was given a score of one (surface abscesses present) or zero (absent). The final sum was divided by the total number of kidneys to obtain a fractional value. In addition, three randomly chosen right-sided kidneys were placed in 10% formalin overnight, embedded, sectioned, and stained with hematoxylin and eosin. For each kidney, four sagittal sections at 200 µM intervals were viewed by microscopy. If a lesion was observed in any plane of inspection, the organ was judged positive for abscess formation. Mice infected with 3 × 10⁷ CFU/ml *S. aureus* Newman displayed visible lesions on 16/20 kidneys (80% surface abscess) and were positive for abscess formation in 3/3 kidneys examined for histopathology (Table 3). In contrast, mice infected with the Δ*srtA* mutant presented with 0% surface abscesses and 0/3 histological lesions (Table 3).

**Table 3. Recovered Recovered CFUs with standard error, log reduction with respect to Newman, P-value (student's t-test), % surface abscesses observed, # histological abscesses observed.**

| **Strain** | **Staphylococcal load in kidney tissue** | | | **Kidney abscess formation** | |
|---|---|---|---|---|---|
| | log₁₀ CFU g⁻¹ | P-value | Reduction | Surface % | Histopathology |
| wild-type | 6.040 ± 0.095 | - | - | 80 | 3 |
| Δ*srtA* | 3.911 ± 0.389 | 0.0002 | 1.830 | 0 | 0 |
| *Surface protein genes* | | | | | |
| *sdrD* | 3.629 ± 0.758 | 0.0040 | 2.411 | 22 | 1 |
| *isdB* | 4.253 ± 0.510 | 0.0027 | 1.790 | 5 | 1 |
| *clfB* | 4.624 ± 0.446 | 0.0067 | 1.398 | 30 | 2 |
| *isdA* | 4.723 ± 0.280 | 0.0002 | 1.320 | 15 | 1 |
| *sasB* | 5.089 ± 0.448 | 0.0433 | 0.951 | 38 | 2 |
| *sasD* | 5.206 ± 0.375 | 0.0371 | 0.833 | 45 | 1 |
| *sasC* | 5.222 ± 0.400 | 0.0594 | 0.824 | 50 | 2 |
| *sasF* | 5.421 ± 0.360 | 0.1051 | 0.619 | 30 | 2 |
| *sasA* | 5.431 ± 0.403 | 0.1217 | 0.609 | 40 | 2 |
| *sasG* | 5.433 ± 0.360 | 0.1051 | 0.607 | 40 | 2 |
| *isdC* | 5.498 ± 0.292 | 0.0945 | 0.541 | 33 | 2 |
| *fnbpB* | 5.530 ± 0.359 | 0.1856 | 0.511 | 30 | 1 |
| *sasI* | 5.599 ± 0.416 | 0.2681 | 0.441 | 38 | 2 |
| *spa* | 5.681 ± 0.455 | 0.4487 | 0.359 | 10 | 1 |
| *fnbpA* | 5.751 ± 0.322 | 0.3800 | 0.289 | 40 | 2 |
| *sdrE* | 5.848 ± 0.334 | 0.5686 | 0.192 | 61 | 3 |
| *clfA* | 5.898 ± 0.296 | 0.1470 | (+) 0.472 | 40 | 2 |
| *PNAG (PIA) genes* | | | | | |
| *icaA* | 5.326 ± 0.452 | 0.1122 | 0.822 | 40 | 3 |
| *icaB* | 5.894 ± 0.306 | 0.4917 | 0.254 | 35 | 2 |
| *icaC* | 5.651 ± 0.441 | 0.3004 | 0.497 | 35 | 2 |
| *icaD* | 5.886 ± 0.278 | 0.4394 | 0.262 | 45 | 2 |
| *icaR* | 6.201 ± 0.309 | 0.8837 | -0.053 | 60 | 2 |
| *ica:tet* | 5.692 ± 0.280 | 0.1909 | 0.456 | 55 | 2 |
| *Envelope protein genes* | | | | | |
| *eap* | 6.530 ± 0.385 | 0.1217 | (+)0.49 | 50 | 2 |
| *emp* | 5.716 ± 0.080 | 0.1051 | 0.324 | 10 | 1 |
| *eap*/Δ*srtA* | 4.708 ± 0.545 | 0.0129 | 1.332 | 0 | 0 |
| *emp*/Δ*srtA* | 3.165 ± 0.496 | 5.53×10⁻⁴ | 2.875 | 0 | 0 |
| *Capsular polysaccharide genes* | | | | | |
| *capO* | --- | --- | --- | --- | --- |
| ^{a}Means of staphylococcal load in colony forming units (CFU) calculated as log₁₀ CFU g⁻¹ in homogenized renal tissues 5 days following infection in cohorts of fifteen BALB/c mice per challenge strain. Standard error of the means (±SEM) is indicated. ^{b}Statistical significance was calculated with the students t-test and P-values recorded. | | | | | |
| ^{c}Reduction in bacterial load calculated as log¹⁰ CFU g⁻¹. | | | | | |
| ^{c}Abscess formation in kidney tissues five days following infection. was measured by macroscopic inspection (% positive) and histopathology of hematoxylene-eosine stained, thin sectioned tissues from three animals, whereby positive tissues were recorded as fractional values (3/n). | | | | | |

Recovered CFUs with standard error, log reduction with respect to Newman, P-value (student's t-test), % surface abscesses observed, # histological abscesses observed.

**Scanning electron microscopy was used to examine infected tissues.** Kidneys were sectioned, fixed, dehydrated in hexamethyldisilazane (HMDS), and sputter coated with 80%Pt/20%Pd prior to viewing. Kidney tissue infected with *S. aureus* Newman harbored bacteria within a central region of the abscess. Wild-type staphylococci were found in tightly associated lawns (FIG. 1G), contained by a fibrous structure that is internal to the larger fibrin capsule. These staphylococcal nests are devoid of leukocytes and appear to be embedded by an adhesive extracellular matrix. Kidneys tissue infected with the *srtA* mutant also harbored staphylococci, however the bacteria were dispersed throughout healthy renal tissue and significantly reduced in number compared to the wild-type (FIG. 1H).

To identify the specific sortase A substrates responsible for abscess formation, the inventors transduced *bursa aurealis* insertions in 17 sortase substrate genes (Bae *et al*., 2004) into *S. aureus* Newman and screened the variants for virulence defects. Mutations in *SdrD*, *isdB*, *clfB*, *isdA*, *sasB*, and *sasD* caused significantly reduced bacterial load (P<0.05) (FIG. 2). Further, mutations in *sdrD*, *isdB*, and *isdA* presented with fewer abscesses when analyzed by macroscopic and microscopic techniques (Table 3). The inventors considered mutants with < 30% surface abscesses and <1/3 histological scores to display a significant defect in abscess formation. Mutants with mismatched surface histological scores were not counted as defective; rather, it is attributed to screening errors. Interestingly, mutations in *clfB* and *sasB* exhibited defects in staphylococcal load but not in abscess formation, whereas mutations in protein A displayed increased load but also displayed a defect in abscess formation. The inventors wondered whether defects in abscess formation and survival in renal tissue were due to the inability of these mutants to form functional biofilms. In other words, is the defect in abscess formation attributable to defects for *in vitro* biofilm growth? To study this the inventors cultured staphylococci in 96 well assay plates or on coverslips and measured safranin-stained biofilm by absorbance at 405 nm. *S*. *aureus* Newman does not form biofilm in laboratory broth, however Morrissey and colleagues reported biofilm growth in iron-depleted RPMI and 5% atmospheric CO₂ (Johnson *et al*., 2008). Using these conditions, all mutant strains tested here grew equally well (data not shown), however mutations in *SrtA*, *isdB*, *sdrD* or *isdA* exhibited significant (P<0.05) defects in biofilm formation. These results were corroborated by scanning electron microscopy experiments (FIG. 3B, 3C, 3D, 3E, 3F, 3G, and Table 4), where *S. aureus* Newman grows as multicellular complexes embedded in a granular extracellular matrix (FIG. 3B). Multicellular complexes are reduced and the extracellular matrix is diminished in staphylococci with mutations in *srtA*, *isdB*, or *sdrD*, the same mutations that also abolished abscess formation in mice. Thus, *in vitro* biofilm formation may indeed be correlated with the ability of staphylococci to form abscesses. The inventors also tested mutations in genes that abrogate the synthesis of other envelope factors in staphylococci, including poly-*N-*acetylglucosamine (PNAG/PIA which is synthesized by products of *ica* genes) (Heilmann *et al*., 1996; Gotz, 2002) as well as the cell wall associated proteins Eap (Scriba *et al*., 2008; Xie *et al*., 2006) and Emp (Hussain *et al*., 2001). Regulatory factors for staphylococcal virulence were also tested: *saeR* (Novick and Jiang, 2003), *sarA* (Cheung *et al*., 1992), *mgrA* (Chen *et al*., 2006), *agrC*, and *agrA* (Novick, 2003). Mutation in *emp* displayed the largest defect in biofilm formation with an 86% reduction in safranin absorbance and the absence of multicellular complexes by scanning EM, results that are in agreement with a similar study of Johnson *et al* (2008). In contrast, the Eap mutant displayed a slight, but significant defect in biofilm formation (FIG. 3A, Table 4). Emp and Eap are envelope adhesins that mediate interaction between staphylococci and host extracellular matrix proteins fibronectin, vibronectin, and collagen (Scriba *et al*., 2008; Xie *et al*., 2006; Hussain *et al*., 2001). All sequenced strains *of S. aureus* harbor genes for both proteins, which are positively regulated by the two-component system SaeRS and the global transcription factor SarA, mutations in which also impact biofilm formation (Harraghy *et al*., 2005) (FIG. 3A, Table 4).

**Table 4. 96 well plate in vitro biofilm assay. Mean absorbance at 450nm with standard error, fraction reduction from Newman absorbance, P-value (student's t-test)**

| Strain | Mean absorbance @ 450nm ± SEEM | % Reduction (0.967-Abs)/0.967 | P-value |
|---|---|---|---|
| Newman | 0.967 ± 0.054 | - | - |
| Eap | 0.621 ± 0.152 | 0.357 | 0.011 |
| Emp | 0.131 ± 0.026 | 0.861 | 2.30 x 10-10 |
| SaeR | 0.161 ± 0.025 | | 6.80 x 10-12 |
| SarA | 0.252 ± 0.051 | 0.83 | 1.37 x 10-5 |
| MgrA | 0.630 ± 0.012 | 0.793 | 0.064 |
| AgrC | 0.701 ± 0.121 | 0.319 | 0.388 |
| AgrA | 0.889 ± 0.106 | 0.272 | 0.204 |
| IcaA | 0.516 ± 0.121 | 0.081 | 4.34 x 10-4 |
| IcaC | 0.653 ± 0.204 | 0.466 | 0.041 |
| IcaD | 0.791 ± 0.158 | 0.325 | 0.194 |
| IcaB | 0.713 ± 0.184 | 0.182 | 0.08 |
| IcaR | 0.511 ± 0.169 | 0.472 | 1.19 x 10-3 |
| Ica:tet | 0.546 ± 0.169 | 0.2 | 4.28 x 10-3 |
| SrtA | 0.524 ± 0.081 | 0.458 | 6.51 x 10-5 |
| IsdB | 0.415 ± 0.042 | 0.57 | 8.79 x 10-8 |
| SdrD | 0.447 ± 0.090 | 0.537 | 0.002 |
| IsdA | 0.658 ± 0.913 | 0.32 | 0.029 |
| SasD | 0.679 ± 0.187 | 0.298 | 0.064 |
| SasA | 0.707 ± 0.077 | 0.268 | 0.388 |
| SasG | 0.774 ± 0.184 | 0.2 | 0.204 |
| SasH | 0.777 ± 0.156 | 0.197 | 0.196 |
| SpA | 0.824 ± 0.136 | 0.176 | 0.136 |
| SasC | 0.833 ± 0.087 | 0.147 | 0.39 |
| SasI | 0.797 ± 0.142 | 0.138 | 0.419 |
| SasB | 0.841 ± 0.127 | 0.13 | 0.374 |
| IsdC | 0.863 ± 0.024 | 0.107 | 0.433 |
| FnbA | 0.886 ± 0.368 | 0.084 | 0.677 |
| ClfB | 0.913 ± 0.181 | 0.056 | 0.705 |
| ClfA | 1.061 ± 0.107 | -0.097 | 0.705 |
| SdrE | 1.070 ± 0.124 | -0.107 | 0.465 |
| FnbB | 1.120 ± 0.118 | -0.158 | 0.419 |

To study if Emp is required for abscess formation, mice were challenged with *emp* mutant staphylococci and kidneys analyzed five days following infection. The *emp* mutant staphylococci were isolated from kidney tissue with similar abundance as the wild-type parent (FIG. 4A), however these mutants failed to form abscesses and instead remained dispersed throughout kidney tissue (FIG. 4B). These results identify Emp as an envelope factor that is uniquely required for abscess and biofilm formation *in vitro* and *in vivo.* It is noted that mutations in ica genes (*icaABCDR*)*,* which mediate PNAG synthesis (Heilmann *et al*., 1997), exhibited only a slight decrease in biofilm formation. Although unable to produce exopolysaccharide, *ica* mutants were able to generate the extracellular matrix that can be detected by electron microscopy. Further, *ica* mutants failed to display *in vivo* defects in abscess formation or bacterial load (FIGs. 6A, 6B, Table 5). These data suggest therefore that the extracellular matrix of *S. aureus* Newman biofilms is not comprised of PNAG. Emp and Eap are cell wall associated surface proteins, whose production can be detected by SDS extraction of staphylococci and separation on Coomassie-stained PAGE (FIG. 5A). With this assay it was observed that *S. aureus* Newman produces large quantities of Eap and Emp. Mutations in *srtA* or *isdB* do not affect production or cell wall association of Emp and Eap, in agreement with the conjecture that the observed defects of *srtA* and *isdB* mutants in abscess and biofilm formation are not due to secondary effects on Eap and Emp. Of note, mutations in *emp* affect the abundance of Eap and it is surmised that envelope deposition of Emp may affect the surface display of Eap.

**Table 5. Ica virulence. Mean recovered CFUs, log reduction from Newman, P-value (student's t-test), % surface abscesses observed, # histological abscesses.**

| Strain | Mean S. aureus per kidneys ± SEM (log10(CFU)/mL) | Reduction (log10(CFU)/mL) | P-value | % surface abscess | # histology abscess | |
|---|---|---|---|---|---|---|
| Newman | 6.148 ± 0.194 | - | - | 0.7 | 3 | |
| IcaA | 5.326 ± 0.452 | 0.822 | 0.1122 | 0.4 | 2 | |
| IcaB | 5.894 ± 0.306 | 0.254 | 0.4917 | 0.35 | 2 | |
| IcaC | 5.651 ± 0.441 | 0.497 | 0.3004 | 0.35 | 2 | |
| IcaD | 5.886 ± 0.278 | 0.262 | 0.4394 | 0.45 | 2 | |
| IcaR | 6.201 ± 0.309 | -0.053 | 0.8837 | 0.6 | 2 | |
| Ica:tet | 5.692 ± 0.280 | 0.456 | 0.1909 | 0.55 | 2 | |
| SrtA | 3.319 ± 0.604 | 2.849 | 2.26 x 10-4 | 0 | 0 | |
| IcaA/SrtA | 2.247 ± 0.559 | 3.901 | 3.45 x 10-6 (N) | 0 | 0 | |
| | | | 1.84 x 10-5 (I) | | | |
| | | | 0.2097 (S) | | | |

As both proteins displayed prominently on the staphylococcal surface, the inventors contemplated that Emp and Eap represent suitable vaccine antigens to prevent staphylococcal disease. The structural genes for each protein were cloned into pET15b and recombinant products purified by affinity chromatography via N-terminal His-6 tag under denaturing conditions. Following purification, Eap could be folded and soluble product purified by a second round of Ni-NTA chromatography in renaturing buffer. Purified Emp could not be refolded and was thenceforth kept in 8 M urea (FIG. 5B). Mice were immunized with PBS, Eap or Emp emulsified in complete Freund adjuvant (CFA) and challenged mice with 3 × 10⁷ CFU *S. aureus* Newman. FIG. 5C shows that immunization with Emp or Eap conferred significant protection (P<0.05) against staphylococcal infection. Mice vaccinated with Eap displayed a two log reduction in staphylococcal load, whereas Emp immunized mice exhibited a 2.5 log reduction. Mice immunized with Eap (1:24,000) or Emp (>64,000) developed high titers of reactive IgG (FIG. 5D). As expected, animal mock immunized with PBS developed 70% surface and 4/5 histological abscesses. In contrast, mice immunized with Eap and Emp presented with < 20% surface abscesses and 1/5 histological lesions (FIG. 5E). Thus, immunization with Eap or Emp generates specific humoral immune responses and protective immunity against staphylococcal infection.

Studies reveal an association between staphylococcal biofilm growth and the ability to form abscesses in infected host tissues. Previous work established that biofilms are required for colonization and persistent infection of implanted medical devices and allow for protection from antibiotics, antibodies, and phagocytic cells. Evidence presented here suggests that biofilms are required for effective seeding and persistent proliferation of staphylococci within organ tissues. Three sortase substrate genes were identified, *sdrD*, *isdB*, and *isdA* that displayed combinatorial defects in abscess formation, staphylococcal load in infected tissues and *in vitro* biofilm growth. Remarkably, the products of these surface protein genes were also identified as premier vaccine candidates in a comparative evaluation of staphylococcal sortase anchored surface proteins (Stranger-Jones *et al.,* 2006). Such attribute can now also be expanded for two cell wall associated factors Emp and Eap. At least *emp* is required for abscess formation and biofilm growth and immunization with Emp product affords protective immunity against staphylococcal disease. Thus, these studies expand the list of suitable vaccine candidates to prevent human infections with *S. aureus* to select cell wall anchored and cell wall associated proteins, whose combined formulation should provide strong protective immunity against all staphylococcal strains.**Animal model for staphylococcal abscess formation and persistent infection.** To characterize the pathogenesis of *S. aureus* abscess formation, the renal abscess model was modified (Albus *et al.,* 1991), wherein BALB/c mice were infected by intravenous injection with 1 × 10⁷ CFU of the human clinical isolate *S. aureus* Newman (Baba *et al*., 2007). Within 6 hours following infection, 99.999% of staphylococci disappeared from the blood stream and were distributed via the vasculature (FIG. 7A). Staphylococcal dissemination to peripheral tissues occurred rapidly, as the bacterial load in kidney and other peripheral organ tissues reached 1 × 10⁵ CFU g⁻¹ within the first three hours (FIG. 7B). The staphylococcal load in kidney tissues increased by 1.5 log CFU within twenty-four hours (FIG. 7B). Forty-eight hours following infection, mice developed disseminated abscesses in multiple organs, detectable by light microscopy of hematoxylin-eosin stained, thin-sectioned kidney tissue (FIG. 7D-K). The initial abscess diameter was 524 µM (± 65 µM); lesions were initially marked by an influx of polymorphonuclear leukocytes (PMNs) and harbored no discernable organization of staphylococci, most of which appeared to reside within PMNs (FIG. 8A-C). On day 5 of infection, abscesses had increased in size and enclosed a central population of staphylococci, surrounded by a layer of eosinophilic, amorphous material and a large cuff of PMNs (FIG. 8D-F). Histopathology revealed massive necrosis of PMNs in proximity to the staphylococcal nidus at the center of abscess lesions as well as a mantle of healthy phagocytes (FIG. 8D-F). A rim of necrotic PMNs at the periphery of abscess lesions, bordering eosinophilic, amorphous material that separated healthy renal tissue from the infected lesion were also observed (FIG. 8D-F). Abscesses eventually reached a diameter of ≥1,524 µM on day 15 or 36 (FIG. 7K). At later time intervals, staphylococcal load was increased to 10⁴-10⁶ CFU g⁻¹ and growing abscess lesions migrated towards the organ's capsule (FIG. 7J-K). Peripheral lesions were prone to rupture, thereby releasing necrotic material and staphylococci into the peritoneal cavity or the retroperitoneal space. These events resulted in bacteremia as well as a secondary wave of abscesses, eventually precipitating a lethal outcome of these infections (data not shown).

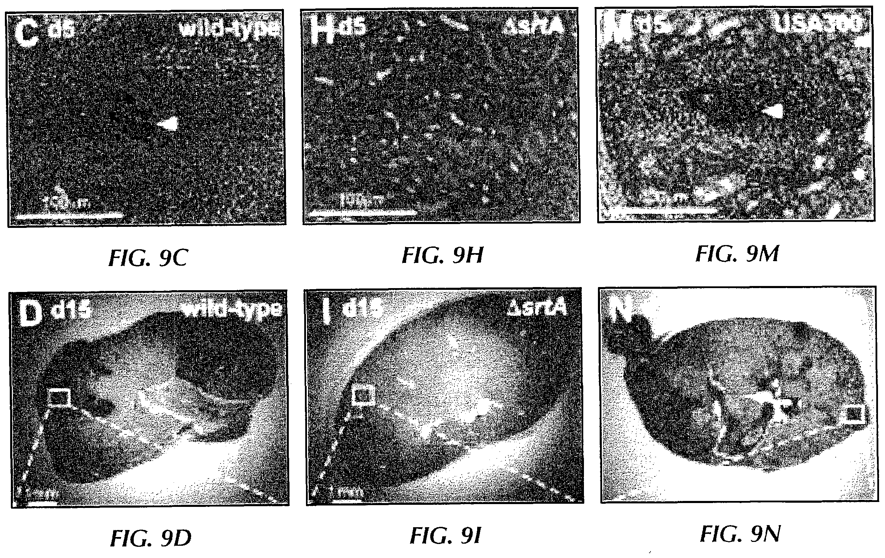

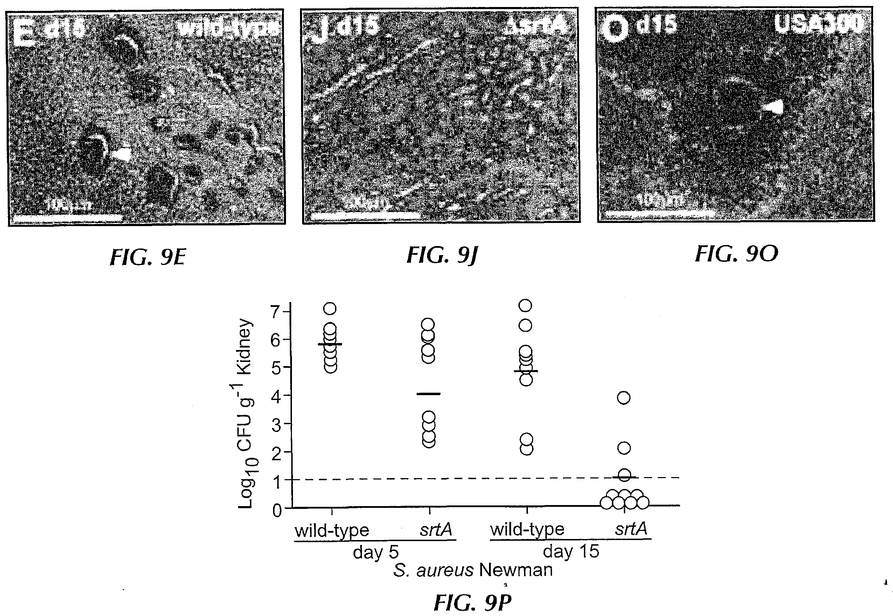

**Staphylococcal abscess communities are enclosed by a pseudocapsule.** To enumerate staphylococcal load in renal tissue, animals were killed, their kidneys excised and tissue homogenate spread on agar media for colony formation. On day 5 of infection, a mean of 1 × 10⁶ CFU g⁻¹ renal tissue for *S. aureus* Newman was observed (FIG. 9P). To quantify abscess formation, kidneys were visually inspected, and each individual organ was given a score of one (FIG. 9A) or zero (FIG. 9F). The final sum was divided by the total number of kidneys to calculate percent surface abscesses (Table 6). In addition, randomly chosen kidneys were fixed in formalin, embedded, thin sectioned, and stained with hematoxylin and eosin. For each kidney, four sagittal sections at 200 µM intervals were viewed by microscopy (FIG. 9). The numbers of lesions were counted for each section and averaged to quantify the number of abscesses within kidneys. *S. aureus* Newman caused 4.364 ± 0.889 abscesses per kidney, and surface abscesses were observed on 14 out of 20 kidneys (70%) (Table 6).

Kidneys were sectioned, fixed, dehydrated and sputter coated with platinum / palladium for scanning electron microscopy. FIG. 10A shows *S. aureus* Newman in tightly associated lawns at the center of abscesses. Staphylococci were contained by an amorphous pseudocapsule (white arrow heads, FIG. 10A) that separated bacteria from the cuff of abscesses leukocytes. No immune cells were observed in these central nests of staphylococci, however occasional red blood cells were located among the bacteria (R, FIG. 10A). Bacterial populations at the abscess center, designated *staphylococcal abscess communities* (SAC), appeared homogenous and were coated by an electron-dense, granular material. The kinetics of the appearance of infectious lesions and the morphological attributes of abscesses formed by *S. aureus* Newman were similar to those observed following mouse infection with *S. aureus* USA300 (LAC), the current epidemic community acquired methicillin-resistant *S. aureus* (CA-MRSA) clone in the United States (Diep *et al*., 2006) (FIG. 9K-O and 10C).

**Table 6. Genetic requirements for S. aureus Newman abscess formation in mice.**

| | **Staphylococcal load in kidney tissue** | | | **Abscess formation in kidney tissue** | | |
|---|---|---|---|---|---|---|
| **Genotype** | **^{a}log₁₀ CFU** g⁻¹ **tissue** | **^{b}Significance (P-value)** | **^{c}Reduction (log₁₀ CFU g⁻¹)** | **^{d}Surface abscesses (%)** | **^{e}Number of abscesses per kidney** | **^{f}Significance (P-value)** |
| wild-type | 6.141 ± 0.192 | - | - | 70 | 4.364 ± 0.889 | - |
| Δs*rtA* | 4.095 ± 0.347 | 6.7×10⁻⁶ | 2.046 | 0 | 0.000 ± 0.000 | 0.0216 |
| *Surface protein genes* | | | | | | |
| *sdrD* | 4.092 ± 0.454 | 0.0001 | 2.049 | 15 | 0.600 ± 0.267 | 0.0265 |
| *isdB* | 4.535 ± 0.298 | 5.7×10⁻⁵ | 1.606 | 5 | 0.500 ± 0.167 | 0.0227 |
| *clfB* | 4.672 ± 0.302 | 0.0001 | 1.469 | 30 | 1.852 ± 0.654 | 0.1298 |
| *isdA* | 4.723 ± 0.299 | 0.0002 | 1.418 | 15 | 0.375 ± 0.182 | 0.0350 |
| *isdC* | 5.050 ± 0.208 | 0.0004 | 1.091 | 27 | 1.000 ± 0.327 | 0.0737 |
| *clfA* | 5.103 ± 0.260 | 0.0025 | 1.038 | 40 | 1.125 ± 0.350 | 0.0848 |
| *spa* | 5.137 ± 0.374 | 0.0144 | 1.004 | 13 | 0.375 ± 0.374 | 0.0356 |
| *sasG* | 5.139 ± 0.287 | 0.0054 | 1.002 | 45 | 1.222 ± 0.425 | 0.0770 |
| *sasC* | 5.193 ± 0.337 | 0.0167 | 0.948 | 56 | 1.375 ± 0.595 | 0.1335 |
| *sasD* | 5.312 ± 0.291 | 0.0212 | 0.829 | 48 | 1.500 ± 0.462 | 0.1272 |
| *sasA* | 5.355 ± 0.217 | 0.0102 | 0.786 | 39 | 2.250 ± 0.453 | 0.2568 |
| *sdrE* | 5.498 ± 0.255 | 0.0475 | 0.643 | 65 | 2.333 ± 0.667 | 0.5023 |
| *sasF* | 5.518 ± 0.318 | 0.0884 | 0.623 | 47 | 1.333 ± 0.408 | 0.3187 |
| *isdH* | 5.555 ± 0.251 | 0.0676 | 0.586 | 44 | 1.125 ± 0.479 | 0.0859 |
| *sasB* | 5.650 ± 0.255 | 0.1641 | 0.491 | 59 | 1.720 ± 0.620 | 0.1651 |
| *fnbA* | 5.678 ± 0.270 | 0.1294 | 0.463 | 51 | 2.125 ± 0.666 | 0.2338 |
| *sdrC* | 5.693 ± 0.287 | 0.1908 | 0.448 | 33 | 1.000 ± 0.378 | 0.0741 |
| *fnbB* | 5.823 ± 0.246 | 0.3124 | 0.318 | 54 | 2.000 ± 0.567 | 0.2074 |
| *PNAG* (*PIA*) *genes* | | | | | | |
| *icaA* | 5.326 ± 0.452 | 0.1122 | 0.815 | 40 | 2.667 ± 1.453 | 0.5768 |
| *icaB* | 5.894 ± 0.306 | 0.4917 | 0.247 | 35 | 1.000 ± 0.270 | 0.2690 |
| *icaC* | 5.651 ± 0.441 | 0.3004 | 0.491 | 35 | 2.000 ± 1.527 | 0.4384 |
| *icaD* | 5.886 ± 0.278 | 0.4394 | 0.255 | 45 | 1.667 ± 0.667 | 0.3741 |
| *icaR* | 6.201 ± 0.309 | 0.8837 | +0.06 | 60 | 2.333 ± 0.333 | 0.5018 |
| *ica:tet* | 5.692 ± 0.280 | 0.1909 | 0.449 | 55 | 2.333 ± 0.667 | 0.5023 |
| *Envelope associated protein genes* | | | | | | |
| *eap* | 6.530 ± 0.385 | 0.1217 | +0.49 | 55 | 1.250 ± 0.412 | 0.0971 |
| *emp* | 5.540 ± 0.040 | 0.0576 | 0.601 | 20 | 0.800 ± 0.416 | 0.0361 |
| *Capsular polysaccharide genes* | | | | | | |
| *capO* | 6.028 ± 0.579 | 0.9825 | 0.113 | 50 | 3.000 ± 1.054 | 0.6035 |

| | | | | | | |
|---|---|---|---|---|---|---|
| ^{a}Means of Staphylococcal load calculated as log₁₀ CFU g⁻¹ in homogenized renal tissues 5 days following infection in cohorts of fifteen BALB/c mice per challenge strain. Standard error of the means (±SEM) is indicated. ^{b}Statistical significance was calculated with the Students *t*-test and P-values recorded: P-values <0.05 were deemed significant. ^{c}Reduction in bacterial load calculated as log₁₀ CFU g⁻¹. ^{d}Abscess formation in Kidney tissues five days following infection was measured by macroscopic inspection (% positive) ^{e}Histopathology of hematoxylene-eosin stained, thin sectioned kidneys from eight to ten animals: the average number of abscesses per kidney was recorded and averaged again for the final mean (±SEM). ^{f}Statistical significance was calculated with the Students *t*-test and P-values recorded; P-values <0.05 were deemed significant. | | | | | | |

**Sortase mutants cannot establish abscess lesions and fail to persist.** Sortase A is a transpeptidase that immobilizes nineteen surface proteins in the envelope of *S. aureus* strain Newman (Mazmanian *et al*., 1999; Mazmanian *et al.,* 2000). Earlier work identified sortase A as a virulence factor in multiple animal model systems, however the contributions of this enzyme and its anchored surface proteins to abscess formation or persistence have not yet been revealed (Jonsson *et al.* 2002; Weiss *et al.,* 2004). Compared to the wild-type parent (Baba *et al.,* 2007), an isogenic *srtA* variant (Δ*srtA*) failed to form abscess lesions on either macroscopic or histopathology examination on days 2, 5 or 15 (FIG. 9F-J and Table 6). In mice infected with the *strA* mutant, only 1×10⁴ CFU g⁻¹ was recovered from kidney tissue on day 5 of infection, which is a 2.046 log₁₀ CFU g⁻¹ reduction compared to the wild-type parent strain (P=6.73×10₋₆)(FIG. 9P). A similar defect was observed for the *SrtA* mutant of MRSA strain USA300 (data not shown). Scanning electron microscopy showed that *SrtA* mutants (arrow heads) were highly dispersed and often associated with leukocytes in otherwise healthy renal tissue (FIG. 10B). On day fifteen following infection, *SrtA* mutants were cleared from renal tissues, a ≥ 3.5 log₁₀ CFU g₋₁ reduction compared to the wild-type (FIG. 9P). Thus, sortase A anchored surface proteins enable the formation of abscess lesions and the persistence of bacteria in host tissues, wherein staphylococci replicate as communities embedded in an extracellular matrix and shielded from surrounding leukocytes by an amorphous pseudocapsule.

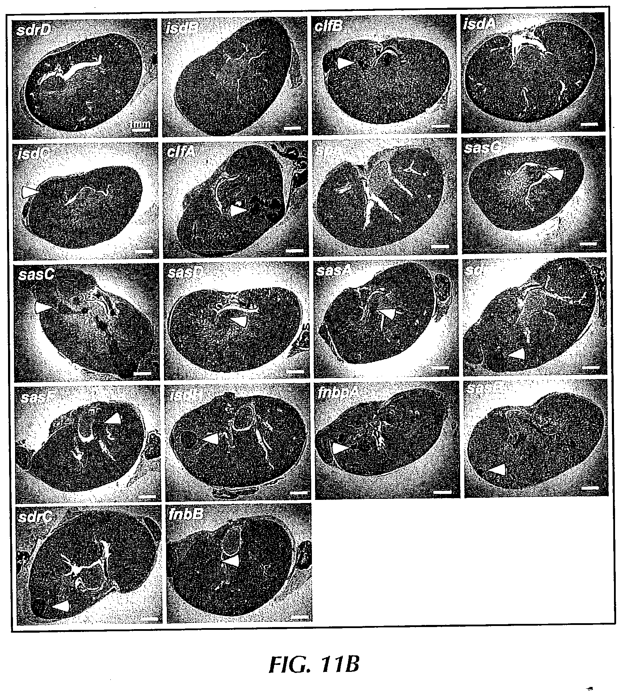

**Genetic requirements for staphylococcal surface proteins.** Sortase A anchors a large spectrum of proteins with LPXTG motif sorting signals to the cell wall envelope, thereby providing for the surface display of many virulence factors (Mazmanian *et al.,* 2002). To identify surface proteins required for staphylococcal abscess formation, *bursa aurealis* insertions were introduced in 5' coding sequences of genes that encode polypeptides with LPXTG motif proteins (Bae *et al.,* 2004) and transduced these mutations into *S. aureus* Newman. Following intravenous infection of mice and analysis through the renal abscess model, the severity of observed virulence defects was rank ordered as the log₁₀ reduction of the means of staphylococcal CFU g⁻¹ (FIG. 11 and Table 6). Mutations in *sdrD, isdB, clfB, isdA, clfA,* and *isdC* caused reduced bacterial load (Table 6). The inventors considered mutants < 30% or less surface abscesses and histology abscess average P < 0.05 as significant for defects in abscess formation, which included variants with mutations in *sdrD, isdB,* and *isdA* (Table 6). Interestingly, mutations in *clfA* and *clfB* exhibited defects in staphylococcal load but not in abscess formation (FIG. 11). These virulence findings are in agreement with previous studies suggesting that clumping factor proteins mediate fibrinogen binding as well as resistance to phagocytic clearance, attributes required for pathogen survival and dissemination in blood (McDevitt *et al.,* 1994; Ni Eidhin *et al.* 1998). Protein A impedes phagocytosis by binding the Fc component of immunoglobulin (Uhlen *et al.,* 1984; Jensen *et al.,* 1958), activates platelet aggregation via the von Willebrand factor (Hartleib *et al.,* 2000), functions as a B cell superantigen by capturing the Fab region of VH3 bearing IgM (Roben *et al.,* 1995), and, through its activation of TNFR1, can initiatestaphylococcal pneumonia (Gomez *et al.,* 2004). Protein A mutants (*spa*) exhibited a modest reduction in staphylococcal load (day 5), however, in contrast to wildtype, *clfA* and *clfB* strains, the ability of *spa* variants to form abscesses was diminished (FIG. 11 and Table 6).

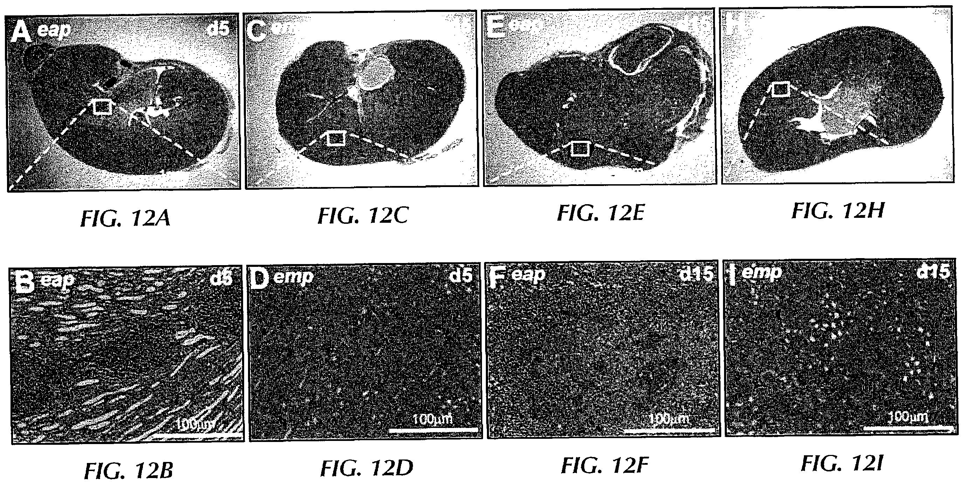

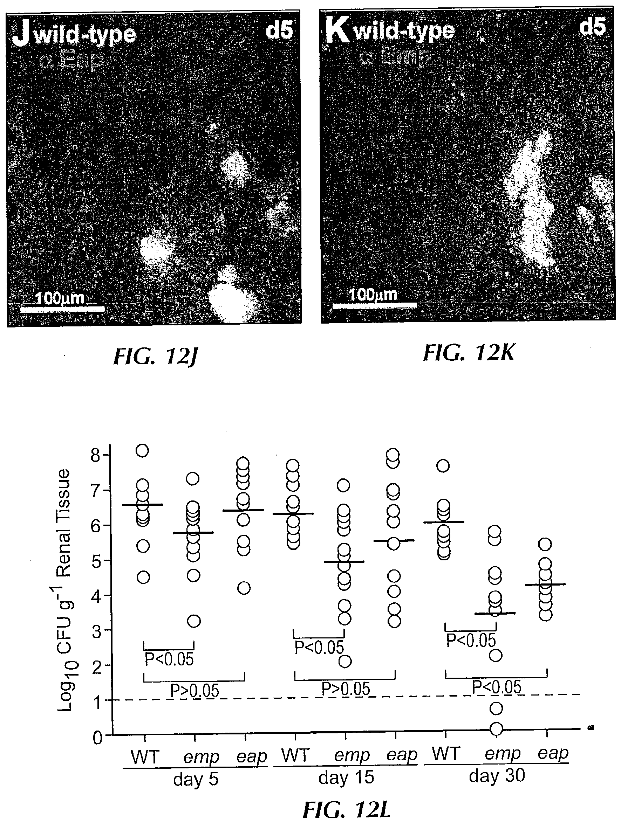

**Staphylococcal carbohydrates and envelope associated proteins.** *S. aureus* elaborates two carbohydrate structures, capsular polysaccharide (CPS) (Jones 2005) and poly-*N*-acetylglucosamine (PNAG) (Gotz 2002). *S. aureus* Newman and USA300 synthesize type 5 CPS, which is composed of a repeating trisaccharide subunit [→4)-β-D-ManAcA-(1→4)-α-L-FucNAc(3OAc)-(1→3)-β-D-FucNAc-(1→] (Baba *et al.,* 2007). Nucleotide sequences of the *cap5* gene cluster comprise a 16 gene operon (*capA-P*) and two of its products, CapP and CapO, function as epimerase and dehydrogenase in the synthesis UDP-*N*-acetylmannosaminuronic acid (UDP-ManNAcA) (O'Riordan and Lee, 2004; Sau *et al.,* 1997). As expected, *bursa aurealis* insertion into *capO* abrogated CPS5 synthesis (data not shown). PNAG (or PIA), a linear β(1-6)-linked glucosaminoglycan, is composed of 2-deoxy-2-amino-D-glucopyranosyl residues, of which 80-85% are *N*9 acetylated (Mack *et al.,* 1996); the remaining glucosamine residues are positively charged and promote association of the polysaccharide with the bacterial envelope (Vuong *et al.,* 2004). PNAG is synthesized by products of the intercellular adhesin locus (*icaADBC*) (Heilmann *et al.,* 1996; Cramton *et al.,* 1999). Both *S. aureus* carbohydrate structures were dispensable for the pathogenesis of animal infections, as mutations in *capO* as well as *icaADBC* or the regulator *icaR* did not affect bacterial load on day 5, the establishment of staphylococcal communities or renal abscess formation (Table 6). The contribution of envelope associated proteins to staphylococcal abscess formation was also examined. The hallmark of envelope associated proteins is that they can be extracted by boiling in hot SDS. This method was used to detect the deposition of two such proteins, Eap and Emp, in the envelope of *S. aureus* Newman. A mutant with *bursa aurealis* insertion in *emp* displayed reduced bacterial load in kidney tissue on day 5 of infection in addition to significant defects in the formation of abscesses and in bacterial persistence within host tissues (FIG. 12, Table 6). No reduction in abscess formation was observed for the *eap* mutant, whereas the reduced staphylococcal load on day 5 and 15 suggests a defect in bacterial peristence within host tissues (FIG. 12, Table 6). Expression of Emp and Eap during infection was detected with immunofluorescence experiments (FIG. 12J-K). Eap was found deposited within the pseudocapsule, whereas Emp was detected in staphylococal abscess communities. These observations support a model whereby Emp contributes to the formation of staphylococcal communities that elicit abscess lesions, whereas Eap deposition in the pseudocapsule promotes bacterial persistence in host tissues.

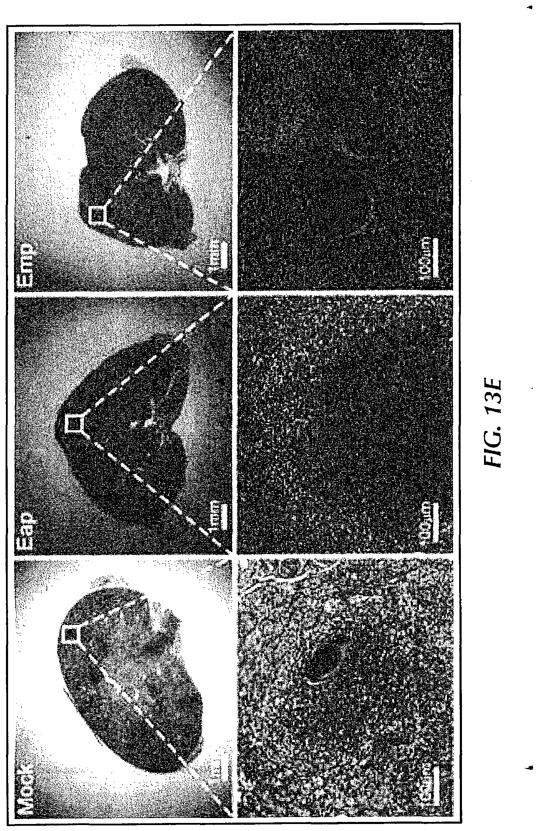

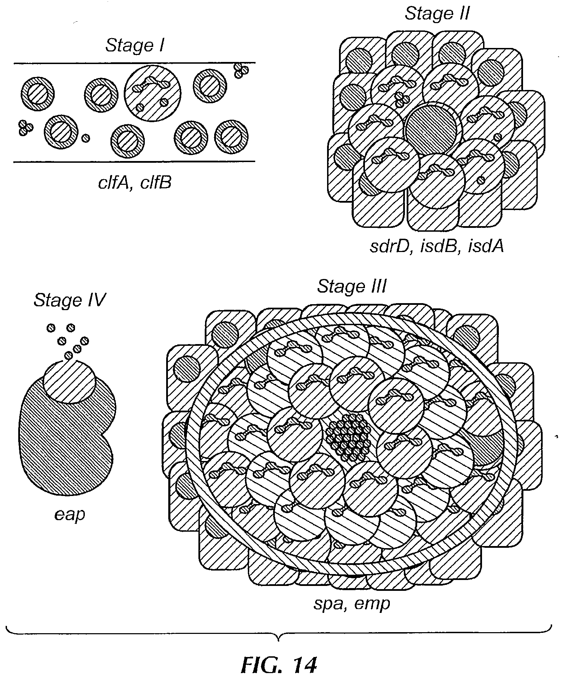

**Envelope associated proteins as vaccine antigens.** Previous work sought to characterize *S. aureus* vaccine antigens by interrogating purified sortase A substrates for their ability to elicit protective immunity towards staphylococcal disease (Stranger-Jones *et al.,* 2006). When used as individual subunit vaccine antigens, surface proteins generated variable degrees of protection; immunization with SdrD, IsdA, IsdB, SdrE, SpA, ClfA as well as ClfB achieved a significant reduction in bacterial load, however none of these vaccines afforded complete protection. In contrast, a combination of four antigens generated much more robust vaccine protection against abscess formation or lethal challenge with several different *S. aureus* strains (Stranger-Jones *et al.,* 2006). Recombinant Eap and Emp was purified from *Escherichia coli* and used these proteins to immunize BALB/c mice for subsequent challenge with *S. aureus* Newman (FIG. 13). Following immunization, mice developed humoral immune responses against both envelope associated proteins (FIG. 13A). Immunization with Emp caused a modest 0.959 log₁₀ CFU g⁻¹ reduction in staphylococcal load within kidney tissues (P=0.5114), whereas a significant level of protection was achieved with Eap (1.939 log₁₀ CFU g⁻¹ reduction in bacterial load, P=0.0079) (FIG. 13B). To test whether Emp or Eap specific antibodies can provide protection against staphylococcal challenge, rabbits were immunized and Emp- as well as Eap-specific antibodies were purified by affinity chromatography.

Passive immunization with 5 mg kg⁻¹ (85 µg per animal) purified antibodies into the peritoneal cavity of naive BALB/c mice resulted in low, but detectable levels of serum IgG 24 hours following transfer (antibody titers of 1,000 ± 110 for Eap and 1,124 ± 236 for Emp, FIG. 13C). In parallel, passively immunized animals were challenged by intravenous inoculation with *S. aureus* Newman, which, when compared to mock controls, resulted in a 1.36 log₁₀ CFU g⁻¹ reduction in staphylococcal load for Eap immunized animals (n=10) on day 4 (P=0.0085) and a reduction in the number of abscesses formed (mock treated 4.64 ± 1.09 abscesses kidney⁻¹ vs. Eap immunized 1.40 ± 0.48, P=0.028, n=14 and 10, FIG. 13D-E).

Animals (n=9) that received Emp-specific antibodies displayed a 1.20 log₁₀ CFU g⁻¹ reduction in staphylococcal load on day 4 (P=0.0132), but only a slightly reduced number of abscesses formed (2.0 ± 0.98, P=0.1362, FIG. 13D-E). In summary, similar to sortase anchored surface proteins, antibodies against envelope associated factors can generate protection against staphylococcal infection in mice.

### G. MATERIALS AND METHODS

**Bacterial Strains and Growth.** Staphylococci were cultured on tryptic soy agar or broth at 37°C. *E. coli* strains DH5α and BL21(DE3) were cultured on Luria agar or broth at 37°C. Ampicillin (100 µg/ml) and erythromycin (10 µg/ml) were used for plasmid and transposon mutant selection, respectively.

**Transposon Mutagenesis.** Insertional mutations from the *Phoenix* library were transduced into human clinical isolate *S. aureus* Newman. Each mutant carries the transposon *bursa aurealis* containing an erythromycin resistance cassette in the gene of interest. The mutations were verified as previously described (Bae *et al.,* 2004). Briefly, chromosomal DNA was extracted (Promega Wizard Kit), digested with Acil (NEB), religated with T4 Ligase (Promega) to form individual plasmids, and PCR amplified using primers specific to the transposon *bursa aurealis.* These products were sequenced to verify the site of transposon insertion in the target gene.

**Cloning, purification, and antibody generation.** Coding sequences for Eap and Emp were PCR-amplified using *S. aureus* Newman template DNA (Baba *et al.,* 2007). PCR products were cloned into pET15b to express recombinant proteins with an N-terminal His6 tag fusion. Bacteria were disrupted in a French press, membrane and insoluble components sedimented by ultracentrifugation. His-tagged Emp was purified by affinity chromatography in its native state. Extract containing Eap was solubilized at room temperature in 8 M urea, 50 mM Tris-HCl pH 8.0 for 4-5 hours, then centrifuged at 10,000 ×*g*. The supernatant containing the denatured protein was subjected to nickel-nitrilotriacetic acid (Ni-NTA) affinity chromatography (Promega). Protein was eluted in PBS-8M urea containing successively higher concentrations of imidazole (100-500 mM). Eluate fractions positive for Eap were pooled, diluted into PBS-1M Urea and passed over a second Ni-NTA column. Refolded Eap was eluted with PBS buffer containing imidazole. Protein concentration was determined by absorbance at 280 nm. Rabbits (6 month old New-Zealand white, females, Charles River Laboratories) were immunized with 500 µg protein emulsified in CFA (Difco) for initial immunization or IFA for booster immunizations on day 24 and 48. On day 60, rabbits were bled and serum recovered for immunoblotting, immune-fluorescence microscopy or passive transfer experiments.

**Scanning Electron Microscopy.** Infected kidneys (right side) were fixed for 24-48 hours in 8% glutaraldehyde at 4°C and sectioned into 2-5mm pieces to expose internal tissues or abscesses. These samples were then dehydrated by successive incubations in 25, 50, 75, 90, 100% ethanol, followed by 100% HMDS. Following dehydration, samples were mounted and sputter coated with 80%Pt/20%Pd to 8 nm before viewing under a Fei NovaNano SEM200 scanning electron microscope. For biofilm assays, staphylococci were grown on coverslips in iron depleted RPMI in 5% CO2 and washed 3 times in PBS. Cover slips were serially dehydrated by incubation in ethanol and HMDS, mounted, and sputter coated prior to viewing under the scanning electron microscope.

**Biofilm formation.** *S. aureus* strains were grown in Chelex (Sigma) treated RPMI 1640 (Gibco) supplemented with 10% RPMI 1640 and 1% Casamino acids (Difco). Overnight cultures were grown at 37°C in 6% CO₂, then inoculated 1:10 in quadruplicate into 96-well flat-bottomed tissue culture plates (Costar) containing fresh media. These plates were incubated statically at 37°C in 6% CO₂ for 24 hours. Wells were washed three times with 1 × PBS, dried for 2 hours at 37°C, and stained with 1% safranin. Absorbance at 450 nm was measured to quantify biofilm formation. Each strain was tested in at least 3 separate experiments and a two-tailed Student *t* test was used to compare mutants to wild-type.

**Renal Abscess.** Overnight cultures of *S. aureus* Newman were inoculated 1:100 into fresh tryptic soy broth and grown for 2 hours at 37°C. Staphylococci were sedimented, washed with 1 × PBS, and suspended in a volume of PBS to yield an *A*600 of 0.6 (3 × 10⁸ CFU/ml). The inoculum was verified by plating and colony enumeration. Mice were anesthetized by intraperitoneal injection of 100 mg/ml of ketamine and 2 mg/ml of xylazine per kilogram of body weight. 6-8 week old female BALB/c mice (Charles River Laboratories) were infected with 100 µl of bacterial suspension (3 × 10⁷ CFU) by retroorbital injection. Cohorts of 10 or 20 mice were infected per staphylococcal strain. On the day 5 following infection, mice were killed by CO₂ inhalation, dissected, and the kidneys were excised and homogenized in 0.01% Triton X-100 using a sonicator. Aliquots (20 µl) were serially diluted and plated for determination of CFU. Three to four right kidneys from each cohort of mice were fixed in 10% formalin for 24 h at room temperature. Tissues were embedded in paraffin, thin-sectioned, stained with hematoxylin and eosin, and examined by microscopy. 3-4 week old female BALB/c mice were used for persistence studies.

**Immunization.** BALB/c mice (24-day-old female, 8-10 mice per group, Charles River Laboratories, Wilmington, MA) were immunized by intramuscular injection into the hind leg with purified protein. Antigen (25 µg purified protein per animal) was administered on days 0 (emulsified 1:1 with complete Freund's adjuvant) and 11 (emulsified 1:1 with incomplete Freund's adjuvant). Mice were bled periorbitally on day 20, followed by retro-orbital challenge in the opposite eye with 10⁷ CFU/ml bacteria on day 21. Mice were killed on day 25 and processed according to the renal abscess model.

**Immunofluorescence microscopy.** Kidneys of infected animals were dissected, placed in 1 × PBS on ice, and then flash frozen in Tissue Tek OCT Compound within cryomolds. Samples were thin sliced (4 µm thick), mounted on slides, and stored at-80°C. Prior to staining, slides were warmed to room temperature for 30 minutes, fixed in ice cold acetone for 10 minutes, and washed twice with ice cold PBS. The slides were blocked in 3% BSA, 1:20 Human IgG (Sigma), 1 × PBS, 0.1% Tween-80 for 1 hour at room temperature with shaking. Specific rabbit antibody (1:2,000) was added to the mixture and slides were allowed to incubate for another hour. The solution was decanted and glass slides were washed 3 times with PBS and 10 minute incubations each. Slides were placed in 3% BSA, 1 × PBS, 0.1% Tween-80, 1:200 AlexaFluor-647 mouse anti-rabbit secondary antibody and allowed to incubate at room temperature in the dark, with shaking. The solution was decanted, slides were washed 3 times with PBS, placed in PBS containing 1:1,000 Hoechst dye (Invitrogen) as well as 1 µg/ml BODIPY-vancomycin and allowed to incubate in the dark for 5 minutes with shaking. The slides were washed once more with PBS, mounted in Npropylgallate, and viewed under a Leica SP5 AOBS spectral two-photon confocal microscope.

**Active and passive immunization.** BALB/c mice (n=15) were immunized with purified Eap or Emp or PBS on day 0, 11. On day 20 following immunization, 5 mice were bled to obtain sera to determine antibody titers and on day 21, all mice were challenged with 1 × 10⁷ CFU *S. aureus* Newman. Five days following infection, kidneys were removed during necropsy and renal tissue analyzed for staphylococcal load or histopathology.

Rabbit Eap or Emp antibodies were purified by affinity chromatography (purified Eap or Emp covalently linked to sepharose) and transferred by intraperitoneal injection into mice. Passively immunized animals were challenged twenty-four hours later by retroorbital injection with 1 × 10⁷ CFU *S. aureus* Newman. Serum IgG titers of actively or passively immunized animals were analyzed by ELISA. Four days following infection, kidneys were removed during necropsy and renal tissue was analyzed for staphylococcal load or histopathology.

### REFERENCES

U.S. Patent 3,791,932
U.S. Patent 3,949,064
U.S. Patent 4,174,384
U.S. Patent 4,197,290
U.S. Patent 4,338,298
U.S. Patent 4,356,170
U.S. Patent 4,367,110
U.S. Patent 4,372,945
U.S. Patent 4,452,901
U.S. Patent 4,474,757
U.S. Patent 4,554,101
U.S. Patent 4,578,770
U.S. Patent 4,596,792
U.S. Patent 4,599,230
U.S. Patent 4,599,231
U.S. Patent 4,601,903
U.S. Patent 4,608,251
U.S. Patent 4,683,195
U.S. Patent 4,683,202
U.S. Patent 4,690,915
U.S. Patent 4,748,018
U.S. Patent 4,800,159
U.S. Patent 4,879,236
U.S. Patent 5,084,269
U.S. Patent 5,199,942
U.S. Patent 5,221,605
U.S. Patent 5,238,808
U.S. Patent 5,310,687
U.S. Patent 5,322,783
U.S. Patent 5,384,253
U.S. Patent 5,512,282
U.S. Patent 5,538,877
U.S. Patent 5,538,880
U.S. Patent 5,548,066
U.S. Patent 5,550,318
U.S. Patent 5,563,055
U.S. Patent 5,580,859
U.S. Patent 5,589,466
U.S. Patent 5,610,042
U.S. Patent 5,620,896
U.S. Patent 5,656,610
U.S. Patent 5,702,932
U.S. Patent 5,736,524
U.S. Patent 5,780,448
U.S. Patent 5,789,215
U.S. Patent 5,801,234
U.S. Patent 5,840,846
U.S. Patent 5,843,650
U.S. Patent 5,846,709
U.S. Patent 5,846,783
U.S. Patent 5,849,497
U.S. Patent 5,849,546
U.S. Patent 5,849,547
U.S. Patent 5,858,652
U.S. Patent 5,866,366
U.S. Patent 5,871,986
U.S. Patent 5,916,776
U.S. Patent 5,922,574
U.S. Patent 5,925,565
U.S. Patent 5,928,905
U.S. Patent 5,928,906
U.S. Patent 5,932,451
U.S. Patent 5,935,819
U.S. Patent 5,935,825
U.S. Patent 5,939,291
U.S. Patent 5,942,391
U.S. Patent 5,945,100
U.S. Patent 5,958,895
U.S. Patent 5,981,274
U.S. Patent 5,994,624
U.S. Patent 6,008,341
U.S. Patent 6,288,214
U.S. Patent 6,294,177
U.S. Patent 6,482,240
U.S. Patent 6,651,655
U.S. Patent 6,656,462
U.S. Patent 6,733,754
U.S. Patent 6,756,361
U.S. Patent 6,770,278
U.S. Patent 6,793,923
U.S. Patent 6,814,971
U.S. Patent 6,936,258
U.S. Patent Appln. 20030153022
U.S. Patent Appln. 20020169288
Albus et al., Infect. Immun., 59:1008-1014, 1991.
An, J. Virol., 71(3):2292-302, 1997.
Anavi, Sc. thesis from the department of Molecular Microbiology and Biotechnology of the Tel-Aviv University, Israel, 1998.
Andersen et al., J. Immunol., 154, 3359-3372, 1995.
Angel et al., Cell, 49:729, 1987b.
Angel et al., Mol. Cell. Biol., 7:2256, 1987a.
Archer, Clin. Infect. Dis., 26, 1179-1181, 1998.
Atchison and Perry, Cell, 46:253, 1986.
Atchison and Perry, Cell, 48:121, 1987.
Ausubel et al., In: Current Protocols in Molecular Biology, John, Wiley & Sons, Inc, New York, 1996.
Baba et al., J. Bacteriol., 190:300-310, 2008.
Baba et al., Lancet., 359:1819-1827, 2007.
Bae and Schneewind, Plasmid, 55:58-63, 2006.
Bae et al., Proc. Natl. Acad. Sci. USA, 101, 12312-12317, 2004.
Banerji et al., Cell, 27(2 Pt 1):299-308, 1981.
Banerji et al., Cell, 33(3):729-740, 1983.
Barany and Merrifield, In: The Peptides, Gross and Meienhofer (Eds.), Academic Press, NY, 1-284, 1979.
Bellus, J. Macromol. Sci. Pure Appl. Chem., A31(1): 1355-1376, 1994.
Berkhout et al., Cell, 59:273-282, 1989.
Blanar et al., EMBO J., 8:1139, 1989.
Bodine and Ley, EMBO J., 6:2997, 1987.
Borges et al., Vet. Parasitol, 143(2):106-11, 2007.
Borrebaeck, In: Antibody Engineering--A Practical Guide, W. H. Freeman and Co., 1992.
Boshart et al., Cell, 41:521, 1985.
Bosze et al., EMBO J., 5(7):1615-1623, 1986.
Braddock et al., Cell, 58:269, 1989.
Brady et al., FEMS Immunol. Med. Microbiol., 52:13-22, 2008.
Brady et al., Infect. Immun., 74:3415-3426, 2006.
Bubeck-Wardenburg et al., Proc. Natl. Acad. Sci. USA, 103:13831-13836, 2006.
Buckley et al. J. Pediatr., 130:378-387, 1997.
Bulla and Siddiqui, J. Virol., 62:1437, 1986.
Burke et al., , J. Inf. Dis., 170:1110-1119, 1994.
Burlak et al., Cell Microbiol., 9:1172-1190, 2007.
Burts et al., Proc. Natl. Acad. Sci. USA, 102:1169-1174, 2005.
Campbell and Villarreal, Mol. Cell. Biol., 8:1993, 1988.
Campere and Tilghman, Genes and Dev., 3:537, 1989.
Campo et al., Nature, 303:77, 1983.
Carbonelli et al., FEMS Microbiol. Lett., 177(1):75-82, 1999.
Carleton et al., Infect. Dis., 190:1730-1738, 2004.
Cassat et al., Methods Mol. Biol., 391:127-144, 2007.
Celander and Haseltine, J. Virology, 61:269, 1987.
Celander et al., J. Virology, 62:1314, 1988.
Champion et al., Science, 313:1632-1636, 2006.
Chandler et al., Cell, 33:489, 1983.
Chandler et al., Proc. Natl. Acad. Sci. USA, 94(8):3596-601, 1997.
Chang et al., Mol. Cell. Biol., 9:2153, 1989.
Chatterjee et al., Proc. Natl. Acad. Sci. USA, 86:9114, 1989.
Chen and Okayama, Mol. Cell Biol., 7(8):2745-2752, 1987.
Chen et al. Nat. Chem. Biol., 2:591-5, 2006.
Cheung et al., Proc. Natl. Acad. Sci. USA 89, 6462-6466, 1992.
Choi et al., Cell, 53:519, 1988.
Cocea, Biotechniques, 23(5):814-816, 1997.
Cohen et al., J. Cell. Physiol., 5:75, 1987.
Costa et al., Mol. Cell. Biol., 8:81, 1988.
Cramton et al., Infect. Immun., 67:5427-5433, 1999.
Cripe et al., EMBO J., 6:3745, 1987.
Cronstein et al., Clin. Invest., 85:1150-1157, 1990.
Culotta and Hamer, Mol. Cell. Biol., 9:1376, 1989.
Dalbey and Wickner, J. Biol. Chem., 260:15925-15931, 1985.
Dandolo et al., J. Virology, 47:55-64, 1983.
de Haas, C. J. et al. J. Exp. Med. 199, 687-695 (2004).
De Villiers et al., Nature, 312(5991):242-246, 1984.
Deschamps et al., Science, 230:1174-1177, 1985.
Deussen et al., J. Am. J. Physiol., 264:692-700, 1993.
Devereux et al., Nucl. Acid Res., 12:387-395, 1984.
Diep et al., J. Infect. Dis., 193:1495-1503, 2006a.
Diep et al., Lancet., 367:731-739, 2006b.
Dinges et al., Clin. Microbiol. Rev., 13:16-34, 2000.
Drenkard and Ausubel, Nature, 416, 740-743, 2002.
Duthie and Lorenz, J. Gen. Microbiol., 6:95-107, 1952.
Edbrooke et al., Mol. Cell. Biol., 9:1908, 1989.
Edlund et al., Science, 230:912-916, 1985.
Eltzschig et al. J. Exp. Med., 198:783-796, 2003.
Emorl and Gaynes, Clin. Microbiol. Rev., 6:428-442, 1993.
EP 0 594 610 B1
EP497524
EP497525
Epitope Mapping Protocols In: Methods in Molecular Biology, Vol. 66, Morris (Ed.), 1996.
Fechheimer, et al., Proc Natl. Acad. Sci. USA, 84:8463-8467, 1987.
Feng and Holland, Nature, 334:6178, 1988.
Firak and Subramanian, Mol. Cell. Biol., 6:3667, 1986.
Firestein, G. S. et alJ. Immunol. 154, 326-334 (1995).
Foecking and Hofstetter, Gene, 45(1):101-105, 1986.
Fortune et al., Proc Natl. Acad. Sci. USA, 102:10676-10681, 2005.
Foster, Nat. Rev. Microbiol., 3:948-958, 2005.
Fournier et al., Infect. Immun. 45:87-93, 1984.
Fraley et al., Proc. Natl. Acad. Sci. USA, 76:3348-3352, 1979.
Fujita et al., Cell, 49:357, 1987.
Gaspar et al., J. Bacteriol., 187, 4646-55, 2005.
GB Appln. 2 202 328
Giblett et al., Lancet., 2:1067-1069, 1972.
Gilles et al., Cell, 33:717, 1983.
Gloss et al., EMBO J., 6:3735, 1987.
Godbout et al., Mol. Cell. Biol., 8:1169, 1988.
Gomez et al., Nature Med., 10:842-848, 2004.
Goodbourn and Maniatis, Proc. Natl. Acad. Sci. USA, 85:1447, 1988.
Goodbourn et al., Cell, 45:601, 1986.
Gopal, Mol. Cell Biol., 5:1188-1190, 1985.
Gotz, Mol. Microbiol., 43:1367-1378, 2002.
Graham and Van Der Eb, Virology, 52:456-467, 1973.
Greene et al., Immunology Today, 10:272, 1989
Grosschedl and Baltimore, Cell, 41:885, 1985.
Guinn et al., Mol. Microbiol., 51:359-370, 2004.
Hall-Stoodley et al., Nat. Rev. Microbiol., 2:95-108, 2004.
Harland and Weintraub, J. Cell Biol., 101(3):1094-1099, 1985.
Harlow and Lane, Antibodies: A Laboratory Manual, 1988.
Harraghy et al. Microbiology, 151:1789-1800. 2005.
Hartleib et al., Blood, 96:2149-2156, 2000.
Haskó and Pacher, Leukoc. Biol., 83:447-455, 2008.
Haslinger and Karin, Proc. Natl. Acad. Sci. USA, 82:8572, 1985.
Hauber and Cullen, J. Virology, 62:673, 1988.
Heilmann et al., Mol. Microbiol., 24:1013-1024, 1997.
Hen et al., Nature, 321:249, 1986.
Hensel et al., Lymphokine Res., 8:347, 1989.
Herr and Clarke, Cell, 45:461, 1986.
Higgins et al., FEMS Microbiol. Lett., 258:290-296, 2006.
Hirochika et al., J. Virol., 61:2599, 1987.
Hirsch et al., Mol. Cell. Biol., 10:1959, 1990.
Holbrook et al., Virology, 157:211, 1987.
Horlick and Benfield, Mol. Cell. Biol., 9:2396, 1989.
Hsu et al., Proc. Natl. Acad. Sci. USA, 100:12420-12425, 2003.
Huang et al., Cell, 27:245, 1981.
Hug et al., Mol. Cell. Biol., 8:3065, 1988.
Hussain et al., J. Bacteriology, 183:6778-86. 2001.
Huston et al., In: Methods in Enzymology, Langone (Ed.), Academic Press, NY, 203:46-88, 1991.
Hwang et al., Mol. Cell. Biol., 10:585, 1990.
Ichiman and Yoshida, J. Appl. Bacterid., 51:229-241, 1981.
Ichiman et al., J. Appl. Bacterid., 71:176-181, 1991.
Imagawa et al., Cell, 51:251, 1987.
Imbra and Karin, Nature, 323:555, 1986.
Imler et al., Mol. Cell. Biol., 7:2558, 1987.
Imperiale and Nevins, Mol. Cell. Biol., 4:875, 1984.
Innis et al., Proc Natl Acad Sci USA, 85(24):9436-9440, 1988.
Inouye and Inouye, Nucleic Acids Res., 13: 3101-3109, 1985.
Iwamoto et al., Planta Med., 54(5):422-5, 1988.
Jakobovits et al., Mol. Cell. Biol., 8:2555, 1988.
Jameel and Siddiqui, Mol. Cell. Biol., 6:710, 1986.
Jardetzky et al., Nature, 368:711-718, 1994.
Jaynes et al., Mol. Cell. Biol., 8:62, 1988.
Jensen, Acta Path. Microbiol. Scandin., 44:421-428, 1958.
Jin et al., J. Immunol., 172:1169-1176, 2004.
Johnson et al., Methods in Enzymol., 203:88-99, 1991.
Johnson et al., Mol. Cell. Biol., 9:3393, 1989.
Johnson et al., Infect. Immun. 76, 1756-1765, 2008.
Johnstone et al., In: Immunochemistry in Practice, Blackwell Scientific Publications, Oxford, 1982.
Jones, Carb. Research, 340:1097-1106, 2005.
Jonsson et al., J. Infect. Dis., 185:1417-1424, 2002.
Joyce et al., Carbohydrate Res., 338:903-922, 2003.
Kadesch and Berg, Mol. Cell. Biol., 6:2593, 1986.
Kaeppler et al., Plant Cell Rep., 8:415-418, 1990.
Kaneda et al., Science, 243:375-378, 1989.
Karin et al., Mol. Cell. Biol., 7:606, 1987.
Katinka et al., Cell, 20:393, 1980.
Kato et al, J. Biol. Chem., 266:3361-3364, 1991.
Kawamoto et al., Mol. Cell. Biol., 8:267, 1988.
Khoa et al. J. Immunol., 167:4026-4032, 2001.
Kiledjian et al., Mol. Cell. Biol., 8:145, 1988.
Klamut et al., Mol. Cell. Biol., 10:193, 1990.
Klevens et al., Clin. Infect. Dis., 42, 389-91, 2006.
Klevens et al., Jama, 298:1763-71, 2007.
Koch et al., Mol. Cell. Biol., 9:303, 1989.
Kohler and Milstein, Nature 256:495-497, 1975.
Kolter and Greenberg, Nature 441, 300-302, 2006.
Kriegler and Botchan, In: Eukaryotic Viral Vectors, Gluzman (Ed.), Cold Spring Harbor: Cold Spring Harbor Laboratory, NY, 1982.
Kriegler and Botchan, Mol. Cell. Biol., 3:325, 1983.
Kriegler et al., Cell, 38:483, 1984a.
Kriegler et al., Cell, 53:45, 1988.
Kriegler et al., In: Cancer Cells 2/Oncogenes and Viral Genes, Van de Woude et al. eds, Cold Spring Harbor, Cold Spring Harbor Laboratory, 1984b.
Kuhl et al., Cell, 50:1057, 1987.
Kunz et al., Nucl. Acids Res., 17:1121, 1989.
Kuroda et al., Lancet., 357:1225-1240, 2001.
Kyte and Doolittle, J. Mol. Biol., 157(1):105-132, 1982.
Larsen et al., Proc Natl. Acad. Sci. USA., 83:8283, 1986.
Laspia et al., Cell, 59:283, 1989.
Latimer et al., Mol. Cell. Biol., 10:760, 1990.
Lee et al., Nature, 294:228, 1981.
Lee et al., Nucleic Acids Res., 12:4191-206, 1984.
LeeTrends Microbiol., 4(4):162-166, 1996.
Levenson et al., Hum. Gene Ther., 9(8):1233-1236, 1998.
Levinson et al., Nature, 295:79, 1982.
Lin et al., Mol. Cell. Biol., 10:850, 1990.
Lowy, New Engl. J. Med., 339:520-532, 1998.
Luria et al., EMBO J., 6:3307, 1987.
Lusky and Botchan, Proc. Natl. Acad. Sci. USA, 83:3609, 1986.
Lusky et al., Mol. Cell. Biol., 3:1108, 1983.
Macejak and Sarnow, Nature, 353:90-94, 1991.
MacGurn et al., Mol. Microbiol., 57:1653-1663,2005.
Macket al., J. Bacteriol. 178, 175-183, 1996.
Maira-Litran et al., Infect. Immun., 70:4433-4440, 2002.
Maira-Litran et al., Vaccine, 22:872-879, 2004.
Majors and Varmus, Proc. Natl. Acad. Sci. USA, 80:5866, 1983.
Mazmanian et al., Mol. Microbiol. 40, 1049-1057, 2001.
Mazmanian et al., Proc. Natl. Acad. Sci. USA, 99:2293-2298, 2002.
Mazmanian et al., Science, 285, 760-763, 1999.
Mazmanian et al., Science, 299:906-909, 2003.
McColl et al. FASEB J., 20:187-189, 2006.
McDevitt et al., Mol. Microbiol., 11:237-248, 1994.
McDougal et al., J. Clin. Microbiol., 41:5113-5120, 2003.
McLaughlin et al., PLoS Pathog., 3:e105, 2007.
McNeall et al., Gene, 76:81, 1989.
Merck Manual Of Diagnosis And Therapy, § 13, Ch. 157, 100th Ed. (Beers & Berkow, Eds., 2004
Mernaugh et al., In: Molecular Methods in Plant Pathology, Singh et al. (Eds.), CRC Press Inc., Boca Raton, FL, 359-365, 1995.
Merrifield, Science, 232(4748):341-347, 1986.
Miksicek et al., Cell, 46:203, 1986.
Mo and Ballard, J. Physiol., 536:593-603, 2001.
Mordacq and Linzer, Genes and Dev., 3:760, 1989.
Moreau et al., Carbohydrate Res., 201:285-297, 1990.
Moreau et al., Nucl. Acids Res., 9:6047, 1981.
Mosmann and Coffman, Ann. Rev. Immunol., 7:145-173, 1989.
Muesing et al., Cell, 48:691, 1987.
Musher et al., Medicine (Baltimore), 73:186-208, 1994.
Needleman & Wunsch, J. Mol. Biol., 48:443, 1970.
Nemeth et al. J. Immunol., 176:5616-5626, 2006.
Ng et al., Nuc. Acids Res., 17:601, 1989.
Ni Eidhin et al., Mol. Microbiol., 30:245-257, 1998.
Nicolau and Sene, Biochim. Biophys. Acta, 721:185-190, 1982.
Nicolau et al., Methods Enzymol., 149:157-176, 1987.
Novick, Mol. Microbiol., 48:1429-1449, 2003.
Novick and Jiang. Microbiology 149, 2709-17 (2003).
Omirulleh et al., Plant Mol. Biol., 21(3):415-28, 1993.
Ondek et al., EMBO J., 6:1017, 1987.
O'Riordan and Lee, Clin. Microbiol Rev., 17:218-234, 2004.
Ornitz et al., Mol. Cell. Biol., 7:3466, 1987.
Pallen, Trends Microbiol, 10:209-212, 2002.
Palmiter et al., Nature, 300:611, 1982.
Palmqvist et al., Microbes Infect., 6:188-195, 2004.
Panther et al., FASEB J., 15:1963-1970, 2001.
PCT Appln. PCT/US89/01025
PCT Appln. WO 00/02523
PCT Appln. WO 00/12689
PCT Appln. WO 01/60852
PCT Appln. WO 03/53462
PCT Appln. WO 04/43405
PCT Appln. WO 04/43407
PCT Appln. WO 95/08348
Pearson & Lipman, Proc. Natl. Acad. Sci. USA, 85:2444, 1988.
Pech et al., Mol. Cell. Biol., 9:396, 1989.
Pelletier and Sonenberg, Nature, 334(6180):320-325, 1988.
Perez-Stable and Constantini, Mol. Cell. Biol., 10:1116, 1990.
Peschel and Sahl, Nat. Rev. Microbiol., 4:529-536, 2006.
Picard and Schaffner, Nature, 307:83, 1984.
Pinkert et al., Genes and Dev., 1:268, 1987.
Ponta et al., Proc. Natl. Acad. Sci. USA, 82:1020, 1985.
Porton et al., Mol. Cell. Biol., 10:1076, 1990.
Potrykus et al., Mol. Gen. Genet., 199(2):169-177, 1985.
Pugsley, Microbiol. Rev., 57:50-108, 1993.
Pym et al., Mol. Microbiol., 46;709-717, 2002.
Pym et al., Nat. Med., 9:533-539, 2003.
Queen and Baltimore, Cell, 35:741, 1983.
Quinn et al., Mol. Cell. Biol., 9:4713, 1989.
Redondo et al., Science, 247:1225, 1990.
Reisman and Rotter, Mol. Cell. Biol., 9:3571, 1989.
Remington's Pharmaceutical Sciences, 18th Ed. Mack Printing Company, 1289-1329, 1990.
Resendez Jr. et al., Mol. Cell. Biol., 8:4579, 1988.
Ripe et al., Mol. Cell. Biol., 9:2224, 1989.
Rippe, et al., Mol. Cell Biol., 10:689-695, 1990.
Rittling et al., Nuc. Acids Res., 17:1619, 1989.
Roben et al., J. Immunol., 154:6437-6445, 1995.
Rooijakkers et al., Trends Microbiol., 13:596-601, 2005.
Rosen et al., Cell, 41:813, 1988.
Sakai et al., Genes and Dev., 2:1144, 1988.
Sambrook et al., In: Molecular cloning, Cold Spring Harbor Laboratory Press, Cold Spring Harbor, NY, 2001.
Sau et al., Microbiology, 143:2395-2405, 1997.
Schaffner et al., J. Mol. Biol., 201:81, 1988.
Schindler and Schuhardt, Proc. Natl. Acad. Sci. USA, 51:414-421, 1964.
Scriba, T. J. et al. The Infect. Immun. 76, 2164-2168 (2008).
Searle et al., Mol. Cell. Biol., 5:1480, 1985.
Sharp and Marciniak, Cell, 59:229, 1989.
Shaul and Ben-Levy, EMBO J., 6:1913, 1987.
Shaw et al., Microbiology, 150:217-228, 2004.
Sherman et al., Mol. Cell. Biol., 9:50, 1989.
Sibbald et al., Microbiol. Mol Biol. Rev., 70:755-788, 2006.
Singh et al., Nature, 417, 552-555, 2002.
Sleigh and Lockett, J. EMBO, 4:3831, 1985.
Smith & Waterman, Adv. Appl. Math., 2:482, 1981.
Sorensen et al., Infect. Immun., 63:1710-1717, 1995.
Spalholz et al., Cell, 42:183, 1985.
Spandau and Lee, J. Virology, 62:427, 1988.
Spandidos and Wilkie, EMBO J., 2:1193, 1983.
Stanley et al., Proc. Natl. Acad. Sci. USA, 100:13001-13006, 2003.
Stephens and Hentschel, Biochem. J., 248:1, 1987.
Stewart and Young, In: Solid Phase Peptide Synthesis, 2d. ed., Pierce Chemical Co., 1984.
Stranger-Jones et al., Proc. Nat. Acad. Sci. USA, 103:16942-16947, 2006.
Stuart et al., Nature, 317:828, 1985.
Sullivan and Peterlin, Mol. Cell. Biol., 7:3315, 1987.
Swartzendruber and Lehman, J. Cell. Physiology, 85:179, 1975.
Takebe et al., Mol. Cell. Biol., 8:466, 1988.
Tam et al., J. Am. Chem. Soc., 105:6442, 1983.
Tavernier et al., Nature, 301:634, 1983.
Taylor and Kingston, Mol. Cell. Biol., 10:165, 1990a.
Taylor and Kingston, Mol. Cell. Biol., 10:176, 1990b.
Taylor et al., J. Biol. Chem., 264:15160, 1989.
Thiel et al., Microbes Infect., 5:515-526, 2003.
Thiesen et al., J. Virology, 62:614, 1988.
Thompson et al., Biochem. Biophys. Res. Commun., 145:118-125, 1987.
Thompson et al., J. Immunol., 143:1815-1821, 1989.
Thomson et al., J. Immunol., 157(2):822-826, 1996.
Tigges et al., J. Immunol., 156(10):3901-3910, 1996.
Toukairin et al., Chem. Pharm.Bull.(Tokyo), 39(6):1480-3, 1991.
Treisman, Cell, 42:889, 1985.
Tronche et al., Mol. Biol. Med., 7:173, 1990.
Trudel and Constantini, Genes and Dev., 6:954, 1987.
Tyndell et al., Nuc. Acids. Res., 9:6231, 1981.
Uchino et al., Planta. Medt.,54(5):419-22, 1988.
van den Ent and Lowe, FEBS Lett., 579:3837-3841, 2005.
van Wely et al., FEMS Microbiol. Rev., 25:437-454, 2001.
Vannice and Levinson, J. Virology, 62:1305, 1988.
Vasseur et al., Proc Natl. Acad. Sci. USA, 77:1068, 1980.
Vaughan et al., Nat. Biotech., 16:535-539, 1998.
Voyich et al., J. Immunol., 175:3907-3919, 2005.
Vuong et al., J. Biol. Chem., 279:54881-54886, 2004.
Wang and Calame, Cell, 47:241, 1986.
Wang et al., Nat. Med., 13:1510-1514,2007.
Weber et al., Cell, 36:983, 1984.
Weinberger et al. Mol. Cell. Biol., 8:988, 1984.
Weiss et al., J. Antimicrob. Chemother., 53:480-486, 2004.
Winoto and Baltimore, Cell, 59:649, 1989.
Wong et al., Gene, 10:87-94, 1980.
Xie, C. et al. J. Exp. Med. 203, 985-994 (2006).
Xiong et al. Antimicrob. Agents Chemother., 49:380-387, 2005.
Xu et al., J. Infect. Dis., 189:2323-2333, 2004.
Xu et al., Mol. Microbiol., 66(3):787-800, 2007.
Yang et al. J. Immunol., 175:6458-6464, 2005.
Yokogawa et al., Antimicrob. Agents Chemother., 6:156-165, 1974.
Yutzey et al. Mol. Cell. Biol., 9:1397, 1989.
Zimmermann, Biochem. J., 285:345-365, 1992.

## Claims

1. An antigen composition comprising:
(a) an isolated Emp antigen that is at least 80% identical to SEQ ID NO:2, or an immunogenic fragment thereof capable of conferring protective immunity to staphylococcal infection; and
(b) at least one additional staphylococcal antigen selected from a group consisting of an isolated ClfA, ClfB, Eap, EsaB, EsxA, EsxB, EsaC, IsdA, IsdB, IsdC, SasF, SasB, SdrC, SdrD, SdrE, SasH, Ebh, Coa, vWa, Hla and SpA antigen, or an immunogenic fragment thereof,
wherein the antigens are comprised in a pharmaceutically acceptable composition and are capable of stimulating an immune response in a subject in need thereof.

2. The composition of claim 1, wherein the at least one additional staphylococcal antigen is selected from the group consisting of an isolated ClfB, Eap, EsxA, EsxB, IsdA, and SrdD.

3. The composition of claim 1, further comprising one or more of a type V capsular saccharide, a type VIII capsular saccharide, and/or a polysaccharide intracellular adhesin (PIA).

4. The composition of claim 3, wherein the type V capsular saccharide, the type VIII capsular saccharide, and/or the polysaccharide intracellular adhesin (PIA) is conjugated to a protein carrier, preferably wherein the protein carrier is selected from the group consisting of tetanus toxoid, diphtheria toxoid, CRM197, Haemophilus influenzae protein D, pneumolysin and alpha toxoid.

5. The composition of claim 1, wherein the composition comprises less than a 1% weight/weight contamination with other staphylococcal bacterial components.

6. The composition of claim 1, wherein one or more isolated antigens are coupled to an adjuvant.

7. The composition of claim 1, wherein the Emp antigen:
(i) comprises at least 5, 10, 15, or 20 consecutive amino acids of SEQ ID NO:2; or
(ii) is at least 90%, 95%, or 98% identical to SEQ ID NO:2.

8. The composition of claim 1, further comprising a pharmaceutically acceptable excipient.

9. An immunogenic composition comprising the composition of any one of the preceding claims and an adjuvant.

10. A composition comprising an isolated antibody, or fragment thereof, that binds an Emp antigen that is at least 80% identical to SEQ ID NO: 2, or an immunogenic fragment thereof, in a pharmaceutically acceptable composition for use in a method of treating staphylococcus bacterial infection.

11. The composition for use in a method of claim 10, wherein the antibody is:
(i) a human or humanized antibody;
(ii) a monoclonal antibody or fragment thereof; or
(iii) a single chain antibody or fragment thereof.

12. The composition for use in a method of claim 10, further comprising at least one additional isolated antibody that binds an antigen selected from a group consisting of an isolated ClfA, ClfB, Eap, EsaB, EsaC, EsxA, EsxB, Hla, IsdA, IsdB, IsdC, SasB, SasF, SasH, Ebh, Coa, vWa, SdrC, SdrD, SdrE, and SpA antigen, or an immunogenic fragment thereof.

13. A method of making a vaccine comprising the steps of mixing:
(a) an isolated Emp antigen that is at least 80% identical to SEQ ID NO: 2, or an immunogenic fragment thereof capable of conferring protective immunity to staphylococcal infection;
(b) at least one additional staphylococcal antigen selected from a group consisting of an isolated ClfA, ClfB, Eap, EsaB, EsaC, EsxA, EsxB, IsdA, IsdB, IsdC, SasF, SasB, SasH, Ebh, Coa, vWa, SdrC, SdrD, SdrE, Hla and SpA antigen, or an immunogenic fragment thereof; and
(c) a pharmaceutically acceptable excipient.

14. The composition of any one of claims 1 to 9 for use in:
(i) a method of eliciting an immune response against a staphylococcus bacterium in a subject, the method comprising administering to the subject an effective amount of the composition; or
(ii) a method of preventing or treating staphylococcal infection, the method comprising the step of administering to a patient in need thereof the composition.

15. Use of the composition of any one of claims 1 to 9 in the manufacture of a medicament for use in:
(i) eliciting an immune response against a staphylococcus bacterium in a subject, by a method comprising administering to the subject an effective amount of the composition; or
(ii) preventing or treating staphylococcal infection, by a method comprising the step of administering to a patient in need thereof the composition.

## Patentansprüche

1. Antigenzusammensetzung, umfassend:
(a) ein isoliertes Emp-Antigen, das zu mindestens 80 % mit SEQ ID NO:2 identisch ist oder ein immunogenes Fragment davon, das einer Staphylokokkeninfektion eine schützende Immunität verleihen kann; und
(b) mindestens ein zusätzliches Staphylokokken-Antigen ausgewählt aus einer Gruppe bestehend aus einem isolierten ClfA-, ClfB-, Eap-, EsaB-, EsxA-, EsxB-, EsaC-, IsdA-, IsdB-, IsdC-, SasF-, SasB-, SdrC-, SdrD-, SdrE-, SasH-, Ebh-, Coa-, vWa-, Hla- und SpA-Antigen oder ein immunogenes Fragment davon,
wobei die Antigene in einer pharmazeutisch akzeptablen Zusammensetzung umfasst sind und eine Immunantwort in einem Subjekt, das dessen bedarf, hervorrufen können.

2. Zusammensetzung nach Anspruch 1, wobei das mindestens eine zusätzliche Staphylokokken-Antigen ausgewählt ist aus der Gruppe bestehend aus einem isolierten ClfB, Eap, EsxA, EsxB, IsdA und SrdD.

3. Zusammensetzung nach Anspruch 1, ferner umfassend ein oder mehrere eines kapselförmigen Typ V-Saccharids, eines kapselförmigen Typ VIII-Saccharids und/oder eines intrazellulären Polysaccharid-Adhäsins (PIA).

4. Zusammensetzung nach Anspruch 3, wobei das kapselförmige Typ V-Saccharid, das kapselförmige Typ VIII Saccharid und/oder das intrazelluläre Polysaccharid-Adhäsin (PIA) auf einen Proteinträger konjugiert wird, vorzugsweise wobei der Proteinträger ausgewählt ist aus der Gruppe bestehend aus Tetanustoxoid, Diphterietoxoid, CRM197, Haemopilus influenzae-Protein D, Pneumolysin und Alphatoxoid.

5. Zusammensetzung nach Anspruch 1, wobei die Zusammensetzung weniger als 1 % Gewicht/Gewicht - Kontamination mit anderen bakteriellen Staphylokokken-Komponenten umfasst.

6. Zusammensetzung nach Anspruch 1, wobei ein oder mehrere isolierte Antigene mit einem Adjuvant verbunden sind.

7. Zusammensetzung nach Anspruch 1, wobei das Emp-Antigen:
(i) mindestens 5, 10, 15 oder 20 fortlaufende Aminosäuren der SEQ ID NO:2 umfasst; oder
(ii) mindestens zu 90 %, 95 % oder 98 % mit SEQ ID NO:2 identisch ist.

8. Zusammensetzung nach Anspruch 1, ferner umfassend einen pharmazeutisch akzeptablen Hilfsstoff.

9. Immunogene Zusammensetzung, umfassend die Zusammensetzung nach einem der vorstehenden Ansprüche und ein Adjuvant.

10. Zusammensetzung, umfassend einen isolierten Antikörper oder ein Fragment davon, der/das ein Emp-Antigen bindet, das mindestens zu 80 % mit SEQ ID NO:2 identisch ist, oder ein immunogenes Fragment davon, in einer pharmazeutisch akzeptablen Zusammensetzung zur Verwendung in einem Verfahren der Behandlung einer bakteriellen Staphylokokkeninfektion.

11. Zusammensetzung zur Verwendung in einem Verfahren nach Anspruch 10, wobei der Antikörper:
(i) ein menschlicher oder humanisierter Antikörper ist;
(ii) ein monoklonaler Antikörper oder ein Fragment davon ist; oder
(iii) ein Einzelkettenantikörper oder ein Fragment davon ist.

12. Zusammensetzung zur Verwendung in einem Verfahren nach Anspruch 10, ferner umfassend mindestens einen zusätzlichen isolierten Antikörper, der ein Antigen bindet, das ausgewählt ist aus einer Gruppe bestehend aus einem isolierten ClfA-, ClfB-, Eap-, EsaB-, EsaC-, EsxA-, EsxB-, Hla-, IsdA-, IsdB-, IsdC-, SasB-, SasF, SasH-, Ebh-, Coa-, vWa-, SdrcC-, SdrD-, SdrE- und SpA-Antigen oder ein immunogenes Fragment davon.

13. Verfahren der Herstellung einer Impfung, umfassend die Schritte des Mischens von:
(a) einem isolierten Emp-Antigen, das zu mindestens 80 % mit SEQ ID NO:2 identisch ist, oder einem immunogenen Fragment davon, das einer Staphylokokkeninfektion eine schützende Immunität verleihen kann;
(b) mindestens einem zusätzlichen Staphylokokken-Antigen ausgewählt aus der Gruppe bestehend aus einem isolierten ClfA-, ClfB-, Eap-, EsaB-, EsaC-, EsxA-, EsxB-, IsdA-, IsdB-, IsdC-, SasF-, SasB-, SasH-, Ebh-, Coa-, vWa-, SdrC-, SdrD-, SdrE-, Hla-, und SpA-Antigen oder einem immunogenen Fragment davon; und
(c) einem pharmazeutisch akzeptablen Hilfsstoff.

14. Zusammensetzung nach einem der Ansprüche 1 bis 9 zur Verwendung bei:
(i) einem Verfahren des Hervorrufens einer Immunantwort gegen ein Staphylokokken-Bakterium in einem Subjekt, wobei das Verfahren das Verabreichen einer wirksamen Menge der Zusammensetzung an das Subjekt umfasst; oder
(ii) ein Verfahren des Vorbeugens oder der Behandlung einer Staphylokokkeninfektion, wobei das Verfahren den Schritt des Verabreichens der Zusammensetzung an einen Patienten, der dessen bedarf, umfasst.

15. Verwendung der Zusammensetzung nach einem der Ansprüche 1 bis 9 bei der Herstellung eines Medikaments für die Verwendung beim:
(i) Hervorrufen einer Immunantwort gegen ein Staphylokokken-Bakterium in einem Subjekt durch ein Verfahren, das das Verabreichen einer wirksamen Menge der Zusammensetzung an das Subjekt umfasst; oder
(ii) Vorbeugen oder Behandeln einer Staphylokokkeninfektion durch ein Verfahren, das den Schritt des Verabreichens der Zusammensetzung an einen Patienten, der dessen bedarf, umfasst.

## Revendications

1. Composition d'antigène, comprenant :
(a) un antigène Emp isolé qui est au moins 80 % identique à SEQ ID NO : 2, ou un fragment immunogène de celui-ci capable de conférer une immunité protectrice contre une infection staphylococcique ; et
(b) au moins un antigène staphylococcique supplémentaire sélectionné parmi un groupe constitué d'un antigène ClfA, ClfB, Eap, EsaB, EsxA, EsxB, EsaC, IsdA, IsdB, IsdC, SasF, SasB, SdrC, SdrD, SdrE, SasH, Ebh, Coa, vWa, Hla et SpA isolé, ou d'un fragment immunogène de celui-ci,
dans laquelle les antigènes sont compris dans une composition pharmaceutiquement acceptable et sont capables de stimuler une réponse immunitaire chez un sujet ayant besoin de celle-ci.

2. Composition selon la revendication 1, dans laquelle l'au moins un antigène staphylococcique supplémentaire est sélectionné parmi le groupe constitué d'un ClfB, Eap, EsxA, EsxB, IsdA, et SrdD isolé.

3. Composition selon la revendication 1, comprenant en outre un ou plusieurs parmi un saccharide capsulaire de type V, un saccharide capsulaire de type VIII, et/ou une adhésine intracellulaire polysaccharide (PIA).

4. Composition selon la revendication 3, dans laquelle le saccharide capsulaire de type V, le saccharide capsulaire de type VIII, et/ou l'adhésine intracellulaire polysaccharide (PIA) est conjugué à un support protéique, de préférence dans laquelle le support protéique est sélectionné parmi le groupe constitué de toxoïde tétanique, de toxoïde diphtérique, de CRM 197, de protéine D d'Haemophilus influenzae, de pneumolysine et de toxoïde alpha.

5. Composition selon la revendication 1, dans laquelle la composition comprend une contamination inférieure à 1 % masse/masse avec d'autres composants bactériens staphylococciques.

6. Composition selon la revendication 1, dans laquelle un ou plusieurs antigènes isolés sont couplés à un adjuvant.

7. Composition selon la revendication 1, dans laquelle l'antigène Emp :
(i) comprend au moins 5, 10, 15, ou 20 acides aminés consécutifs de SEQ ID NO : 2 ; ou
(ii) est au moins 90 %, 95 %, ou 98 % identique à SEQ ID NO : 2.

8. Composition selon la revendication 1, comprenant en outre un excipient pharmaceutiquement acceptable.

9. Composition immunogène comprenant la composition de l'une quelconque des revendications précédentes et un adjuvant.

10. Composition comprenant un anticorps isolé, ou un fragment de celui-ci, qui lie un antigène Emp qui est au moins 80 % identique à SEQ ID NO : 2, ou un fragment immunogène de celui-ci, une composition pharmaceutiquement acceptable pour l'utilisation dans un procédé de traitement d'infection bactérienne staphylococcique.

11. Composition, pour l'utilisation dans un procédé, selon la revendication 10, dans laquelle l'anticorps est :
(i) un anticorps humain ou humanisé ;
(ii) un anticorps monoclonal ou fragment de celui-ci ; ou
(iii) un anticorps monocaténaire ou fragment de celui-ci.

12. Composition, pour l'utilisation dans un procédé, selon la revendication 10, comprenant en outre au moins un anticorps isolé supplémentaire qui lie un antigène sélectionné parmi un groupe constitué d'un antigène ClfA, ClfB, Eap, EsaB, EsaC, EsxA, EsxB, Hla, IsdA, IsdB, IsdC, SasB, SasF, SasH, Ebh, Coa, vWa, SdrC, SdrD, SdrE, et SpA isolé, ou d'un fragment immunogène de celui-ci.

13. Procédé de fabrication d'un vaccin comprenant les étapes de mélange de :
(a) un antigène Emp isolé qui est au moins 80 % identique à SEQ ID NO : 2, ou un fragment immunogène de celui-ci capable de conférer une immunité protectrice contre une infection staphylococcique ;
(b) au moins un antigène staphylococcique supplémentaire sélectionné parmi un groupe constitué d'un antigène ClfA, ClfB, Eap, EsaB, EsaC, EsxA, EsxB, IsdA, IsdB, IsdC, SasF, SasB, SasH, Ebh, Coa, vWa, SdrC, SdrD, SdrE, Hla et SpA isolé, ou d'un fragment immunogène de celui-ci ; et
(c) un excipient pharmaceutiquement acceptable.

14. Composition selon l'une quelconque des revendications 1 à 9 pour l'utilisation dans :
(i) un procédé pour susciter une réponse immunitaire contre une bactérie staphylococcique chez un sujet, le procédé comprenant l'administration, au sujet, d'une quantité efficace de la composition ; ou
(ii) un procédé pour empêcher ou traiter une infection staphylococcique, le procédé comprenant l'étape de l'administration, à un patient ayant besoin de celle-ci, de la composition.

15. Utilisation de la composition selon l'une quelconque des revendications 1 à 9 dans la fabrication d'un médicament pour l'utilisation pour :
(i) susciter une réponse immunitaire contre une bactérie staphylococcique chez un sujet, par un procédé comprenant l'administration, au sujet, d'une quantité efficace de la composition ; ou
(ii) empêcher ou traiter l'infection staphylococcique, par un procédé comprenant l'étape de l'administration, à un patient ayant besoin de celle-ci, de la composition.
